# EUROPEAN PATENT SPECIFICATION

(11) **EP 1 845 973 B1**
(45) Date of publication and mention of the grant of the patent: **12.08.2015**
(21) Application number: 06700879.7
(22) Date of filing: 20.01.2006
(51) Int. Cl.: A61K 31/415, A61K 31/4155, A61P 35/00, A61K 31/4196, A61K 31/5685, A61K 31/135, A61K 31/565, A61K 31/4535, A61K 31/138, A61K 39/395, A61K 31/255, A61K 31/675, A61K 31/195, A61K 31/4188

(54) **PHARMACEUTICAL COMPOUNDS**
PHARMAZEUTISCHE VERBINDUNGEN
COMPOSES PHARMACEUTIQUES

(30) Priority: 21.01.2005 US 645988 P; 21.01.2005 US 645974 P; 21.01.2005 US 646216 P; 21.01.2005 US 646001 P; 21.01.2005 US 646003 P
(43) Date of publication of application: 24.10.2007
(73) Proprietor: Astex Therapeutics Limited, Cambridgeshire CB4 0QA (GB)
(72) Inventor: CURRY, Jayne Elizabeth, Cambridge CB4 0QA (GB); LYONS, John Francis, Cambridge CB4 0QA (GB); SQUIRES, Matthew Simon, Cambridge CB4 0QA (GB); THOMPSON, Neil Thomas, Cambridge CB4 0QA (GB); THOMPSON, Kyla Merriom, Cambridge CB4 0QA (GB); WYATT, Paul Graham, Cambridge CB4 0QA (GB)
(74) Representative: Cooke, Richard Spencer
(86) International application number: PCT/GB2006/000210
(87) International publication number: WO 2006/077428

(56) References cited:
- EP-A- 1 020 471
- WO-A-97/41824
- WO-A-03/101953
- WO-A-2004/110381
- WO-A-2005/012256
- WO-A-2005/082340
- WO-A-2005/087724
- US-A1- 2003 092 095
- US-A1- 2003 149 001
- US-A1- 2004 097 517
- US-A1- 2004 127 523
- US-A1- 2004 204 339
- US-B1- 6 306 393
- RETZLOFF ET AL: "GnRH antagonist compares favorably, but is not superior, to GnRH agonist microflare for poor responders in IVF." FERTILITY AND STERILITY, vol. 80, no. S3, September 2003 (2003-09), pages S189-S189, XP009063252
- EMANUEL ET AL: "Inhibition of human tumor xenograft growth by a novel, potent and selective CDK inhibitor" PROCEEDINGS OF THE AMERICAN ASSOCIATION FOR CANCER RESEARCH ANNUAL MEETING, vol. 44, July 2003 (2003-07), pages 135-136, XP001208382
- MAZUCCO ROY; WILLIAMS LAURA: "American Association for Cancer Research - 93rd Annual Meeting. Immunotherapy, chemoprevention and angiogenesis. 6-10 April 2002, San Francisco, CA, USA." IDRUGS : THE INVESTIGATIONAL DRUGS JOURNAL, vol. 5, no. 5, April 2002 (2002-04), pages 408-411, XP002371684
- SAITO R ET AL: "CONVECTION-ENHANCED DELIVERY OF TUMOR NECROSIS FACTOR-RELATED APOPTOSIS-INDUCING LIGAND WITH SYSTEMIC ADMINISTRATION OF TEMOZOLOMIDE PROLONGS SURVIVAL IN AN INTRACRANIAL GLIOBLASTOMA XENOGRAFT MODEL" CANCER RESEARCH, AMERICAN ASSOCIATION FOR CANCER RESEARCH, BALTIMORE, MD, US, vol. 64, no. 19, 1 October 2004 (2004-10-01), pages 6858-6862, XP009059977 ISSN: 0008-5472
- WONG ET AL: "Phase II study of two-weekly temozolomide in patients with high-grade gliomas" JOURNAL OF CLINICAL NEUROSCIENCE, CHURCHILL LIVINGSTONE, GB, vol. 13, no. 1, January 2006 (2006-01), pages 18-22, XP005235974 ISSN: 0967-5868
- MISRA RAJ N ET AL: "N-(cycloalkylamino)acyl-2-aminothiazole inhibitors of cyclin-dependent kinase 2. N-[5-[[[5-(1,1-dimethylethyl)-2-oxazolyl]m ethyl]thio]-2-thi azolyl]-4- piperidinecarboxamide (BMS-387032), a highly efficacious and selective antitumor agent" JOURNAL OF MEDICINAL CHEMISTRY, AMERICAN CHEMICAL SOCIETY. WASHINGTON, US, vol. 47, no. 7, 25 March 2004 (2004-03-25), pages 1719-1728, XP002302339 ISSN: 0022-2623
- HANDRICK R ET AL: "651 Effects of celecoxib and irradiation treatment on prostate cancer cell lines" EUROPEAN JOURNAL OF CANCER. SUPPLEMENT, PERGAMON, OXFORD, GB, vol. 2, no. 8, September 2004 (2004-09), page 196, XP004640095 ISSN: 1359-6349
- TAKIGAWA N ET AL: "203 Modulation of DNA damage induced apoptosis by the proteasome inhibitor bortezomib (PS) in human colorectal and non-small cell lung cancer cells is p53-dependent and NK-kB-independent" EUROPEAN JOURNAL OF CANCER. SUPPLEMENT, PERGAMON, OXFORD, GB, vol. 2, no. 8, September 2004 (2004-09), page 63, XP004639647 ISSN: 1359-6349
- BISCHOFF ET AL: "Tamoxifen resistance: the RXR-selective ligand LGD1069 causes complete regression of tamoxifen-resistant mammary carcinoma" PROCEEDINGS OF THE ANNUAL MEETING OF THE AMERICAN ASSOCIATION FOR CANCER RESEARCH, NEW YORK, NY, US, vol. 40, March 1999 (1999-03), page 309, XP002115216 ISSN: 0197-016X
- TEIXEIRA M ET AL: "Temozolamide (TMZ) in second-line treatment after pcv in glioblastoma multiforme (GBM). Experience from a single Portuguese institution" EUROPEAN JOURNAL OF CANCER, PERGAMON PRESS, OXFORD, GB, vol. 37, April 2001 (2001-04), page S344, XP004478517 ISSN: 0959-8049
- CAMPBELL D A ET AL: "MOLECULAR PATHOLOGY IN ONCOLOGY - THE ASTRAZENECA PERSPECTIVE" PHARMACOGENOMICS, ASHLEY PUBLICATIONS, GB, vol. 5, no. 8, 2004, pages 1167-1173, XP009052350 ISSN: 1462-2416
- GRANT S: "TARGETED THERAPIES IN CANCER" IDRUGS, CURRENT DRUGS LTD, GB, vol. 6, no. 10, 2003, pages 946-948, XP008042930 ISSN: 1369-7056
- KRISTELEIT R ET AL: "HISTONE MODIFICATION ENZYMES: NOVEL TARGETS FOR CANCER DRUGS" EXPERT OPINION ON EMERGING DRUGS, ASHLEY PUBLICATIONS, GB, vol. 9, no. 1, 2004, pages 135-154, XP008041959 ISSN: 1472-8214

## Description

This invention relates to combinations of pyrazole compounds that inhibit or modulate the activity of cyclin dependent kinase (CDK) and/or glycogen synthase kinase (GSK, e.g. GSK-3) with an ancillary agent selected from: a monoclonal antibody, an alkylating agent, an anticancer agent, a further CDK inhibitor and a hormone, hormone agonist, hormone antagonist or hormone modulating agent, and to the therapeutic uses of such combinations.

### Background of the Invention

The compounds of Formula (II) and subgroups thereof and the compound 4-(2,6-dichloro-benzoylamino)-1H-pyrazole-3-carboxylic acid piperidin-4-ylamide and the hydrochloric acid addition salt thereof are disclosed in our earlier International patent application number PCT/GB2004/003179 (Publication No. WO 2005/012256) as being inhibitors of Cyclin Dependent Kinases (CDK kinases) and Glycogen Synthase Kinase-3 (GSK3).

The methanesulphonic acid and acetic acid addition salts of compound 4-(2,6-dichloro-benzoylamino)-1H-pyrazole-3-carboxylic acid piperidin-4-ylamide and crystals thereof and method of making them are disclosed in our earlier applications USSN 60/645,973 and GB 0501475.8.

Protein kinases constitute a large family of structurally related enzymes that are responsible for the control of a wide variety of signal transduction processes within the cell (Hardie, G. and Hanks, S. (1995) The Protein Kinase Facts Book. I and II, Academic Press, San Diego, CA). The kinases may be categorized into families by the substrates they phosphorylate (e.g., protein-tyrosine, protein-serine/threonine, lipids, etc.). Sequence motifs have been identified that generally correspond to each of these kinase families (e.g., Hanks, S.K., Hunter, T., FASEB J., 9:576-596 (1995); Knighton, et al., Science, 253:407-414 (1991); Hiles, et al., Cell, 70:419-429 (1992); Kunz, et al., Cell, 73:585-596 (1993); Garcia-Bustos, et al., EMBO J., 13:2352-2361 (1994)).

Protein kinases may be characterized by their regulation mechanisms. These mechanisms include, for example, autophosphorylation, transphosphorylation by other kinases, protein-protein interactions, protein-lipid interactions, and protein-polynucleotide interactions. An individual protein kinase may be regulated by more than one mechanism.

Kinases regulate many different cell processes including, but not limited to, proliferation, differentiation, apoptosis, motility, transcription, translation and other signalling processes, by adding phosphate groups to target proteins. These phosphorylation events act as molecular on/off switches that can modulate or regulate the target protein biological function. Phosphorylation of target proteins occurs in response to a variety of extracellular signals (hormones, neurotransmitters, growth and differentiation factors, etc.), cell cycle events, environmental or nutritional stresses, etc. The appropriate protein kinase functions in signalling pathways to activate or inactivate (either directly or indirectly), for example, a metabolic enzyme, regulatory protein, receptor, cytoskeletal protein, ion channel or pump, or transcription factor. Uncontrolled signalling due to defective control of protein phosphorylation has been implicated in a number of diseases, including, for example, inflammation, cancer, allergy/asthma, disease and conditions of the immune system, disease and conditions of the central nervous system, and angiogenesis.

### Cyclin Dependent Kinases

The process of eukaryotic cell division may be broadly divided into a series of sequential phases termed G1, S, G2 and M. Correct progression through the various phases of the cell cycle has been shown to be critically dependent upon the spatial and temporal regulation of a family of proteins known as cyclin dependent kinases (cdks) and a diverse set of their cognate protein partners termed cyclins. Cdks are cdc2 (also known as cdk1) homologous serine-threonine kinase proteins that are able to utilise ATP as a substrate in the phosphorylation of diverse polypeptides in a sequence dependent context Cyclins are a family of proteins characterised by a homology region, containing approximately 100 amino acids, termed the "cyclin box" which is used in binding to, and defining selectivity for, specific cdk partner proteins.

Modulation of the expression levels, degradation rates, and activation levels of various cdks and cyclins throughout the cell cycle leads to the cyclical formation of a series of cdk/cyclin complexes, in which the cdks are enzymatically active. The formation of these complexes controls passage through discrete cell cycle checkpoints and thereby enables the process of cell division to continue. Failure to satisfy the pre-requisite biochemical criteria at a given cell cycle checkpoint, i.e. failure to form a required cdk/cyclin complex, can lead to cell cycle arrest and/or cellular apoptosis. Aberrant cellular proliferation, as manifested in cancer, can often be attributed to loss of correct cell cycle control. Inhibition of cdk enzymatic activity therefore provides a means by which abnormally dividing cells can have their division arrested and/or be killed. The diversity of cdks, and cdk complexes, and their critical roles in mediating the cell cycle, provides a broad spectrum of potential therapeutic targets selected on the basis of a defined biochemical rationale.

Progression from the G1 phase to the S phase of the cell cycle is primarily regulated by cdk2, cdk3, cdk4 and cdk6 via association with members of the D and E type cyclins. The D-type cyclins appear instrumental in enabling passage beyond the G1 restriction point, where as the cdk2/cyclin E complex is key to the transition from the G1 to S phase. Subsequent progression through S phase and entry into G2 is thought to require the cdk2/cyclin A complex. Both mitosis, and the G2 to M phase transition which triggers it, are regulated by complexes of cdk1 and the A and B type cyclins.

During G1 phase Retinoblastoma protein (Rb), and related pocket proteins such as p130, are substrates for cdk(2, 4, & 6)/cyclin complexes. Progression through G1 is in part facilitated by hyperphosphorylation, and thus inactivation, of Rb and p130 by the cdk(4/6)/cyclin-D complexes. Hyperphosphorylation of Rb and p130 causes the release of transcription factors, such as E2F, and thus the expression of genes necessary for progression through G1 and for entry into S-phase, such as the gene for cyclin E. Expression of cyclin E facilitates formation of the cdk2/cyclin E complex which amplifies, or maintains, E2F levels via further phosphorylation of Rb. The cdk2/cyclin E complex also phosphorylates other proteins necessary for DNA replication, such as NPAT, which has been implicated In histone biosynthesis. G1 progression and the G1/S transition are also regulated via the mitogen stimulated Myc pathway, which feeds into the cdk2/cyclin E pathway. Cdk2 is also connected to the p53 mediated DNA damage response pathway via p53 regulation of p21 levels. p21 is a protein inhibitor of cdk2/cyclin E and is thus capable of blocking, or delaying, the G1/S transition. The cdk2/cyclin E complex may thus represent a point at which biochemical stimuli from the Rb, Myc and p53 pathways are to some degree integrated. Cdk2 and/or the cdk2/cyclin E complex therefore represent good targets for therapeutics designed at arresting, or recovering control of, the cell cycle in aberrantly dividing cells.

The exact role of cdk3 in the cell cycle is not clear. As yet no cognate cyclin partner has been identified, but a dominant negative form of cdk3 delayed cells in G1, thereby suggesting that cdk3 has a role in regulating the G1/S transition.

Although most cdks have been implicated in regulation of the cell cycle there is evidence that certain members of the cdk family are involved in other biochemical processes. This is exemplified by cdk5 which is necessary for correct neuronal development and which has also been implicated in the phosphorylation of several neuronal proteins such as Tau, NUDE-1, synapsin1, DARPP32 and the Munc18/Syntaxin1A complex. Neuronal cdk5 is conventionally activated by binding to the p35/p39 proteins. Cdk5 activity can, however, be deregulated by the binding of p25, a truncated version of p35. Conversion of p35 to p25, and subsequent deregulation of cdk5 activity, can be induced by ischemia, excitotoxicity, and β-amyloid peptide. Consequently p25 has been implicated in the pathogenesis of neurodegenerative diseases, such as Alzheimer's, and is therefore of interest as a target for therapeutics directed against these diseases.

Cdk7 is a nuclear protein that has cdc2 CAK activity and binds to cyclin H. Cdk7 has been identified as component of the TFIIH transcriptional complex which has RNA polymerase II C-terminal domain (CTD) activity. This has been associated with the regulation of HIV-1 transcription via a Tat-mediated biochemical pathway. Cdk8 binds cyclin C and has been implicated in the phosphorylation of the CTD of RNA polymerase II. Similarly the cdk9/cyclin-T1 complex (P-TEFb complex) has been implicated in elongation control of RNA polymerase II. PTEF-b is also required for activation of transcription of the HIV-1 genome by the viral transactivator Tat through its interaction with cyclin T1. Cdk7, cdk8, cdk9 and the P-TEFb complex are therefore potential targets for anti-viral therapeutics.

At a molecular level mediation of cdk/cyclin complex activity requires a series of stimulatory and inhibitory phosphorylation, or dephosphorylation, events. Cdk phosphorylation is performed by a group of cdk activating kinases (CAKs) and/or kinases such as wee1, Myt1 and Mik1. Dephosphorylation is performed by phosphatases such as cdc25(a & c), pp2a, or KAP.

Cdk/cyclin complex activity may be further regulated by two families of endogenous cellular proteinaceous inhibitors: the Kip/Cip family, or the INK family. The INK proteins specifically bind cdk4 and cdk6. p16^{ink4} (also known as MTS1) is a potential tumour suppressor gene that is mutated, or deleted, in a large number of primary cancers. The Kip/Cip family contains proteins such as p21^{Cip1,Waf1}, p27^{Kip1} and p57^{kip2}. As discussed previously p21 is induced by p53 and is able to inactivate the cdk2/cyclin(E/A) and cdk4/cyclin(D1/D2/D3) complexes. Atypically low levels of p27 expression have been observed in breast, colon and prostate cancers. Conversely over expression of cyclin E in solid tumours has been shown to correlate with poor patient prognosis. Over expression of cyclin D1 has been associated with oesophageal, breast, squamous, and non-small cell lung carcinomas.

The pivotal roles of cdks, and their associated proteins, in co-ordinating and driving the cell cycle in proliferating cells have been outlined above. Some of the biochemical pathways in which cdks play a key role have also been described. The development of monotherapies for the treatment of proliferative disorders, such as cancers, using therapeutics targeted generically at cdks, or at specific cdks, is therefore potentially highly desirable. Cdk inhibitors could conceivably also be used to treat other conditions such as viral infections, autoimmune diseases and neuro-degenerative diseases, amongst others. Cdk targeted therapeutics may also provide clinical benefits in the treatment of the previously described diseases when used in combination therapy with either existing, or new, therapeutic agents. Cdk targeted anticancer therapies could potentially have advantages over many current antitumour agents as they would not directly interact with DNA and should therefore reduce the risk of secondary tumour development.

### Glycogen Synthase Kinase

Glycogen Synthase Kinase-3 (GSK3) is a serine-threonine kinase that occurs as two ubiquitously expressed isoforms in humans (GSK3α & beta GSK3β). GSK3 has been implicated as having roles in embryonic development, protein synthesis, cell proliferation, cell differentiation, microtubule dynamics, cell motility and cellular apoptosis. As such GSK3 has been implicated in the progression of disease states such as diabetes, cancer, Alzheimer's disease, stroke, epilepsy, motor neuron disease and/or head trauma. Phylogenetically GSK3 is most closely related to the cyclin dependent kinases (CDKs).

The consensus peptide substrate sequence recognised by GSK3 is (Ser/Thr)-X-X-X-(pSer/pThr), where X is any amino acid (at positions (n+1), (n+2), (n+3)) and pSer and pThr are phospho-serine and phospho-threonine respectively (n+4). GSK3 phosphorylates the first serine, or threonine, at position (n). Phospho-serine, or phospho-threonine, at the (n+4) position appears necessary for priming GSK3 to give maximal substrate turnover. Phosphorylation of GSK3α at Ser21, or GSK3β at Ser9, leads to inhibition of GSK3. Mutagenesis and peptide competition studies have led to the model that the phosphorylated N-terminus of GSK3 is able to compete with phospho-peptide substrate (S/TXXXpS/pT) via an autoinhibitory mechanism. There are also data suggesting that GSK3α and GSKβ may be subtly regulated by phosphorylation of tyrosines 279 and 216 respectively. Mutation of these residues to a Phe caused a reduction in in vivo kinase activity. The X-ray crystallographic structure of GSK-3β has helped to shed light on all aspects of GSK3 activation and regulation.

GSK3 forms part of the mammalian insulin response pathway and is able to phosphorylate, and thereby inactivate, glycogen synthase. Upregulation of glycogen synthase activity, and thereby glycogen synthesis, through inhibition of GSK3, has thus been considered a potential means of combating type II, or non-insulin-dependent diabetes mellitus (NIDDM): a condition in which body tissues become resistant to insulin stimulation. The cellular insulin response in liver, adipose, or muscle tissues is triggered by insulin binding to an extracellular insulin receptor. This causes the phosphorylation, and subsequent recruitment to the plasma membrane, of the insulin receptor substrate (IRS) proteins. Further phosphorylation of the IRS proteins initiates recruitment of phosphoinositide-3 kinase (Pl3K) to the plasma membrane where it is able to liberate the second messenger phosphatidylinosityl 3,4,5-trisphosphate (PIP3). This facilitates co-localisation of 3-phosphoinositide-dedependent protein kinase 1 (PDK1) and protein kinase B (PKB or Akt) to the membrane, where PDK1 activates PKB. PKB is able to phosphorylate, and thereby inhibit, GSK3α and/or GSKβ through phosphorylation of Ser9, or ser21, respectively. The inhibition of GSK3 then triggers upregulation of glycogen synthase activity. Therapeutic agents able to inhibit GSK3 may thus be able to induce cellular responses akin to those seen on insulin stimulation. A further in vivo substrate of GSK3 is the eukaryotic protein synthesis initiation factor 2B (elF2B). elF2B is inactivated via phosphorylation and is thus able to suppress protein biosynthesis. Inhibition of GSK3, e.g. by inactivation of the "mammalian target of rapamycin" protein (mTOR), can thus upregulate protein biosynthesis. Finally there is some evidence for regulation of GSK3 activity via the mitogen activated protein kinase (MAPK) pathway through phosphorylation of GSK3 by kinases such as mitogen activated protein kinase activated protein kinase 1 (MAPKAP-K1 or RSK). These data suggest that GSK3 activity may be modulated by mitogenic, insulin and/or amino acid stimulii.

It has also been shown that GSK-3β is a key component in the vertebrate Wnt signalling pathway. This biochemical pathway has been shown to be critical for normal embryonic development and regulates cell proliferation in normal tissues. GSK3 becomes inhibited in response to Wnt stimulii. This can lead to the dephosphorylation of GSK3 substrates such as Axin, the adenomatous polyposis coli (APC) gene product and β-catenin. Aberrant regulation of the Wnt pathway has been associated with many cancers. Mutations in APC. and/or β-catenin, are common in colorectal cancer and other tumours. β-catenin has also been shown to be of importance in cell adhesion. Thus GSK3 may also modulate cellular adhesion processes to some degree. Apart from the biochemical pathways already described there are also data implicating GSK3 in the regulation of cell division via phosphorylation of cyclin-D1, in the phosphorylation of transcription factors such as c-Jun, CCAAT/enhancer binding protein α (C/EBPα), c-Myc and/or other substrates such as Nuclear Factor of Activated T-cells (NFATc), Heat Shock Factor-1 (HSF-1) and the c-AMP response element binding protein (CREB). GSK3 also appears to play a role, albeit tissue specific, in regulating cellular apoptosis. The role of GSK3 in modulating cellular apoptosis, via a pro-apoptotic mechanism, may be of particular relevance to medical conditions in which neuronal apoptosis can occur. Examples of these are head trauma, stroke, epilepsy, Alzheimer's and motor neuron diseases, progressive supranuclear palsy, corticobasal degeneration, and Pick's disease. In vitro it has been shown that GSK3 is able to hyper-phosphorylate the microtubule associated protein Tau. Hyperphosphorylation of Tau disrupts its normal binding to microtubules and may also lead to the formation of intra-cellular Tau filaments. It is believed that the progressive accumulation of these filaments leads to eventual neuronal dysfunction and degeneration. Inhibition of Tau phosphorylation, through inhibition of GSK3, may thus provide a means of limiting and/or preventing neurodegenerative effects.

### Diffuse Large B-cell Lymphomas (DLBCL)

Cell cycle progression is regulated by the combined action of cyclins, cyclin-dependent kinases (CDKs), and CDK-inhibitors (CDKi), which are negative cell cycle regulators. p27KIP1 is a CDKi key in cell cycle regulation, whose degradation is required for G1/S transition. In spite of the absence of p27KIP1 expression in proliferating lymphocytes, some aggressive B-cell lymphomas have been reported to show an anomalous p27KIP1 staining. An abnormally high expression of p27KIP1 was found in lymphomas of this type. Analysis of the clinical relevance of these findings showed that a high level of p27KIP1 expression in this type of tumour is an adverse prognostic marker, in both univariate and multivariate analysis. These results show that there is abnormal p27KIP1 expression in Diffuse Large B-cell Lymphomas (DLBCL), with adverse clinical significance, suggesting that this anomalous p27KIP1 protein may be rendered non-functional through interaction with other cell cycle regulator proteins. (Br. J. Cancer. 1999 Jul;80(9):1427-34. p27KIP1 is abnormally expressed in Diffuse Large B-cell Lymphomas and is associated with an adverse clinical outcome. Saez A, Sanchez E, Sanchez-Beato M, Cruz MA, Chacon I, Munoz E, Camacho Fl, Martinez-Montero JC, Mollejo M, Garcia JF, Piris MA. Department of Pathology, Virgen de la Salud Hospital, Toledo, Spain.)

### Chronic Lymphocytic Leukemia

B-Cell chronic lymphocytic leukaemia (CLL) is the most common leukaemia in the Western hemisphere, with approximately 10,000 new cases diagnosed each year (Parker SL, Tong T, Bolden S, Wingo PA: Cancer statistics, 1997. Ca. Cancer. J. Clin. 47:5, (1997)). Relative to other forms of leukaemia, the overall prognosis of CLL is good, with even the most advanced stage patients having a median survival of 3 years.

The addition of fludarabine as initial therapy for symptomatic CLL patients has led to a higher rate of complete responses (27% v 3%) and duration of progression-free survival (33 v 17 months) as compared with previously used alkylator-based therapies. Although attaining a complete clinical response after therapy is the initial step toward improving survival In CLL. the majority of patients either do not attain complete remission or fail to respond to fludarabine. Furthermore, all patients with CLL treated with fudarabine eventually relapse, making its role as a single agent purely palliative (Rai KR, Peterson B, Elias L, Shepherd L, Hines J, Nelson D, Cheson B, Kolitz J, Schiffer CA: A randomized comparison of fludarabine and chlorambucil for patients with previously untreated chronic lymphocytic leukemia. A CALGB SWOG, CTG/NCI-C and ECOG Inter-Group Study. Blood 88:141 a, 1996 (abstr 552, suppl 1 Therefore, identifying new agents with novel mechanisms of action that complement fludarabine's cytotoxicity and abrogate the resistance induced by intrinsic CLL drug-resistance factors will be necessary if further advances in the therapy of this disease are to be realized.

The most extensively studied, uniformly predictive factor for poor response to therapy and inferior survival In CLL patients is aberrant p53 function, as characterized by point mutations or chromosome 17p13 deletions. Indeed, virtually no responses to either alkylator or purine analog therapy have been documented in multiple single institution case series for those CLL patients with abnormal p53 function. Introduction of a therapeutic agent that has the ability to overcome the drug resistance associated with p53 mutation in CLL would potentially be a major advance for the treatment of the disease.

Flavopiridol and CYC 202, inhibitors of cyclin-dependent kinases induce in vitro apoptosis of malignant cells from B-cell chronic lymphocytic leukemia (B-CLL).

Flavopiridol exposure results in the stimulation of caspase 3 activity and in caspase-dependent cleavage of p27(kip1), a negative regulator of the cell cycle, which is overexpressed in B-CLL (Blood. 1998 Nov 15;92(10):3804-16 Flavopiridol induces apoptosis in chronic lymphocytic leukemia cells via activation of caspase-3 without evidence of bcl-2 modulation or dependence on functional p53. Byrd JC, Shinn C, Waselenko JK, Fuchs EJ, Lehman TA, Nguyen PL, Flinn IW, Diehl LF, Sausville E, Grever MR).

### Ancillary agents

A wide variety of ancillary agents selected from monoclonal antibodies, alkylating agents, anticancer agents, further CDK inhibitors and hormones, hormone agonists, hormone antagonists and hormone modulating agents, find application in the combinations of the invention, as described in detail below.

It is an object of the invention to provide therapeutic combinations of pyrazole compounds that inhibit or modulate (in particular inhibit) the activity of cyclin dependent kinases (CDK) and/or glycogen synthase kinase (e.g. GSK-3) with an ancillary agent selected from: a monoclonal antibody, an alkylating agent, an anticancer agent, a further CDK inhibitor and a hormone, hormone agonist, hormone antagonist or hormone modulating agent. Such combinations may have an advantageous efficacious effect against tumour cell growth, in comparison with the respective effects shown by the individual components of the combination.

### Prior Art

WO 02/34721 from Du Pont discloses a class of indeno [1,2-c]pyrazol-4-ones as inhibitors of cyclin dependent kinases.

WO 01/81348 from Bristol Myers Squibb describes the use of 5-thio-, sulphinyl- and sulphonylpyrazolo[3,4-b]-pyridines as cyclin dependent kinase inhibitors.

WO 00/62778 also from Bristol Myers Squibb discloses a class of protein tyrosine kinase inhibitors.

WO 01/72745A1 from Cyclacel describes 2-substituted 4-heteroaryl-pyrimidines and their preparation, pharmaceutical compositions containing them and their use as Inhibitors of cyclin-dependant kinases (CDKs) and hence their use in the treatment of proliferative disorders such as cancer, leukaemia, psoriasis and the like.

WO 99/21845 from Agouron describes 4-aminothiazole derivatives for inhibiting cyclin-dependent kinases (CDKs), such as CDK1, CDK2, CDK4, and CDK6. The invention is also directed to the therapeutic or prophylactic use of pharmaceutical compositions containing such compounds and to methods of treating malignancies and other disorders by administering effective amounts of such compounds.

WO 01/53274 from Agouron discloses as CDK kinase inhibitors a class of compounds which can comprise an amide-substituted benzene ring linked to an N-containing heterocyclic group.

WO 01/98290 (Pharmacia & Upjohn) discloses a class of 3-aminocarbonyl-2-carboxamido thiophene derivatives as protein kinase inhibitors.

WO 01/53268 and WO 01/02369 from Agouron disclose compounds that mediate or inhibit cell proliferation through the inhibition of protein kinases such as cyclin dependent kinase or tyrosine kinase. The Agouron compounds have an aryl or heteroaryl ring attached directly or though a CH=CH or CH=N group to the 3-position of an indazole ring.

WO 00/39108 and WO 02/00651 (both to Du Pont Pharmaceuticals) describe heterocyclic compounds that are inhibitors of trypsin-like serine protease enzymes, especially factor Xa and thrombin. The compounds are stated to be useful as anticoagulants or for the prevention of thromboembolic disorders.

US 2002/0091116 (Zhu et al.), WO 01/19798 and WO 01/64642 each disclose diverse groups of heterocyclic compounds as inhibitors of Factor Xa. Some 1-substituted pyrazole carboxamides are disclosed and exemplified.

WO 02/070510 (Bayer) describes a class of amino-dicarboxylic acid compounds for use in the treatment of cardiovascular diseases. Although pyrazoles are mentioned generically, there are no specific examples of pyrazoles in this document.

WO 97/03071 (Knoll AG) discloses a class of heterocyclyl-carboxamide derivatives for use in the treatment of central nervous system disorders. Pyrazoles are mentioned generally as examples of heterocyclic groups but no specific pyrazole compounds are disclosed or exemplified.

WO 97/40017 (Novo Nordisk) describes compounds that are modulators of protein tyrosine phosphatases.

WO 031020217 (Univ. Connecticut) discloses a class of pyrazole 3-carboxamides as cannabinoid receptor modulators for treating neurological conditions. It is stated (page 15) that the compounds can be used In cancer chemotherapy but it is not made clear whether the compounds are active as anti-cancer agents or whether they are administered for other purposes.

WO 01/58869 (Bristol Myers Squibb) discloses cannabinoid receptor modulators that can be used inter alia to treat a variety of diseases. The main use envisaged is the treatment of respiratory diseases, although reference is made to the treatment of cancer.

WO 01/02385 (Aventis Crop Science) discloses 1-(quinoline-4-yl)-1H-pyrazole derivatives as fungicides. 1-Unsubsituted pyrazoles are disclosed as synthetic intermediates.

WO 2004/039795 (Fujisawa) discloses amides containing a 1-substituted pyrazole group as inhibitors of apolipoprotein B secretion. The compounds are stated to be useful In treating such conditions as hyperlipidemia.

WO 2004/000318 (Cellular Genomics) discloses various amino-substituted monocycles as kinase modulators. None of the exemplified compounds are pyrazoles.

WO2005/002552 (Astex) discloses benzoimidazolylpyrazoles and aza-analogues thereof as inhibitors of cyclin dependent kinases, glycogen synthase kinases and Aurora kinases.

### Summary of the Invention

The invention provides combinations of an ancillary agent selected from monoclonal antibodies, alkylating agents, anticancer agents, further CDK inhibitors and hormones, hormone agonists, hormone antagonists and hormone modulating agents, with pyrazole compounds that have cyclin dependent kinase inhibiting or modulating activity, wherein the combinations have efficacy against abnormal cell growth. The invention further provides combinations of the invention which are further combined with other classes of therapeutic agents or treatments that may be administered together (whether concurrently or at different time intervals) with the combinations of the invention, as described below.

Thus, for example, it is envisaged that the combinations of the invention will be useful in alleviating or reducing the incidence of cancer.

Accordingly, in one aspect, the invention provides a combination comprising an ancillary agent and a compound of the formula (II): 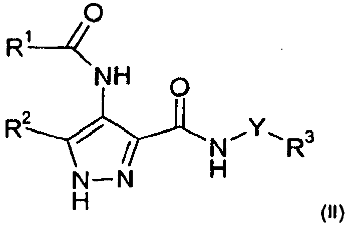 or salts or tautomers or N-oxides or solvates thereof; wherein the ancillary agent is selected from:
(a) a monoclonal antibody to a cell surface antigen; or
(b) an alkylating agent wherein said alkylating agent is selected from nitrogen mustards; nitrosoureas; bifunctional alkylating agents having a bis-alkanesulfonate group; and aziridlne compounds; and wherein the alkylating agents are other than mitomycin C or cyclophosphamide: or
(c) an anticancer agent selected from COX-2 inhibitors. HDAC inhibitors, DNA methyltransferase (methylase) inhibitors and proteasome inhibitors; or
(d) a further CDK inhibitor, and
(e) an anti-androgen including an anti-oestrogen;
   Y is a bond or an alkylene chain of 1, 2 or 3 carbon atoms in length;
   R¹ is a carbocyclic or heterocyclic group having from 3 to 12 ring members, wherein the carbocyclic or heterocyclic group is unsubstituted or substituted by one or more substituent groups R¹⁰; or a C₁₋₈ hydrocarbyl group optionally substituted by one or more substituents selected from fluorine, hydroxy, C₁₋₄ hydrocarbyloxy, amino, mono- or di-C₁₋₄ hydrocarbylamino, and carbocyclic or heterocyclic groups having from 3 to 12 ring members wherein the carbocyclic or heterocyclic groups are unsubstituted or substituted by one or more substituent groups R¹⁰;
   R² is hydrogen or methyl;
   R³ is selected from non-aromatic carbocyclic and heterocyclic groups having from 3 to 12 ring members, wherein the carbocyclic or heterocyclic group is unsubstituted or substituted by one or more substituent groups R¹⁰; and
   R¹⁰ is selected from halogen, hydroxy, trifluoromethyl, cyano, nitro, carboxy, amino, mono- or di-C₁₋₄ hydrocarbylamino, carbocyclic and heterocyclic groups having from 3 to 12 ring members; a group R^{a}-R^{b} wherein R^{a} is a bond, O, CO, X¹C(X²), C(X²)X¹, X¹C(X²)X¹, S, SO, SO₂, NR^{c}, SOₐNR^{c} or NR^{c}SO₂; and R^{b} is selected from hydrogen, carbocyclic and heterocyclic groups having from 3 to 12 ring members, and a C₁₋₈ hydrocarbyl group optionally substituted by one or more substituents selected from hydroxy, oxo, halogen, cyano, nitro, carboxy, amino, mono- or di-C₁₋₄ hydrocarbylamino, carbocyclic and heterocyclic groups having from 3 to 12 ring members;
   R^{c} is selected from hydrogen and C₁₋₄ hydrocarbyl; and
   X¹ is O, S or NR^{c} and X² is =O, =S or =NR^{c};
   and provided that where the substituent group R¹⁰ comprises or includes a carbocyclic or heterocyclic group, the said carbocyclic or heterocyclic group may be unsubstituted or may itself be substituted with one or more further substituent groups R¹⁰ and wherein (a) such further substituent groups R¹⁰ include carbocyclic or heterocyclic groups, which are not themselves further substituted; or (b) the said further substituents do not include carbocyclic or heterocyclic groups but are otherwise selected from the groups listed above in the definition of R¹⁰.

Any one or more of the following optional provisos, in any combination, may apply to the compounds of formula (II) and sub-groups thereof:
(a-i) R¹ is other than a moiety containing a purine nucleoside group.
(a-ii) When Y-R³ is an alkyl, cycloalkyl, optionally substituted phenyl or optionally substituted phenylalkyl group, then R¹ is other than a substituted or unsubstituted tetrahydronaphthalene, tetrahydroquinolinyl, tetrahydrochromanyl or tetrahydrothiochromanyl group.
(a-iii) R¹ is other than 4-(tert-butyloxycarbonylamino)-3-methylimidazol-2-yl.
(b-i) R³ is other than a bridged azabicyclo group.
(b-ii) When R¹ or R³ contain a moiety in which a heterocyclic ring having an S(=O)₂ ring member is fused to a carbocyclic ring, the said carbocyclic ring is other than a substituted or unsubstituted benzene ring

The reference in proviso (a-i) to a purine nucleoside group refers to substituted and unsubstituted purine groups having attached thereto a monosaccharide group (e.g. a pentose or hexose) or a derivative of a monosaccharide group, for example a deoxy monosaccharide group or a substituted monosaccharide group.

The reference in proviso (b-i) to a bridged azabicyclo group refers to bicycloalkane bridged ring systems in which one of the carbon atoms of the bicycloalkane has been replaced by a nitrogen atom. In bridged ring systems, two rings share more than two atoms, see for example Advanced Organic Chemistry, by Jerry March, 4th Edition, Wiley Interscience, pages 131-133, 1992.

The provisos (a-i) to (a-x), (b-i) to (b-vii), (c-i) and (c-ii) in formulae (I), (Ia) and (Ib) above refer to the disclosures in the following prior art documents.
(a-i) WO 03/014137
(a-ii) WO 97/48672, WO 97/19052
(b-i) WO 03/040147
(b-ii) WO 00/59902

Any one or more of the foregoing optional provisos may apply to the compounds of formulae (II), (IV), (IVa), (Va), (Vb), (VIa) or (VIb) and sub-groups thereof or salts or tautomers or N-oxides or solvates thereof as defined herein.

In the following aspects and embodiments of the invention, references to "a combination according to the invention" refer to the combination of an ancillary agent as hereinabove described and a compound of formula (II), (IV), (IVa), (Va), (Vb), (VIa) and (VIb). In this section, as in all other sections of this application, unless the context indicates otherwise, references to a compound of formula (II), (IV), (IVa), (Va), (Vb), (Via) or (Vlb) includes all other subgroups defined herein. The term 'subgroups' includes all preferences, examples and particular compounds defined herein.

Moreover a reference to a compound of formula (II), (IV), (IVa), (Va), (Vb), (Via) or (VIb) and sub-groups thereof includes ionic, salt, solvate, isomers, tautomers, N-oxides, ester, prodrugs, isotopes and protected forms thereof, as discussed below. Preferably the salts or tautomers or isomers or N-oxides or solvates thereof. More preferably, the salts or tautomers or N-oxides or solvates.

The invention also provides:
- A combination according to the invention for use in alleviating or reducing the incidence of a disease or condition comprising or arising from abnormal cell growth in a mammal.
- A combination of the invention for use in the prophylaxis or treatment of a disease state or condition mediated by a cyclin dependent kinase or glycogen synthase kinase-3.
- A combination of the invention for use in a method for the prophylaxis or treatment of a disease state or condition mediated by a cyclin dependent kinase or glycogen synthase kinase-3, which method comprises administering to a subject in need thereof the combination of the invention.
- A combination of the invention for use in a method for alleviating or reducing the incidence of a disease state or condition mediated by a cyclin dependent kinase or glycogen synthase kinase-3, which method comprises administering to a subject in need thereof the combination of the invention.
- A combination of the invention for use In a method for alleviating or reducing the Incidence of a disease or condition comprising or arising from abnormal cell growth in a mammal, which method comprises administering to the mammal the combination according to the invention in an amount effective in inhibiting abnormal cell growth.
- A combination of the invention for use in a method for treating a disease or condition comprising or arising from abnormal cell growth in a mammal, which method comprises administering to the mammal the combination according to the invention in an amount effective in inhibiting abnormal cell growth.
- A combination according to the invention for use in inhibiting tumour growth in a mammal.
- A combination of the invention for use in a method of inhibiting tumour growth in a mammal, which method comprises administering to the mammal an effective tumour growth-inhibiting amount of the combination according to the invention.
- A combination according to the invention for use in inhibiting the growth of tumour cells.
- A combination of the invention for use in a method of inhibiting the growth of tumour cells, which method comprises contacting the tumour cells with an effective tumour cell growth-inhibiting amount of the combination according to the invention.
- A pharmaceutical composition comprising a combination according to the invention and a pharmaceutically acceptable carrier.
- A combination according to the invention for use in medicine.
- The use of a combination according to the invention, for the manufacture of a medicament for the prophylaxis or treatment of any one of the disease states or conditions disclosed herein.
- A combination of the invention for use in a method for the treatment or prophylaxis of any one of the disease states or conditions disclosed herein, which method comprises administering to a patient (e.g. a patient in need thereof) the combination according to the invention.
- A combination of the invention for use in a method for alleviating or reducing the incidence of a disease state or condition disclosed herein, which method comprises administering to a patient (e,g, a patient in need thereof) the combination according to the Invention.
- A combination of the invention for use in a method for the diagnosis and treatment of a cancer in a mammalian patient, which method comprises (i) screening a patient to determine whether a cancer from which the patient is or may be suffering is one which would be susceptible to treatment with a compound having activity against cyclin dependent kinases and an ancillary agent as hereinabove described and (ii) where it is indicated that the disease or condition from which the patient is thus susceptible, thereafter administering to the patient the combination according to the invention.
- The use of a combination according to the invention for the manufacture of a medicament for the treatment or prophylaxis of a cancer in a patient who has been screened and has been determined as suffering from, or being at risk of suffering from, a cancer which would be susceptible to treatment with a combination of an ancillary agent as hereinabove described and a compound having activity against cyclin dependent kinase.
- A combination of the invention for use in a method for treating a cancer in a patient comprising administration of the combination according to the invention to said patient in an amount and in a schedule of administration that is therapeutically efficacious in the treatment of said cancer.
- A combination of the invention for use in a method for preventing, treating or managing cancer in a patient in need thereof, said method comprising administering to said patient a prophylactically or therapeutically effective amount of the combination according to the invention.
- The use of a combination according to the invention for the manufacture of a medicament for use in the production of an anti-cancer effect in a warm-blooded animal such as a human.
- A kit comprising a combination according to the invention.
- A pharmaceutical kit for anticancer therapy comprising an ancillary agent as hereinabove described in dosage form and a compound of the formula (II), (IV), (IVa), (Va), (Vb), (Via) or (VIb) and sub-groups thereof as defined herein, also in dosage form (e.g. wherein the dosage forms are packaged together in common outer packaging).
- A compound of the formula (II), (IV), (IVa), (Va), (Vb), (Via) or (VIb) and sub-groups thereof as defined herein for use in combination therapy with an ancillary agent as hereinabove described to alleviate or reduce the incidence of a disease or condition comprising or arising from abnormal cell growth in a mammal.
- A compound of the formula (II), (IV), (IVa), (Va), (Vb), (VIa) or (VIb) and sub-groups thereof as defined herein for use in combination therapy with an ancillary agent as hereinabove described to inhibit tumour growth in a mammal.
- A compound of the formula (II), (IV), (IVa), (Va), (Vb), (VIa) or (VIb) and sub-groups thereof as defined herein for use in combination therapy with an ancillary agent as hereinabove described to prevent, treat or manage cancer in a patient in need thereof.
- A compound of the formula (II), (IV), (IVa), (Va), (Vb), (Via) or (VIb) and sub-groups thereof as defined herein for use in enhancing or potentiating the response rate in a patient suffering from a cancer where the patient is being treated with an ancillary agent as hereinabove described.
- The use of a combination according to the invention for the manufacture of a medicament for any of the therapeutic uses as defined herein.

In each of the foregoing uses, methods and other aspects of the invention, as well as any aspects and embodiments of the invention as set out below, references to compounds of the formulae (II), (IV), (IVa), (Va), (Vb), (Via) or (VIb) and sub-groups thereof as defined herein include within their scope the salts or solvates or tautomers or N-oxides of the compounds.

The invention also provides the further combinations, uses, methods, compounds and processes as set out in the claims below.

### General Preferences and Definitions

As used herein, the term "modulation", as applied to the activity of cyclin dependent kinase (CDK) and glycogen synthase kinase (GSK, e.g. GSK-3), is intended to define a change in the level of biological activity of the kinase(s). Thus, modulation encompasses physiological changes which effect an increase or decrease in the relevant kinase activity. In the latter case, the modulation may be described as "inhibition". The modulation may arise directly or indirectly, and may be mediated by any mechanism and at any physiological level, including for example at the level of gene expression (including for example transcription, translation and/or post-translational modification), at the level of expression of genes encoding regulatory elements which act directly or indirectly on the levels of cyclin dependent kinase (CDK) and/or glycogen synthase kinase-3 (GSK-3) activity, or at the level of enzyme (e.g. cyclin dependent kinase (CDK) and/or glycogen synthase kinase-3 (GSK-3)) activity (for example by allosteric mechanisms, competitive inhibition, active-site inactivation, perturbation of feedback inhibitory pathways etc.). Thus, modulation may imply elevated/suppressed expression or over- or under-expression of the cyclin dependent kinase (CDK) and/or glycogen synthase kinase-3 (GSK-3), including gene amplification (i.e. multiple gene copies) and/or increased or decreased expression by a transcriptional effect, as well as hyper- (or hypo-)activity and (de)activation of the cyclin dependent kinase (CDK) and/or glycogen synthase kinase-3 (GSK-3) (including (de)activation) by mutation(s). The terms "modulated" and "modulate" are to be interpreted accordingly.

As used herein, the term "mediated", as used e.g. in conjunction with the cyclin dependent kinases (CDK) and/or glycogen synthase kinase-3 (GSK-3) as described herein (and applied for example to various physiological processes, diseases, states, conditions, therapies, treatments or interventions) is intended to operate limitatively so that the various processes, diseases, states, conditions, treatments and interventions to which the term is applied are those in which cyclin dependent kinase (CDK) and/or glycogen synthase klnase-3 (GSK-3) plays a biological role. In cases where the term is applied to a disease, state or condition, the biological role played by cyclin dependent kinase (CDK) and/or glycogen synthase kinase-3 (GSK-3) may be direct or indirect and may be necessary and/or sufficient for the manifestation of the symptoms of the disease, state or condition (or its aetiology or progression). Thus, cyclin dependent kinase (CDK) and/or glycogen synthase kinase-3 (GSK-3) activity (and in particular aberrant levels of cyclin dependent kinase (CDK) and/or glycogen synthase kinase-3 (GSK-3)activity, e.g. cyclin dependent kinases (CDK) and/or glycogen synthase kinase-3 (GSK-3) over-expression) need not necessarily be the proximal cause of the disease, state or condition: rather, it is contemplated that the CDK- and/or GSK- (e.g. GSK-3-) mediated diseases, states or conditions include those having multifactorial aetiologies and complex progressions in which CDK and/or GSK-3 is only partially involved. In cases where the term is applied to treatment, prophylaxis or intervention (e.g. in the "CDK-mediated treatments" and "GSK-3-mediated prophylaxis" of the invention), the role played by CDK and/or GSK-3 may be direct or indirect and may be necessary and/or sufficient for the operation of the treatment, prophylaxis or outcome of the intervention.

The term "intervention" is a term of art used herein to define any agency which effects a physiological change at any level. Thus, the intervention may comprises the induction or repression of any physiological process, event, biochemical pathway or cellular/biochemical event. The interventions of the invention typically effect (or contribute to) the therapy, treatment or prophylaxis of a disease or condition.

The combinations of the invention are combinations of an ancillary agent as hereinbefore described and a compound of the formulae (II), (IV), (IVa), (Va), (Vb), (VIa) or (VIb) and sub-groups thereof that produce a therapeutically efficacious effect.

The term 'efficacious' includes advantageous effects such as additivity, synergism, reduced side effects, reduced toxicity, increased time to disease progression, increased time of survival, sensitization or resensitization of one agent to another, or improved response rate. Advantageously, an efficacious effect may allow for lower doses of each or either component to be administered to a patient, thereby decreasing the toxicity of chemotherapy, whilst producing and/or maintaining the same therapeutic effect.

A "synergistic" effect in the present context refers to a therapeutic effect produced by the combination which is larger than the sum of the therapeutic effects of the components of the combination when presented individually.

An "additive" effect in the present context refers to a therapeutic effect produced by the combination which is larger than the therapeutic effect of any of the components of the combination when presented individually.

The term "response rate" as used herein refers, in the case of a solid tumour, to the extent of reduction in the size of the tumour at a given time point, for example 12 weeks. Thus, for example, a 50% response rate means a reduction in tumour size of 50%. References herein to a "clinical response" refer to response rates of 50% or greater. A "partial response" is defined herein as being a response rate of less than 50%.

As used herein, the term "combination", as applied to two or more compounds and/or agents (also referred to herein as the *components*), may define material In which the two or more compounds/agents are associated. The terms "combined" and "combining" in this context are to be interpreted accordingly.

The association of the two or more compounds/agents in a combination may be physical or non-physical. Examples of physically associated combined compounds/agents include:
- compositions (e.g. unitary formulations) comprising the two or more compounds/agents in admixture (for example within the same unit dose);
- compositions comprising material in which the two or more compounds/agents are chemically/physicochemically linked (for example by crosslinking, molecular agglomeration or binding to a common vehicle moiety);
- compositions comprising material in which the two or more compounds/agents are chemically/physioochemically co-packaged (for example, disposed on or within lipid vesicles, particles (e.g. micro- or nanoparticles) or emulsion droplets);
- pharmaceutical kits, pharmaceutical packs or patient packs in which the two or more compounds/agents are co-packaged or co-presented (e.g. as part of an array of unit doses);

Examples of non-physically associated combined compounds/agents include:
- material (e.g. a non-unitary formulation) comprising at least one of the two or more compounds/agents together with instructions for the extemporaneous association of the at least one compound to form a physical association of the two or more compounds/agents;
- material (e.g. a non-unitary formulation) comprising at least one of the two or more compounds/agents together with instructions for combination therapy with the two or more compounds/agents;
- material comprising at least one of the two or more compounds/agents together with instructions for administration to a patient population in which the other(s) of the two or more compounds/agents have been (or are being) administered;
- material comprising at least one of the two or more compounds/agents in an amount or in a form which is specifically adapted for use in combination with the other(s) of the two or more compou nd s/agents.

As used herein, the term "combination therapy" is intended to define therapies which comprise the use of a combination of two or more compounds/agents (as defined above). Thus, references to "combination therapy", "combinations" and the use of compounds/agents "in combination" in this application may refer to compounds/agents that are administered as part of the same overall treatment regimen. As such, the posology of each of the two or more compounds/agents may differ each may be administered at the same time or at different times. It will therefore be appreciated that the compounds/agents of the combination may be administered sequentially (e.g. before or after) or simultaneously, either in the same pharmaceutical formulation (i.e. together), or in different pharmaceutical formulations (i.e. separately). Simultaneously in the same formulation is as a unitary formulation whereas simultaneously in different pharmaceutical formulations is non-unitary. The posologies of each of the two or more compounds/agents in a combination therapy may also differ with respect to the route of administration.

As used herein, the term "pharmaceutical kit" defines an array of one or more unit doses of a pharmaceutical composition together with dosing means (e.g. measuring device) and/or delivery means (e.g. inhaler or syringe), optionally all contained within common outer packaging. In pharmaceutical kits comprising a combination of two or more compounds/agents, the individual compounds/agents may unitary or non-unitary formulations. The unit dose(s) may be contained within a blister pack. The pharmaceutical kit may optionally further comprise instructions for use.

As used herein, the term "pharmaceutical pack" defines an array of one or more unit doses of a pharmaceutical composition, optionally contained within common outer packaging. In pharmaceutical packs comprising a combination of two or more compounds/agents, the individual compounds/agents may unitary or non-unitary formulations. The unit dose(s) may be contained within a blister pack. The pharmaceutical pack may optionally further comprise instructions for use.

As used herein, the term "patient pack" defines a package, prescribed to a patient, which contains pharmaceutical compositions for the whole course of treatment. Patient packs usually contain one or more blister pack(s). Patient packs have an advantage over traditional prescriptions, where a pharmacist divides a patient's supply of a pharmaceutical from a bulk supply, in that the patient always has access to the package insert contained in the patient pack, normally missing in patient prescriptions. The inclusion of a package insert has been shown to improve patient compliance with the physician's instructions.

The combinations of the invention may produce a therapeutically efficacious effect relative to the therapeutic effect of the individual compounds/agents when administered separately.

The following general preferences and definitions shall apply to each of the moieties Y, R¹ to R⁴ and any sub-definition, sub-group or embodiment thereof, unless the context indicates otherwise.

In this specification, references to formula (II) include formulae (IV), (IVa), (Va), (Vb), (Via) or (Vlb) and sub-groups, examples or embodiments of formulae (II), (IV), (IVa), (Va), (Vb), (Via) or (Vlb) unless the context indicates otherwise.

Thus for example, references to inter alia therapeutic uses, pharmaceutical formulations and processes for making compounds, where they refer to formula (II), are also to be taken as referring to formulae (IV), (IVa), (Va), (Vb), (VIa) or (VIb) and sub-groups, examples or embodiments of formulae (III), (IV), (IVa), (Va), (Vb), (VIa) or (Vlb).

Similarly, where preferences, embodiments and examples are given for compounds of the formula (II), they are also applicable to formulae (IV), (IVa), (Va), (Vb), (VIa) or (VIb) and sub-groups, examples or embodiments of formulae (IV), (IVa), (Va), (Vb), (VIa) or (VIb) unless the context requires otherwise.

References to "carbocyclic" and "heterocyclic" groups as used herein shall, unless the context indicates otherwise, include both aromatic and non-aromatic ring systems. Thus, for example, the term "carbocyclic and heterocyclic groups" includes within its scope aromatic, non-aromatic, unsaturated, partially saturated and fully saturated carbocyclic and heterocyclic ring systems. In general, such groups may be monocyclic or bicyclic and may contain, for example, 3 to 12 ring members, more usually 5 to 10 ring members. Examples of monocyclic groups are groups containing 3, 4, 5, 6, 7, and 8 ring members, more usually 3 to 7, and preferably 5 or 6 ring members. Examples of bicyclic groups are those containing 8, 9, 10, 11 and 12 ring members, and more usually 9 or 10 ring members.

The carbocyclic or heterocyclic groups can be aryl or heteroaryl groups having from 5 to 12 ring members, more usually from 5 to 10 ring members. The term "aryl" as used herein refers to a carbocyclic group having aromatic character and the term "heteroaryl" is used herein to denote a heterocyclic group having aromatic character. The terms "aryl" and "heteroaryl" embrace polycyclic (e.g. bicyclic) ring systems wherein one or more rings are non-aromatic, provided that at least one ring is aromatic. In such polycyclic systems, the group may be attached by the aromatic ring, or by a non-aromatic ring. The aryl or heteroaryl groups can be monocyclic or bicyclic groups and, where stated, can be unsubstituted or substituted with one or more substituents, for example one or more groups R¹⁰ as defined herein.

The term "non-aromatic group" embraces unsaturated ring systems without aromatic character, partially saturated and fully saturated carbocyclic and heterocyclic ring systems. The terms "unsaturated" and "partially saturated" refer to rings wherein the ring structure(s) contains atoms sharing more than one valence bond i.e. the ring contains at least one multiple bond e.g. a C=C, C≡C or N=C bond. The term "fully saturated" refers to rings where there are no multiple bonds between ring atoms. Saturated carbocyclic groups include cycloalkyl groups as defined below. Partially saturated carbocyclic groups include cycloalkenyl groups as defined below, for example cyclopentenyl, cycloheptenyl and cyclooctenyl. A further example of a cycloalkenyl group is cyclohexenyl.

Examples of heteroaryl groups are monocyclic and bicyclic groups containing from five to twelve ring members, and more usually from five to ten ring members. The heteroaryl group can be, for example, a five membered or six membered monocyclic ring or a bicyclic structure formed from fused five and six membered rings or two fused six membered rings or, by way of a further example, two fused five membered rings. Each ring may contain up to about four heteroatoms typically selected from nitrogen, sulphur and oxygen. Typically the heteroaryl ring will contain up to 4 heteroatoms, more typically up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general the number of basic nitrogen atoms present in the heteroaryl group, including any amino group substituents of the ring, will be less than five.

Examples of five membered heteroaryl groups include but are not limited to pyrrole, furan, thiophene, imidazole, furazan, oxazole, oxadiazole, oxatriazole, isoxazole, thiazole, isothiazole, pyrazole, triazole and tetrazole groups.

Examples of six membered heteroaryl groups include but are not limited to pyridine, pyrazine, pyridazine, pyrimidine and triazine.

A bicyclic heteroaryl group may be, for example, a group selected from:
a) a benzene ring fused to a 5- or 6-membered ring containing 1, 2 or 3 ring heteroatoms;
b) a pyridine ring fused to a 5- or 6-membered ring containing 1, 2 or 3 ring heteroatoms;
c) a pyrimidine ring fused to a 5- or 6-membered ring containing 1 or 2 ring heteroatoms;
d) a pyrrole ring fused to a a 5- or 6-membered ring containing 1, 2 or 3 ring heteroatoms;
e) a pyrazole ring fused to a a 5- or 6-membered ring containing 1 or 2 ring heteroatoms;
f) an imidazole ring fused to a 5- or 6-membered ring containing 1 or 2 ring heteroatoms;
g) an oxazole ring fused to a 5- or 6-membered ring containing 1 or 2 ring heteroatoms;
h) an isoxazole ring fused to a 5- or 6-membered ring containing 1 or 2 ring heteroatoms;
i) a thiazole ring fused to a 5- or 6-membered ring containing 1 or 2 ring heteroatoms;
j) an isothiazole ring fused to a 5- or 6-membered ring containing 1 or 2 ring heteroatoms;
k) a thiophene ring fused to a 5- or 6-membered ring containing 1, 2 or 3 ring heteroatoms;
l) a furan ring fused to a 5- or 6-membered ring containing 1, 2 or 3 ring heteroatoms;
m) an oxazole ring fused to a 5- or 6-membered ring containing 1 or 2 ring heteroatoms;
n) an isoxazole ring fused to a 5- or 6-membered ring containing 1 or 2 ring heteroatoms;
o) a cyclohexyl ring fused to a 5- or 6-membered ring containing 1, 2 or 3 ring heteroatoms; and
p) a cyclopentyl ring fused to a 5- or 6-membered ring containing 1, 2 or 3 ring heteroatoms.

Particular examples of bicyclic heteroaryl groups containing a five membered ring fused to another five membered ring include but are not limited to imidazothiazole (e.g. imidazo[2,1-b]thiazole) and imidazoimidazole (e.g. imidazo[1,2-a]imidazole).

Particular examples of bicyclic heteroaryl groups containing a six membered ring fused to a five membered ring include but are not limited to benzfuran, benzthiophene, benzimidazole, benzoxazole, isobenzoxazole, benzisoxazole, benzthiazole, benzisothiazole, isobenzofuran, indole, isoindole, indolizine, indoline, isoindoline, purine (e.g., adenine, guanine), indazole, pyrazolopyrimidine (e.g. pyrazolo[1,5-a]pyrimidine), triazolopyrimidine (e.g. [1,2,4]triazolo[1,5-a]pyrimidine), benzodloxole and pyrazolopyridine (e.g. pyrazolo[1,5-a]pyridine) groups.

Particular examples of bicyclic heteroaryl groups containing two fused six membered rings include but are not limited to quinoline, isoquinoline, chroman, thlochroman, chromene, isochromene, chroman, isochroman, benzodioxan, quinolizine, benzoxazine, benzodiazine, pyridopyridine, quinoxaline, quinazoline, cinnoline, phthalazine, naphthyridine and pteridine groups.

One sub-group of heteroaryl groups comprises pyridyl, pyrrolyl, furanyl, thienyl, imidazolyl, oxazolyl, oxadiazolyl, oxatriazolyl, isoxazolyl, thiazolyl, isothiazolyl, pyrazolyl, pyrazinyl, pyridazinyl, pyrimidinyl, triazinyl, triazolyl, tetrazolyl, quinolinyl, isoquinolinyl, benzfuranyl, benzthienyl, chromanyl, thiochromanyl, benzimidazolyl, benzoxazolyl, benzisoxazole, benzthiazolyl and benzisothiazole, isobenzofuranyl, indolyl, isoindolyl, indolizinyl, indolinyl, isoindolinyl, purinyl (e.g., adenine, guanine), indazolyl, benzodioxolyl, chromenyl, isochromenyl, isochromanyl, benzodioxanyl, quinolizinyl, benzoxazinyl, benzodiazinyl, pyridopyridinyl, quinoxalinyl, quinazolinyl, cinnolinyl, phthalazinyl, naphthyridinyl and pteridinyl groups.

Examples of polycyclic aryl and heteroaryl groups containing an aromatic ring and a non-aromatic ring include tetrahydronaphthalene, tetrahydroisoquinoline, tetrahydroquinoline, dihydrobenzthiene, dihydrobenzfuran, 2,3-dihydro-benzo[1,4]dioxine, benzo[1,3]dioxole, 4,5,6,7-tetrahydrobenzofuran, indoline and indane groups.

Examples of carbocyclic aryl groups include phenyl, naphthyl, indenyl, and tetrahydronaphthyl groups.

Examples of non-aromatic heterocyclic groups include unsubstituted or substituted (by one or more groups R¹⁰) heterocyclic groups having from 3 to 12 ring members, typically 4 to 12 ring members, and more usually from 5 to 10 ring members. Such groups can be monocyclic or bicyclic, for example, and typically have from 1 to 5 heteroatom ring members (more usually 1,2,3 or 4 heteroatom ring members) typically selected from nitrogen, oxygen and sulphur.

When sulphur is present, it may, where the nature of the adjacent atoms and groups permits, exist as -S-, - S(O)- or -S(O)₂-.

The heterocylic groups can contain, for example, cyclic ether moieties (e.g. as in tetrahydrofuran and dioxane), cyclic thioether moieties (e.g. as in tetrahydrothiophene and dithiane), cyclic amine moieties (e.g. as in pyrrolidine), cyclic amide moieties (e.g. as in pyrrolidone), cyclic thioamides, cyclic thioesters, cyclic ester moieties (e.g. as in butyrolactone), cyclic sulphones (e.g. as in sulpholane and sulpholene), cyclic sulphoxides, cyclic sulphonamides and combinations thereof (e.g. morpholine and thiomorpholine and its S-oxide and S,S-dioxide). Further examples of heterocyclic groups are those containing a cyclic urea moiety (e.g. as in imidazolidin-2-one),

In one sub-set of heterocyclic groups, the heterocyclic groups contain cyclic ether moieties (e.g as in tetrahydrofuran and dioxane), cyclic thioether moieties (e.g. as in tetrahydrothiophene and dithiane), cyclic amine moieties (e.g. as in pyrrolidine), cyclic sulphones (e.g. as in sulpholane and sulpholene), cyclic sulphoxides, cyclic sulphonamides and combinations thereof (e.g. thiomorpholine).

Examples of monocyclic non-aromatic heterocyclic groups Include 5-, 6-and 7-membered monocyclic heterocyclic groups. Particular examples include morpholine, piperidine (e.g. 1-piperidlnyl, 2-piperidinyl, 3-piperidinyl and 4-piperidinyl), pyrrolidine (e.g. 1-pyrrolidinyl, 2-pyrrolidinyl and 3-pyrrolidinyl), pyrrolidone, pyran (2H-pyran or 4H-pyran), dihydrothiophene, dihydropyran, dihydrofuran, dihydrothiazole, tetrahydrofuran, tetrahydrothiophene, dioxane, tetrahydropyran (e.g. 4-tetrahydro pyranyl), imldazoline, imidazolidinone, oxazoline, thiazoline, 2-pyrazoline, pyrazolidine, piperazine, and N-alkyl piperazines such as N-methyl piperazine. Further examples include thiomorpholine and its S-oxide and S,S-dioxide (particularly thiomorpholine). Still further examples Include azetidine, piperidone, piperazone, and N-alkyl piperidines such as N-methyl piperidine.

One preferred sub-set of non-aromatic heterocyclic groups consists of saturated groups such as azetidine, pyrrolidine, piperidine, morpholine, thiomorpholine, thiomorpholine S,S-dioxide, piperazine, N-alkyl piperazines, and N-alkyl piperidines.

Another sub-set of non-aromatic heterocyclic groups consists of pyrrolidine, piperidine, morpholine, thiomorpholine, thiomorpholine S,S-dioxide, piperazine and N-alkyl piperazines such as N-methyl piperazine.

One particular sub-set of heterocyclic groups consists of pyrrolidine, piperidine, morpholine and N-alkyl piperazines (e.g. N-methyl piperazine), and optionally thiomorpholine.

Examples of non-aromatic carbocyclic groups include cycloalkane groups such as cyclohexyl and cyclopentyl, cycloalkenyl groups such as cyclopentenyl, cyclohexenyl, cycloheptenyl and cyclooctenyl, as well as cyclohexadienyl, cyclooctatetraene, tetrahydronaphthenyl and decalinyl.

Preferred non-aromatic carbocyclic groups are monocyclic rings and most preferably saturated monocyclic rings.

Typical examples are three, four, five and six membered saturated carbocyclic rings, e.g. optionally substituted cyclopentyl and cyclohexyl rings.

One sub-set of non-aromatic carboyclic groups includes unsubstituted or substituted (by one or more groups R¹⁰) monocyclic groups and particularly saturated monocyclic groups, e.g. cycloalkyl groups. Examples of such cycloalkyl groups include cyclopropyl, cyclobutyl, cyclopentyl, cyclohexyl and cycloheptyl; more typically cyclopropyl, cyclobutyl, cyclopentyl and cyclohexyl, particularly cyclohexyl.

Further examples of non-aromatic cyclic groups include bridged ring systems such as bicycloalkanes and azabicycloalkanes although such bridged ring systems are generally less preferred. By "bridged ring systems" is meant ring systems in which two rings share more than two atoms, see for example Advanced Organic Chemistry, by Jerry March, 4th Edition, Wiley Interscience, pages 131-133, 1992. Examples of bridged ring systems include bicyclo[2.2.1]heptane, aza-bicyclo[2.2.1]heptane, bicyclo[2.2.2]octane, aza-bicyclo[2.2.2]octane, bicyclo[3.2.1]octane and aza-bicyclo[3.2.1]octane. A particular example of a bridged ring system Is the 1-aza-bicyclo[2.2.2]octan-3-yl group.

Where reference is made herein to carbocyclic and heterocyclic groups, the carbocyclic or heterocyclic ring is, where stated, and unless the context Indicates otherwise, unsubstituted or substituted by one or more substituent groups R¹⁰ selected from halogen, hydroxy, trifluoromethyl, cyano, nitro, carboxy, amino, mono- or di-C₁₋₄ hydrocarbylamino, carbocyclic and heterocyclic groups having from 3 to 12 ring members; a group R^{a}-R^{b} wherein R^{a} is a bond, O, CO, X¹C(X²), C(X²)X¹, X¹C(X²)X¹, S, SO, SO₂, NR^{c}, SO₂NR^{c} or NR^{c}SO₂; and R^{b} is selected from hydrogen, carbocyclic and heterocyclic groups having from 3 to 12 ring members, and a C₁₋₈ hydrocarbyl group optionally substituted by one or more substituents selected from hydroxy, oxo, halogen, cyano, nitro, carboxy, amino, mono- or di-C₁₋₄ hydrocarbylamino, carbocyclic and heterocyclic groups having from 3 to 12 ring members;
R^{c} is selected from hydrogen and C₁₋₄ hydrocarbyl; and
X¹ is O, S or NR^{c} and X² is =O, =S or =NR^{c}; provided that where the substituent group R¹⁰ comprises or includes a carbocyclic or heterocyclic group, the said carbocyclic or heterocyclic group may be unsubstituted or may itself be substituted with one or more further substituent groups R¹⁰ and wherein (a) such further substituent groups R¹⁰ carbocyclic or heterocyclic groups, which are not themselves further substituted; or (b) the said further substituents do not include carbocyclic or heterocyclic groups but are otherwise selected from the groups listed above in the definition of R¹⁰.

The substituents R¹⁰ may be selected such that they contain no more than 20 non-hydrogen atoms, for example, no more than 15 non-hydrogen atoms, e.g. no more than 12, or 11, or 10, or 9, or 8, or 7, or 6, or 5 non-hydrogen atoms.

Examples of halogen substituents include fluorine, chlorine, bromine and iodine. Fluorine and chlorine are particularly preferred.

In the definition of the compounds of the formula (II) above and as used hereinafter, the term "hydrocarbyl" is a generic term encompassing aliphatic, alicyclic and aromatic groups having an all-carbon backbone and consisting of carbon and hydrogen atoms, except where otherwise stated.

In certain cases, as defined herein, one or more of the carbon atoms making up the carbon backbone may be replaced by a specified atom or group of atoms.

Examples of hydrocarbyl groups include alkyl, cycloalkyl, cycloalkenyl, carbocyclic aryl, alkenyl, alkynyl, cycloalkylalkyl, cycloalkenylalkyl, and carbocyclic aralkyl, aralkenyl and aralkynyl groups. Such groups can be unsubstituted or, where stated, substituted by one or more substituents as defined herein. The examples and preferences expressed below apply to each of the hydrocarbyl substituent groups or hydrocarbyl-containing substituent groups referred to in the various definitions of substituents for compounds of the formula (II) unless the context indicates otherwise.

Preferred non-aromatic hydrocarbyl groups are saturated groups such as alkyl and cycloalkyl groups.

Generally by way of example, the hydrocarbyl groups can have up to eight carbon atoms, unless the context requires otherwise. Within the sub-set of hydrocarbyl groups having 1 to 8 carbon atoms, particular examples are C₁₋₆ hydrocarbyl groups, such as C₁₋₄ hydrocarbyl groups (e.g. C₁₋₃ hydrocarbyl groups or C₁₋₂ hydrocarbyl groups), specific examples being any individual value or combination of values selected from C₁, C₂, C₃, C₄, C₅, C₆, C₇ and C₈ hydrocarbyl groups.

The term "alkyl" covers both straight chain and branched chain alkyl groups. Examples of alkyl groups include methyl, ethyl, propyl, isopropyl, n-butyl, isobutyl, tert-butyl, n-pentyl, 2-pentyl, 3-pentyl, 2-methyl butyl, 3-methyl butyl, and n-hexyl and its isomers. Within the sub-set of alkyl groups having 1 to 8 carbon atoms, particular examples are C₁₋₆ alkyl groups, such as C₁₋₄ alkyl groups (e.g. C₁₋₃ alkyl groups or C₁₋₂ alkyl groups).

Examples of cycloalkyl groups are those derived from cyclopropane, cyclobutane, cyclopentane, cyclohexane and cycloheptane. Within the sub-set of cycloalkyl groups the cycloalkyl group will have from 3 to 8 carbon atoms, particular examples being C₃₋₆ cycloalkyl groups.

Examples of alkenyl groups include, but are not limited to, ethenyl (vinyl), 1-propenyl, 2-propenyl (allyl), isopropenyl, butenyl, buta-1,4-dienyl, pentenyl, and hexenyl. Within the sub-set of alkenyl groups the alkenyl group will have 2 to 8 carbon atoms, particular examples being C₂₋₆ alkenyl groups, such as C₂₋₄ alkenyl groups.

Examples of cycloalkenyl groups include, but are not limited to, cyclopropenyl, cyclobutenyl, cyclopentenyl, cyclopentadienyl and cyclohexenyl. Within the sub-set of cycloalkenyl groups the cycloalkenyl groups have from 3 to 8 carbon atoms, and particular examples are C₃₋₆ cycloalkenyl groups.

Examples of alkynyl groups include, but are not limited to, ethynyl and 2-propynyl (propargyl) groups. Within the sub-set of alkynyl groups having 2 to 8 carbon atoms, particular examples are C₂₋₆ alkynyl groups, such as C₂₋₄ alkynyl groups.

Examples of carbocyclic aryl groups include substituted and unsubstituted phenyl groups.

Examples of cycloalkylalkyl, cycloalkenylalkyl, carbocyclic aralkyl, aralkenyl and aralkynyl groups include phenethyl, benzyl, styryl, phenylethynyl, cyclohexylmethyl, cyclopentylmethyl, cyclobutylmethyl, cyclopropylmethyl and cyclopentenylmethyl groups.

When present, and where stated, a hydrocarbyl group can be optionally substituted by one or more substituents selected from hydroxy, oxo, alkoxy, carboxy, halogen, cyano, nitro, amino, mono- or di-C₁₋₄ hydrocarbylamino, and monocyclic or bicyclic carbocyclic and heterocyclic groups having from 3 to 12 (typically 3 to 10 and more usually 5 to 10) ring members. Preferred substituents include halogen such as fluorine. Thus, for example, the substituted hydrocarbyl group can be a partially fluorinated or perfluorinated group such as difluoromethyl or trifluoromethyl. In one embodiment preferred substituents include monocyclic carbocyclic and heterocyclic groups having 3-7 ring members, more usually 3, 4, 5 or 6 ring members.

Where stated, one or more carbon atoms of a hydrocarbyl group may optionally be replaced by O, S, SO, SO₂, NR^{c}, X¹C(X²), C(X²)X¹ or X¹C(X²)X¹ (or a sub-group thereof) wherein X¹ and X² are as hereinbefore defined, provided that at least one carbon atom of the hydrocarbyl group remains. For example, 1, 2, 3 or 4 carbon atoms of the hydrocarbyl group may be replaced by one of the atoms or groups listed, and the replacing atoms or groups may be the same or different. In general, the number of linear or backbone carbon atoms replaced will correspond to the number of linear or backbone atoms in the group replacing them. Examples of groups in which one or more carbon atom of the hydrocarbyl group have been replaced by a replacement atom or group as defined above include ethers and thioethers (C replaced by O or S), amides, esters, thioamides and thioesters (C-C replaced by X¹C(X²) or C(X²)X¹), sulphones and sulphoxides (C replaced by SO or SO₂), amines (C replaced by NR^{c}). Further examples include ureas, carbonates and carbamates (C-C-C replaced by X¹C(X²)X¹).

Where an amino group has two hydrocarbyl substituents, they may, together with the nitrogen atom to which they are attached, and optionally with another heteroatom such as nitrogen, sulphur, or oxygen, link to form a ring structure of 4 to 7 ring members, more usually 5 to 6 ring members.

The term "aza-cycloalkyl" as used herein refers to a cycloalkyl group in which one of the carbon ring members has been replaced by a nitrogen atom. Thus examples of aza-cycloalkyl groups include piperidine and pyrrolidine. The term "oxa-cycloalkyl" as used herein refers to a cycloalkyl group in which one of the carbon ring members has been replaced by an oxygen atom. Thus examples of oxa-cycloalkyl groups include tetrahydrofuran and tetrahydropyran. In an analogous manner, the terms "diaza-cycloalkyl", "dioxa-cycloalkyl" and "aza-oxa-cycloalkyl" refer respectively to cycloalkyl groups in which two carbon ring members have been replaced by two nitrogen atoms, or by two oxygen atoms, or by one nitrogen atom and one oxygen atom.

The definition "R^{a}-R^{b}" as used herein, either with regard to substituents present on a carbocyclic or heterocyclic moiety, or with regard to other substituents present at other locations on the compounds of the formula (II), includes inter alia compounds wherein R^{a} is selected from a bond, O, CO, OC(O), SC(O), NR^{c}C(O), OC(S), SC(S), NR^{c}C(S), OC(NR^{c}), SC(NR^{c}), NR^{c}C(NR^{c}), C(O)O, C(O)S, C(O)NR^{c}, C(S)O, C(S)S, C(S) NR^{c}, C(NR^{c})O, C(NR^{c})S, C(NR^{c})NR^{c}, OC(O)O, SC(O)O, NR^{c}C(O)O, OC(S)O, SC(S)O, NR-C(S)O, OC(NR^{c})O, SC(NR^{c})O, NR^{c}C(NR^{c})O, OC(O)S, SC(O)S, NF^{c}C(O)S, OC(S)S, SC(S)S, NR^{c}C(S)S, OC(NR^{c})S, SC(NR^{c})S, NR^{c}C(NR^{c})S, OC(O)NR^{c}, SC(O)NR^{c}, NR^{c}C(O) NR^{c}, OC(S)NR^{c}, SC(S) NR^{c}, NR^{c}C(S)NR^{c}, OC(NR^{c})NR^{c}, SC(NR^{c})NR^{c}, NRC^{c}C(NR^{c}NR^{c}, S, SO, SO₂, NR^{c}, SO₂NR^{c} and NRSO₂ wherein R^{c} is as hereinbefore defined.

The moiety R^{b} can be hydrogen or it can be a group selected from carbocyclic and heterocyclic groups having from 3 to 12 ring members (typically 3 to 10 and more usually from 5 to 10), and a C₁₋₈ hydrocarbyl group optionally substituted as hereinbefore defined. Examples of hydrocarbyl, carbocyclic and heterocyclic groups are as set out above.

When R^{a} is O and R^{b} is a C₁₋₆ hydrocarbyl group, R^{a} and R^{b} together form a hydrocarbyloxy group. Preferred hydrocarbyloxy groups include saturated hydrocarbyloxy such as alkoxy (e.g. C₁₋₆ alkoxy, more usually C₁₋₄ alkoxy such as ethoxy and methoxy, particularly methoxy), cycloalkoxy (e.g. C₃₋₆ cycloalkoxy such as cyclopropyloxy, cyclobutyloxy, cyclopentyloxy and cyclohexyloxy) and cycloalkyalkoxy (e.g. C₃₋₆ cycloalkyl-C₁₋₂ alkoxy such as cyclopropylmethoxy).

The hydrocarbyloxy groups can be substituted by various substituents as defined herein. For example, the alkoxy groups can be substituted by halogen (e.g. as in difluoromethoxy and trifluoromethoxy), hydroxy (e.g. as in hydroxyethoxy), C₁₋₂ alkoxy (e.g. as in methoxyethoxy), hydroxy-C₁₋₂ alkyl (as in hydroxyethoxyethoxy) or a cyclic group (e.g. a cycloalkyl group or non-aromatic heterocyclic group as hereinbefore defined). Examples of alkoxy groups bearing a non-aromatic heterocyclic group as a substituent are those in which the heterocyclic group is a saturated cyclic amine such as morpholine, piperidine, pyrrolidine, piperazine, C₁₋₄-alkyl-piperazines, C₃₋₇-cycloalkyl-piperazines, tetrahydropyran or tetrahydrofuran and the alkoxy group is a C₁₋₄ alkoxy group, more typically a C₁₋₃ alkoxy group such as methoxy, ethoxy or n-propoxy.

Alkoxy groups substituted by a monocyclic group such as pyrrolidine, piperidine, morpholine and piperazine and N-substituted derivatives thereof such as N-benzyl, N-C₁₋₄ acyl and N-C₁₋₄ alkoxycarbonyl. Particular examples include pyrrolidinoethoxy, piperidinoethoxy and piperazinoethoxy.

When R^{a} is a bond and R^{b} is a C₁₋₈ hydrocarbyl group, examples of hydrocarbyl groups R^{a}-R^{b} are as hereinbefore defined. The hydrocarbyl groups may be saturated groups such as cycloalkyl and alkyl and particular examples of such groups include methyl, ethyl and cyclopropyl. The hydrocarbyl (e.g. alkyl) groups can be substituted by various groups and atoms as defined herein. Examples of substituted alkyl groups include alkyl groups substituted by one or more halogen atoms such as fluorine and chlorine (particular examples including bromoethyl, chloroethyl and trifluoromethyl), or hydroxy (e.g. hydroxymethyl and hydroxyethyl), C₁₋₈ acyloxy (e.g. acetoxymethyl and benzyloxymethyl), amino and mono- and dialkylamino (e.g. aminoethyl, methylaminoethyl, dimethylaminomethyl, dimethylaminoethyl and tert-butylaminomethyl), alkoxy (e.g. C₁₋₂ alkoxy such as methoxy - as in methoxyethyl), and cyclic groups such as cycloalkyl groups, aryl groups, heteroaryl groups and non-aromatic heterocyclic groups as hereinbefore defined).

Particular examples of alkyl groups substituted by a cyclic group are those wherein the cyclic group is a saturated cyclic amine such as morpholine, piperidine, pyrrolidine, piperazine, C₁₋₄-alkyl-piperazines, C₃₋₇-cycloalkyl-piperazines, tetrahydropyran or tetrahydrofuran and the alkyl group is a C₁₋₄ alkyl group, more typically a C₁₋₃ alkyl group such as methyl, ethyl or n-propyl. Specific examples of alkyl groups substituted by a cyclic group include pyrrolidinomethyl, pyrrolidinopropyl, morpholinomethyl, morpholinoethyl, morpholinopropyl, piperidinylmethyl, piperazinomethyl and N-substituted forms thereof as defined herein.

Particular examples of alkyl groups substituted by aryl groups and heteroaryl groups include benzyl and pyridylmethyl groups.

When R^{a} is SO₂NR^{c}, R^{b} can be, for example, hydrogen or an optionally substituted C₁₋₈ hydrocarbyl group, or a carbocyclic or heterocyclic group. Examples of R^{a}-R^{b} where R^{a} is SO₂NR^{c} include aminosulphonyl, C₁₋₄ alkylaminosulphonyl and di-C₁₋₄ alkylaminosulphonyl groups, and sulphonamides formed from a cyclic amino group such as piperidine, morpholine, pyrrolidine, or an optionally N-substituted piperazine such as N-methyl piperazine.

Examples of groups R^{a}-R^{b} where R^{a} is SO₂ include alkylsulphonyl, heteroarylsulphonyl and arylsulphonyl groups, particularly monocyclic aryl and heteroaryl sulphonyl groups. Particular examples include methylsulphonyl, phenylsulphonyl and toluenesulphonyl.

When R^{a} is NR^{c}, R^{b} can be, for example, hydrogen or an optionally substituted C₁₋₈ hydrocarbyl group, or a carbocyclic or heterocyclic group. Examples of R^{a}-R^{b} where R^{a} is NR^{c} include amino, C₁₋₄ alkylamino (e.g. methylamino, ethylamino, propylamino, isopropylamino, tert-butylamino), di-C₁₋₄ alkylamino (e.g. dimethylamino and diethylamino) and cycloalkylamino (e.g. cyclopropylamino, cyclopentylamlno and cyclohexylamino).

### Specific Embodiments of and Preferences for the Moieties Y, R^{g}, R¹ to R³ and R¹⁰

### R²

R² is hydrogen or methyl, most preferably hydrogen. *

### R¹

R¹ is a carbocyclic or heterocyclic group having from 3 to 12 ring members and being optionally substituted by one or more substituent groups R¹⁰ as defined herein, or a C₁₋₈ hydrocarbyl group optionally substituted by one or more substituents selected from halogen (e.g. fluorine), hydroxy, C₁₋₄ hydrocarbyloxy, amino, mono- or di-C₁₋₄ hydrocarbylamino, and carbocyclic or heterocyclic groups having from 3 to 12 ring members. Examples of carbocyclic or heterocyclic groups and hydrocarbyl groups and general preferences for such groups are as set out above in the General Preferences and Definitions section, and as set out below.

In one embodiment, R¹ is an aryl or heteroaryl group.

When R¹ is a heteroaryl group, particular heteroaryl groups include monocyclic heteroaryl groups containing up to three heteroatom ring members selected from O, S and N, and bicyclic heteroaryl groups containing up to 2 heteroatom ring members selected from O, S and N and wherein both rings are aromatic.

Examples of such groups include furanyl (e.g. 2-furanyl or 3-furanyl), indolyl (e.g. 3-indolyl, 6-indolyl), 2,3-dihydro-benzo[1,4]dioxinyl(e.g. 2,3-dihydro-benzo[1,4]dioxin-5-yl), pyrazolyl (e.g. pyrazole-5-yl), pyrazolo[1,5-a]pyridinyl (e.g. pyrazolo[1,5-a]pyridine-3-yl), oxazolyl (e.g.), isoxazolyl (e.g. isoxazol-4-yl), pyridyl (e.g. 2-pyridyl, 3-pyridyl, 4-pyridyl), quinolinyl (e.g. 2-quinolinyl), pyrrolyl (e.g. 3-pyrrolyl), imidazolyl and thienyl (e.g. 2-thienyl, 3-thienyl).

One sub-group of heteroaryl groups R¹ consists of furanyl (e.g. 2-furanyl or 3-furanyl), indolyl, oxazolyl, isoxazolyl, pyridyl, quinolinyl, pyrrolyl, imidazolyl and thienyl.

A preferred sub-set of R¹ heteroaryl groups includes 2-furanyl, 3-furanyl, pyrrolyl, imidazolyl and thienyl.

Preferred aryl groups R¹ are phenyl groups.

The group R¹ can be an unsubstituted or substituted carbocylic or heterocyclic group in which one or more substituents can be selected from the group R¹⁰ as hereinbefore defined. In one embodiment, the substituents on R¹ may be selected from the group R^{10a} consisting of halogen, hydroxy, trifluoromethyl, cyano, nitro, carboxy, a group R^{a}-R^{b} wherein R^{a} is a bond, O, CO, X³C(X⁴), C(X⁴)X³, X³C(X⁴)X³, S, SO, or SO₂, and R^{b} is selected from hydrogen and a C₁₋₈ hydrocarbyl group optionally substituted by one or more substituents selected from hydroxy, oxo, halogen, cyano, nitro, carboxy and monocyclic non-aromatic carbocyclic or heterocyclic groups having from 3 to 6 ring members; wherein one or more carbon atoms of the C₁₋₈ hydrocarbyl group may optionally be replaced by O, S, SO, SO₂, X³C(X⁴), C(X⁴)X³ or X³C(X⁴)X³; X³ is O or S; and X⁴ is =O or =S.

Where the carbocyclic and heterocyclic groups have a pair of substituents on adjacent ring atoms, the two substituents may be linked so as to form a cyclic group. Thus, two adjacent groups R¹⁰, together with the carbon atoms or heteroatoms to which they are attached may form a 5-membered heteroaryl ring or a 5- or 6-membered non-aromatic carbocyclic or heterocyclic ring, wherein the said heteroaryl and heterocyclic groups contain up to 3 heteroatom ring members selected from N, O and S. In particular the two adjacent groups R¹⁰, together with the carbon atoms or heteroatoms to which they are attached, may form a 6-membered non-aromatic heterocyclic ring, containing up to 3, in particular 2, heteroatom ring members selected from N, O and S. More particularly the two adjacent groups R¹⁰ may form a 6-membered non-aromatic heterocyclic ring, containing 2 heteroatom ring members selected from N, or O, such as dioxan e.g. [1,4 dioxan]. In one embodiment R¹ is a carbocyclic group e.g. phenyl having a pair of substituents on adjacent ring atoms linked so as to form a cyclic group e.g. to form 2,3-dihydro-benzo[1,4]dioxine.

More particularly, the substituents on R¹ may be selected from halogen, hydroxy, trifluoromethyl, a group R^{a}-R^{b} wherein R^{a} is a bond or O, and R^{b} is selected from hydrogen and a C₁₋₄ hydrocarbyl group optionally substituted by one or more substituents selected from hydroxyl, halogen (preferably fluorine) and 5 and 6 membered saturated carbocyclic and heterocyclic groups (for example groups containing up to two heteroatoms selected from O, S and N, such as unsubstituted piperidine, pyrrolidino, morpholino, piperazino and N-methyl piperazino).

The group R¹ may be substituted by more than one substituent. Thus, for example, there may be 1 or 2 or 3 or 4 substituents. In one embodiment, where R¹ is a six membered ring (e.g. a carbocyclic ring such as a phenyl ring), there may be one, two or three substituents and these may be located at the 2-, 3-, 4- or 6-positions around the ring. By way of example, a phenyl group R¹ may be 2-monosubstituted, 3-monosubstituted, 2,6-disubstituted, 2,3-disubstituted, 2,4-disubstituted 2,5-disubstituted, 2,3,6-trisubstituted or 2,4,6-trisubstituted. More particularly, a phenyl group R¹ may be monosubstituted at the 2-position or disubstituted at positions 2-and 6- with substituents selected from fluorine, chlorine and R^{a}-R^{b}, where R^{a} is O and R^{b} is C₁₋₄ alkyl (e.g. methyl or ethyl). In one embodiment, fluorine is a preferred substituent. In another embodiment, preferred substituents are selected from fluorine, chlorine and methoxy.

Particular examples of non-aromatic groups R¹ include unsubstituted or substituted (by one or more groups R¹⁰) monocyclic cycloalkyl groups. Examples of such cycloalkyl groups include cyclopropyl, cyclobutyl, cyclopentyl, cyclohexyl and cycloheptyl; more typically cyclopropyl, cyclobutyl, cyclopentyl and cyclohexyl, particularly cyclohexyl.

Further examples of non-aromatic groups R¹ include unsubstituted or substituted (by one or more groups R¹⁰) heterocyclic groups having from 3 to 12 ring members, typically 4 to 12 ring members, and more usually from 5 to 10 ring members. Such groups can be monocyclic or bicyclic, for example, and typically have from 1 to 5 heteroatom ring members (more usually 1,2,3 or 4 heteroatom ring members) typically selected from nitrogen , oxygen and sulphur.

When sulphur Is present, it may, where the nature of the adjacent atoms and groups permits, exist as -S-,-S(O)- or -S(O)₂-.

The heterocylic groups can contain, for example, cyclic ether moieties (e.g as in tetrahydrofuran and dioxane), cyclic thioether moieties (e.g. as in tetrahydrothiophene and dithiane), cyclic amine moieties (e.g. as in pyrrolidine), cyclic amides (e.g. as in pyrrolidone), cyclic esters (e.g. as in butyrolactone), cyclic thioamides and thloesters, cyclic sulphones (e.g. as in sulpholane and sulpholene), cyclic sulphoxides, cyclic sulphonamides and combinations thereof (e.g. morpholine and thiomorpholine and its S-oxide and S,S-dioxide).

In one sub-set of heterocyclic groups R¹, the heterocyclic groups contain cyclic ether moieties (e.g as in tetrahydrofuran and dioxane), cyclic thioether moieties (e.g. as in tetrahydrothiophene and dithiane), cyclic amine moieties (e.g. as in pyrrolidine), cyclic sulphones (e.g. as in sulpholane and sulpholene), cyclic sulphoxides, cyclic sulphonamides and combinations thereof (e.g. thiomorpholine).

Examples of monocyclic non-aromatic heterocyclic groups R¹ include 5-, 6-and 7-membered monocyclic heterocyclic groups such as morpholine, piperidine (e.g. 1-piperidinyl, 2-piperidinyl 3-piperidinyl and 4-piperidinyl), pyrrolidine (e.g. 1-pyrrolidinyl, 2-pyrrolidinyl and 3-pyrrolidinyl), pyrrolidone, pyran (2H-pyran or 4H-pyran), dihydrothiophene, dihydropyran, dihydrofuran, dihydrothiazole, tetrahydrofuran, tetrahydrothiophene, dioxane, tetrahydropyran (e.g. 4-tetrahydro pyranyl), imidazoline, imidazolidinone, oxazoline, thiazoline, 2-pyrazoline, pyrazolidine, piperazine, and N-alkyl piperazines such as N-methyl piperazine. Further examples include thiomorpholine and its S-oxide and S,S-dioxide (particularly thiomorpholine). Still further examples include N-alkyl piperidines such as N-methyl piperidine.

One sub-group of non-aromatic heterocyclic groups R¹ includes unsubstituted or substituted (by one or more groups R¹⁰) 5-, 6-and 7-membered monocyclic heterocyclic groups such as morpholine, piperidine (e.g. 1-piperidinyl, 2-piperidinyl 3-piperidinyl and 4-piperidinyl), pyrrolidine (e.g. 1-pyrrolidinyl, 2-pyrrolidinyl and 3-pyrrolidinyl), pyrrolidone, piperazine, and N-alkyl piperazines such as N-methyl piperazine, wherein a particular sub-set consists of pyrrolidine, piperidine, morpholine, thiomorpholine and N-methyl piperazine.

In general, preferred non-aromatic heterocyclic groups include pyrrolidine, piperidine, morpholine, thiomorpholine, thiomorpholine S,S-dioxide, piperazine, N-alkyl piperazines, and N-alkyl piperidines.

Another particular sub-set of heterocyclic groups consists of pyrrolidine, piperidine, morpholine and N-alkyl piperazines, and optionally, N-methyl piperazine and thiomorpholine.

When R¹ is a C₁₋₈ hydrocarbyl group substituted by a carbocyclic or heterocyclic group, the carbocyclic and heterocyclic groups can be aromatic or non-aromatic and can be selected from the examples of such groups set out hereinabove. The substituted hydrocarbyl group is typically a saturated C₁₋₄ hydrocarbyl group such as an alkyl group, preferably a CH₂ or CH₂CH₂ group. Where the substituted hydrocarbyl group is a C₂₋₄ hydrocarbyl group, one of the carbon atoms and its associated hydrogen atoms may be replaced by a sulphonyl group, for example as in the moiety SO₂CH₂.

When the carbocyclic or heterocylic group attached to the a C₁₋₈ hydrocarbyl group is aromatic, examples of such groups include monocyclic aryl groups and monocyclic heteroaryl groups containing up to four heteroatom ring members selected from O, S and N, and bicyclic heteroaryl groups containing up to 2 heteroatom ring members selected from O, S and N and wherein both rings are aromatic.

Examples of such groups are set out in the "General Preferences and Definitions" section above.

Particular examples of such groups include furanyl (e.g. 2-furanyl or 3-furanyl), indolyl, oxazolyl, isoxazolyl, pyridyl, quinolinyl, pyrrolyl, imidazolyl and thienyl. Particular examples of aryl and heteroaryl groups as substituents for a C₁₋₈ hydrocarbyl group include phenyl, imidazolyl, tetrazolyl, triazolyl, indolyl, 2-furanyl, 3-furanyl, pyrcolyl and thienyl. Such groups may be substituted by one or more substituents R¹⁰ or R^{10a} as defined herein.

When R¹ is a C₁₋₈ hydrocarbyl group substituted by a non-aromatic carbocyclic or heterocyclic group, the non-aromatic or heterocyclic group may be a group selected from the lists of such groups set out hereinabove. For example, the non-aromatic group can be a monocyclic group having from 4 to 7 ring members, e.g. 5 to 7 ring members, and typically containing from 0 to 3, more typically 0, 1 or 2, heteroatom ring members selected from O, S and N. When the cyclic group is a carbocyclic group, it may additionally be selected from monocyclic groups having 3 ring members. Particular examples Include monocyclic cycloalkyl groups such as cyclopropyl, cyclobutyl, cyclopentyl, cyclohexyl and cycloheptyl, and 5-, 6-and 7-membered monocyclic heterocyclic groups such as morpholine, piperidine (e.g. 1-piperidinyl, 2-piperidinyl, 3-piperidinyl and 4-piperidinyl), pyrrolidine (e.g. 1-pyrrolidinyl, 2-pyrrolidinyl and 3-pyrrolidinyl), pyrrolidone, piperazine, and N-alkyl piperazines such as N-methyl piperazine. In general, preferred non-aromatic heterocyclic groups include pyrrolidine, piperidine, morpholine, thiomorpholine and N-methyl piperazine.

When R¹ is an optionally substituted C₁₋₈ hydrocarbyl group, the hydrocarbyl group may be as hereinbefore defined, and is preferably up to four carbon atoms in length, more usually up to three carbon atoms in length for example one or two carbon atoms in length.

In one embodiment, the hydrocarbyl group is saturated and may be acyclic or cyclic, for example acyclic. An acyclic saturated hydrocarbyl group (i.e. an alkyl group) may be a straight chain or branched alkyl group.

Examples of straight chain alkyl groups R¹ include methyl, ethyl, propyl and butyl.

Examples of branched chain alkyl groups R¹ include isopropyl, isobutyl, tert-butyl and 2,2-dimethylpropyl.

In one embodiment, the hydrocarbyl group is a linear saturated group having from 1-6 carbon atoms, more usually 1-4 carbon atoms, for example 1-3 carbon atoms, e.g. 1, 2 or 3 carbon atoms. When the hydrocarbyl group is substituted, particular examples of such groups are substituted (e.g. by a carbocyclic or heterocyclic group) methyl and ethyl groups.

A C₁₋₈ hydrocarbyl group R¹ can be optionally substituted by one or more substituents selected from halogen (e.g. fluorine), hydroxy, C₁₋₄ hydrocarbyloxy, amino, mono- or di-C₁₋₄ hydrocarbylamino, and carbocyclic or heterocyclic groups having from 3 to 12 ring members. Particular substituents for the hydrocarbyl group include hydroxy, chlorine, fluorine (e.g. as in trifluoromethyl), methoxy, ethoxy, amino, methylamino and dimethylamino, preferred substituents being hydroxy and fluorine.

Particular groups R¹-CO are the groups set out in Table 1 below.

In Table 1, the point of attachment of the group to the nitrogen atom of the pyrazole-4-amino group is represented by the terminal single bond extending from the carbonyl group. Thus, by way of illustration, group B in the table is the trifluoroacetyl group, group D in the table is the phenylacetyl group and group I in the table is the 3-(4-chlorophenyl)propionyl group.

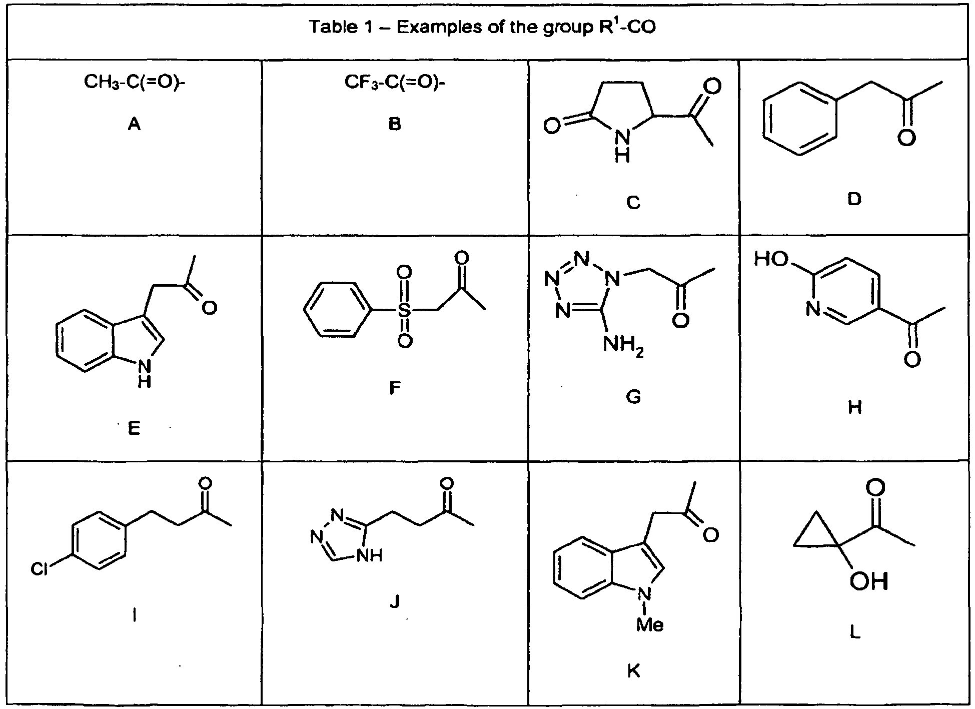

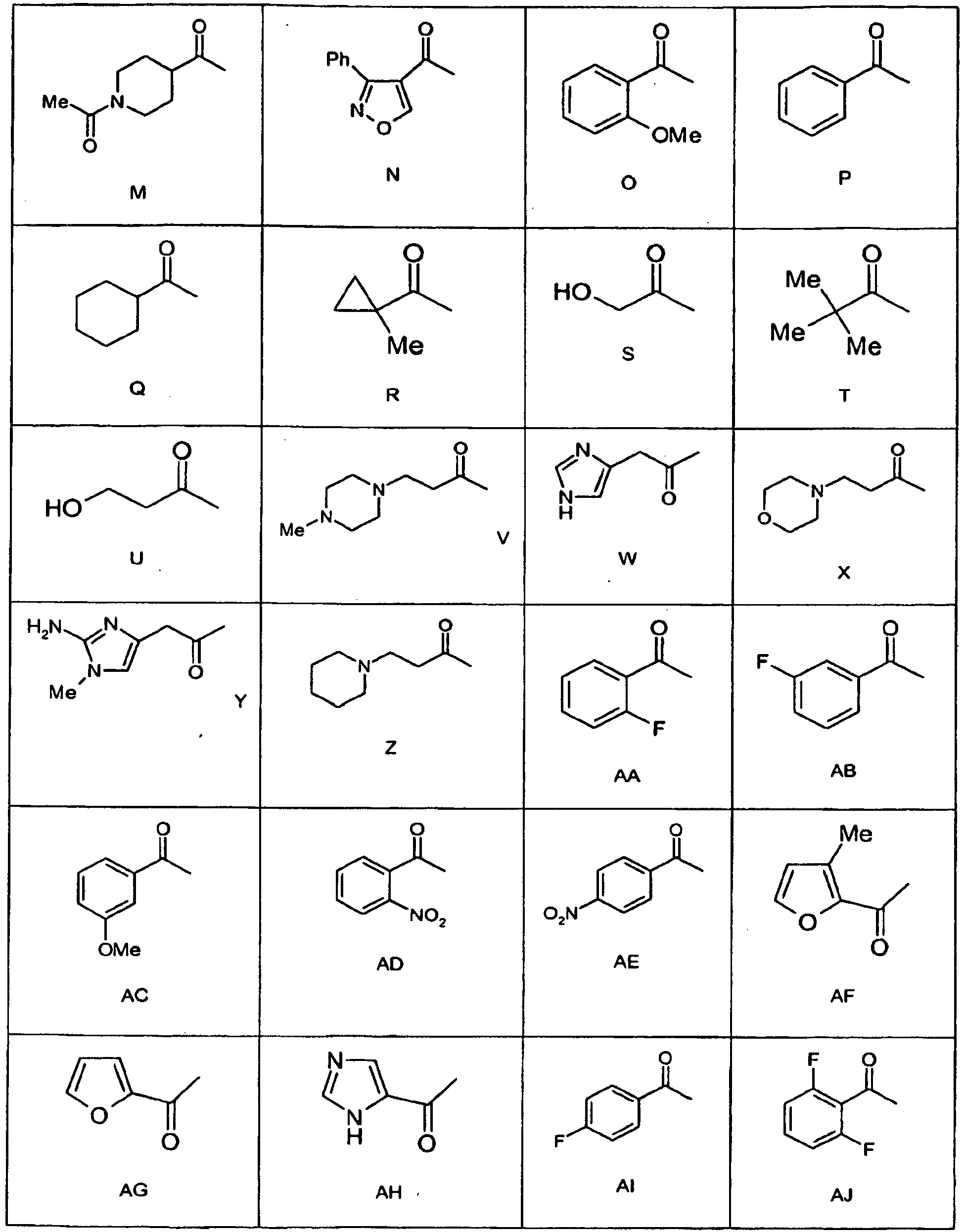

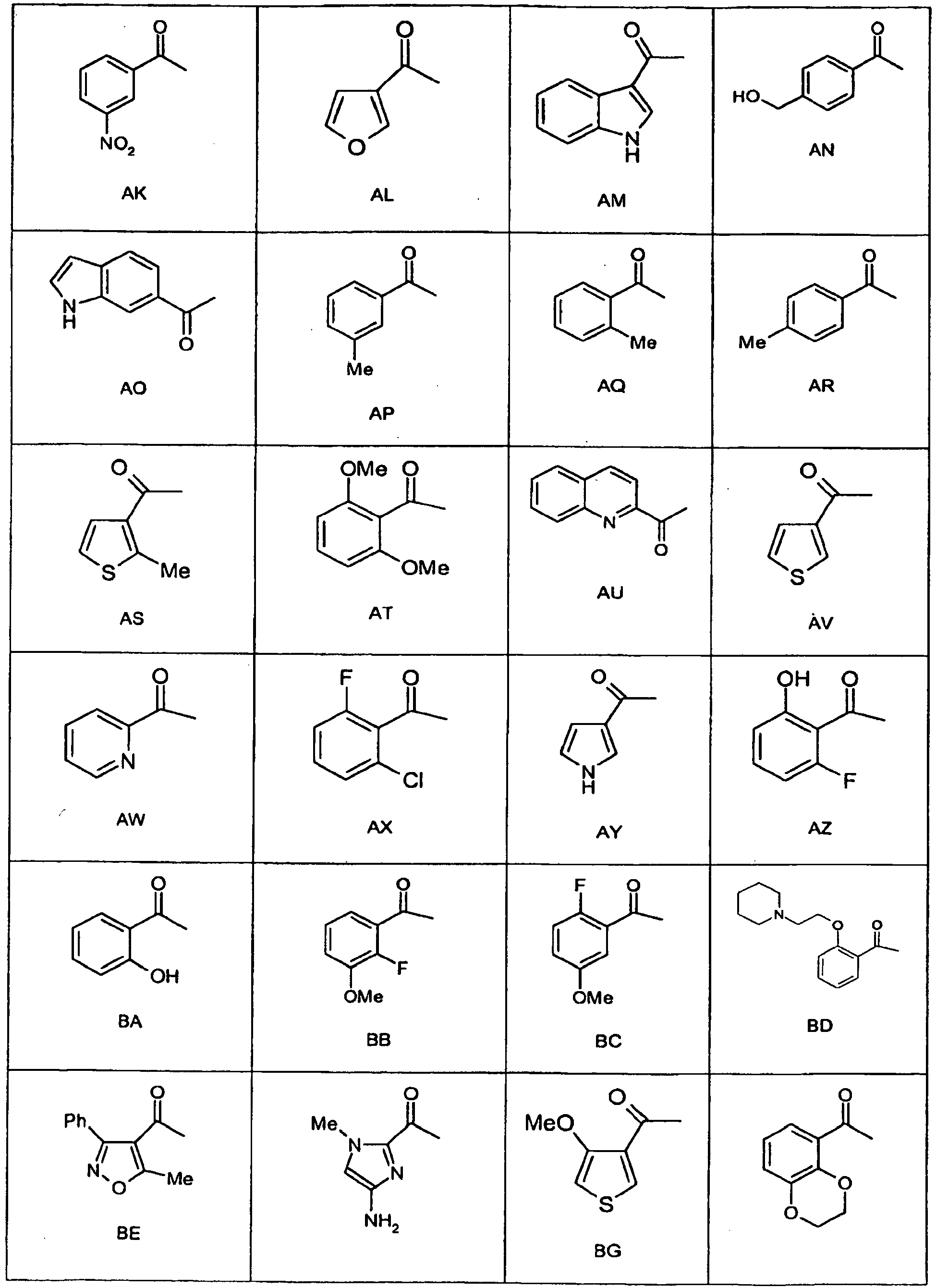

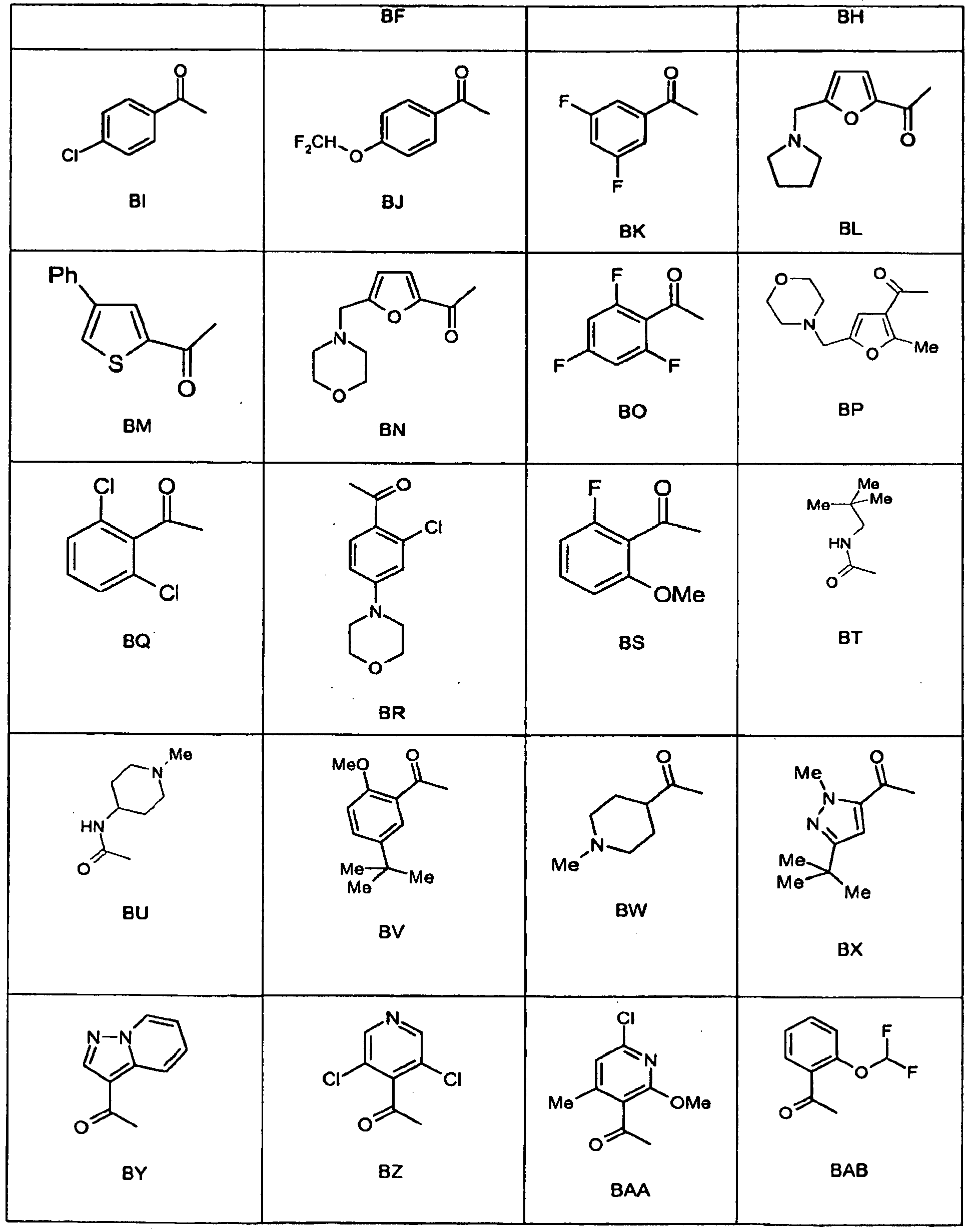

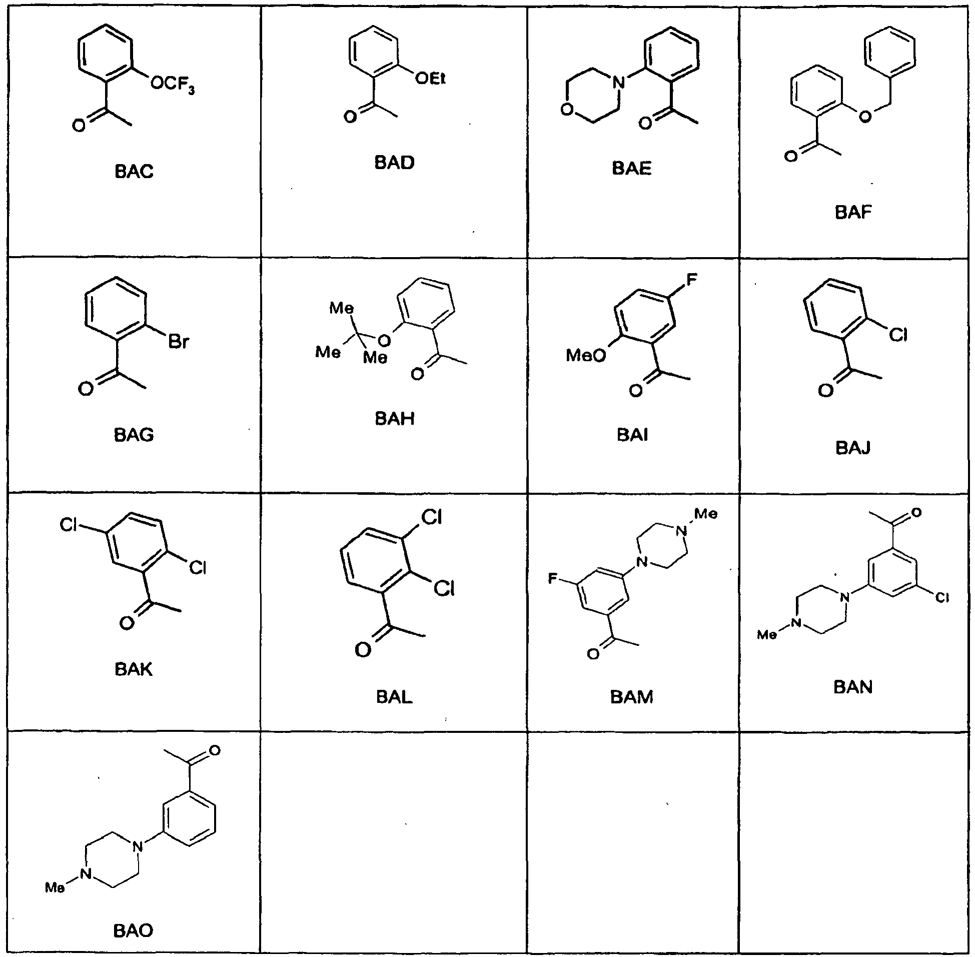

One sub-group of groups R¹-CO consists of groups A to BF in Table 1 above.

Another sub-group of groups R¹-CO consists of groups A to BS in Table 1 above.

One set of preferred groups R¹-CO consists of the groups J, AB, AH, AJ, AL, AS, AX, AY, AZ, BA, BB, BD, BH, BL, BQ, BS and BAI

Another set of preferred groups R¹-CO consists of the groups J, AB, AH, AJ, AL, AS, AX, AY, AZ, BA, BB, BD, BH, BL, BQ and BS.

More preferred groups R¹-CO- are AJ, AX, BQ, BS and BAI.

One particularly preferred sub-set of groups R¹-CO- consists of AJ, BQ and BS.

Another particularly preferred sub-set of groups R¹-CO- consists of AJ and BQ.

When R¹ is a phenyl ring bearing a substituent at the 4-position, the substituent at the 4-position is preferably other than a phenyl group having a group SO₂NH₂ or SO₂Me at the ortho-position.

In one general embodiment, R¹ may be other than a substituted or unsubstituted tetrahydroquinoline, chroman, chromene, thiochroman, thiochromene, dihydro-naphthalene or tetrahydronaphthalene group. More particularly, R¹ may be other than a substituted or unsubstituted tetrahydroquinoline, chroman, chromene, thiochroman, thiochromene, dihydro-naphthalene or tetrahydronaphthalene group linked by its aromatic ring to the moiety C(=O)-NH-.

In another general embodiment, when R¹ is a substituted or unsubstituted phenyl group, the moiety Y-R³ may be other than unsubstituted C₅₋₁₀ cycloalkyl.

When R¹ Is an optionally substituted hydrocarbyl group and the hydrocarbyl group comprises or contains a substituted or unsubstituted alkene group, it is preferred that the carbon-carbon double bond of the alkene group is not directly bonded to the group A.

When R¹ is an optionally substituted hydrocarbyl group, the hydrocarbyl group may be other than an alkene group.

### Y

In the compounds of the formula (II), Y is a bond or an alkylene chain of 1, 2 or 3 carbon atoms in length.

The term "alkylene" has its usual meaning and refers to a divalent saturated acyclic hydrocarbon chain. The hydrocarbon chain may be branched or unbranched. Where an alkylene chain is branched, it may have one or more methyl group side chains. Examples of alkylene groups include -CH₂-, -CH₂-CH₂-, -CH₂-CH₂-CH₂-, CH(CH₃)-, -C(CH₃)₂-, -CH₂-CH(CH₃)-, -CH₂-C(CH₃)₂- and -CH(CH₃)-CH(CH₃)-.

In one embodiment, Y is a bond.

In another embodiment, Y is an alkylene chain.

When Y is an alkylene chain, preferably it is unbranched and more particularly contains 1 or 2 carbon atoms, preferably 1 carbon atom. Thus preferred groups Y are -CH₂- and -CH₂-CH₂-, a most preferred group being (CH₂)-.

Where Y is a branched chain, preferably it has no more than two methyl side chains. For example, it may have a single methyl side chain. In one embodiment, Y is a group -CH(Me)-.

In one sub-group of compounds, Y is a bond, CH₂, CH₂CH₂ or CH₂CH(CH₃).

### R³

The group R⁵ is selected from non-aromatic carbocyclic and heterocyclic groups having from 3 to 12 ring members.

In a another sub-group of compounds, Y is a bond or an alkylene chain (e.g. a group -(CH₂)-) and R³ is a non-aromatic carbocyclic or heterocyclic group.

In a further sub-group of compounds, Y is a bond.

In a still further sub-group of compounds, Y is an alkylene chain (e.g. a group -(CH₂)-) and R³ is a non-aromatic carbocyclic or heterocyclic group.

The carbocyclic and heterocyclic groups R³ are non-aromatic carbocyclic or non-aromatic heterocyclic and examples of such groups are as set out in detail above in the General Preferences and Definitions section, and as set out below.

Examples of non-aromatic groups R³ include optionally substituted (by R¹⁰ or R^{10a}) cycloalkyl, oxa-cydoalkyl, aza-cycloalkyl, diaza-cycloalkyl, dioxa-cycloalkyl and aza-oxa-cycloalkyl groups. Further examples include C_{7- 10} aza-bicycloalkyl groups such as 1-aza-bicyclo[2.2.2]octan-3-yl.

Particular examples of such groups include unsubstituted or substituted cyclopropyl, cyclobutyl, cyclopentyl, cyclohexyl, tetrahydropyran, morpholine, tetrahydrofuran, piperidine and pyrrolidine groups.

One sub-set of non-aromatic groups R³ consists of cyclopropyl, cyclobutyl, cyclopentyl, cyclohexyl, tetrahydropyran, tetrahydrofuran, piperidine and pyrrolidine groups.

Preferred non-aromatic groups R³ include unsubstituted or substituted cyclopentyl, cyclohexyl, tetrahydropyran, tetrahydrofuran, piperidine and pyrrolidine groups,

The non-aromatic groups may be unsubstituted or substituted with one or more groups R¹⁰ or R^{10a} as hereinbefore defined.

Particular substituents for R³ are selected from the group R^{10a} consisting of halogen; hydroxy; monocyclic carbocyclic and heterocyclic groups having from 3 to 6 ring members and containing up to 2 heteroataom ring members selected from O, N and S; and a group R^{a}-R^{b} wherein R^{a} is a bond, O, CO, CO₂, SO₂, NH, SO₂NH or NHSO₂; and R^{b} is selected from hydrogen, a carbocyclic or heterocyclic group with 3-6 ring members and containing up to 2 heteroatom ring members selected from O, N and S; and a C₁₋₈ hydrocarbyl group optionally substituted by one or more substituents selected from hydroxy, oxo, halogen, carboxy, amino, mono- or di-C₁₋₄ hydrocarbylamlno, a carbocyclic or heterocyclic group with 3-6 ring members and containing up to 2 heteroatom ring members selcted from O, N and S; and wherein one or two carbon atoms of the C₁₋₈ hydrocarbyl group may optionally be replaced by O, S, SO, SO₂ or NH.

In one embodiment, preferred R^{10a} substituent groups on R³ include halogen, a group R^{a}-R^{b} wherein R⁸ is a bond, O, CO, C(X²)X¹, and R^{b} is selected from hydrogen, heterocyclic groups having 3-7 ring members and a C₁₋₄ hydrocarbyl group optionally substituted by one or more substituents selected from hydroxy, carboxy, amino, mono- or di-C₁₋₄ hydrocarbylamino, and heterocyclic groups having 3-7 ring members.

Particularly preferred substituent groups R^{10a} on R³ include halogen, especially fluorine, C₁₋₃ alkoxy such as methoxy, and C₁₋₃ hydrocarbyl optionally substituted by fluorine, hydroxy (e.g. hydroxymethyl), C₁₋₂ alkoxy or a 5- or 6-membered saturated heterocyclic ring such as piperidino, morpholino, piperazino and N-methylpiperazino.

In another embodiment, the substituents for R³ are selected from:
- halogen (e.g. fluorine and chlorine)
- C₁₋₄ alkoxy (e.g. methoxy and ethoxy) optionally substituted by one or substituents selected from halogen, hydroxy, C₁₋₂ alkoxy and five and six membered saturated heterocyclic rings containing 1 or 2 heteroatoms selected from O, N and S, the heterocyclic rings being optionally further substituted by one or more C₁₋₄ groups (e.g. methyl) and wherein the S, when present, may be present as S, SO or SO₂;
- C₁₋₄ alkyl optionally substituted by one or substituents selected from halogen, hydroxy, C₁₋₄ alkoxy, amino, C₁₋₄ alkylsulphonylamino, 3 to 6 membered cycloalkyl groups (e.g. cyclopropyl), phenyl (optionally substituted by one or more substituents selected from halogen, methyl, methoxy and amino) and five and six membered saturated heterocyclic rings containing 1 or 2 heteroatoms selected from O, N and S, the heterocyclic rings being optionally further substituted by one or more C₁₋₄ groups (e.g. methyl) and wherein the S, when present, may be present as S, SO or SO₂;
- hydroxy;
- amino, mono-C₁₋₄ alkylamino, di-C₁₋₄ alkylamino, benzyloxycarbonylamino and C₁₋₄ alkoxycarbonylamino;
- carboxy and C₁₋₄ alkoxycarbonyl;
- C₁₋₄ alkylaminosulphonyl and C₁₋₄ alkylsulphonylamino;
- C₁₋₄ alkylsulphonyl;
- a group O-Het^{s} or NH-Het^{s} where Het^{s} is a five or six membered saturated heterocyclic ring containing 1 or 2 heteroatoms selected from O, N and S, the heterocyclic rings being optionally further substituted by one or more C₁₋₄ groups (e.g. methyl) and wherein the S, when present, may be present as S, SO or SO₂;
- five and six membered saturated heterocyclic rings containing 1 or 2 heteroatoms selected from O, N and S, the heterocyclic rings being optionally further substituted by one or more C₁₋₄ groups (e.g. methyl) and wherein the S, when present, may be present as S, SO or SO₂;
- oxo; and
- six membered aryl and heteroaryl rings containing up to two nitrogen ring members and being optionally substituted by one or substituents selected from halogen, methyl and methoxy.

In a further embodiment, R³ is selected from:
- C₃-C₇ cycloalkyl groups optionally substituted by 1-4 (for example 1-2, e.g. 1) substituents R¹⁰ or R^{10a};
- saturated five membered heterocyclic rings containing 1 ring heteroatom selected from O, N and S and being optionally substituted by an oxo group and/or by 1-4 (for example 1-2, e.g. 1) substituents R¹⁰ or R^{10a};
- saturated six membered heterocyclic rings containing 1 or 2 ring heteroatoms selected from O, N and S and being optionally substituted by an oxo group and/or by 1-4 (for example 1-2, e.g. 1) substituents R¹⁰ or R^{10a};
- mono-azabicycloalkyl and diazabicycloalkyl groups each having 7 to 9 ring members and being optionally substituted by 1-4 (for example 1-2, e.g. 1) substituents R¹⁰ or R^{10a}.

Specific examples of the group Y-R³ are set out in Table 2. In Table 2, the point of attachment of the group to the nitrogen atom of the pyrazole-3-carboxamide group is represented by the terminal single bond extending from the group. Thus, by way of illustration, group CA in the table is the 4-fluorophenyl, group CB in the table is the 4-methoxybenzyl group and group CC in the table is the 4-(4-methylpiperazino)-phenylmethyl group.

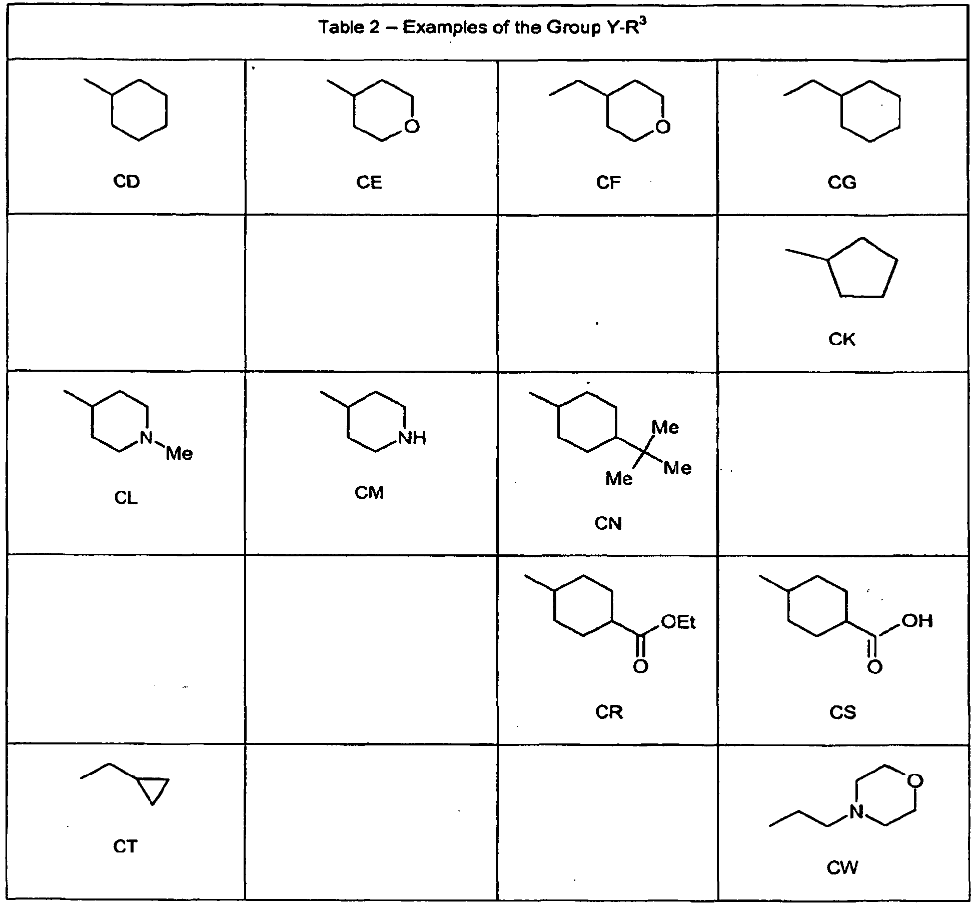

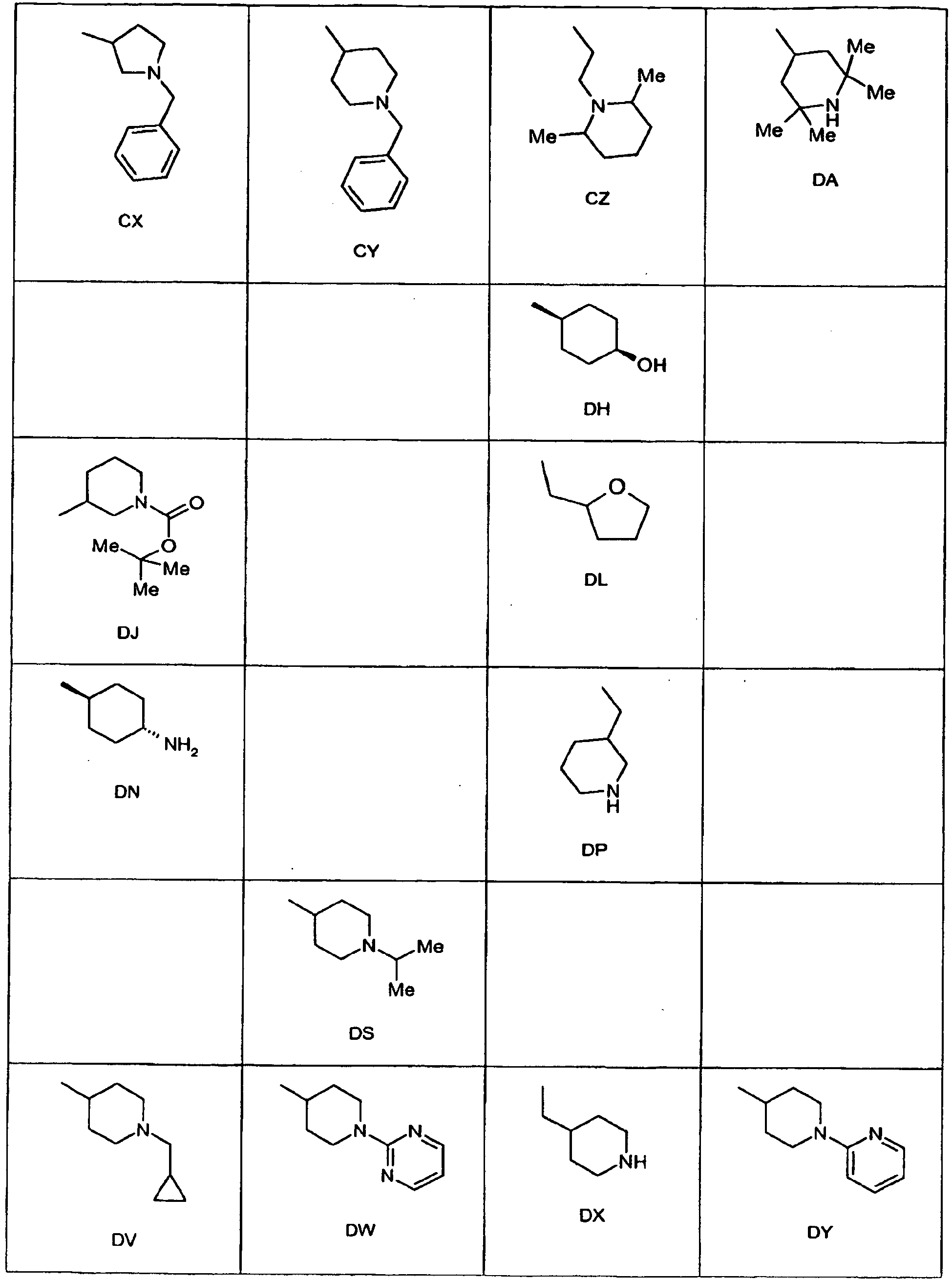

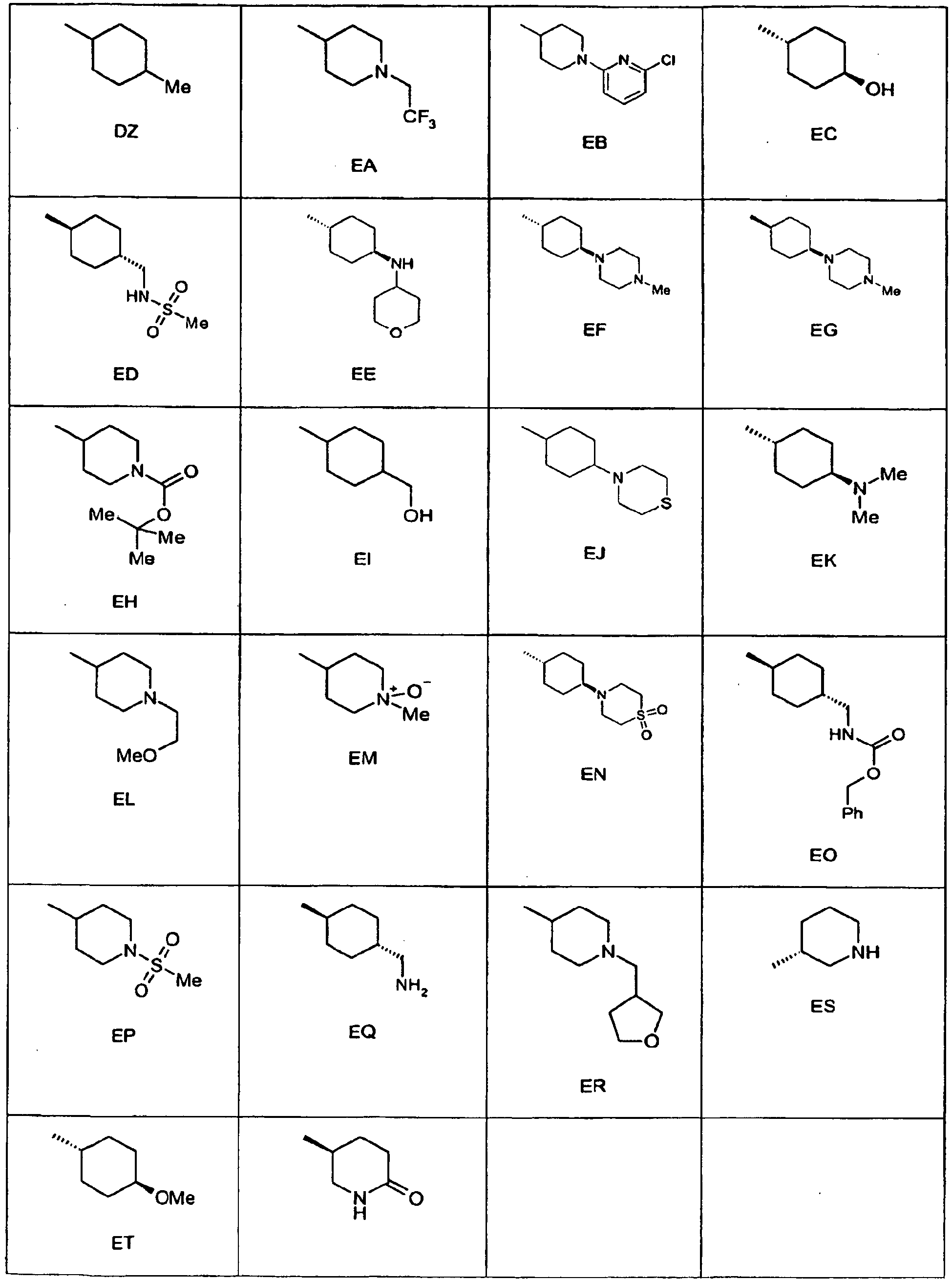

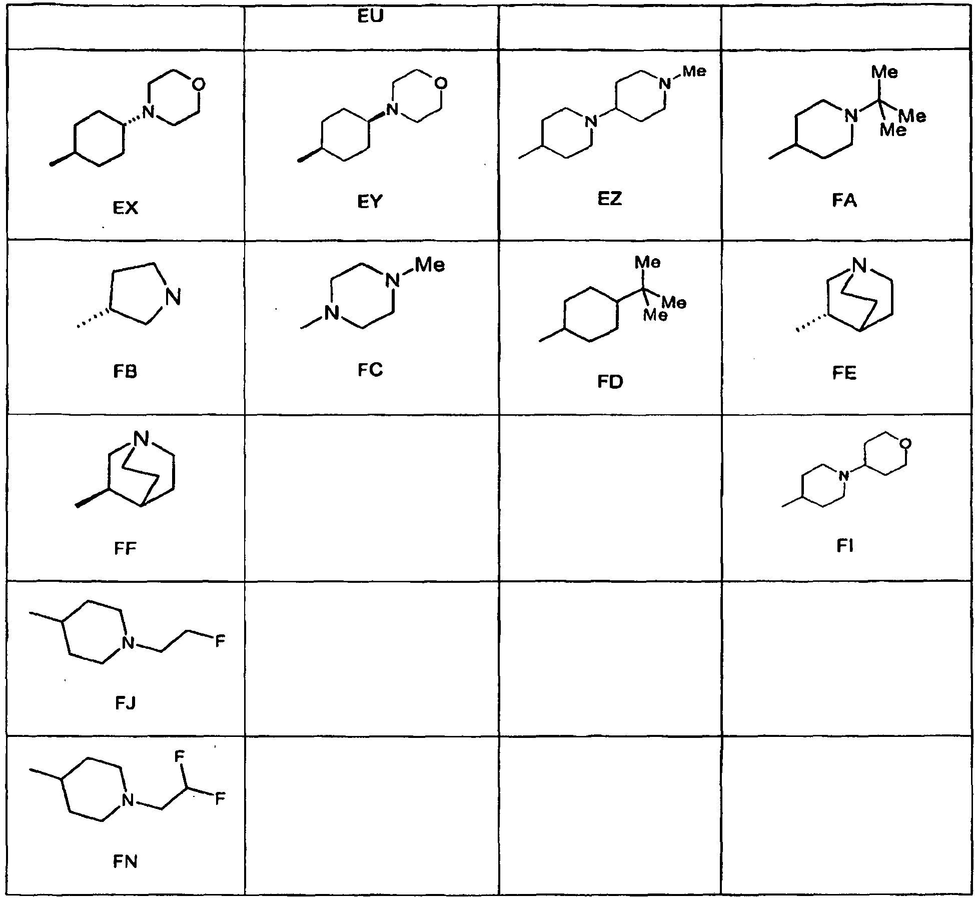

One sub-set of groups selected from table 2 consists of groups CD to EU.

Another sub-set of groups selected from table 2 consists of groups CD to CT.

Preferred groups selected from Table 2 include groups CL, CM, ES, ET and FC.

Particularly preferred groups selected from Table 2 include groups CL, CM and ES, and most preferably CL and CM.

In another general embodiment, when R³ is an aza-cycloalkyl group, the nitrogen atom of the aza-cycloalkyl group is preferably not substituted with an alkylene chain linked to a 2,3-dihydro-benzo[1,4]dioxine or tetrahydronaphthalene group.

In another general embodiment, R³ is other than a moiety containing a five membered heteroaryl ring linked directly by a single bond to a monocyclic or bicyclic aryl group or R³ is other than a moiety containing a bis heteroaryl group comprising two five membered heteroaryl rings linked together by a single bond.

In a further general embodiment, R¹ is other than a moiety containing a five membered heteroaryl ring linked directly by a single bond to a monocyclic or bicyclic aryl group or R¹ Is other than a moiety containing a bis heteroaryl group comprising two five membered heteroaryl rings linked together by a single bond.

In another general embodiment, R¹-C(=)NH- is other than an optionally substituted benzoyl-amino group when Y-R³ is an alkyl, cycloalkyl, optionally substituted phenyl or optionally substituted phenylalkyl group.

Y-R³ may be other than a cycloalkyl group substituted at the 1-position with a hydrocarbon chain simultaneously bearing an oxy substituent such as hydroxy, an aryl substituent and a diazole or triazole substituent.

Preferably, R¹ or R³ each are other than a moiety containing a substituted phenyl group having thio and/or oxy substituents such as hydroxy, alkoxy and alkylthio at both the 3- and 4-positions of the phenyl ring.

The group Y-R³ preferably does not include a benzo-fused lactam group having attached thereto an unsubstituted or substituted imidazole group.

The group Y-R³ preferably does not include the moiety -CH=C(CO₂R^{q})-S- where R^{q} is hydrogen or alkyl.

In another general embodiment, neither R¹ nor R³ contain a moiety in which a five membered nitrogen-containing heteroaryl group is linked directly or via an alkylene, oxa-alkylene, thia-alkylene or aza-alkylene group to an unsubstituted pyridyl group or to a substituted aryl, heteroaryl or piperidine ring, each said ring having attached thereto a subsitutent selected from cyano, and substituted or unsubstituted amino, aminoalkyl, amidine, guanidine, and carbamoyl groups.

In a further general embodiment, R¹ and R³ are each other than an unsaturated nitrogen-containing heterocyclic group or a benzfuran or benzthiophene group wherein the said nitrogen-containing heterocyclic group, bicyclic benzfuran or benzthiophene group are linked directly by a single bond to a substituted pyridyl or phenyl group.

In another general embodiment, neither R¹ nor R³ contain a moiety in which a five membered nitrogen-containing heteroaryl group is linked directly or via an alkylene, oxa-alkylene, thia-alkylene or aza-alkylene group to a substituted aryl, heteroaryl or piperidine group or to an unsubstituted pyridyl group.

In general, it is preferred that the compounds of the invention, where they contain a carboxylic acid group, contain no more than one such group.

Within formula (II), it is preferred that R² is hydrogen.

In one sub-group of compounds of the formula (II), R¹ is:
(i) phenyl optionally substituted by one or more substituents (e.g. 1, 2 or 3) selected from fluorine; chlorine; hydroxy; 5- and 6-membered saturated heterocyclic groups containing 1 or 2 heteroatoms selected from O, N and S, the heterocyclic groups being optionally substituted by one or more C₁₋₄ alkyl groups; C₁₋₄ hydrocarbyloxy; and C₁₋₄ hydrocarbyl; wherein the C₁₋₄ hydrocarbyl and C₁₋₄ hydrocarbyloxy groups are optionally substituted by one or more substituents chosen from hydroxy, fluorine, C₁₋₂ alkoxy, amino, mono and di-C₁₋₄ alkylamino, phenyl, halophenyl, saturated carbocyclic groups having 3 to 7 ring members (more preferably 4, 5 or 6 ring members, e.g. 5 or 6 ring members) or saturated heterocyclic groups of 5 or 6 ring members and containing up to 2 heteroatoms selected from O, S and N; or 2, 3-dihydro-benzo[1,4]dioxine; or
(ii) a monocyclic heteroaryl group containing one or two heteroatoms selected from O, S and N; or a bicyclic heteroaryl group containing a single heteroatom selected from O, S and N; the monocyclic and bicyclic heteroaryl groups each being optionally substituted by one or more substituents selected from fluorine; chlorine; C₁₋₃ hydrocarbyloxy; and C₁₋₃ hydrocarbyl optionally substituted by hydroxy, fluorine, methoxy or a five or six membered saturated carbocyclic or heterocyclic group containing up to two heteroatoms selected from O, S and N; or
(iii) a substituted or unsubstituted cycloalkyl group having from 3 to 6 ring members; or
(iv) a C₁₋₄ hydrocarbyl group optionally substituted by one or more substituents selected from fluorine; hydroxy; C₁₋₄ hydrocarbyloxy; amino; mono- or di-C₁₋₄ hydrocarbylamino; and carbocyclic or heterocyclic groups having from 3 to 12 ring members, and wherein one of the carbon atoms of the hydrocarbyl group may optionally be replaced by an atom or group selected from O, NH, SO and SO₂.

Within group (i), a sub-group of groups R¹ consists of phenyl optionally substituted by one or more substituents selected from fluorine; chlorine; hydroxy; C₁₋₃ hydrocarbyloxy; and C₁₋₃ hydrocarbyl wherein the C₁₋₃ hydrocarbyl group is optionally substituted by one or more substituents chosen from hydroxy, fluorine, C₁₋₂ alkoxy, amino, mono and di-C₁₋₄ alkylamino, saturated carbocyclic groups having 3 to 7 ring members (more preferably 4, 5 or 6 ring members, e.g. 5 or 6 ring members) or saturated heterocyclic groups of 5 or 6 ring members and containing up to 2 heteroatoms selected from O, S and N.

In another sub-group of compounds of the formula (II), R¹ is selected from (i) and (iii) above and additionally from a sub-set (aii) where sub-set (aii) consists of 2-furanyl, 3-furanyl, imidazolyl, 2-pyridyl, indolyl, 2-thienyl and 3-thienyl, each optionally substituted by one or more substituents selected from fluorine, chlorine, C₁₋₃ hydrocarbyloxy, and C₁₋₃ hydrocarbyl optionally substituted by hydroxy, fluorine or methoxy.

Within the group of compounds defined by the formula (II), where R¹ is (i) an optionally substituted phenyl group, it may be, for example, an unsubstituted phenyl group or a 2-monosubstituted, 3-monosubstituted, 2,3 disubstituted, 2,5 disubstituted or 2,6 disubstituted phenyl group or 2, 3-dihydro-benzo[1,4]dioxine, where the substituents are selected from halogen; hydroxyl; C₁₋₃ alkoxy; and C₁₋₃ alkyl groups wherein the C₁₋₃ alkyl group is optionally substituted by hydroxy, fluorine, C₁₋₂ alkoxy, amino, mono and di-C₁₋₄ alkylamino, or saturated carbocyclic groups having 3 to 6 ring members and/or saturated heterocyclic groups of 5 or 6 ring members and containing 1 or 2 heteroatoms selected from N and O.

In one embodiment, R¹ is selected from unsubstituted phenyl, 2-fluorophenyl, 2-hydroxyphenyl, 2-methoxyphenyl, 2-methylphenyl, 2-(2-(pyrrolidin-1-yl)ethoxy)-phenyl, 3-fluorophenyl, 3-methoxyphenyl, 2,6-difluorophenyl, 2-fluoro-6-hydroxyphenyl, 2-fluoro-3-methoxyphenyl, 2-fluoro-5-methoxyphenyl, 2-chloro-6-methoxyphenyl, 2-fluoro-6-methoxyphenyl, 2,6-dichlorophenyl and 2-chloro-6-fluorophenyl, and is optionally further selected from 5-fluoro-2-methoxyphenyl.

In another embodiment, R¹ is selected from unsubstituted phenyl, 2-fluorophenyl, 2-hydroxyphenyl, 2-methoxyphenyl, 2-methylphenyl, 2-(2-(pyrrolidin-1-yl)ethoxy)-phenyl, 3-fluorophenyl, 3-methoxyphenyl, 2,6-difluorophenyl, 2-fluoro-6-hydroxyphenyl, 2-fluoro-3-methoxyphenyl and 2-fluoro-5-methoxyphenyl.

Particular groups R¹ are 2,6-difluorophenyl, 2-fluoro-6-methoxyphenyl and 2,6-dichlorophenyl.

One particularly preferred group R¹ is 2,6-difluorophenyl.

Another particularly preferred group R¹ is 2,6-dichlorophenyl.

When R¹ is (ii) a monocyclic heteroaryl group containing one or two heteroatoms selected from O, S and N or a bicyclic heteroaryl group containing a single heteroatom, examples of monocyclic and bicyclic heteroaryl groups include furanyl (e.g. 2-furanyl and 3-furanyl), imidazolyl, pyridyl (e.g. 2-pyridyl), indolyl, thienyl (e.g. 2-thienyl and 3-thienyl) groups. The optional substituents for such groups can include chlorine, fluorine, methyl, methoxy, hydroxymethyl, methoxymethyl, morpholinomethyl, piperazinomethyl, N-methylypiperazinomethyl and plperidinylmethyl groups. Particular examples of groups (ii) include unsubstituted 2-furanyl, 3-methyl-2-furanyl, unsubstituted 4-(1H)-imidazolyl, unsubstituted 5-(1H)-imidazolyl, unsubstituted 3-furanyl, unsubstituted 3-thienyl, 2-methyl-3-thienyl and unsubstituted 3-pyrrolyl, and further examples include 4-methoxy-3-thienyl, 5-(1-pyrrolidinyl)methyl-2-furyl and 5-(4-morpholino)methyl-2-furyl groups.

When R¹ is (iii) an optionally substituted cycloalkyl group, it can be for example a substituted or unsubstituted cyclopropyl, cyclobutyl, cyclopentyl or cyclohexyl group. When the cycloalkyl group is substituted, preferred substituents include methyl, fluorine and hydroxyl. Particular examples of cycloalkyl groups include 1-methylcyclopropyl, 1-hydroxycyclopropyl, and unsubstituted cyclohexyl, cyclopentyl and cyclobutyl.

In the context of formula (II) and the group R¹, examples of optionally substituted hydrocarbyl groups are optionally substituted methyl, ethyl and propyl groups wherein one of the carbon atoms of the hydrocarbyl group is optionally replaced by O, NH, SO or SO₂. Particular examples of such groups include methyl, ethyl, trifluoromethyl, methyl and ethyl substituted with a carbocyclic or heterocyclic group having from 3 to 12 ring members, sulphonylmethyl substituted with a carbocyclic or heterocyclic group having from 3 to 12 ring members, hydroxymethyl, hydroxyethyl, 3-hydroxy-2-propyl, propyl, isopropyl, butyl and tertiary butyl. Examples of hydrocarbyl groups and carbocylic and heteroacyclic groups are as set out above in the general definitions of such groups. Particular carbocyclic and heterocyclic groups include unsubstituted or substituted phenyl, indolyl, tetrazolyl, triazolyl, piperidlnyl, morpholinyl, piperazinyl, N-methylpiperazinyl, imidazolyl wherein the optional substituents may be selected from the group R¹⁰, and sub-groups thereof, as defined herein.

In another sub-group of compounds of the formula (II), R¹ is a C₁₋₄ hydrocarbyl group optionally substituted by one or more substituents selected from fluorine, hydroxy, C₁₋₄ hydrocarbyloxy, amino, mono- or di-C₁₋₄ hydrocarbylamino, and carbocyclic or heterocyclic groups having from 3 to 12 ring members, and wherein 1 of the carbon atoms of the hydrocarbyl group may optionally be replaced by an atom or group selected from O, NH, SO and SO₂.

In one embodiment, R¹ is a group R^{1a}-(V)ₙ- where:
n is 0 or 1;
V is selected from CH₂, CH₂CH₂ and SO₂CH₂; and
R^{1a} is a carbocyclic or heterocyclic group selected from phenyl;
   five membered heteroaryl rings having up to 4 heteroatom ring members selected from N, O and S;
   six membered heteroaryl rings containing one or two nitrogen ring members;
   five or six membered saturated non-aromatic heterocyclic rings containing one or two heteroatom ring members selected from N, O, S and SO₂;
   C₃₋₆ cycloalkyl groups; indole; and quinoline;
   wherein each of the carbocyclic and heterocyclic groups R^{1a} can be optionally substituted by one or more substituents selected from five or six membered saturated non-aromatic carbocyclic and heterocyclic groups containing up to two heteroatom ring members selected from N, O, S and SO₂; hydroxy; amino; oxo; mono-C₁₋₄ alkylamino; di-C₁₋₄ alkylamino; fluorine; chlorine; nitro; C₁₋₄ alkyl-(O)_{q}- wherein q is 0 or 1 and the C₁₋₄ alkyl moiety is optionally substituted by fluorine, hydroxy, C₁₋₂ alkoxy or a five or six membered saturated non-aromatic carbocyclic or heterocyclic group containing up to two heteroatom ring members selected from N, O, S and SO₂; phenyl and C₁₋₂-alkylene dioxy.

Specific examples of groups R¹-CO- in formula (II) are set out in Table 1 above.

One sub-group of preferred groups R¹-CO consists of the groups J, AB, AH, AJ, AL, AS, AX, AY, AZ, BA, BB, BD, BH, BL, BQ and BS.

Another sub-group of groups R¹-CO consists of the groups A to BF.

A further sub-group of groups R¹-CO consists of the groups A to BS.

Particularly preferred groups are the groups AJ, BQ and BS in Table 1, e.g. the sub-set consisting of AJ and BQ.

One sub-group of compounds of the formula (II) can be represented by the formula (IV):

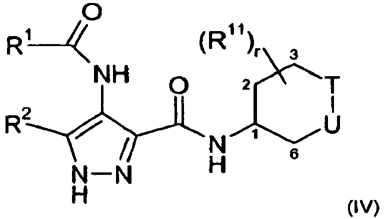

or salts or tautomers or N-oxides or solvates thereof;
wherein R¹ and R² are as defined herein;
an optional second bond may be present between carbon atoms numbered 1 and 2;
one of U and T is selected from CH₂, CHR¹³, CR¹¹R¹³, NR¹⁴, N(O)R¹⁵, O and S(O)ₜ; and the other of U and T is selected from , NR¹⁴, O, CH₂, CHR¹¹, C(R¹¹)₂, and C=O; r is 0, 1, 2, 3 or 4; t is 0, 1 or 2;
R¹¹ is selected from hydrogen, halogen (particularly fluorine), C₁₋₃ alkyl (e.g. methyl) and C₁₋₃ alkoxy (e.g. methoxy);
R¹³ is selected from hydrogen, NHR¹⁴, NOH, NOR¹⁴ and R^{a}-R^{b};
R¹⁴ is selected from hydrogen and R^{d}-R^{b};
R^{d} is selected from a bond, CO, C(X²)X¹, SO₂ and SO₂NR^{c};
R^{a}, R^{b} and R^{c} are as hereinbefore defined; and
R¹⁵ is selected from C₁₋₄ saturated hydrocarbyl optionally substituted by hydroxy, C₁₋₂ alkoxy, halogen or a monocyclic 5- or 6-membered carbocyclic or heterocyclic group, provided that U and T cannot be O simultaneously.

Examples of, and preferences, for the groups R¹ and R² are as set out above for compounds of the formula (II) unless the context indicates otherwise.

Within formula (IV), r can be 0, 1, 2, 3 or 4. In one embodiment, r is 0. In another embodiment, r is 2, and in a further embodiment r Is 4.

Within formula (IV), one sub-set of preferred compounds is the set of compounds where there is only a single bond between the carbon atoms numbered 1 and 2.

However, in another sub-set of compounds, there is a double bond between the carbon atoms numbered 1 and 2.

Another sub-set of compounds is characterised by gem disubstitution at the 2-carbon (when there is a single bond between carbon atoms numbers 1 and 2) and/or the 6-carbon. Preferred gem disubstituents include difluoro and dimethyl.

A further sub-set of compounds is characterised by the presence of an alkoxy group, for example a methoxy group at the carbon atom numbered 3, i.e. at a position α with respect to the group T.

Within formula (IV) are compounds wherein, for example, R³ Is selected from any of the following ring systems:

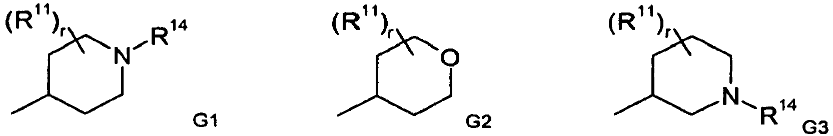

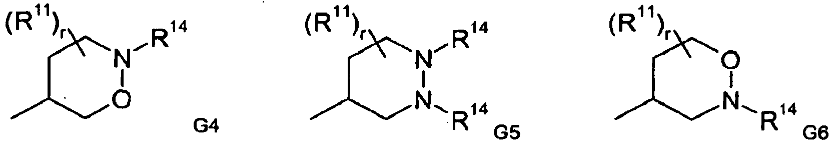

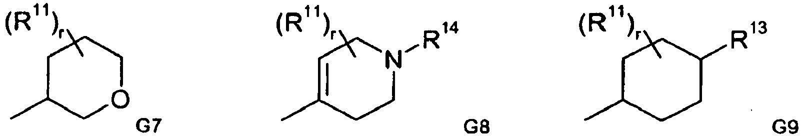

Preferred ring systems include G1 and G3.

A preferred sub-group of compounds within formula (IV) can be represented by the formula (IVa): 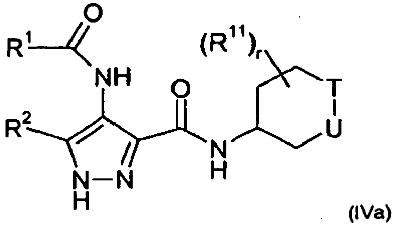 or salts or tautomers or N-oxides or solvates thereof;
wherein R¹ and R² are as hereinbefore defined;
one of U and T is selected from CH₂, CHR¹³, CR¹¹R¹³, NR¹⁴, N(O)R¹⁵, O and S(O)ₜ; and the other of U and T is selected from CH₂, CHR¹¹, C(R¹¹)₂, and C=O; r is 0, 1 or 2; t is 0, 1 or 2;
R¹¹ is selected from hydrogen and C₁₋₃ alkyl;
R¹³ is selected from hydrogen and R^{a}-R^{b};
R¹⁴ is selected from hydrogen and R^{d}-R^{b};
R^{d} is selected from a bond, CO, C(X²)X¹, SO₂ and SO₂NR^{c};
R^{a}, R^{b} and R^{c} are as hereinbefore defined; and
R¹⁵ is selected from C₁₋₄ saturated hydrocarbyl optionally substituted by hydroxy, C₁₋₂ alkoxy, halogen or a monocyclic 5- or 6-membered carbocyclic or heterocyclic group.

Examples of, and preferences, for the groups R¹ and R² are as set out above for compounds of the formula (II) unless the context indicates otherwise.

In formula (IVa), T is preferably selected from CH₂, CHR¹³, CR¹¹R¹³, NR¹⁴, N(O)R¹⁵, O and S(O)ₜ; and U is preferably selected from CH₂, CHR¹¹, C(R¹¹)₂, and C=O.

In the definitions for substituents R¹¹ and R¹⁴, R^{b} is preferably selected from hydrogen; monocyclic carbocyclic and heterocyclic groups having from 3 to 7 ring members; and C₁₋₄ hydrocarbyl (more preferably acyclic saturated C₁₋₄ groups) optionally substituted by one or more substituents selected from hydroxy, oxo, halogen, amino, mono- or di-C₁₋₄ hydrocarbylamino, and monocyclic carbocyclic and heterocyclic groups having from 3 to 7 ring members (more preferably 3 to 6 ring members) and wherein one or more carbon atoms of the C₁₋₄ hydrocarbyl group may optionally be replaced by O, S, SO, SO₂, NR^{c}, X¹C(X²), C(X²)X¹ ;
R^{c} is selected from hydrogen and C₁₋₄ hydrocarbyl; and
X¹ Is O, S or NR^{c} and X² is =O, =S or =NR^{c}.

R¹¹ is preferably selected from hydrogen and methyl and most preferably is hydrogen.

R¹³ is preferably selected from hydrogen; hydroxy; halogen; cyano; amino; mono-C₁₋₄ saturated hydrocarbylamino; dl-C₁₋₄ saturated hydrocarbylamino; monocyclic 5- or 6-membered carbocyclic and heterocyclic groups; C₁₋₄ saturated hydrocarbyl optionally substituted by hydroxy, C₁₋₂ alkoxy, halogen or a monocyclic 5- or 6-membered carbocyclic or heterocyclic group.

Particular examples of R¹³ are hydrogen, hydroxy, amino, C₁₋₂ alkylamino (e.g. methylamino) C₁₋₄ alkyl (e.g. methyl, ethyl, propyl and butyl), C₁₋₂ alkoxy (e.g. methoxy), C₁₋₂ alkylsulphonamido (e.g. methanesulphonamldo), hydroxy-C₁₋₂ alkyl (e.g. hydroxymethyl), C₁₋₂-alkoxy-C₁₋₂ alkyl (e.g. methoxymethyl and methoxyethyl), carboxy. C₁₋₄ alkoxycarbonyl (e.g.ethoxycarbonyl) and aminoC₁₋₂-alkyl (e.g. aminomethyl).

Particular examples of R¹⁴ are hydrogen; alkyl optionally substituted by fluoro or a five or six membered saturated heterocyclic group (e.g. a group selected from (i) methyl, ethyl, n-propyl, i-propyl, butyl, 2,2,2-trifluoroethyl and tetrahydrofuranylmethyl; and/or (ii) 2-fluoroethyl and 2,2-difluoroethyl); cyclopropylmethyl; substituted or unsubstituted pyridyl-C₁₋₂ alkyl (e.g. 2-pyridylmethyl); substituted or unsubstituted phenyl-C₁₋₂ alkyl (e.g. benzyl); C₁₋₄ alkoxycarbonyl (e.g.ethoxycarbonyl and t-butyloxycarbonyl); substituted and unsubstituted phenyl-C₁₋₂ alkoxycarbonyl (e.g. benzyloxycarbonyl); substituted and unsubstituted 5- and 6-membered heteroaryl groups such as pyridyl (e.g. 2-pyridyl and 6-chloro-2-pyridyl) and pyrimidinyl (e.g. 2-pyrimidinyl); C₁₋₂-alkoxy-C₁₋₂ alkyl (e.g. methoxymethyl and methoxyethyl); C₁₋₄ alkylsulphonyl (e.g. methanesulphonyl).

Preferred compounds include those in which (i) U is CHR¹³ (more preferably CH₂) and T is NR₁₄, and (II) T is CHR¹³ (more preferably CH₂) and U is NR¹⁴.

One particular preferred sub-group of compounds of the formula (IV) can be represented by the formula (Va): 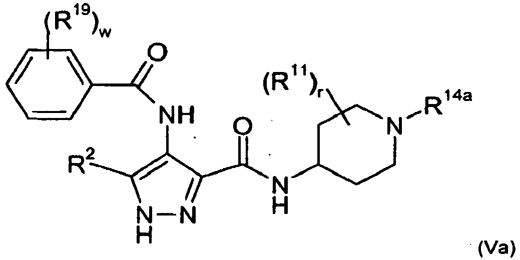 or salts or tautomers or N-oxides or solvates thereof;
wherein R^{14a} is selected from hydrogen, C₁₋₄ alkyl optionally substituted by fluoro (e.g. methyl, ethyl, n-propyl, i-propyl, butyl and 2,2,2-trifluoroethyl), cyclopropylmethyl, phenyl-C₁₋₂ alkyl (e.g. benzyl), C₁₋₄ alkoxycarbonyl (e.g.ethoxycarbonyl and t-butyloxycarbonyl), phenyl-C₁₋₂ alkoxycarbonyl (e.g. benzyloxycarbonyl), C₁₋₂-alkoxy-C₁₋₂ alkyl (e.g. methoxymethyl and methoxyethyl), and C₁₋₄ alkylsulphonyl (e.g.methanesulphonyl), wherein the phenyl moieties when present are optionally substituted by one to three substituents selected from fluorine, chlorine, C₁₋₄ alkoxy optionally substituted by fluoro or C₁₋₂-alkoxy, and C₁₋₄ alkyl optionally substituted by fluoro or C₁₋₂-alkoxy;
w is 0, 1, 2 or 3;
R² is hydrogen or methyl, most preferably hydrogen;
R¹¹ and r are as hereinbefore defined; and
R¹⁹ is selected from fluorine; chlorine; C₁₋₄ alkoxy optionally substituted by fluoro or C₁₋₂-alkoxy, and C₁₋₄ alkyl optionally substituted by fluoro or C₁₋₂-alkoxy.

Another particular preferred sub-group of compounds of the formula (IV) can be represented by the formula (Vb): 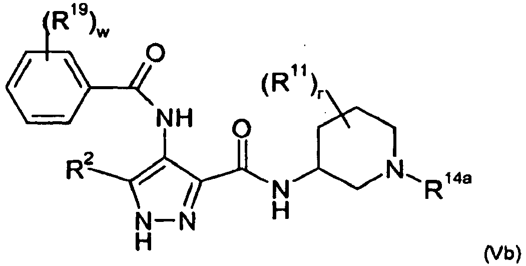 or salts or tautomers or N-oxides or solvates thereof;
wherein R^{14a} is selected from hydrogen, C₁₋₄ alkyl optionally substituted by fluoro (e.g. methyl, ethyl, n-propyl, i-propyl, butyl and 2,2,2-trifluoroethyl), cyclopropylmethyl, phenyl-C₁₋₂ alkyl (e.g. benzyl), C₁₋₄ alkoxycarbonyl (e.g.ethoxycarbonyl and t-butyloxycarbonyl), phenyl-C₁₋₂ alkoxycarbonyl (e.g. benzyloxycarbonyl), C₁₋₂-alkoxy-C₁₋₂ alkyl (e.g. methoxymethyl and methoxyethyl), and C₁₋₄ alkylsulphonyl (e.g.methanesulphonyl), wherein the phenyl moieties when present are optionally substituted by one to three substituents selected from fluorine, chlorine, C₁₋₄ alkoxy optionally substituted by fluoro or C₁₋₂-alkoxy, and C₁₋₄ alkyl optionally substituted by fluoro or C₁₋₂-alkoxy;
w is 0, 1, 2 or 3;
R² is hydrogen or methyl, most preferably hydrogen;
R¹¹ and r are as hereinbefore defined; and
R¹⁹ is selected from fluorine; chlorine; C₁₋₄ alkoxy optionally substituted by fluoro or C₁₋₂-alkoxy; and C₁₋₄ alkyl optionally substituted by fluoro or C₁₋₂-alkoxy.

In formulae (Va) and (Vb), when w Is 1, 2 or 3, it is preferred that the phenyl ring is 2-monosubstituted, 3-monosubstituted, 2,6-disubstituted, 2,3-disubstituted, 2,4-disubstituted 2,5-disubstituted, 2,3,6-trisubstituted or 2,4,6-trisubstituted. Most preferably the phenyl ring is disubstituted at positions 2- and 6- with substituents selected from fluorine, chlorine and methoxy.

R¹¹ is preferably hydrogen (or r is 0).

R^{14a} is most preferably hydrogen or methyl.

One preferred sub-group of compounds of the formula (Va) can be represented by the formula (Via): 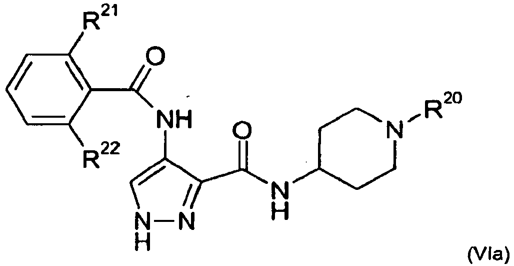 or salts or tautomers or N-oxides or solvates thereof;
wherein R²⁰ is selected from hydrogen and methyl;
R²¹ is selected from fluorine and chlorine; and
R²² is selected from fluorine, chlorine and methoxy; or
one of R²¹ and R²² is hydrogen and the other is selected from chlorine, methoxy, ethoxy, difluoromethoxy, trifluoromethoxy and benzyloxy.

Another preferred sub-group of compounds of the formula (Va) can be represented by the formula (Vlb): 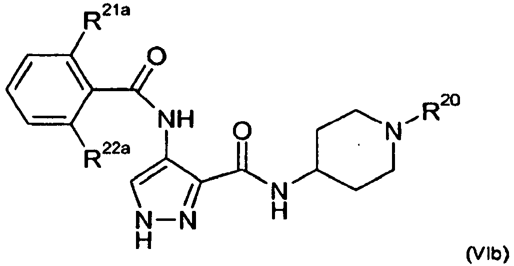 or salts or tautomers or N-oxides or solvates thereof;
wherein R²⁰ is selected from hydrogen and methyl;
R^{21a} is selected from fluorine and chlorine; and
R^{22a} is selected from fluorine, chlorine and methoxy.

Particular compounds within formula (VIb) include:
4-(2,6-difluoro-benzoylamino)-1H-pyrazole-3-carboxylic acid piperidin-4-ylamide;
4-(2,6-difluoro-benzoylamino)-1H-pyrazole-3-carboxylic acid (1-methyl-piperidin-4-yl)-amide;
4-(2,6-dichloro-benzoylamino)-1H-pyrazole-3-carboxylic acid piperidln-4-ylamide; and
4-(2-fluoro-6-methoxy-benzoylamino)-1H-pyrazole-3-carboxylic acid piperidin-4-ylamide;
   or salts or tautomers or N-oxides or solvates thereof.

For the avoidance of doubt, it is to be understood that each general and specific preference, embodiment and example of the groups R¹ may be combined with each general and specific preference, embodiment and example of the groups R² and/or R³ and/or R¹⁰ and/or Y and/or sub-groups thereof as defined herein and that all such combinations are embraced by this application.

The various functional groups and substituents making up the compounds of the formula (II) are typically chosen such that the molecular weight of the compound of the formula (II) does not exceed 1000. More usually, the molecular weight of the compound will be less than 750, for example less than 700, or less than 650, or less than 600, or less than 550. More preferably, the molecular weight is less than 525 and, for example, is 500 or less.

Particular compounds of the formulae (II), (IV), (IVa), (Va), (Vb), (Via) or (Vlb) and sub-groups thereof are as illustrated in the examples below.

One particularly preferred compound is 4-(2,6-dichloro-benzoylamino)-1H-pyrazole-3-carboxylic acid piperidin-4-ylamide and salts therof, particularly acid addition salts such as the methanesulphonic acid, acetic acid and hydrochloric acid salts.

### Salts, Solvates, Tautomers, Isomers, N-Oxides, Esters, Prodrugs and Isotopes

A reference to an ancillary agent as hereinabove described or compound of the formulae (II), (IV), (IVa), (Va), (Vb), (VIa) or (VIb) and sub-groups thereof also includes ionic, salt, solvate, isomers, tautomers, N-oxides, esters, prodrugs, isotopes and protected forms thereof, for example, as discussed below. Preferably the salts or tautomers or isomers or N-oxides or solvates thereof. More preferably, the salts or tautomers or N-oxides or solvates thereof.

Many compounds of the formula (II) can exist in the form of salts, for example acid addition salts or, in certain cases salts of organic and inorganic bases such as carboxylate, sulphonate and phosphate salts. All such salts are within the scope of this invention, and references to compounds of the formula (II) include the salt forms of the compounds. As in the preceding sections of this application, all references to formula (II) should be taken to refer also to formulae (II), (IV), (IVa), (Va), (Vb), (Via) or (VIb) and sub-groups thereof unless the context indicates otherwise.

Salt forms may be selected and prepared according to methods described in Pharmaceutical Salts: Properties, Selection, and Use, P. Heinrich Stahl (Editor), Camille G. Wermuth (Editor), ISBN: 3-90639-026-8, Hardcover, 388 pages, August 2002.

Acid addition salts may be formed with a wide variety of acids, both inorganic and organic. Examples of acid addition salts include salts formed with an acid selected from the group consisting of acetic, 2,2-dichloroacetic, adipic, alginic, ascorbic (e.g. L-ascorbic), L-aspartic, benzenesulphonic, benzoic, 4-acetamidobenzoic, butanoic, (+) camphoric, camphor-sulphonic, (+)-(1S)-camphor-10-sulphonic, capric, caproic, caprylic, carbonic, cinnamic, citric, cyclamic, dodecylsulphuric, ethane-1,2-disulphonic, ethanesulphonic, 2-hydroxyethanesulphonic, formic, fumaric, galactaric, gentisic, glucoheptonic, D-gluconic, glucuronic (e.g. D-glucuronic), glutamic (e.g. L-glutamic), α-oxoglutaric, glycolic, hippuric, hydrobromic, hydrochloric, hydriodic, isethionic, (+)-L-lactic, (±)-DL-lactic, lactobionic, maleic, malic, (-)-L-malic, malonic, (±)-DL-mandelic, methanesulphonic, naphthalene-2-sulphonic, naphthalene-1,5-disulphonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulphuric, tannic, (+)-L-tartaric, thiocyanic, p-toluenesulphonic, undecylenic and valeric acids, as well as acylated amino acids and cation exchange resins.

One particular group of salts includes salts formed with an acid selected from the group consisting of acetic, adipic, alginic, ascorbic (e.g. L-ascorbic), aspartic (e.g. L-aspartic), benzenesulphonic, benzoic, camphoric (e.g. (+) camphoric), capric, caprylic, carbonic, citric, cyclamic, dodecanoate, dodecylsulphuric, ethane-1,2-disulphonic, ethanesulphonic, fumaric, galactaric, gentisic, glucoheptonic, D-gluconic, glucuronic (e.g. D-glucuronic), glutamic (e.g. L-glutamic), α-oxoglutaric, glycolic, hippuric, hydrochloric, isethionic, isobutyric, lactic (e.g. (+)-L-lactic and (±)-DL-lactic), lactobionic, laurylsulphonic, maleic, malic, (-)-L-malic, malonic, methanesulphonic, mucic, naphthalenesulphonic (e.g. naphthalene-2-sulphonic), naphthalene-1,5-disulphonic, nicotinic, oleic, orotic, oxalic, palmitic, pamoic, phosphoric, propionic, sebacic, stearic, succinic, sulphuric, tartaric (e.g. (+)-L-tartaric), thiocyanic, toluenesulphonic (e.g. p-toluenesulphonic), valeric and xinafoic acids.

Another particular group of salts consists of salts formed from hydrochloric, hydriodic, phosphoric, nitric, sulphuric, citric, lactic, succinic, maleic, malic, isethionic, fumaric, benzenesulphonic, toluenesulphonic, methanesulphonic, ethanesulphonic, naphthalenesulphonic, valeric, acetic, propanoic, butanoic, malonic, glucuronic and lactobionic acids.

One preferred group of salts consists of salts formed from methanesulphonic, hydrochloric, acetic, adipic, L-aspartic and DL-lactic acids.

Particular salts are salts formed with hydrochloric, methanesulphonic and acetic acids.

One preferred salt is the salt formed with methanesulphonic acid.

Another preferred salt is the salt formed with acetic acid.

A further preferred salt is the salt formed with hydrochloric acid.

For example, if the compound is anionic, or has a functional group which may be anionic (e.g., -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al³⁺. Examples of suitable organic cations include, but are not limited to, ammonium ion (i.e., NH₄⁺) and substituted ammonium ions (e.g., NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

Where the compounds of the formula (II) contain an amine function, these may form quaternary ammonium salts, for example by reaction with an alkylating agent according to methods well known to the skilled person. Such quaternary ammonium compounds are within the scope of formula (II).

The salt forms of the compounds of the invention are typically pharmaceutically acceptable salts, and examples of Pharmaceutical acceptable salts are discussed in Berge et al., 1977, "Pharmaceutically Acceptable Salts," J. Pharm. Sci., Vol. 66, pp. 1-19. However, salts that are not pharmaceutically acceptable may also be prepared as intermediate forms which may then be converted into pharmaceutically acceptable salts. Such non-pharmaceutically acceptable salts forms, which may be useful, for example, in the purification or separation of the compounds of the invention, also form part of the invention.

Particular salts for use in the preparation of liquid (e.g. aqueous) compositions of the compounds of formulae (I) and sub-groups and examples thereof as described herein are salts having a solubility in a given liquid carrier (e.g. water) of greater than 25 mg/ml of the liquid carrier (e.g. water), more typically greater than 50 mg/ml and preferably greater than 100 mg/ml.

In one embodiment of the invention, the compound of the formula (II) as defined herein is provided in the form of a pharmaceutical composition comprising an aqueous solution containing the said compound in the form of a salt in a concentration of greater than 25 mg/ml, typically greater than 50 mg/ml and preferably greater than 100 mg/ml.

Compounds of the formula (II) containing an amine function may also form N-oxides. A reference herein to a compound of the formula (II) that contains an amine function also includes the N-oxide.

Where a compound contains several amine functions, one or more than one nitrogen atom may be oxidised to form an N-oxide. Particular examples of N-oxides are the N-oxides of a tertiary amine or a nitrogen atom of a nitrogen-containing heterocycle.

N-Oxides can be formed by treatment of the corresponding amine with an oxidizing agent such as hydrogen peroxide or a per-acid (e.g. a peroxycarboxylic acid), see for example Advanced Organic Chemistry, by Jerry March, 4th Edition, Wiley Interscience, pages. More particularly, N-oxides can be made by the procedure of L. W. Deady (Syn. Comm. 1977, 7, 509-514) in which the amine compound is reacted with m-chloroperoxybenzoic acid (MCPBA), for example, in an inert solvent such as dichloromethane.

Compounds of the formula (II) may exist in a number of different geometric isomeric, and tautomeric forms and references to compounds of the formula (II) include all such forms. For the avoidance of doubt, where a compound can exist in one of several geometric isomeric or tautomeric forms and only one is specifically described or shown, all others are nevertheless embraced by formula (II).

For example, in compounds of the formula (II) the pyrazole group may take either of the following two tautomeric forms A and B (where the moiety "X" is R¹-C(=O)-. For simplicity, the general formula (II) illustrates form A but the formula is to be taken as embracing both tautomeric forms.

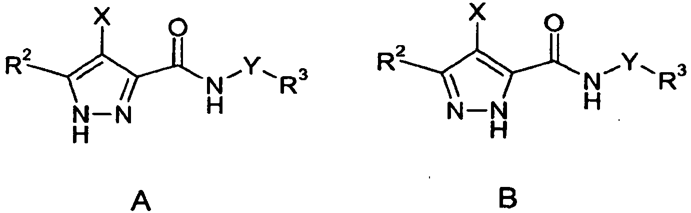

Other examples of tautomeric forms include, for example, keto-, enol-, and enolate-forms, as in, for example, the following tautomeric pairs: keto/enol (illustrated below), imine/enamine, amide/imino alcohol, amidine/amidine, nitroso/oxime, thioketone/enethiol, and nitro/aci-nitro.

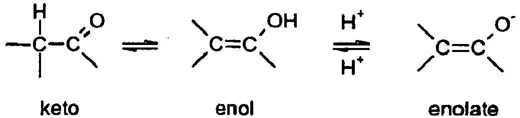

Where compounds of the formula (II) contain one or more chiral centres, and can exist in the form of two or more optical isomers, references to compounds of the formula (II) include all optical isomeric forms thereof (e.g. enantiomers, epimers and diastereoisomers), either as individual optical isomers, or mixtures (e.g. racemic mixtures) or two or more optical isomers, unless the context requires otherwise.

The optical isomers may be characterised and identified by their optical activity (i.e. as + and - isomers, or d and I isomers) or they may be characterised in terms of their absolute stereochemistry using the "R and S" nomenclature developed by Cahn, Ingold and Prelog, see Advanced Organic Chemistry by Jerry March, 4th Edition, John Wiley & Sons, New York, 1992, pages 109-114, and see also Cahn, Ingold & Prelog, Angew. Chem. Int. Ed. Engl., 1966, 5, 385-415.

Optical isomers can be separated by a number of techniques including chiral chromatography (chromatography on a chiral support) and such techniques are well known to the person skilled in the art.

As an alternative to chiral chromatography, optical isomers can be separated by forming diastereoisomeric salts with chiral acids such as (+)-tartaric acid, (-)-pyroglutamic acid, (-)-di-toluoyl-L-tartaric acid, (+)-mandelic acid, (-)-malic acid, and (-)-camphorsulphonic, separating the diastereoisomers by preferential crystallisation, and then dissociating the salts to give the individual enantiomer of the free base.

Where compounds of the formula (II) exist as two or more optical isomeric forms, one enantiomer in a pair of enantiomers may exhibit advantages over the other enantiomer, for example, in terms of biological activity. Thus, in certain circumstances, it may be desirable to use as a therapeutic agent only one of a pair of enantiomers, or only one of a plurality of diastereoisomers. Accordingly, the invention provides compositions containing a compound of the formula (II) having one or more chiral centres, wherein at least 55% (e.g. at least 60%, 65%, 70%, 75%, 80%, 85%, 90% or 95%) of the compound of the formula (II) is present as a single optical isomer (e.g. enantiomer or diastereoisomer). In one general embodiment, 99% or more (e.g. substantially all) of the total amount of the compound of the formula (II) may be present as a single optical isomer (e.g. enantiomer or diastereoisomer).

The compounds of the invention include compounds with one or more isotopic substitutions, and a reference to a particular element includes within its scope all isotopes of the element. For example, a reference to hydrogen includes within its scope ¹H, ²H (D), and ³H (T). Similarly, references to carbon and oxygen include within their scope respectively ¹²C, ¹³C and ¹⁴C and ¹⁶O and ¹⁸O.

The isotopes may be radioactive or non-radioactive. In one embodiment of the invention, the compounds contain no radioactive isotopes. Such compounds are preferred for therapeutic use. In another embodiment, however, the compound may contain one or more radioisotopes. Compounds containing such radioisotopes may be useful in a diagnostic context.

Esters such as carboxylic acid esters and acyloxy esters of the compounds of formula (II) bearing a carboxylic acid group or a hydroxyl group are also embraced by Formula (II). Examples of esters are compounds containing the group -C(=O)OR, wherein R is an ester substituent, for example, a C₁₋₇ alkyl group, a C₃₋₂₀ heterocyclyl group, oraC₅₋₂₀ aryl group, preferably a C₁₋₇ alkyl group. Particular examples of ester groups include, but are not limited to, -C(=O)OCH₃, -C(=O)OCH₂CH₃, -C(=O)OC(CH₃)₃, and -(=O)OPh. Examples of acyloxy (reverse ester) groups are represented by -OC(=O)R, wherein R is an acyloxy substituent, for example, a C₁₋₇ alkyl group, a C₃₋₂₀ heterocyclyl group, or a C₅₋₂₀ aryl group, preferably a C₁₋₇ alkyl group. Particular examples of acyloxy groups include, but are not limited to, -OC(=O)CH₃ (acetoxy), -OC(=O)CH₂CH₃, -OC(=O)C(CH₃)₃, -OC(=O)Ph, and -OC(=O)CH₂Ph.

Also encompassed by formula (II) are any polymorphic forms of the compounds, solvates (e.g. hydrates), complexes (e.g. inclusion complexes or clathrates with compounds such as cyclodextrins, or complexes with metals) of the compounds, and pro-drugs of the compounds. By "prodrugs" is meant for example any compound that is converted in vivo into a biologically active compound of the formula (II).

For example, some prodrugs are esters of the active compound (e.g., a physiologically acceptable metabolically labile ester). During metabolism, the ester group (-C(=O)OR) is cleaved to yield the active drug. Such esters may be formed by esterification, for example, of any of the carboxylic acid groups (-C(=O)OH) in the parent compound, with, where appropriate, prior protection of any other reactive groups present in the parent compound, followed by deprotection if required.

Examples of such metabolically labile esters include those of the formula -C(=O)OR wherein R is:
C₁₋₇alkyl
(e.g., -Me, -Et, -nPr, -iPr, -nBu, -sBu, -iBu, -tBu);
C₁₋₇aminoalkyl
(e.g., aminoethyl; 2-(N,N-diethylamino)ethyl; 2-(4-morpholino)ethyl); and
acyloxy-C₁₋₇alkyl
(e.g., acyloxymethyl;
acyloxyethyl;
pivaloyloxymethyl;
acetoxymethyl;
1-acetoxyethyl;
1-(1-methoxy-1-methyl)ethyl-carbonxyloxyethyl;
1-(benzoyloxy)ethyl; isopropoxy-carbonyloxymethyl;
1-isopropoxy-carbonyloxyethyl; cyclohexyl-carbonyloxymethyl;
1-cyclohexyl-carbonyloxyethyl;
cyclohexyloxy-carbonyloxymethyl;
1-cyclohexyloxy-carbonyloxyethyl;
(4-tetrahydropyranyloxy) carbonyloxymethyl;
1-(4-tetrahydropyranyloxy)carbonyloxyethyl;
(4-tetrahydropyranyl)carbonyloxymethyl; and
1-(4-tetrahydropyranyl)carbonyloxyethyl).

Also, some prodrugs are activated enzymatically to yield the active compound, or a compound which, upon further chemical reaction, yields the active compound (for example, as in ADEPT, GDEPT, LIDEPT, etc.). For example, the prodrug may be a sugar derivative or other glycoside conjugate, or may be an amino acid ester derivative.

### Methanesulphonic acid and acetic acid addition salts of compound 4-(2,6-dichloro-benzoylamino)-1H-pyrazole-3-carboxylic acid piperidin-4-ylamide

The combinations of the invention may comprise any of the compounds, salts, solvates, tautomers and isotopes thereof and, where the context admits, N-oxides, other ionic forms and prodrugs, as described below.

References to the compound 4-(2,6-dichloro-benzoylamino)-1H-pyrazole-3-carboxylic acid piperidin-4-ylamide and its acid addition salts include within their scope all solvates, tautomers and isotopes thereof and, where the context admits, N-oxides, other ionic forms and prodrugs.

The acid addition salt may be selected from salts formed with an acid selected from the group consisting of acetic, adipic, alginic, ascorbic (e.g. L-ascorbic), aspartic (e.g. L-aspartic), benzenesulphonic, benzoic, camphoric (e.g. (+) camphoric), capric, caprylic, carbonic, citric, cyclamic, dodecanoate, dodecylsulphuric, ethane-1,2-disulphonic, ethanesulphonic, fumaric, galactaric, gentisic, glucoheptonic, D-gluconic, glucuronic (e.g. D-glucuronic), glutamic (e.g. L-glutamic), α-oxoglutaric, glycolic, hippuric, isethionic, isobutyric, lactic (e.g. (+)-L-lactic and (±)-DL-lactic), lactobionic, laurylsulphonic, maleic, malic, (-)-L-malic, malonic, methanesulphonic, mucic, naphthalenesulphonic (e.g. naphthalene-2-sulphonic), naphthalene-1,5-disulphonic, nicotinic, oleic, orotic, oxalic, palmitic, pamoic, phosphoric, propionic, sebacic, stearic, succinic, sulphuric, tartaric (e.g. (+)-L-tartaric), thiocyanic, toluenesulphonic (e.g. *p*-toluenesulphonic), valeric and xinafoic acids.

One sub-group of acid addition salts includes salts formed with an acid selected from the group consisting of acetic, adipic, ascorbic (e.g. L-ascorbic), aspartic (e.g. L-aspartic), caproic, carbonic, citric, dodecanoic, fumaric, galactaric, glucoheptonic, gluconic (e.g. D-gluconic), glucuronic (e.g. D-glucuronic), glutamic (e.g. L-glutamic), glycolic, hippuric, lactic (e.g. (+)-L-lactic and (±)-DL-lactic), maleic, palmitic, phosphoric, sebacic, stearic, succinic, sulphuric, tartaric (e.g. (+)-L-tartaric) and thiocyanic acids.

More particularly the salts are acid addition salts formed with an acid selected from methanesulphonic acid and acetic acid, and mixtures thereof.

In one embodiment, the salt is an acid addition salt formed with methanesulphonic acid.

In another embodiment, the salt is an acid addition salt formed with acetic acid.

For convenience the salts formed from methanesulphonic acid and acetic acid may be referred to herein as the methanesulphonate or mesylate salts and acetate salts respectively.

In the solid state, the salts can be crystalline or amorphous or a mixture thereof.

In one embodiment, the salts are amorphous.

In an amorphous solid, the three dimensional structure that normally exists in a crystalline form does not exist and the positions of the molecules relative to one another in the amorphous form are essentially random, see for example Hancock et al. J. Pharm. Sci. (1997), 86, 1).

In another embodiment, the salts are substantially crystalline; i.e. they are from 50% to 100% crystalline, and more particularly they may be at least 50% crystalline, or at least 60% crystalline, or at least 70% crystalline, or at least 80% crystalline, or at least 90% crystalline, or at least 95% crystalline, or at least 98% crystalline, or at least 99% crystalline, or at least 99.5% crystalline, or at least 99.9% crystalline, for example 100% crystalline.

In a further embodiment, the salts are selected from the group consisting of salts that are from 50% to 100% crystalline, salts that are at least 50% crystalline, salts that are at least 60% crystalline, salts that are at least 70% crystalline, salts that are at least 80% crystalline, salts that are at least 90% crystalline, salts that are at least 95% crystalline, salts that are at least 98% crystalline, salts that are at least 99% crystalline, salts that are at least 99.5% crystalline, and salts that are at least 99.9% crystalline, for example 100% crystalline.

More preferably the salts may be those (or may be selected from the group consisting of those) that are 95% to 100 % crystalline, for example at least 98% crystalline, or at least 99% crystalline, or at least 99.5% crystalline, or at least 99.6% crystalline or at least 99.7% crystalline or at least 99.8% crystalline or at least 99.9% crystalline, for example 100% crystalline.

One example of a substantially crystalline salt is a crystalline salt formed with methanesulphonic acid.

Another example of a substantially crystalline salt is a crystalline salt formed with acetic acid.

The salts, in the solid state, can be solvated (e.g. hydrated) or non-solvated (e.g. anhydrous).

In one embodiment, the salts are non-solvated (e.g. anhydrous). An example of a non-solvated salt is the crystalline salt formed with methanesulphonic acid as defined herein.

The term "anhydrous" as used herein does not exclude the possibility of the presence of some water on or in the salt (e.g a crystal of the salt). For example, there may be some water present on the surface of the salt (e.g. salt crystal), or minor amounts within the body of the salt (e.g. crystal). Typically, an anhydrous form contains fewer than 0.4 molecules of water per molecule of compound, and more preferably contains fewer than 0.1 molecules of water per molecule of compound, for example 0 molecules of water.

In another embodiment, the salts are solvated. Where the salts are hydrated, they can contain, for example, up to three molecules of water of crystallisation, more usually up to two molecules of water, e.g. one molecule of water or two molecules of water. Non-stoichiometric hydrates may also be formed in which the number of molecules of water present is less than one or is otherwise a non-integer. For example, where there is less than one molecule of water present, there may be for example 0.4, or 0.5, or 0.6, or 0.7, or 0.8, or 0.9 molecules of water present per molecule of compound.

Other solvates include alcoholates such as ethanolates and isopropanolates.

The salts can be synthesized from the parent compound 4-(2,6-dichloro-benzoylamino)-1H-pyrazole-3-carboxylic acid piperidin-4-ylamide by conventional chemical methods such as methods described in Pharmaceutical Salts: Properties, Selection, and Use, P. Heinrich Stahl (Editor), Camille G. Wermuth (Editor), ISBN: 3-90639-026-8, Hardcover, 388 pages, August 2002. Generally, such salts can be prepared by reacting the parent compound 4-(2,6-dichloro-benzoylamino)-1H-pyrazole-3-carboxylic acid piperidin-4-ylamide with the appropriate acid in water or in an organic solvent, or in a mixture of the two; generally, nonaqueous media such as ether, ethyl acetate, ethanol, isopropanol, or acetonitrile are used.

One method of preparing an acid addition salt of 4-(2,6-dichloro-benzoylamino)-1H-pyrazole-3-carboxylic acid piperidin-4-ylamide comprises forming a solution of 4-(2,6-dichloro-benzoylamino)-1H-pyrazole-3-carboxylic acid piperidin-4-ylamide free base in a solvent (typically an organic solvent) or mixture of solvents, and treating the solution with an acid to form a precipitate of the acid addition salt.

The acid may be added as a solution in a solvent which is miscible with the solvent in which the free base is dissolved. The solvent in which the free base is initially dissolved may be one in which the acid addition salt thereof is insoluble. Alternatively, the solvent in which the free base is initially dissolved may be one in which the acid addition salt is at least partially soluble, a different solvent in which the acid addition salt is less soluble subsequently being added such that the salt precipitates out of solution.

In an alternative method of forming an acid addition salt, 4-(2,6-dichloro-benzoylamino-1H-pyrazole-3-carboxylic acid piperidin-4-ylamide is dissolved in a solvent comprising a volatile acid and optionally a cosolvent, thereby to form a solution of the acid addition salt with the volatile acid, and the resulting solution is then concentrated or evaporated to isolate the salt. An example of an acid addition salt that can be made in this way is the acetate salt.

In another aspect, the combination of the invention includes an acid addition salt of 4-(2,6-dichloro-benzoylamino)-1H-pyrazole-3carboxylic acid piperidin-4-ylamide as defined herein, obtained (or obtainable) by treating a compound of the formula (X): 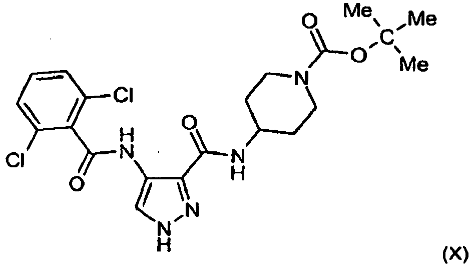 with an organic or inorganic acid as defined herein, other than hydrochloric acid, in an organic solvent to remove the *tert*-butyloxycarbonyl group and form an acid addition salt of 4-(2,6-dichloro-benzoylamino)-1H-pyrazole-3-carboxylic acid piperidin-4-ylamide with the organic or inorganic acid, and optionally isolating the acid addition salt thus formed.

The salt is typically precipitated from the organic solvent as it is formed and hence can be isolated by separation of the solid from the solution, e.g. by filtration.

One salt form can be converted to the free base and optionally to another salt form by methods well known to the skilled person. For example, the free base can be formed by passing the salt solution through a column containing an amine stationary phase (e.g. a Strata-NH₂ column). Alternatively, a solution of the salt in water can be treated with sodium bicarbonate to decompose the salt and precipitate out the free base. The free base may then be combined with another acid by one of the methods described above or elsewhere herein.

The methanesulphonate salt form is particularly advantageous because of its good stability at elevated temperatures and in conditions of high relative humidity, its non-hygroscopicity (as defined herein), absence of polymorph and hydrate formation, and stability in aqueous conditions. Moreover, it has excellent water solubility and has better physiochemical properties (such as a high melting point) relative to other salts.

The term 'stable' or 'stability' as used herein includes chemical stability and solid state (physical) stability. The term 'chemical stability' means that the compound can be stored in an isolated form, or in the form of a formulation in which it is provided in admixture with for example, pharmaceutically acceptable carriers, diluents or adjuvants as described herein, under normal storage conditions, with little or no chemical degradation or decomposition. 'Solid-state stability' means the compound can be stored in an isolated solid form, or the form of a solid formulation In which it is provided in admixture with, for example, pharmaceutically acceptable carriers, diluents or adjuvants as described herein, under normal storage conditions, with little or no solid-state transformation (e.g. hydration, dehydration, solvatisation, desolvatisation, crystallisation, recrystallisation or solid-state phase transition).

The terms "non-hygroscopic" and "non-hygroscopicity" and related terms as used herein refer to substances that absorb less than 5% by weight (relative to their own weight) of water when exposed to conditions of high relative humidity, for example 90% relative humidity, and/or do not undergo changes In crystalline form In conditions of high humidity and/or do not absorb water into the body of the crystal (internal water) in conditions of high relative humidity.

Preferred salts for use in the combinations of the invention are acid addition salts (such as the mesylate and acetate and mixtures thereof as defined herein) having a solubility in a given liquid carrier (e.g. water) of greater than 15 mg/ml of the liquid carrier (e.g. water), more typically greater than 20 mg/ml, preferably greater than 25 mg/ml, and more preferably greater than 30 mg/ml.

In another aspect, there is provided a combination comprising an aqueous solution containing an acid addition salt of 4-(2,6-dichloro-benzoylamino)-1H-pyrazole-3-carboxylic acid piperidin-4-ylamide (such as the mesylate and acetate and mixtures thereof as defined herein, and preferably the mesylate) in a concentration of greater than 15 mg/ml, typically greater than 20 mg/ml, preferably greater than 25 mg/ml, and more preferably greater than 30 mg/ml.

In a preferred embodiment, the combination comprises an aqueous solution containing an acid addition salt of 4-(2,6-dichloro-benzoylamino)-1H-pyrazole-3-carboxylic acid piperidin-4-ylamide selected from an acetate or methanesulphonate salt or a mixture thereof in a concentration of greater than 15 mg/ml, typically greater than 20 mg/ml, preferably greater than 25 mg/ml, and more preferably greater than 30 mg/ml.

In another aspect, the combination of the invention includes an aqueous solution of an acid addition salt of 4-(2,6-dichloro-benzoylamino)-1H-pyrazole-3-carboxylic acid piperidin-4-ylamide (such as the mesylate and acetate and mixtures thereof as defined herein), wherein the aqueous solution has a pH of 2 to 12, for example 2 to 9, and more particularly 4 to 7.

In the aqueous solutions defined above, the acid addition salt may be any of the salts described herein but, in one preferred embodiment, is a mesylate or acetate salt as defined herein, and in particular the mesylate salt.

The combinations of the invention may include an aqueous solution of 4-(2,6-dichloro-benzoylamino)-1H-pyrazole-3-carboxylic acid piperidin-4-ylamide in protonated form together with one or more counter ions and optionally one or more further counter ions. In one embodiment one of the counter ions is selected from methanesulphonate and acetate. In another embodiment one of the counter ions is from the formulation buffer as described herein such as acetate. In a further embodiment there may be one or more further counter ions such as a chloride ion (e.g. from saline).

The combinations of the invention may include an aqueous solution of 4-(2,6-dichloro-benzoylamino)-1H-pyrazole-3-carboxylic acid piperidin-4-ytamide in protonated form together with one or more counter ions selected from methanesulphonate and acetate and optionally one or more further counter ions such as a chloride ion.

In the situation where there Is more than one counter ion the aqueous solution of 4-(2,6-dichloro-benzoylamino)-1H-pyrazole-3-carboxylic acid piperldin-4-ylamide in protonated form will potentially contain a mixture of counter ions for example a mixture of methanesulphonate and acetate counter ions and optionally one or more further counter ions such as a chloride ion.

The combinations of the invention may include an aqueous solution of 4-(2,6-dichloro-benzoylamino)-1H-pyrazole-3-carboxylic acid piperidin-4-ylamide in protonated form together with one or more counter ions selected from methanesulphonate and acetate and optionally one or more further counter ions such as a chloride ion, and a mixture thereof.

The aqueous solutions can be formed *inter alia* by dissolving a mesylate salt in a solution of acetate ions (e.g an acetate buffer) or by dissolving an acetate salt In a solution of mesylate ions. The mesylate and acetate ions may be present in the solution in a mesylate:acetate ratio of from 10:1 or less, for example 10:1 to 1:10, more preferably less then 8:1, or less than 7:1, or less than 6:1, or less than 5:1 or less than 4:1 or less than 3:1 or less than 2:1 or less than 1:1, more particularly from 1:1 to 1:10. In one embodiment, the mesylate and acetate ions are present in the solution in a mesylate:acetate ratio of from 1:1 to 1:10, for example 1:1 to 1:8, or 1:1 to 1:7 or 1:1 to 1:6 or 1:1 to 1:5, e.g. approximately 1:4.8.

The aqueous solutions of the salts may be buffered or unbuffered but in one embodiment are buffered.

In the context of the acid addition salt formed with methanesulphonic acid, a preferred buffer is a buffer formed from acetic acid and sodium acetate, for example at a solution pH of approximately 4.6. At this pH and in the acetate buffer, the methanesulphonic acid salt has a solubility of about 35 mg/ml.

The salts for use in the combinations of the invention are typically pharmaceutically acceptable salts, and examples of pharmaceutically acceptable salts are discussed in Berge et a/., 1977, "Pharmaceutically Acceptable Salts," J. Pharm. Sci., Vol. 66, pp. 1-19. However, salts that are not pharmaceutically acceptable may also be prepared as intermediate forms which may then be converted into pharmaceutically acceptable salts. Such non-pharmaceutically acceptable salt forms therefore also form part of the invention.

### Biological Activity

The ancillary agents of the combinations of the invention have activity against various cancers.

The compounds of the formulae (II), (IV), (IVa), (Va), (Vb), (Via) or (VIb) and sub-groups thereof are inhibitors or modulators (in particular inhibitors) of one or more cyclin dependent kinases and/or glycogen synthase kinases, and in particular one or more cyclin dependent kinases selected from CDK1, CDK2, CDK3, CDK4, CDK5, CDK6 and CDK9, and more particularly selected from CDK1, CDK2, CDK3, CDK4, CDK5 and CDK9.

Preferred compounds of the formulae (II), (IV), (IVa), (Va), (Vb), (Via) or (VIb) and sub-groups thereof are compounds that inhibit one or more CDK kinases selected from CDK1, CDK2, CDK4 and CDK9, for example CDK1 and/or CDK2.The compounds of the formulae (II), (IV), (IVa), (Va), (Vb), (Via) or (VIb) and sub-groups thereof may modulate or inhibit GSKs such as glycogen synthase kinase-3 (GSK3).

As a consequence of their activity in modulating or inhibiting CDK kinases and/or glycogen synthase kinases, and the acivity of the ancillary agents described herein, the combinations of the invention are expected to be useful in providing a means of arresting, or recovering control of, the cell cycle in abnormally dividing cells. It is therefore anticipated that the compounds will prove useful in treating or preventing proliferative disorders such as cancers.

CDKs play a role in the regulation of the cell cycle, apoptosis, transcription, differentiation and CNS function. Therefore, CDK inhibitors could be useful in the treatment of diseases in which there is a disorder of proliferation, apoptosis or differentiation such as cancer. In particular RB+ve tumours may be particularly sensitive to CDK inhibitors. RB-ve tumours may also be sensitive to CDK inhibitors.

Examples of cancers which may be inhibited include, but are not limited to, a carcinoma, for example a carcinoma of the bladder, breast, colon (e.g. colorectal carcinomas such as colon adenocarcinoma and colon adenoma), kidney, epidermis, liver, lung, for example adenocarcinoma, small cell lung cancer and non-small cell lung carcinomas, oesophagus, gall bladder, ovary, pancreas e.g. exocrine pancreatic carcinoma, stomach, cervix, thyroid, prostate, or skin, for example squamous cell carcinoma; a hematopoietic tumour of lymphoid lineage, for example leukemia, acute lymphocytic leukemia, B-cell lymphoma, T-cell lymphoma, Hodgkin's lymphoma, non-Hodgkin's lymphoma, hairy cell lymphoma, or Burkett's lymphoma; a hematopoietic tumour of myeloid lineage, for example acute and chronic myelogenous leukemias, myelodysplastic syndrome, or promyelocytic leukemia; thyroid follicular cancer; a tumour of mesenchymal origin, for example fibrosarcoma or habdomyosarcoma, a tumour of the central or peripheral nervous system, for example astrocytoma, neuroblastoma, glioma or schwannoma; melanoma; seminoma; teratocarcinoma; osteosarcoma; xeroderma pigmentosum; keratoctanthoma; thyroid follicular cancer, Kaposi's sarcoma, B-cell lymphoma and chronic lymphocytic leukaemia.

The cancers may be cancers which are sensitive to inhibition of any one or more cyclin dependent kinases selected from CDK1, CDK2, CDK3, CDK4, CDK5 and CDK6, for example, one or more CDK kinases selected from CDK1, CDK2, CDK4 and CDK5, e.g. CDK1 and/or CDK2.

Whether or not a particular cancer is one which is sensitive to inhibition by a cyclin dependent kinase may be determined by means of a cell growth assay as set out in the examples below or by a method as set out in the section headed "Methods of Diagnosis".

Thus, in the pharmaceutical compositions, uses or methods of this invention for treating a disease or condition comprising abnormal cell growth, the disease or condition comprising abnormal cell growth in one embodiment is a cancer.

One group of cancers includes human breast cancers (e.g. primary breast tumours, node-negative breast cancer, invasive duct adenocarcinomas of the breast, non-endometrioid breast cancers); and mantle cell lymphomas. In addition, other cancers are colorectal and endometrial cancers.

Another sub-set of cancers includes breast cancer, ovarian cancer, colon cancer, prostate cancer, oesophageal cancer, squamous cancer and non-small cell lung carcinomas.

A further sub-set of cancers includes non small cell lung cancer, colon cancer, breast cancer, non-hodgkin's lymphoma, multiple myeloma and chromic lymphocytic leukemia.

A yet further sub-set of cancers includes breast cancer, colorectal cancer, ovarian cancer and non-small cell lung carcinoma.

A yet further sub-set of cancers includes colorectal cancer, ovarian cancer and non-small cell lung carcinoma.

Another sub-set of cancers includes hematopoietic tumours of lymphoid lineage, for example leukemia, chronic lymphocytic leukaemia, mantle cell lymphoma and B-cell lymphoma (such as diffuse large B cell lymphoma).

One particular cancer is chronic lymphocytic leukaemia.

Another particular cancer is mantle cell lymphoma.

Another particular cancer is diffuse large B cell lymphoma

The activity of the compounds of the invention as inhibitors or modulators of cyclin dependent kinases and/or glycogen synthase kinases (e.g. GSK-3) can be measured using the assays set forth in the examples below and the level of activity exhibited by a given compound can be defined in terms of the IC₅₀ value. Preferred compounds of the present invention are compounds having an IC₅₀ value of less than 1 micromole, more preferably less than 0.1 micromole.

### Methods for the Preparation of Compounds of Formula (II) of the Invention

Compounds of the formula (II) and the various sub-groups thereof can be prepared in accordance with synthetic methods well known to the skilled person. Unless stated otherwise, R¹, R², R³ and Y are as hereinbefore defined.

In this section, as in all the other sections of this application, references to formula (II) should be taken to refer also to formulae (IV), (IVa), (Va), (Vb), (Via) and (VIb) and sub-groups thereof unless the context indicates otherwise.

Compounds of the formula (II) can be prepared by reacting a carboxylic acid of the formula R¹-CO₂H or an activated derivative thereof with an appropriately substituted 4-amino-pyrazole as shown in Scheme 1.

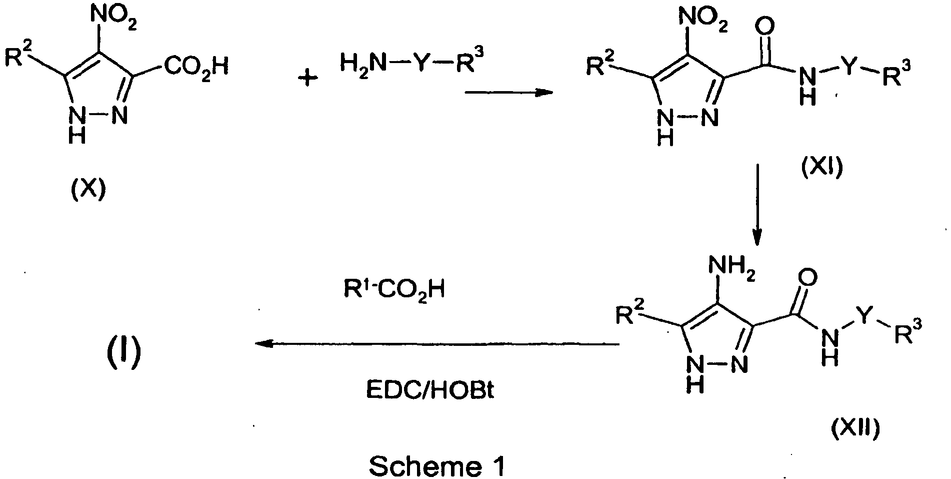

The starting material for the synthetic route shown in Scheme 1 is the 4-nitro-pyrazole-3-carboxylic acid (X) which can either be obtained commercially or can be prepared by nitration of the corresponding 4-unsubstituted pyrazole carboxy compound.

The 4-nitro-pyrazole carboxylic acid (X), or a reactive derivative thereof, is reacted with the amine H₂N-Y-R³ to give the 4-nitro-amide (XI). The coupling reaction between the carboxylic acid (X) and the amine is preferably carried out in the presence of a reagent of the type commonly used in the formation of peptide linkages. Examples of such reagents include 1,3-dicyclohexylcarbodiimide (DCC) (Sheehan et al, J. Amer. Chem Soc. 1955, 77, 1067), 1-ethyl-3-(3'-dimethylaminopropyl)-carbodiimide (referred to herein either as EDC or EDAC but also known in the art as EDCI and WSCDI) (Sheehan et al, J. Org. Chem., 1961, 26, 2525), uronium-based coupling agents such as O-(7-azabenzotriazol-1-yl)-N,N,N',N'-tetramethyluronium hexafluorophosphate (HATU) and phosphonium-based coupling agents such as 1-benzo-triazolyloxytris-(pyrrolidino)phosphonium hexafluorophosphate (PyBOP) (Castro et al, Tetrahedron Letters, 1990, 31, 205). Carbodiimide-based coupling agents are advantageously used in combination with 1-hydroxy-7-azabenzotriazole (HOAt) (L. A. Carpino, J. Amer. Chem. Soc., 1993, 115, 4397) or 1-hydroxybenzotriazole (HOBt) (Konig et al, Chem. Ber., 103, 708, 2024-2034). Preferred coupling reagents include EDC (EDAC) and DCC in combination with HOAt or HOBt.

The coupling reaction is typically carried out in a non-aqueous, non-protic solvent such as acetonitrile, dioxan, dimethylsulphoxlde, dichloromethane, dimethylformamide or N-methylpyrrolidine, or in an aqueous solvent optionally together with one or more miscible co-solvents. The reaction can be carried out at room temperature or, where the reactants are less reactive (for example in the case of electron-poor anilines bearing electron withdrawing groups such as sulphonamide groups) at an appropriately elevated temperature. The reaction may be carried out in the presence of a non-interfering base, for example a tertiary amine such as triethylamine or N,N-diisopropylethylamine.

As an alternative, a reactive derivative of the carboxylic acid, e.g. an anhydride or acid chloride, may be used. Reaction with a reactive derivative such an anhydride is typically accomplished by stirring the amine and anhydride at room temperature in the presence of a base such as pyridine.

Amines of the formula H₂N-Y-R³ can be obtained from commercial sources or can be prepared by any of a large number of standard synthetic methods well known those skilled in the art, see for example see Advanced Organic Chemistry by Jerry March, 4th Edition, John Wiley & Sons, 1992, and and Organic Syntheses, Volumes 1-8, John Wiley, edited by Jeremiah P. Freeman (ISBN: 0-471-31192-8), 1995, and see also the methods described in the experimental section below.

The nitro-pyrazole amide (XI) is reduced to give the corresponding 4-amino-compound of the formula (XII). The reduction may be carried out by standard methods such as catalytic hydrogenation, for example in the presence of palladium on carbon in a polar solvent such as ethanol or dimethylformamide at room temperature. As an alternative, reduction may be effected using a reducing agent such as tin (II) chloride in ethanol, typically with heating, for example to the reflux temperature of the solvent.

The 4-amino-pyrazole compound (XII) is then reacted with a carboxylic acid of the formula R¹-CO₂H, or a reactive derivative thereof, using the methods and conditions described above for the formation of the amide (XI), to give a compound of the formula (II).

Carboxylic acids of the formula R¹-CO₂H can be obtained commercially or can be synthesised according to methods well known to the skilled person, see for example Advanced Organic Chemistry and Organic Syntheses, the details for which are given above.

In an alternative synthetic route, compounds of the formula (II) can be prepared by reaction of a compound of the formula (XIII), wherein X is R¹-C(=O)-NH-, with a compound of the formula R³-Y-NH₂. The reaction can be carried out using the amide coupling conditions described above.

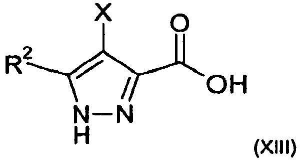

Once formed, one compound of the formula (II) may be transformed into another compound of the formula (II) using standard chemistry procedures well known in the art. For examples of functional group interconversions, see for example, Fiesers' Reagents for Organic Synthesis, Volumes 1-17, John Wiley, edited by Mary Fieser (ISBN: O-471-58283-2), and Organic Syntheses, Volumes 1-8, John Wiley, edited by Jeremiah P. Freeman (ISBN: 0-471-31192-8), 1995.

The starting materials for the synthetic routes shown in the Schemes above, e.g. the pyrazoles of formula (X), can either be obtained commercially or can be prepared by methods known to those skilled in the art. They can be obtained using known methods e.g. from ketones, such as in a process described in EP308020 (Merck), or the methods discussed by Schmidt in Helv. Chim. Acta., 1956, 39, 986-991 and Helv. Chim. Acta., 1958, 41, 306-309. Alternatively they can be obtained by conversion of a commercially available pyrazole, for example those containing halogen, nitro, ester, or amide functionalities, to pyrazoles containing the desired functionality by standard methods known to a person skilled in the art. For example, in 3-carboxy-4-nitropyrazole, the nitro group can be reduced to an amine by standard methods. 4-Nitro-pyrazole-3-carboxylic acid (XII) can either be obtained commercially or can be prepared by nitration of the corresponding 4-unsubstituted pyrazole carboxy compound, and pyrazoles containing a halogen, may be utilized in coupling reactions with tin or palladium chemistry.

### Protecting Groups

In many of the reactions described above, it may be necessary to protect one or more groups to prevent reaction from taking place at an undesirable location on the molecule. Examples of protecting groups, and methods of protecting and deprotecting functional groups, can be found in Protective Groups in Organic Synthesis (T. Green and P. Wuts; 3rd Edition; John Wiley and Sons, 1999).

A hydroxy group may be protected, for example, as an ether (-OR) or an ester (-OC(=O)R), for example, as: a t-butyl ether, a tetrahydropyranyl (THP) ether, a benzyl, benzhydryl (diphenylmethyl), or trityl (triphenylmethyl) ether, a trimethylsilyl or t-butyldimethylsilyl ether; or an acetyl ester (-OC(=O)CH₃, OAc).

An aldehyde or ketone group may be protected, for example, as an acetal (R-CH(OR)₂) or ketal (R₂C(OR)₂), respectively, in which the carbonyl group (>C=O) is converted to a diether (>C(OR)₂), by reaction with, for example, a primary alcohol. The aldehyde or ketone group Is readily regenerated by hydrolysis using a large excess of water in the presence of acid.

An amine group may be protected, for example, as an amide (-NRCO-R) or a urethane (-NRCO-OR), for example, as: a methyl amide (-NHCO-CH₃); a benzyloxy amide (-NHCO-OCH₂C₆H₅, -NH-Cbz or NH-Z); as a t-butoxy amide (-NHCO-OC(CH₃)₃, -NH-Boc); a 2-biphenyl-2-propoxy amide (-NHCO-OC(CH₃)₂C₆H₄C₆H₅, -NH-Bpoc), as a 9-fluorenylmethoxy amide (-NH-Fmoc), as a 6-nitroveratryloxy amide (-NH-Nvoc), as a 2-trimethylsilylethyloxy amide (-NH-Teoc), as a 2,2,2-trichloroethyloxy amide (-NH-Troc), as an allyloxy amide (-NH-Alloc), or as a 2(-phenylsulphonyl)ethyloxy amide (-NH-Psec).

For example, in Scheme 1 above, when the moiety R³ in the amine H₂N-Y-R³ contains a second amino group, such as a cyclic amino group (e.g. a piperidine or pyrrolidine group), the second amino group can be protected by means of a protecting group as hereinbefore defined, one preferred group being the tert-butyloxycarbonyl (Boc) group. Where no subsequent modification of the second amino group is required, the protecting group can be carried through the reaction sequence to give an N-protected form of a compound of the formula (II) which can then be de-protected by standard methods (e.g. treatment with acid in the case of the Boc group) to give the compound of formula (II).

Other protecting groups for amines, such as cyclic amines and heterocyclic N-H groups, include toluenesulphonyl (tosyl) and methanesulphonyl (mesyl) groups, benzyl groups such as a para-methoxybenzyl (PMB) group and tetrahydropyranyl (THP) groups.

A carboxylic acid group may be protected as an ester for example, as: an C₁₋₇ alkyl ester (e.g., a methyl ester; a t-butyl ester); a C₁₋₇ haloalkyl ester (e.g., a C₁₋₇ trihaloalkyl ester); a triC₁₋₇ alkylsilyl-C₁-₇alkyJ ester; or a C₅₋₂₀ aryl-C₁₋₇ alkyl ester (e.g., a benzyl ester, a nitrobenzyl ester); or as an amide, for example, as a methyl amide. A thiol group may be protected, for example, as a thioether (-SR), for example, as: a benzyl thioether; an acetamidomethyl ether (-S-CH₂NHC(=O)CH₃).

### Isolation and purification of the compounds of the invention

The compounds of the invention can be isolated and purified according to standard techniques well known to the person skilled in the art. One technique of particular usefulness in purifying the compounds is preparative liquid chromatography using mass spectrometry as a means of detecting the purified compounds emerging from the chromatography column.

Preparative LC-MS is a standard and effective method used for the purification of small organic molecules such as the compounds described herein. The methods for the liquid chromatography (LC) and mass spectrometry (MS) can be varied to provide better separation of the crude materials and improved detection of the samples by MS. Optimisation of the preparative gradient LC method will involve varying columns, volatile eluents and modifiers, and gradients. Methods are well known in the art for optimising preparative LC-MS methods and then using them to purify compounds. Such methods are described in Rosentreter U, Huber U.; Optimal fraction collecting in preparative LC/MS; J Comb Chem.; 2004; 6(2), 159-64 and Leister W, Strauss K, Wisnoski D, Zhao Z, Lindsley C., Development of a custom high-throughput preparative liquid chromatography/mass spectrometer platform for the preparative purification and analytical analysis of compound libraries; J Comb Chem.; 2003; 5(3); 322-9.

An example of such a system for purifying compounds via preparative LC-MS is described below in the Examples section of this application (under the heading "Mass Directed Purification LC-MS System"). However, it will be appreciated that alternative systems and methods to those described could be used. In particular, normal phase preparative LC based methods might be used in place of the reverse phase methods described here. Most preparative LC-MS systems utilise reverse phase LC and volatile acidic modifiers, since the approach is very effective for the purification of small molecules and because the eluents are compatible with positive ion electrospray mass spectrometry. Employing other chromatographic solutions e.g. normal phase LC, alternatively buffered mobile phase, basic modifiers etc as outlined in the analytical methods described below could alternatively be used to purify the compounds.

### Ancillary agents for use according to the invention

The ancillary agent for use in combination with the compounds of the invention are selected from the following classes:
1. hormones, hormone agonists, hormone antagonists and hormone modulating agents (including antiandrogens, antiestrogens and GNRAs);
2. monoclonal antibodies (e.g. to one or more cell surface antigens);
3. alkylating agents selected from nitrogen mustards; nitrosoureas; bifunctional alkylating agents having a bis-alkanesulfonate group; and aziridine compounds; and wherein the alkylating agents are other than mitomycin C or cyclophosphamide;
4. one or more further CDK inhibitors;
5. anticancer agents; and
6. a combination of two or more of the foregoing classes.

Preferred anticancer agents for use in the combinations of the invention may be selected from the following classes:
1. COX-2 inhibitors;
2. HDAC inhibitors;
3. DNA methyltransferase inhibitors;
4. proteasome inhibitors;
5. a combination of two or more of the foregoing classes.

A reference to a particular ancillary agent herein (for example, a reference to a hormone, hormone agonist, hormone antagonist, hormone modulating agent) is intended to Include ionic, salt, solvate, isomers, tautomers, N-oxides, ester, prodrugs, isotopes and protected forms thereof (preferably the salts or tautomers or isomers or N-oxides or solvates thereof, and more preferably, the salts or tautomers or N-oxides or solvates thereof).

### 1. Hormones, hormone agonists, hormone antagonists and hormone modulating agents

In one embodiment of the invention, the ancillary agent is a hormone, hormone agonist, hormone antagonist or hormone modulating agent.

*Definition*: The terms "antiandrogen", "antiestrogen" , "antiandrogen agent" and "antiestrogen agent" as used herein refers to those described herein and analogues thereof, including the ionic, salt, solvate, isomers, tautomers, N-oxides, ester, prodrugs, isotopes and protected forms thereof (preferably the salts or tautomers or isomers or N-oxides or solvates thereof, and more preferably, the salts or tautomers or N-oxides or solvates thereof), as described above.

*Biological activity:* The hormones, hormone agonists, hormone antagonists and hormone modulating agents (including the antiandrogens and antiestrogen agents) working via one or more pharmacological actions as described herein have been identified as suitable anti-cancer agents.

*Technical background*: Hormonal therapy plays an important role in the treatment of certain types of cancer where tumours are formed in tissues that are sensitive to hormonal growth control such as the breast and prostate. Thus, for example, estrogen promotes growth of certain breast cancers and testosterone promotes growth of some prostate cancers. Since the growth of such tumours is dependent on specific hormones, considerable research has been carried out to investigate whether it is possible to affect tumour growth by increasing or decreasing the levels of certain hormones in the body. Hormonal therapy attempts to control tumour growth in these hormone-sensitive tissues by manipulating the activity of the hormones.

With regard to breast cancer, tumour growth is stimulated by estrogen, and antiestrogen agents have therefore been proposed and widely used for the treatment of this type of cancer. One of the most widely used of such agents is tamoxifen which is a competitive inhibitor of estradiol binding to the estrogen receptor (ER). When bound to the ER, tamoxifen induces a change in the three-dimensional shape of the receptor, inhibiting its binding to the estrogen responsive element on DNA. Under normal physiological conditions, estrogen stimulation increases tumour cell production of transforming growth cell b (TGF-b), an autocrine inhibitor of tumour cell growth. By blocking these pathways, the net effect of tamoxifen treatment is to decrease the autocrine stimulation of breast cancer growth. In addition, tamoxifen decreases the local production of insulin-like growth factor (IGF-1) by surrounding tissues: IGF- I is a paracrine growth factor for the breast cancer cell (Jordan and Murphy, Endocr. Rev., 1990, 1 1; 578-610). Tamoxifen is the endocrine treatment of choice for post-menopausal women with metastatic breast cancer or at a high risk of recurrences from the disease. Tamoxifen is also used in pre-menopausal women with ER-positive tumours. There are various potential side-effects of long-term tamoxifen treatment, for example the possibility of endometrial cancer and the occurrence of thrombo-embolic events.

Other estrogen receptor antagonists or selective estrogen receptor modulators (SERMs) include fulvestrant, toremifene and raloxifene. Fulvestrant which has the chemical name 7-α-[9-(4,4,5,5,5-pentafluoropentylsulphinyl)-nonyl] estra-1,3,5-(10)- triene-3,17-beta-diol, is used as a second line treatment of advanced breast cancer but side-effects include hot flushes and endometrial stimulation. Toremifene is a non-steroidal SERM, which has the chemical name 2-(4-[(Z)-4-chloro-1,2-diphenyl-1-butenyl]-phenoxy)- N,N-dimethylethylamine, and is used for the treatment of metastatic breast cancer, side-effects including hot flushes, nausea and dizziness. Raloxifene is a benzothiophene SERM, which has the chemical name [6-hydroxy-2-(4-hydroxyphenyl)benzo[*b*]thien-3-yl]-[4-[2-(1-piperidinyl)ethoxy]-phenyl]-methanone hydrochloride, and is being investigated for the treatment of breast cancer, side-effects including hot flushes and leg cramps.

With regard to prostate cancer, such cancer cells have a high level of expression of androgen receptor, and antiandrogens have therefore been used to treat the disease. Antiandrogens are androgen receptor antagonists which bind to the androgen receptor and prevent dihydrotestosterone from binding. Dihydrotestosterone stimulates new growth of prostate cells, including cancerous prostate cells. An example of an antiadrogen is bicalutamide, which has the chemical name (R,S)-N-(4-cyano-3-(4-fluorophenylsulfonyl)-2-hydroxy-2-methyl-3(trifluoromethyl)propanamide, and has been approved for use In combination with luteinizing hormone-releasing hormone (LHRH) analogs for the treatment of advanced prostate cancer, side effects including hot flushes, bone pain, hematuria and gastro-intestinal symptoms.

A further type of hormonal cancer treatment comprises the use of progestin analogs. Progestin is the synthetic form of progesterone. Progesterone is a hormone secreted by the ovaries and endometrial lining of the uterus. Acting with estrogen, progesterone promotes breast development and growth of endometrial cells during the menstrual cycle. It is believed that progestins may act by suppressing the production of estrogen from the adrenal glands (an alternate source particularly in post-menopausal women), lowering estrogen receptor levels, or altering tumour hormone metabolism.

Progestin analogs are commonly used in the management of advanced uterine cancer. They can also be used for treating advanced breast cancer, although this use is less common, due to the numerous anti-estrogen treatment options available. Occasionally, progestin analogs are used as hormonal therapy for prostate cancer. An example of a progestin analog is megestrol acetate (a.k.a. megestrel acetate), which has the chemical name 17α-acetyloxy-6-methylpregna-4,6-diene-3, 20-dione, and is a putative inhibitor of pituitary gonadotrophin production with a resultant decrease in estrogen secretion, The drug is used for the palliative treatment of advanced carcinoma of the breast or endometrium (i.e., recurrent, inoperable, or metastatic disease), side-effects including oedema and thromoembolic episodes.

*Preferences and specific embodiments*: A particularly preferred antiestrogen agent for use In accordance with the invention is tamoxifen. Tamoxifen is commercially available for example from AstraZeneca plc under the trade name Nolvadex, or may be prepared for example as described in U.K. patent specifications 1064629 and 1354939, or by processes analogous thereto.

Other preferred antiestrogen agents include fulvestrant, raloxifene and toremifene. Yet another preferred antiestrogen agent is droloxifene. Fulvestrant is commercially available for example from AstraZeneca plc under the trade name Faslodex, or may be prepared for example as described in European patent specification No.138504, or by processes analogous thereto. Raloxifene is commercially available for example from Eli Lilly and Company under the trade name Evista, or may be prepared for example as described in U.S. patent specification No. 4418068, or by processes analogous thereto. Toremifene is commercially available for example from Schering Corporation under the trade name Fareston, or may be prepared for example as described in U.S. patent specification No. 4696949, or by processes analogous thereto. The antiestrogen agent droloxifene, which may be prepared for example as described in U.S. patent specification No. 5047431, or by processes analogous thereto, can also be used in accordance with the invention.

A preferred antiandrogen for use in accordance with the invention is bicalutamide which Is commercially available for example from AstraZeneca plc under the trade name Casodex, or may be prepared for example as described in European patent specification No. 100172, or by processes analogous thereto. Other preferred antiandrogens for use in accordance with the Invention include tamoxifen, fulvestrant, raloxifene, toremifene, droloxifene, letrazole, anastrozole, exemestane, bicalutamide, luprolide,megestrol/megestrel acetate, aminoglutethimide and bexarotene.

A preferred progestin analog is megestrol/megestrel acetate which is commercially available for example from Bristol-Myers Squibb Corporation under the trade name Megace, or may be prepared for example as described in US Patent Specification No. 2891079, or by processes analogous thereto.

Thus, specific embodiments of these anti-cancer agents for use in the combinations of the invention include: tamoxifen; toremifene; raloxifene; medroxyprogesterone; megestrol/megestrel; aminoglutethimide; letrozole; anastrozole; exemestane; goserelin; leuprolide; abarelix; fluoxymestrone; diethylstilbestrol; ketacanazole; fulvestrant; flutamide; bicalutimide; nilutamide; cyproterone and buserelin.

Thus, contemplated for use in the combinations of the invention are antiandrogens and antiestrogens.

In other embodiments, the hormone, hormone agonist, hormone antagonist or hormone modulating agent Is fulvestrant, raloxifene, droloxifene, toremifene, megestrol/megestrel and bexarotene.

*Posology.* The antiandrogen or antiestrogen agent is advantageously administered in a dosage of about 1 to 100mg daily depending on the particular agent and the condition being treated. Tamoxifen is advantageously administered orally in a dosage of 5 to 50 mg, preferably 10 to 20 mg twice a day, continuing the therapy for sufficient time to achieve and maintain a therapeutic effect.

With regard to the other preferred antiestrogen agents: fulvestrant is advantageously administered in the form of a 250 mg monthly injection; toremifene is advantageously administered orally in a dosage of about 60 mg once a day, continuing the therapy for sufficient time to achieve and maintain a therapeutic effect; droloxifene is advantageously administered orally in a dosage of about 20-100 mg once a day; and raloxifene is advantageously administered orally in a dosage of about 60 mg once a day.

With regard to the preferred antiandrogen bicalutamide, this is generally administered in an oral dosage of 50 mg daily.

With regard to the preferred progestin analog megestrol/megestrel acetate, this is generally administered in an oral dosage of 40 mg four times daily.

The dosages noted above may generally be administered for example once, twice or more per course of treatment, which may be repeated for example every 7,14, 21 or 28 days.

### Aromatase inhibitors

Of the hormones, hormone agonists, hormone antagonists and hormone modulating agents for use in the combinations of the invention, preferred are aromatase inhibitors.

In post-menopausal women, the principal source of circulating estrogen is from conversion of adrenal and ovarian androgens (androstenedione and testosterone) to estrogens (estrone and estradiol) by the aromatase enzyme in peripheral tissues. Estrogen deprivation through aromatase inhibition or inactivation is an effective and selective treatment for some post-menopausal patients with hormone-dependent breast cancer. Examples of such hormone modulating agents include aromatase inhibitors or inactivators, such as exemestane, anastrozole, letrozole and aminoglutethimide.

Exemestane, which has the chemical name 6-methylenandrosta-1,4-diene-3,17-dione, is used for the treatment of advanced breast cancer in post-menopausal women whose disease has progressed following tamoxifen therapy, side effects including hot flashes and nausea. Anastrozole, which has the chemical name, α,α,α',α'-tetramethyl-5-(1H-1.2,4-triazol-1-ylmethyl)-1,3-benzenediacetonitrile, is used for adjuvant treatment of post-menopausal women with hormone receptor-positive early breast cancer, and also for the first-line treatment of post-menopausal women with hormone receptor-positive or hormone receptor-unknown locally advanced or metastatic breast cancer, and for the treatment of advanced breast cancer in post-menopausal women with disease progression following tamoxifen therapy. Administration of anastozole usually. results in side-effects including gastrointestinal disturbances, rashes and headaches. Letrozole, which has the chemical name 4,4'-(1H-1,2,4-triazol-1-ylmethylene)-dibenzonitrile, is used for first-line treatment of post-menopausal women with hormone receptor-positive or hormone receptor-unknown locally advanced or metastatic breast cancer, and for the treatment of advanced breast cancer in post-menopausal women with disease progression following antiestrogen therapy, possible side-effects including occasional transient thrombocytopenia and elevation of liver transaminases. Aminoglutethimide, which has the chemical name 3-(4-aminophenyl)-3-ethyl-2,6-piperidinedione, is also used for treating breast cancer but suffers from the side-effects of skin rashes and less commonly thrombocytopenia and leukopenia.

Preferred aromatase inhibitors include letrozole, anastrozole, exemestane and aminoglutethimide. Letrozole is commercially available for example from Novartis A.G. under the trade name Femara, or may be prepared for example as described in U.S. patent specification No. 4978672, or by processes analogous thereto. Anastrozole is commercially available for example from AstraZeneca p1c under the trade name Arimidex, or may be prepared for example as described in U.S. Patent Specification No. 4935437, or by processes analogous thereto. Exemestane is commercially available for example from Pharmacia Corporation under the trade name Aromasin, or may be prepared for example as described in U.S. patent specification No. 4978672, or by processes analogous thereto. Aminoglutethimide is commercially available for example from Novartis A.G. under the trade name Cytadren, or may be prepared for example as described in U.S. patent specification No 2848455, or by processes analogous thereto. The aromatase inhibitor vorozole, which may be prepared for example as described in European patent specification No. 293978, or by processes analogous thereto, can also be used in accordance with the invention.

With regard to the preferred aromatase inhihibitors, these are generally administered in an oral daily dosage in the range 1 to 1000 mg, for example letrozole in a dosage of about 2.5 mg once a day; anastrozole in a dosage of about 1 mg once a day; exemestane in a dosage of about 25 mg once a day; and aminoglutethimide in a dosage of 250 mg 2-4 times daily.

Particularly preferred are aromatase inhibitors selected from the agents described herein, for example, letrozole, anastrozole, exemestane and aminoglutethimide.

### GNRAs

Of the hormones, hormone agonists, hormone antagonists and hormone modulating agents for use in the combinations of the invention, preferred are agents of the GNRA class.

*Definition*: As used herein the term GNRA is intended to define gonadotropin-releasing hormone (GnRH) agonists and antagonists (including those described below), together with the ionic, salt, solvate, isomers, tautomers, N-oxides, ester, prodrugs, isotopes and protected forms thereof (preferably the salts or tautomers or isomers or N-oxides or solvates thereof, and more preferably, the salts or tautomers or N-oxides or solvates thereof), as described above.

*Technical background*: When released from the hypothalamus in the brain, gonadotropin-releasing hormone (GnRH) agonists stimulate the pituitary gland to produce gonadotropins. Gonadotropins are hormones that stimulate androgen synthesis in the testes and estrogen synthesis in the ovaries. When GnRH agonists are first administered, they can cause an increase in gonadotropin release, but with continued administration, GnRH will block gonadotropin release, and therefore decrease the synthesis of androgen and estrogen. GnRH analogs are used to treat metastatic prostate cancer. They have also been approved for treatment of metastatic breast cancer in pre-menopausal women. Examples of GnRH analogs include goserelin acetate and leuprolide acetate. In contrast GnRH antagonists such as aberelix cause no initial GnRH surge since they have no agonist effects. However, due to their narrow therapeutic index, their use is currently limited to advanced prostate cancer that is refractory to other hormonal treatment such as GnRH agonists and anti-androgens. Goserelin acetate is a synthetic decapeptide analog of LHRH or GnRH, and has the chemical structure is pyro-Glu-His-Trp-Ser-Tyr-D-Ser(Bu)-Leu-Arg-Pro-Azgly-NH₂ acetate, and is used for the treatment of breast and prostate cancers and also endometriosis, side effects including hot flashes, bronchitis, arrhythmias, hypertension, anxiety and headaches.Leuprolide acetate is a synthetic nonapeptide analog of GnRH or LHRH, and has the chemical name 5-oxo-L-prolyl-L-histidyl-L-tryptophyl-L-seryl-L-tyrosyl-D-leucyl-L-leucyl-L-arginyl-N-ethyl-L-prolinamide acetate. Leuprolide acetate is used for the treatment of prostate cancer, endometriosis, and also breast cancer, side effects being similar to those of goserelin acetate.

Abarelix is a synthetic decapeptide Ala-Phe-Ala-Ser-Tyr-Asn-Leu-Lys-Pro-Ala, and has the chemical name N-Acetyl-3-(2-naphthalenyl)-D-alanyl-4-chloro-D-phenylalanyl-3(3pyridinyl)-D-alanyl-L-seryl-N-methyl-L-tyrosyl-D-asparaginyl-L-leucyl-N6-(1-methylethyl)-L-lysyl-L-prolyl-D-alaninamide. Abarelix can be prepared according to R. W. Roeske, WO9640757 (1996 to Indiana Univ. Found.).

*Preferences and specific embodiments*: Preferred GnRH agonists and antagonists for use in accordance with the invention include any of the GNRAs described herein, including in particular goserelin, leuprolide/leuporelin, triptorelin, buserelin, abarelix, goserelin acetate and leuprolide acetate. Particularly preferred are goserelin and leuprolide. Goserelin acetate is commercially available for example from AstraZeneca plc under the trade name Zoladex, or may be prepared for example as described in U.S. Patent Specification No. 5510460, or by processes analogous thereto. Leuprolide acetate is commercially available for example from TAP Pharmaceuticals Inc. under the trade name Lupron, or may be prepared for example as described in U.S. Patent Specification No. 3914412, or by processes analogous thereto. Goserelin is commercially available from AstraZeneca under the trade name Zoladex may be prepared for example as described in ICI patent publication US4100274 or Hoechst patent publication EP475184 or by processes analagous thereto. Leuprolide is commercially available in the USA from TAP Pharmaceuticals Inc. under the trade name Lupron and in Europe from Wyeth under the trade name Prostap and may be prepared for example as described in Abbott patent publication US4005063 or by processes analogous thereto. Triptorelin is commercially available from Watson Pharma under the trade name Trelstar and may be prepared for example as described in Tulane patent publication US5003011 or by processes analagous thereto. Buserelin Is commercially available under the trade name Suprefact and may be prepared for example as described in Hoechst patent publication US4024248 or by processes analogous thereto. Abarelix is commercially available from Praecis Pharmaceuticals under the trade name Plenaxis and may be prepared for example as described by Jiang et al., J Med Chem (2001). 44(3), 453-467 or Polypeptide Laboratories patent publication WO2003055900 or by processes analogous thereto.

Other GnRH agonists and antagonists for use in accordance with the invention include, but are not limited to, Histrelin from Ortho Pharmaceutical Corp, Nafarelin acetate from Roche, and Deslorelin from Shire Pharmaceuticals.

*Posology.* The GnRH agonists and antagonists are advantageously administered in dosages of 1.8mg to 100mg, for example 3.6mg monthly or 10.8mg every three months for goserelin or 7.5mg monthly, 22.5mg every three months or 30mg every four months for leuprolide.

With regard to the preferred GnRH analogs, these are generally administered in the following dosages, namely goserelin acetate as a 3.6 mg subcutaneous implant every 4 weeks, and leuprolide as a 7.5 mg intramuscular depot every month.

### 2. Monoclonal antibodies for use according to the invention

In one embodiment of the invention, the ancillary agent is a monoclonal antibody to one or more cell surface antigen (s).

Any monoclonal antibody to one or more cell surface antigen(s)) may be used in the combinations of the invention. Antibody specificity may be assayed or determined using any of a wide variety of techniques well-known to those skilled in the art.

*Definition*: The term "monoclonal antibody" used herein refers to antibodies from any source, and so includes those that are fully human and also those which contain structural or specificity determining elements derived from other species (and which can be referred to as, for example, chimeric or humanized antibodies).

*Technical background.* The use of monoclonal antibodies is now widely accepted in anticancer chemotherapy as they are highly specific and can therefore bind and affect disease specific targets, thereby sparing normal cells and causing fewer side-effects than traditional chemotherapies.

One group of cells which have been investigated as targets for antibody chemotherapy for the treatment of various cancers are those bearing the cell-surface antigens comprising the cluster designation (CD) molecules which are over-expressed or aberrantly expressed in tumour cells, for example CD20, CD22, CD33 and CD52 which are over-expressed on the tumour cell surface, most notably in tumours of hematopoietic origin. Antibodies to these CD targets (anti-CD antibodies) include the monoclonal antibodies rituximab (a.k.a. rituxamab), tositumomab and gemtuzumab ozogamicin.

Rituximab/rituxamab is a mouse/human chimeric anti-CD20 monoclonal antibody which has been used extensively for the treatment of B-cell non-Hodgkin's lymphoma including relapsed, refractory low-grade or follicular lymphoma. The product is also being developed for various other indications including chronic lymphocytic leukaemia. Side effects of rituximab/rituxamab may include hypoxia, pulmonary infiltrates, acute respiratory distress syndrome, myocardial infarction, ventricular fibrillation or cardiogenic shock. Tositumomab is a cell-specific anti-CD20 antibody labelled with iodine-131, for the treatment of non-Hodgkin's lymphoma and lymphocytic leukaemia. Possible side-effects of tositumomab include thrombocytopenia and neutropenia. Gemtuzumab ozogamicin is a cytotoxic drug (calicheamicin) linked to a human monoclonal antibody specific for CD33. Calicheamicin is a very potent antitumour agent, over 1,000 times more potent than adriamycin. Once released inside the cell, calicheamicin binds in a sequence-specific manner to the minor groove of DNA, undergoes rearrangement, and exposes free radicals, leading to breakage of double-stranded DNA, and resulting in cell apoptosis (programmed cell death). Gemtuzumab ozogamicin is used as a second-line treatment for acute myeloid leukaemia, possible side-effects including severe hypersensitivity reactions such as anaphylaxis, and also hepatotoxicity.

Alemtuzumab (Millennium Pharmaceuticals, also known as Campath) is a humanized monoclonal antibody against CD52 useful for the treatment of chronic lymphocytic leukaemia and Non-Hodgkin lymphoma which induces the secretion of TNF-alpha, IFN-gamma and IL-6.

*Preferences:* Preferred monoclonal antibodies for use according to the Invention include anti-CD antibodies, including alemtuzumab, CD20, CD22 and CD33. Particularly preferred monoclonal antibody to cell surface antigens, include CD20, CD22, CD33 as described above.

*Specific embodiments*: In one embodiment, the monoclonal antibody is an antibody to the cluster designation CD molecules, for example, CD20, CD22, CD33 and CD52. In another embodiment, the monoclonal antibody to cell surface antigen is selected from rituximab/rituxamab, tositumomab and gemtuzumab ozogamicin. Other monoclonal antibodies that may be used according to the invention include bevacizumab.

*Exemplary formulations*: Monoclonal antibodies to cell surface antigen(s) for use according to the invention include CD52 antibodies (e.g. alemtuzumab) and other anti-CD antibodies (for example, CD20, CD22 and CD33) as described herein. Preferred are therapeutic combinations comprising a monoclonal antibody to cell surface antigen (selected from CD20, CD22 and CD33) which exhibit an advantageous efficacious effect, for example, against tumour cell growth, in comparison with the respective effects shown by the individual components of the combination.

Preferred examples of monoclonal antibodies to cell surface antigens (anti-CD antibodies) include rituximab/rituxamab, tositumomab and gemtuzumab ozogamicin. Rituximab/rituxamab is commercially available from F Hoffman-La Roche Ltd under the trade name Mabthera, or may be obtained as described in PCT patent specification No. WO 94/11026. Tositumomab is commercially available from GlaxoSmithKline pic under the trade name Bexxar, or may be obtained as described in U.S. Patent specification No 5595721. Gemtuzumab ozogamicin is commercially available from Wyeth Research under the trade name Mylotarg, or may be obtained as described in U.S. Patent specification 5,877,296.

*Biological activity.* Monoclonal antibodies to one or more cell surface antigen(s)) have been identified as suitable anti-cancer agents. Antibodies are effective through a variety of mechanisms. They can block essential cellular growth factors or receptors, directly induce apoptosis, bind to target cells or deliver cytotoxic payloads such as radioisotopes and toxins.

*Posology.* The anti-CD antibodies may be administered for example in dosages of 5 to 400 mg per square meter (mg/m²) of body surface; in particular gemtuzumab ozogamicin may be administered for example in a dosage of about 9 mg/m² of body surface; rituximab/rituxamab may be administered for example in a dosage of about 375 mg/m² as an IV infusion once a week for four doses; the dosage for tositumomab must be individually quantified for each patient according to the usual clinical parameters such as age, weight, sex and condition of the patient.

These dosages may be administered for example once, twice or more per course of treatment, which may be repeated for example every 7, 14, 21 or 28 days.

### 3. Alkylating agents

In one embodiment of the invention, the ancillary agent is an alkylating agent as defined herein.

Definition: The term "alkylating agent" or "alkylating agents" as used herein refers to alkylating agents or analogues of alkylating agents as described herein, including the ionic, salt, solvate, isomers, tautomers, N-oxides, ester, prodrugs, isotopes and protected forms thereof (preferably the salts or tautomers or isomers or N-oxides or solvates thereof, and more preferably, the salts or tautomers or N-oxides or solvates thereof), as described above.

Technical background: Alkylating agents used in cancer chemotherapy encompass a diverse group of chemicals that have the common feature that they have the capacity to contribute, under physiological conditions, alkyl groups to biologically vital macromolecules such as DNA. With most of the more important agents such as the nitrogen mustards and the nitrosoureas, the active alkylating moieties are generated in vivo after complex degradative reactions, some of which are enzymatic. The most important pharmacological actions of the alkylating agents are those that disturb the fundamental mechanisms concerned with cell proliferation, in particular DNA synthesis and cell division. The capacity of alkylating agents to interfere with DNA function and integrity in rapidly proliferating tissues provides the basis for their therapeutic applications and for many of their toxic properties. Alkylating agents as a class have therefore been investigated for their anti-tumour activity and certain of these compounds have been widely used in anti-cancer therapy although they tend to have in common a propensity to cause dose-limiting toxicity to bone marrow elements and to a lesser extent the intestinal mucosa.

Among the alkylating agents, the nitrogen mustards represent an important group of anti-tumour compounds which are characterised by the presence of a bis-(2-chloroethyl) grouping and include chlorambucil, which has the chemical name 4-[bis(2-chloroethyl)amino]-benzenebutoic acid.

Ifosfamide (a.k.a. ifosphamide) is a structural analogue of cyclophosphamide and its mechanism of action is presumed to be Identical. It has the chemical name 3-(2-chloroethyl)-2-[(2-chloroethyl)amino]tetrahydro-2H-1,3,2-oxazaphosphorin-2-oxide, and is used for the treatment of cervical cancer, sarcoma, and testicular cancer but may have severe urotoxic effects. Chlorambucil has been used for treating chronic leukocytic leukaemia and malignant lymphomas including lymphosarcoma.

Another important class of alkylating agents are the nitrosoureas which are characterised by the capacity to undergo spontaneous non-enzymatic degradation with the formation of the 2-chloroethyl carbonium ion. Examples of such nitrosourea compounds include carmustine (BCNU) which has the chemical name 1,3-bis(2-chloroethyl)-1-nitrosourea, and lomustine (CCNU) which has the chemical name 1-(2-chloroethyl)cyclohexyl-1-nitrosourea. Carmustine and lomustine each have an important therapeutic role in the treatment of brain tumours and gastrointestinal neoplasms although these compounds cause profound, cumulative myelosuppression that restricts their therapeutic value.

Another class of alkylating agent is represented by the bifunctional alkylating agents having a bis-alkanesulfonate group and represented by the compound busulfan which has the chemical name 1,4-butanediol dimethanesulfonate, and is used for the treatment of chronic myelogenous (myeloid, myelocytic or granulocytic) leukaemia. However, it can induce severe bone marrow failure resulting in severe pancytopenia.

Another class of alkylating agent are the aziridine compounds containing a three-membered nitrogen-containing ring which act as anti-tumour agents by binding to DNA, leading to cross-linking and inhibition of DNA synthesis and function.

Biological activity: One of the most important pharmacological actions of the alkylating agent in the combinations of the invention is its ability to disturb the fundamental mechanisms concerned with cell proliferation as herein before defined. This capacity to interfere with DNA function and integrity in rapidly proliferating tissues provides the basis for their therapeutic application against various cancers.

Problems: This class of cytotoxic compound is associated with side effects, as mentioned above. Thus, there is a need to provide a means for the use of lower dosages to reduce the potential of adverse toxic side effects to the patient.

Preferences: Preferred alkylating agents for use in accordance with the invention include the nitrogen mustard compounds ifosfamide/ ifosphamide and chlorambucil and the nitrosourea compounds carmustine and lomustine referred to above. Preferred nitrogen mustard compounds for use In accordance with the invention include ifosfamide/ifosphamide and chlorambucil referred to above. Chlorambucil is commercially available for example from GlaxoSmithKline plc under the trade name Leukeran, or may be prepared for example as described in U.S. patent specification No. 3046301, or by processes analogous thereto. Ifosfamide/ifosphamide is commercially available for example from Baxter Oncology under the trade name Mitoxana, or may be prepared for example as described in U. S. patent specification No. 3732340, or by processes analogous thereto. Preferred nitrosourea compounds for use in accordance with the invention include carmustine and lomustine referred to above. Carmustine is commercially available for example from Bristol-Myers Squibb Corporation under the trade name BiCNU, or may be prepared for example as described in European patent specification No. 902015, or by processes analogous thereto. Lomustine is commercially available for example from Bristol-Myers Squibb Corporation under the trade name CeeNU, or may be prepared for example as described in U. S. patent specification No. 4377687, or by processes analogous thereto. Busulfan is commercially available for example from GlaxoSmithKline pic under the trade name Myleran, or may be prepared for example as described in U. S. patent specification No. 2917432, or by processes analogous thereto. Others include estramustine, mechlorethamine, melphalan, bischloroethylnitrosurea, cyclohexylchloroethylnitrosurea, methylcyclohexylchloroethylnitrosurea, nimustine, procarbazine, dacarbazine, temozolimide and thiotepa.

Specific embodiments: In one embodiment, the alkylating agent is a nitrogen mustard compound selected from ifosfamide/ifosphamide and chlorambucil. In another embodiment, the alkylating agent is a nitrosurea selected from carmustine and lomustine. The alkylating agents further include Busulfan. The alkylating agents are as herein before defined other than mitomycin C or cyclophosphamide.

Posology: The nitrogen mustard or nitrosourea alkylating agent is advantageously administered in a dosage of 100 to 2500 mg per square meter (mg/m²) of body surface area, for example 120 to 500 mg/m², for ifosfamide/ ifosphamide In a dosage of 500-2500mg/m², for chlorambucil in a dosage of about 0.1 to 0.2 mg/kg, for carmustine in a dosage of about 150 to 200 mg/m² and for lomustine in a dosage of about 100 to 150 mg/m². For bis-alkanesulfonate compounds such as busulphan a typical dose may be 1-2 mg/m², e.g. about 1.8 mg/m².

Aziridine alkylating agents can be administered for example in a dosage of 15 to 25 mg/m² preferably about 20 mg/m².

The dosages noted above may be administered for example once, twice or more per course of treatment, which may be repeated for example every 7, 14, 21 or 28 days.

### 4. Further CDK inhibitors

In one embodiment of the invention, the ancillary agent is a further CDK inhibitor.

*Definition*: The term "CDK inhibitor" as used herein refers to compounds that inhibit or modulate the activity of cyclin dependent kinases (CDK), including the ionic, salt, solvate, isomers, tautomers, N-oxides, ester, prodrugs, isotopes and protected forms thereof (preferably the salts or tautomers or isomers or N-oxides or solvates thereof, and more preferably, the salts or tautomers or N-oxides or solvates thereof), as described above.

*Technical background*: CDKs play a role in the regulation of the cell cycle, apoptosis, transcription, differentiation and CNS function. Therefore, CDK inhibitors may find application in the treatment of diseases in which there is a disorder of proliferation, apoptosis or differentiation such as cancer. In particular RB+ve tumours may be particularly sensitive to CDK inhibitors. RB-ve tumours may also be sensitive to CDK inhibitors.

In addition to the CDK compounds of formula I herein, the combinations of the present invention may include further CDK compounds being one or more further CDK inhibitors or modulators selected from the compounds of formula I and the various further CDK inhibitors described herein.

Examples of further CDK inhibitors which may be used in combinations according to the invention include seliciclib, alvocidib, 7-hydroxy-staurosporine, JNJ-7706621, BMS-387032, PHA533533, PD332991, ZK-304709 and AZD-5438.

Seliciclib, which is the R isomer of roscovitine, and otherwise known as CYC 202, has the chemical name (2R)-2-[[9-(1-methylethyl)-6-[(phenylmethyl)-amino]-9H-purin-2-yl]amino]-1-butanol. It is being evaluated in clinical trials for the potential treatment of various cancers including lymphoid leukaemia, non-small-cell lung cancer, glomerulonephritis, mantle cell lymphoma, multiple myeloma, and breast cancer. Observed toxicities in clinical trials include nausea/vomiting and asthenia, skin rash and hypokalemia. Other toxicities included reversible renal impairment and transaminitis, and emesis.

Alvocidib, which is otherwise known as flavopiridol, HMR 1275 or L 86-8275, and which has the chemical name 5,7-dihydroxy-8-(4-N-methyl-2-hydroxypyridyl)-6'-chloroflavone, is being investigated in clinical trials for the potential treatment of various cancers including cancer of the esophagus, stomach, prostate, lung and colon, and also chronic lymphocytic leukaemia, and multiple myeloma, lymphoma; the most common toxicities observed were diarrhea, tumour pain, anemia, dyspnea and fatigue.

7-Hydroxystaurosporine, which is otherwise known as UCN-01 is being evaluated in clinical trials for the potential treatment of various cancers including chronic lymphocytic leukaemia, pancreas tumours and renal tumours; adverse events observed included nausea, headache and hyperglycemia.

JNJ-7706621, which has the chemical name N3-[4-(aminosulfonyl)-phenyl]-1-(2,6-difluorobenzoyl)-1H-1,2,4-triazole-3,5-diamine, is the subject of pre-clinical testing for the potential treatment of melanoma and prostate cancer. BMS-387032 which has the chemical name N-[5-[[[5(1,1-dimethylethyl)-2-oxazolyl]-methyl]thio]-2-thiazolyl]-4- piperidinecarboxamide, has been evaluated in phase I studies as a potential anticancer drug for patients with metastatic solid tumours such as renal cell carcinomas, non-small-cell lung cancer, head and neck cancers and leiomyosarcoma The drug was well tolerated with transient neutropenia noted as the primary toxicity. Other side-effects included transient liver aminase elevations, gastrointestinal toxicity, nausea, vomiting, diarrhea and anorexia. PHA533533, which has the chemical name (αS)- N-(5-cyclopropyl-1 H-pyrazol-3-yl)-α-methyl-4-(2-oxo-1-pyrrolidinyl)-benzene-acetamide, is the subject of pre-clinical testing for the potential treatment of various cancers such as tumours of the prostate, colon and ovary. PD332991, which has the chemical name 8-cyclohexyl-2-[[4-(4-methyl-1-piperazinyl)phenyl]amino]- pyrido[2,3-d]pyrimidin-7(8H)-one, is the subject of pre-clinical testing for the potential treatment of various cancers. Pre-clinical data suggests that it is a highly selective and potent CDK4 inhibitor, demonstrating marked tumour regression in vivo models.

ZK-304709 is an oral dual specificity CDK and VEGFR kinase inhibitor, described in PCT patent specification No. WO 02/096888, and is the subject of pre-clinical testing for the potential treatment of various cancers. AZD-5438 is a selective cyclin-dependent kinase (CDK) inhibitor, which is In pre-clinical development for the treatment of solid cancers. Seliciclib may be prepared for example as described in PCT patent specification No. WO 97/20842, or by processes analogous thereto. Alvocidib, may be prepared for example as described in U.S. patent specification No. 4900727 or by processes analogous thereto. 7-Hydroxystaurosporine may be prepared for example as described in U.S. patent specification No. 4935415, or by processes analogous thereto. JNJ-7706621 may be prepared for example as described in PCT patent specification No. WO 02/057240, or by processes analogous thereto. BMS-387032 may be prepared for example as described in PCT patent specification No. WO 01/44242, or by processes analogous thereto. PHA533533 may be prepared for example as described in U.S. patent specification No. 6455559, or by processes analogous thereto. PD332991, may be prepared for example as described in PCT patent specification No. WO 98/33798, or by processes analogous thereto. ZK-304709 may be prepared for example as described in PCT patent specification No. WO 02/096888, or by processes analogous thereto.

*Preferences and specific embodiments*: In addition to the CDK compounds of formula I herein, the combinations of the present invention may include further CDK compounds being one or more further CDK inhibitors or modulators selected from the compounds of formula I and the various further CDK inhibitors described herein. Thus, the one or more further CDK inhibitors or modulators for use in the combinations of the invention may be selected from the compounds of formula (II), (IV), (IVa), (Va), (Vb), (Via) or (VIb). Alternatively, they may not conform to the aforementioned formulae, and may for example correspond to any of the various further CDK inhibitors described herein. Thus, embodiments contemplated include combinations in which the anti-cancer agent is a CDK inhibitor selected from one or more of the specific compounds described above. Thus, preferred CDK inhibitors for use in combinations according to the invention include seliciclib, alvocidib, 7-hydroxystaurosporine, JNJ-7706621, BMS-387032, PHA533533, PD332991, ZK-304709 and AZD-5438.

*Posology*: The CDK inhibitor may be administered for example in a daily dosage of for example 0.5 to 2500 mg, more preferably 10 to 1000mg, or alternatively 0.001 to 300 mg /kg, more preferably 0.01 to 100 mg/kg, particularly for seliciclib, in a dosage of 10 to 50 mg; for alvocidib, in a dosage in accordance with the above-mentioned U.S. patent specification No. 4900727; for 7-hydroxystaurosporine in a dosage of 0.01 to 20mg/kg; for JNJ-7706621 in a dosage of 0.001 to 300mg/kg; for BMS-387032 in a dosage of 0.001 to 100mg/kg more preferably 0.01 to 50 mg/kg, and most preferably 0.01 to 20 mg/kg; for PHA533533 in a dosage of 10 to 2500 mg; for PD332991 in a dosage of 1 to 100 mg/kg; and for ZK-304709 in a dosage of 0.5 to 1000 mg preferably 50 to 200 mg.

These dosages may be administered for example once, twice or more per course of treatment, which may be repeated for example every 7, 14, 21 or 28 days.

### 5. Anticancer agents

### (a) COX-2 inhibitors

In one embodiment of the invention, the ancillary agent is a COX-2 inhibitor.

*Definition*: The term "COX-2 inhibitor" is used herein to define compounds which inhibit or modulate the activity of the cyclo-oxygenase-2 (COX-2) enzyme, including the ionic, salt, solvate, isomers, tautomers, N-oxides, ester, prodrugs, isotopes and protected forms thereof (preferably the salts or tautomers or isomers or N-oxides or solvates thereof, and more preferably, the salts or tautomers or N-oxides or solvates thereof), as described above.

*Biological activity.* The COX-2 inhibitors working via one or more pharmacological actions as described herein have been identified as suitable anti-cancer agents.

*Technical background*: Recently, research in cancer chemotherapy has focused on the role of the cyclo-oxygenase-2 (COX-2) enzyme. Epidemiological studies have shown that people who regularly take non-steroidal anti-inflammatory drugs (NSAIDs), for example aspirin and ibuprofen to treat conditions such as arthritis, have lower rates of colorectal polyps, colorectal cancer, and death due to colorectal cancer. NSAIDs block cyclooxygenase enzymes, which are produced by the body in inflammatory processes, and which are also produced by pre-cancerous tissues. For example in colon cancers, a dramatic increase of COX-2 levels is observed. One of the key factors for tumour growth is the supply of blood to support its increased size. Many tumours can harness chemical pathways that prompt the body to create a web of new blood vessels around the cancer, a process called angiogenesis. COX-2 is believed to have a role in this process. It has therefore been concluded that inhibition of COX-2 may be effective for treating cancer, and COX-2 inhibitors have been developed for this purpose. For example celecoxib, which has the chemical name 4-[5-(4-methylphenyl)-3-(trifluoromethyl)-1H-pyrazol-1-yl]benzenesulfonamide, is a selective COX-2 inhibitor that is being investigated for the treatment of various cancers including bladder and esophageal cancer, renal cell carcinoma, cervical cancer, breast cancer, pancreatic cancer non-Hodgkin's lymphoma and non-small cell lung cancer.

*Posology.* The COX-2 inhibitor (for example celecoxib) can be administered in a dosage such as 100 to 200 mg.

These dosages may be administered for example once, twice or more per course of treatment, which may be repeated for example every 7, 14, 21 or 28 days.

*Problems*: The most common adverse effects are headache, abdominal pain, dyspepsia, diarrhea, nausea, flatulence and insomnia. There is a need to provide a means for the use of lower dosages of COX-2 inhibitors to reduce the potential for adverse toxic side effects to the patient.

*Preferences and specific embodiments*: In one embodiment the COX-2 inhibitor is celecoxib. Celecoxib is commercially available for example from Pfizer Inc under the trade name Celebrex, or may be prepared for example as described in PCT patent specification No. WO 95/15316, or by processes analogous thereto.

### (b) HDAC inhibitors

In one embodiment of the invention, the ancillary agent is a HDAC inhibitor.

*Definition*: The term "HDAC inhibitor" is used herein to define compounds which inhibit or modulate the activity of histone deacetylases (HDAC), including the ionic, salt, solvate, isomers, tautomers, N-oxides, ester, prodrugs, isotopes and protected forms thereof (preferably the salts or tautomers or isomers or N-oxides or solvates thereof, and more preferably, the salts or tautomers or N-oxides or solvates thereof), as described above.

*Biological activity*: The HDAC inhibitors working via one or more pharmacological actions as described herein have been identified as suitable anti-cancer agents.

*Technical background*: Reversible acetylation of histones is a major regulator of gene expression that acts by altering accessibility of transcription factors to DNA. In normal cells, histone deacetylase (HDA or HDAC) and histone acetyltrasferase (HDA) together control the level of acetylation of histones to maintain a balance. Inhibition of HDA results in the accumulation of hyperacetylated histones, which results in a variety of cellular responses. Inhibitors of HDA (HDAI) have been studied for their therapeutic effects on cancer cells. Recent developments in the field of HDAI research have provided active compounds, both highly efficacious and stable, that are suitable for treating tumours.

Accruing evidence suggests that HDAI are even more efficacious when used in combination with other chemotherapeutic agents. There are both synergistic and additive advantages, both for efficacy and safety. Therapeutic effects of combinations of chemotherapeutic agents with HDAI can result in lower safe dosage ranges of each component in the combination.

The study of inhibitors of histone deacetylases (HDAC) indicates that indeed these enzymes play an important role in cell proliferation and differentiation. The inhibitor Trichostatin A (TSA) causes cell cycle arrest at both G1 and G2 phases, reverts the transformed phenotype of different cell lines, and induces differentiation of Friend leukaemia cells and others. TSA (and suberoylanilide hydroxamic acid SAHA) have been reported to inhibit cell growth, induce terminal differentiation, and prevent the formation of tumours in mice (Finnin et al., Nature, 401:188 - 193, 1999).

Trichostatin A has also been reported to be useful in the treatment of fibrosis, e.g. liver fibrosis and liver chirrhosis. (Geerts et al., European Patent Application EP0 827 742, published 11 March 1998).

*Preferences and specific embodiments*: Preferred HDAC inhibitors for use in accordance with the invention are selected from TSA, SAHA, JNJ-16241199, LAQ-824, MGCD-0103 and PXD-101.

Thus, synthetic inhibitors of histone deacetylases (HDAC) which are suitable for use in the present Invention include JNJ-16241199 from Johnson and Johnson Inc, LAQ-824 from Novartis, MGCD-0103 from MethylGene, and PXD-101 from Prolifix.

JNJ-16241199 has the following structure:

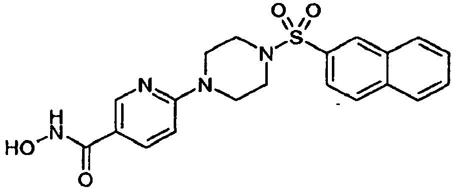

MGCD-0103 has the structure:

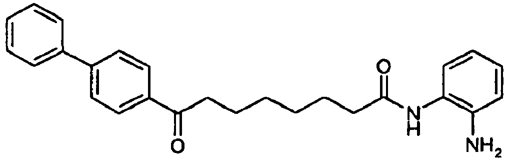

LAQ-824 has the structure:

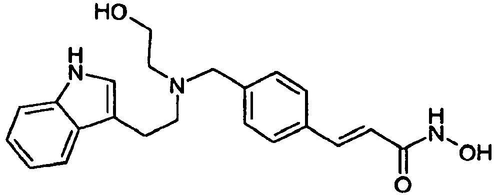

Other inhibitors of histone deacetylases (HDAC) which are suitable for use in the present invention include, but are not limited to, the peptide chlamydocin, and A-173, also from Abbott Laboratories.

A-173 is a succinimide macrocyclic compound with the following structure:

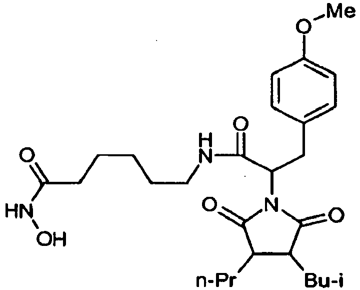

*Posology*: In general, for HDAC inhibitors it is contemplated that a therapeutically effective amount would be from 0.005 mg/kg to 100 mg/kg body weight, and in particular from .005 mg/kg to 10 mg/kg body weight It may be appropriate to administer the required dose as two, three, four or more sub-doses at appropriate intervals throughout the day. Said sub-doses may be formulated as unit dosage forms, for example, containing 0.5 to 500 mg, and in particular 10 mg to 500 mg of active ingredient per unit dosage form.

These dosages may be administered for example once, twice or more per course of treatment, which may be repeated for example every 7, 14, 21 or 28 days.

### (c) DNA methylase inhibitors

In one embodiment of the invention, the ancillary agent is a DNA methylase inhibitor.

*Definition:* The term "DNA methylase inhibitor" or "DNA methyltransferase inhibitor" as used herein refers to a compound which directly or indirectly perturbs, disrupts, blocks, modulates or inhibits the methylation of DNA, including the ionic, salt, solvate, isomers, tautomers, N-oxides, ester, prodrugs, isotopes and protected forms thereof (preferably the salts or tautomers or isomers or N-oxides or solvates thereof, and more preferably, the salts or tautomers or N-oxides or solvates thereof), as described above.

*Biological activity:* The DNA methylase inhibitors working via one or more pharmacological actions as described herein have been identified as suitable anti-cancer agents.

*Technical background*: One target for cancer chemotherapy is DNA synthesis, which may depend on appropriate methylation of tumour DNA. Compounds which directly or indirectly perturb, disrupt, block, modulate or inhibit the methylation of DNA may therefore be useful anticancer drugs.

The DNA methylase inhibitor temozolomide is used for the treatment of glioblastoma multiforme, and is also being investigated and used for the treatment of malignant glioma at first relapse and first-line treatment of patients with advanced metastatic malignant melanoma. This compound undergoes rapid chemical conversion at physiological pH to the active compound, monomethyl triazeno imidazole carboxamide (MTIC) which is responsible for the methylation of DNA at the O⁸ position of guanine residues (which appears to lead to a suppression in expression of DNA methyltransferase and so produce hypomethylation).

*Problems:* The most common side effects associated with temozolomide therapy are nausea, vomiting, headache, fatigue, and constipation. There Is a need to increase the inhibitory efficacy of DNA\methylase inhibitors and to provide a means for the use of lower dosages of signaling inhibitors to reduce the potential for adverse toxic side effects to the patient.

*Preferences and specific embodiments*: In one embodiment, the DNA methylase inhibitor is temozolomide (3,4-dihydro-3-methyl-4-oxoimidazo[5,1-d]-as-tetrazine-8-carboxamide). Temozolomide is commercially available for example from Schering Corporation under the trade name Temodar, or may be prepared for example as described in German patent specification No. 3231255, or by processes analogous thereto.

*Posology.* The DNA methylating agent (for example temozolomide) can be administered In a dosage such as 0.5 to 2.5 mg per square meter (mg/m²) of body surface area, particularly about 1.3 mg/m². These dosages may be administered for example once, twice or more per course of treatment, which may be repeated for example every 7, 14, 21 or 28 days.

### (d) Proteasome inhibitors

In one embodiment of the invention, the ancillary agent is a proteasome inhibitor.

Definition: The term "proteasome inhibitor" as used herein refers to compounds which directly or indirectly perturb, disrupt, block, modulate or inhibit the half-life of many short-lived biological processes, such as those involved in the cell cycle. The term therefore embraces compounds which block the action of proteasomes (large protein complexes that are involved in the turnover of other cellular proteins). The term also embraces the ionic, salt, solvate, isomers, tautomers, N-oxides, ester, prodrugs, isotopes and protected forms thereof (preferably the salts or tautomers or isomers or N-oxides or solvates thereof, and more preferably, the salts or tautomers or N-oxides or solvates thereof), as described above.

*Biological activity*: The proteasome inhibitors working via one or more pharmacological actions as described herein have been identified as suitable anti-cancer agents.

Technical background: Another class of anticancer agents are the proteasome inhibitors. Proteasomes control the half-life of many short-lived biological processes, such as those involved in the cell cycle. Therefore, proteasome malfunction can lead to abnormal regulation of the cell cycle and uncontrolled cell growth.

The cell cycle is controlled by both positive and negative signals. In a normal cell, proteasomes break down proteins that inhibit the cell cycle, such as cyclin-dependent kinase inhibitors. Inhibition of proteasome function causes cell cycle arrest and cell death. Tumour cells are more susceptible to these effects than normal cells, in part because they divide more rapidly and in part because many of their normal regulatory pathways are disrupted. The mechanism for the differential response of normal and cancer cells to proteasome inhibition is not fully understood. Overall, cancer cells are more susceptible to proteasome inhibitors and, as a result, these inhibitors may be an effective treatment for certain cancers.

One such proteasome inhibitor is bortezomib, which has the chemical name [(1R)-3-methyl-1-[[(2S)-1-oxo-3-phenyl-2-[(pyrazinylcarbonyl)amlno]propyl]amino]butyl]-boronic acid. Bortezomib specifically interacts with a key amino acid, namely threonine, within the catalytic site of the proteasome. Bortezomib is being used for the treatment of multiple myeloma and also for a number of other cancers, including leukemia and lymphoma, and prostate, pancreatic and colorectal carcinoma.

Problems: The most common side effects with bortezomib are nausea, tiredness, diarrhea, constipation, decreased platelet blood count, fever, vomiting, and decreased appetite. Bortezomib can also cause peripheral neuropathy.

Thus, there is a need to provide a means for the use of lower dosages to reduce the potential of adverse toxic side effects to the patient.

Preferences and specific embodiments: Preferred proteasome inhibitors for use in accordance with the invention include bortezomib. Bortezomib is commercially available for example from Millennium Pharmaceuticals Inc under the trade name Velcade, or may be prepared for example as described In PCT patent specification No. WO 96/13266, or by processes analogous thereto.

Posology: The proteasome inhibitor (such as bortezomib) can be administered In a dosage such as 100 to 200 mg/m².

These dosages may be administered for example once, twice or more per course of treatment, which may be repeated for example every 7, 14, 21 or 28 days.

### Pharmaceutical Formulations

While it is possible for the active compounds/agents in the combinations of the invention to be administered without any accompanying pharmaceutical excipients or carriers, it is preferable to present them In the form of pharmaceutical compositions (e.g. formulations). As such, they may be formulated for simultaneous or sequential administration.

Where they are intended for sequential administration, they will typically be formulated in separate compositions which may be of the same type or a different type. Thus, for example, the components of the combination may be formulated for delivery by the same route (e.g. both by the oral route or both by injection) or they may be formulated for administration by different routes (e.g. one by the oral route and another by a parenteral route such as by i.v. injection or infusion). In a preferred embodiment the compound 4-(2,6-dichloro-benzoylamino)-1H-pyrazole-3-carboxylic acid piperidin-4-ylamide and salts therof, particularly acid addition salts such as the methanesulphonic acid, acetic acid and hydrochloric acid salts is administered sequentially (either before or after) or simulatenously with the ancillary agent as hereinabove described. Preferably the compound 4-(2,6-dichloro-benzoylamino)-1H-pyrazole-3-carboxylic acid piperidin-4-ylamide and salts therof, particularly acid addition salts such as the methanesulphonic acid, acetic acid and hydrochloric acid salts is administered using an i.v. formulation as defined herein.

When they are intended for simultaneous administration, they may be formulated together or separately and, as above, may be formulated for administration by the same route or by different routes.

The compositions typically comprise at least one active compound of the combination together with one or more pharmaceutically acceptable carriers, adjuvants, excipients, diluents, fillers, buffers, stabilisers, preservatives, lubricants, or other materials well known to those skilled in the art. The compositions may also include other therapeutic or prophylactic agents, for example agents that reduce or alleviate some of the side effects associated with chemotherapy. Particular examples of such agents include anti-emetic agents and agents that prevent or decrease the duration of chemotherapy-associated neutropenia and prevent complications that arise from reduced levels of red blood cells or white blood cells, for example erythropoietin (EPO), granulocyte macrophage-colony stimulating factor (GM-CSF), and granulocyte-colony stimulating factor (G-CSF).

Also included are agents that inhibit bone resorption such as bisphosphonate agents e.g. zoledronate, pamidronate and ibandronate, as well as agents that suppress inflammatory responses (such as dexamethazone, prednisone, and prednisolone). Also included are agents used to reduce blood levels of growth hormone and IGF-I in acromegaly patients such as synthetic forms of the brain hormone somatostatin, which includes octreotide acetate which is a long-acting octapeptide with pharmacologic properties mimicking those of the natural hormone somatostatin. Further included are agents such as leucovorin, which is used as an antidote to drugs that decrease levels of folic acid, or folinic acid it self. In one particular embodiment is the combination of 5FU and leucovorin or 5FU and folinic acid. In addition megestrol acetate can be used for the treatment of side-effects Including oedema and thromoembolic episodes.

Therefore in one embodiment the combinations further include an additional agent selected from erythropoietin (EPO), granulocyte macrophage-colony stimulating factor (GM-CSF), granulocyte-colony stimulating factor (G-CSF), zoledronate, pamidronate, ibandronate, dexamethazone, prednisone, prednisolone, leucovorin, folinic acid and megestrol acetate.

In particular the combinations further include an additional agent selected from erythropoietin (EPO), granulocyte macrophage-colony stimulating factor (GM-CSF), granulocyte-colony stimulating factor (G-CSF), zoledronate, pamidronate, dexamethazone, prednisone, prednisolone, leucovorin, and folinic acid such as erythropoietin (EPO), granulocyte macrophage-colony stimulating factor (GM-CSF) and granulocyte-colony stimulating factor (G-CSF).

Zoledronic acid is available from Novartis under the Tradename Zometa®. It is used in the treatment of bone metastasis in a variety of tumor types and for the treatment of hypercalcemia.

Pamidronate disodium (APD) available from Novartis under the tradename Aredia Is a bone-resorption inhibitor and is used in the treatment of moderate or severe hypercalcemia. Pamidronate disodium is for i.v. injection.

Octreotide acetate is available from Novartis as Sandostatin LAR ® (octreotide acetate for injectable suspension) and Sandostatin® (octreotide acetate for injection ampuls or for vials). Octreotide is known chemically as L-Cysteinamide, D-phenylalanyl-L-cysteinyl-L-phenylalanyl-D-tryptophyl-L-lysyl-L-threonyl-N-[2-hydroxy-1-(hydroxy-methyl) propyl]-, cyclic (2, 7)-disulfide; (R-(R*,R*)]. Synthetic forms of the brain hormone somatostatin, such as octreotide, work at the site of the tumour. They bind to sst-2/sst-5 receptors to regulate gastrointestinal hormone secretion and affect tumour growth.

Thus, the present invention further provides pharmaceutical compositions, as defined above, and methods of making a pharmaceutical composition comprising admixing at least one active compound, as defined above, together with one or more pharmaceutically acceptable carriers, excipients, buffers, adjuvants, stabilizers, or other materials, as described herein.

The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

Accordingly, in a further aspect, the invention provides combinations of an ancillary agent as hereinabove described and a compound of the formula (0) or a sub-group thereof such as formulae (I⁰), (I), (Ia), (Ib), (II), (III), (IV), (IVa), (Va), (Vb), (VIa), (VIb), (VII) or (VIII) and sub-groups thereof as defined herein in the form of pharmaceutical compositions.

The pharmaceutical compositions can be in any form suitable for oral, parenteral, topical, intranasal, ophthalmic, otic, rectal, intra-vaginal, or transdermal administration. Where the compositions are intended for parenteral administration, they can be formulated for intravenous, intramuscular, intraperitoneal, subcutaneous administration or for direct delivery into a target organ or tissue by injection, infusion or other means of delivery. The delivery can be by bolus injection, short term infusion or longer term infusion and can be via passive delivery or through the utilisation of a suitable infusion pump.

Pharmaceutical formulations adapted for parenteral administration include aqueous and non-aqueous sterile injection solutions which may contain anti-oxidants, buffers, bacteriostats, co-solvents, organic solvent mixtures, cyclodextrin complexalion agents, emulsifying agents (for forming and stabilizing emulsion formulations), liposome components for forming liposomes, gellable polymers for forming polymeric gels, lyophilisation protectants and combinations of agents for, inter alia, stabilising the active ingredient in a soluble form and rendering the formulation isotonic with the blood of the intended recipient. Pharmaceutical formulations for parenteral administration may also take the form of aqueous and non-aqueous sterile suspensions which may include suspending agents and thickening agents (R. G. Strickly, Solubilizing Excipients in oral and injectable formulations, Pharmaceutical Research, Vol 21(2) 2004, p 201-230).

A drug molecule that is ionizable can be solubilized to the desired concentration by pH adjustment if the drug's pKₐ is sufficiently away from the formulation pH value. The acceptable range is pH 2-12 for intravenous and intramuscular administration, but subcutaneously the range is pH 2.7-9.0. The solution pH is controlled by either the salt form of the drug, strong acids/bases such as hydrochloric acid or sodium hydroxide, or by solutions of buffers which include but are not limited to buffering solutions formed from glycine, citrate, acetate, maleate, succinate, histidine, phosphate, tris(hydroxymethyl)aminomethane (TRIS), or carbonate.

The combination of an aqueous solution and a water-soluble organic solvent/surfactant (i.e., a cosolvent) is often used in injectable formulations. The water-soluble organic solvents and surfactants used in injectable formulations include but are not limited to propylene glycol, ethanol, polyethylene glycol 300, polyethylene glycol 400, glycerin, dimethylacetamide (DMA), N-methyl-2-pyrrolidone (NMP; Pharmasolve), dimethylsulphoxide (DMSO), Solutol HS 15, Cremophor EL, Cremophor RH 60, and polysorbate 80. Such formulations can usually be, but are not always, diluted prior to injection.

Propylene glycol, PEG 300, ethanol, Cremophor EL, Cremophor RH 60, and polysorbate 80 are the entirely organic water-miscible solvents and surfactants used in commercially available injectable formulations and can be used in combinations with each other. The resulting organic formulations are usually diluted at least 2-fold prior to IV bolus or IV infusion.

Alternatively increased water solubility can be achieved through molecular complexation with cyclodextrins Liposomes are closed spherical vesicles composed of outer lipid bilayer membranes and an inner aqueous core and with an overall diameter of <100 µm. Depending on the level of hydrophobicity, moderately hydrophobic drugs can be solubilized by liposomes if the drug becomes encapsulated or intercalated within the liposome. Hydrophobic drugs can also be solubilized by liposomes if the drug molecule becomes an integral part of the lipid bilayer membrane, and in this case, the hydrophobic drug is dissolved in the lipid portion of the lipid bilayer. A typical liposome formulation contains water with phospholipid at -5-20 mg/ml, an isotonicifier, a pH 5-8 buffer, and optionally cholesterol.

The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use.

The pharmaceutical formulation can be prepared by lyophilising a compound of Formula (II) or acid addition salt thereof. Lyophilisation refers to the procedure of freeze-drying a composition. Freeze-drying and lyophilisation are therefore used herein as synonyms. A typical process is to solubilise the compound and the resulting formulation is clarified, sterile filtered and aseptically transferred to containers appropriate for lyophilisation (e.g. vials). In the case of vials, they are partially stoppered with lyo-stoppers. The formulation can be cooled to freezing and subjected to lyophilisation under standard conditions and then hermetically capped forming a stable, dry lyophile formulation. The composition will typically have a low residual water content, e.g. less than 5% e.g. less than 1% by weight based on weight of the lyophile.

The lyophilsation formulation may contain other excipients for example, thickening agents, dispersing agents, buffers, antioxidants, preservatives, and tonicity adjusters. Typical buffers include phosphate, acetate, citrate and glycine. Examples of antioxidants include ascorbic acid, sodium bisulphite, sodium metabisulphite, monothioglycerol, thiourea, butylated hydroxytoluene, butylated hydroxyl anisole, and ethylenediamietetraacetic acid salts. Preservatives may include benzoic acid and its salts, sorbic acid and its salts, alkyl esters of para-hydroxybenzoic acid, phenol, chlorobutanol, benzyl alcohol, thimerosal, benzalkonium chloride and cetylpyridinium chloride. The buffers mentioned previously, as well as dextrose and sodium chloride, can be used for tonicity adjustment if necessary.

Bulking agents are generally used in lyophilisation technology for facilitating the process and/or providing bulk and/or mechanical integrity to the lyophilized cake. Bulking agent means a freely water soluble, solid particulate diluent that when co-lyophilised with the compound or salt thereof, provides a physically stable lyophilized cake, a more optimal freeze-drying process and rapid and complete reconstitution. The bulking agent may also be utilised to make the solution isotonic.

The water-soluble bulking agent can be any of the pharmaceutically acceptable inert solid materials typically used for lyophilisation. Such bulking agents include, for example, sugars such as glucose, maltose, sucrose, and lactose; polyalcohols such as sorbitol or mannitol; amino acids such as glycine; polymers such as polyvinylpyrrolidine; and polysaccharides such as dextran.

The ratio of the weight of the bulking agent to the weight of active compound is typically within the range from about 1 to about 5, for example of about 1 to about 3, e.g. in the range of about 1 to 2.

Alternatively they can be provided in a solution form which may be concentrated and sealed in a suitable vial. Sterilisation of dosage forms may be via filtration or by autoclaving of the vials and their contents at appropriate stages of the formulation process. The supplied formulation may require further dilution or preparation before delivery for example dilution into suitable sterile infusion packs.

Extemporaneous injection solutions and suspensions may be prepared from sterile powders, granules and tablets.

In one preferred embodiment of the invention, the pharmaceutical composition is in a form suitable for i.v. administration, for example by injection or infusion.

Pharmaceutical compositions of the present invention for parenteral injection can also comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

The compositions of the present invention may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption such as aluminum monostearate and gelatin.

If a compound is not stable in aqueous media or has low solubility in aqueous media, it can be formulated as a concentrate in organic solvents. The concentrate can then be diluted to a lower concentration in an aqueous system, and can be sufficiently stable for the short period of time during dosing. Therefore in another aspect, there is provided a pharmaceutical composition comprising a non aqueous solution composed entirely of one or more organic solvents, which can be dosed as is or more commonly diluted with a suitable IV excipient (saline, dextrose; buffered or not buffered) before administration (Solubilizing excipients in oral and injectable formulations, Pharmaceutical Research, 21(2), 2004, p201-230). Examples of solvents and surfactants are propylene glycol, PEG300, PEG400, ethanol, dimethylacetamide (DMA), N-methyl-2-pyrrolidone (NMP, Pharmasolve), Glycerin, Cremophor EL, Cremophor RH 60 and polysorbate. Particular non aqueous solutions are composed of 70-80% propylene glycol, and 20-30% ethanol. One particular non aqueous solution is composed of 70% propylene glycol, and 30% ethanol. Another is 80% propylene glycol and 20% ethanol. Normally these solvents are used in combination and usually diluted at least 2-fold before IV bolus or IV infusion. The typical amounts for bolus IV formulations are ∼50% for Glycerin, propylene glycol, PEG300, PEG400, and ∼20% for ethanol. The typical amounts for IV infusion formulations are ∼15% for Glycerin, 3% for DMA, and ∼10% for propylene glycol, PEG300, PEG400 and ethanol.

In one preferred embodiment of the invention, the pharmaceutical composition is in a form suitable for i.v. administration, for example by injection or infusion. For intravenous administration, the solution can be dosed as is, or can be injected into an infusion bag (containing a pharmaceutically acceptable excipient, such as 0.9% saline or 5% dextrose), before administration.

In another preferred embodiment, the pharmaceutical composition is in a form suitable for sub-cutaneous (s.c.) administration.

Pharmaceutical dosage forms suitable for oral administration include tablets, capsules, caplets, pills, lozenges, syrups, solutions, powders, granules, elixirs and suspensions, sublingual tablets, wafers or patches and buccal patches.

Pharmaceutical compositions containing compounds of the formula (II) can be formulated in accordance with known techniques, see for example, Remington's Pharmaceutical Sciences, Mack Publishing Company, Easton, PA, USA.

Thus, tablet compositions can contain a unit dosage of active compound together with an inert diluent or carrier such as a sugar or sugar alcohol, eg; lactose, sucrose, sorbitol or mannitol; and/or a non-sugar derived diluent such as sodium carbonate, calcium phosphate, calcium carbonate, or a cellulose or derivative thereof such as methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Capsule formulations may be of the hard gelatin or soft gelatin variety and can contain the active component in solid, semi-solid, or liquid form. Gelatin capsules can be formed from animal gelatin or synthetic or plant derived equivalents thereof.

The solid dosage forms (eg; tablets, capsules etc.) can be coated or un-coated, but typically have a coating, for example a protective film coating (e.g. a wax or varnish) or a release controlling coating. The coating (e.g. a Eudragit ™ type polymer) can be designed to release the active component at a desired location within the gastro-intestinal tract. Thus, the coating can be selected so as to degrade under certain pH conditions within the gastrointestinal tract, thereby selectively release the compound in the stomach or in the ileum or duodenum.

Instead of, or in addition to, a coating, the drug can be presented in a solid matrix comprising a release controlling agent, for example a release delaying agent which may be adapted to selectively release the compound under conditions of varying acidity or alkalinity in the gastrointestinal tract. Alternatively, the matrix material or release retarding coating can take the form of an erodible polymer (e.g. a maleic anhydride polymer) which is substantially continuously eroded as the dosage form passes through the gastrointestinal tract. As a further alternative, the active compound can be formulated in a delivery system that provides osmotic control of the release of the compound. Osmotic release and other delayed release or sustained release formulations may be prepared in accordance with methods well known to those skilled in the art.

Compositions for topical use include ointments, creams, sprays, patches, gels, liquid drops and inserts (for example intraocular inserts). Such compositions can be formulated in accordance with known methods.

Compositions for parenteral administration are typically presented as sterile aqueous or oily solutions or fine suspensions, or may be provided in finely divided sterile powder form for making up extemporaneously with sterile water for injection.

Examples of formulations for rectal or intra-vaginal administration include pessaries and suppositories which may be, for example, formed from a shaped moldable or waxy material containing the active compound.

Compositions for administration by inhalation may take the form of inhalable powder compositions or liquid or powder sprays, and can be administrated in standard form using powder inhaler devices or aerosol dispensing devices. Such devices are well known. For administration by inhalation, the powdered formulations typically comprise the active compound together with an inert solid powdered diluent such as lactose.

The compounds of the formula (II) will generally be presented in unit dosage form and, as such, will typically contain sufficient compound to provide a desired level of biological activity. For example, a formulation may contain from 1 nanogram to 2 grams of active ingredient, e.g. from 1 nanogram to 2 milligrams of active ingredient. Within this range, particular sub-ranges of compound are, or 0.1 milligrams to 2 grams of active ingredient (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredient).

The active compound will be administered to a patient In need thereof (for example a human or animal patient) in an amount sufficient to achieve the desired therapeutic effect.

Where the compounds of the combination of the invention are presented together, they can be formulated together as tablets, capsules, solutions for infusion or injection or any of the other solid or liquid dosage forms described above. For example, where they are formulated together, they may be Intimately mixed, or physically separated within the same formulation, for example by virtue of being present in different layers or granules within a tablet, or a separate beads or granules within a capsule. More typically, however, they are formulated separately for separate or concurrent administration.

In one embodiment, the the individual components of the combination may be formulated separately and presented together in the form of a kit, optionally under common outer packaging and optionally with instructions for their use.

More commonly these days, pharmaceutical formulations are prescribed to a patient in "patient packs" containing the whole course of treatment in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in patient prescriptions. The inclusion of a package insert has been shown to improve patient compliance with the physicians instructions.

Accordingly, in a further embodiment, the invention provides a package containing separate dosage units, one or more of which contain a compound of the formula (II), (IV), (IVa), (Va), (Vb), (VIa) or (Vlb) and sub-groups thereof as defined herein, and one or more of which contain an ancillary agent as hereinabove described. Dosage units containing a compound of the formula (II), (IV), (IVa), (Va), (Vb), (Via) or (VIb) and sub-groups thereof as defined herein and an ancillary agent as hereinabove described have suitable amounts of active ingredient as defined herein. A package contains enough tablets, capsules or the like to treat a patient for a pre-determined period of time, for instance for 2 weeks, 1 month or 3 months.

### Methods of Treatment

It is envisaged that the combinations containing an ancillary agent as hereinabove described and compounds of the formula (II) and sub-groups thereof such as formulae (IV), (IVa), (Va), (Vb), (VIa) or (VIb) and sub-groups thereof as defined herein will be useful in the prophylaxis or treatment of a range of disease states or conditions mediated by cyclin dependent kinases. Examples of such disease states and conditions are set out herein.

The combinations are generally administered to a subject in need of such administration, for example a human or animal patient, preferably a human.

The compounds will typically be administered in amounts that are therapeutically or prophylactically useful and which generally are non-toxic. However, in certain situations (for example in the case of life threatening diseases), the benefits of administering a compound of the formula (II) may outweigh the disadvantages of any toxic effects or side effects, in which case it may be considered desirable to administer compounds in amounts that are associated with a degree of toxicity.

The compounds may be administered over a prolonged term to maintain beneficial therapeutic effects or may be administered for a short period only. Alternatively they may be administered in a pulsatile or continuous manner.

The compounds of the combination can be administered simultaneously or sequentially. When administered sequentially, they can be administered at closely spaced intervals (for example over a period of 5-10 minutes) or at longer intervals (for example 1, 2, 3, 4 or more hours apart, or even longer periods, e.g. 1, 2, 3, 4, 5, 6, or 7 days, apart where required), the precise dosage regimen being commensurate with the properties of the therapeutic agent(s). With sequential administration, the delay in administering the second (or additional) active ingredient should not be such as to lose the advantageous benefit of the efficacious effect of the combination of the active ingredients. In addition, the delay in administering the second (or additional) active ingredient is typically timed so as to allow for any adverse side effects of the first compound to subside to an acceptable level before adminstration of the second compound, whilst not losing the advantageous benefit of the efficacious effect of the combination of the active ingredients.

The two or more treatments may be given in individually varying dose schedules and via the same or different routes.

For example, one compound may be administered by the oral route and the other compound administered by parenteral administration such as administration by injection (e.g. i.v.) or infusion. In an alternative, both compounds may be administered by injection or infusion. In a further alternative, both compounds may be given orally. In one particular embodiment, the compound of formula (II), (IV), (IVa), (Va), (Vb), (Via) or (VIb) and sub-groups is administered by injection or infusion and the an ancillary agent as hereinabove described is adminstered orally.

When administered at different times, the administration of one component of the combination may alternate with or interleaf with administration of the other component or the components of the combination may be administered in sequential blocks of therapy. As indicated above, the administration of the components of the combination may be spaced apart in time, for example by one or more hours, or days, or even weeks, provided that they form part of the same overall treatment.

In one embodiment of the invention, the compound of the formula (II), (IV), (IVa), (Va), (Vb), (Via) or (Vlb) and sub-groups thereof as defined herein is administered sequentially or simultaneously with the ancillary agent as hereinabove described. In another embodiment of the invention, the compound of the formula (II), (IV), (IVa), (Va), (Vb), (VIa) or (Vlb) and sub-groups thereof as defined herein is administered sequentially with the ancillary agent as hereinabove described in either order.

In a further embodiment, the ancillary agent as hereinabove described is administered prior to the compound of the formula (II), (IV), (IVa), (Va), (Vb), (Via) or (VIb) and sub-groups thereof as defined herein.

In another embodiment, the ancillary agent as hereinabove described is administered after the compound of the formula (II), (IV), (IVa), (Va), (Vb), (Via) or (VIb) and sub-groups thereof as defined herein.

In another embodiment of the invention, the compound of the formula (II), (IV), (IVa), (Va), (Vb), (Via) or (VIb), and sub-groups thereof as defined herein and the ancillary agent as hereinabove described are administered simultaneously.

In another embodiment, the compound of the formula (II), (IV), (IVa), (Va), (Vb), (Via) or (VIb) and sub-groups thereof as defined herein and the ancillary agent as hereinabove described are each administered in a therapeutically effective amount with respect to the individual components; in other words, the the compound of the formula (II), (IV), (IVa), (Va), (Vb), (Via) or (VIb) and sub-groups thereof as defined herein and the ancillary agent as hereinabove described are administered in amounts that would be therapeutically effective even if the components were administered other than in combination.

In another embodiment, the compound of the formula (II), (IV), (IVa), (Va), (Vb), (Via) or (VIb) and sub-groups thereof as defined herein and the ancillary agent as hereinabove described are each administered in a sub-therapeutic amount with respect to the individual components; in other words, the compound of the formula (II), (IV), (IVa), (Va), (Vb), (Via) or (Vlb) and sub-groups thereof as defined herein and an ancillary agent as hereinabove described are administered in amounts that would be therapeutically ineffective if the components were administered other than in combination.

Preferably, the ancillary agent as hereinabove described and the compound of the formula (II), (IV), (IVa), (Va), (Vb), (Via) or (VIb) and sub-groups thereof as defined herein interact in a synergistic or additive manner, and in particular an additive manner.

A typical daily dose of the compound of formula (II) can be in the range from 100 picograms to 100 milligrams per kilogram of body weight, more typically 5 nanograms to 25 milligrams per kilogram of bodyweight, and more usually 10 nanograms to 15 milligrams per kilogram (e.g. 10 nanograms to 10 milligrams, and more typically 1 microgram per kilogram to 20 milligrams per kilogram, for example 1 microgram to 10 milligrams per kilogram) per kilogram of bodyweight although higher or lower doses may be administered where required. The compound of the formula (II) can be administered on a daily basis or on a repeat basis every 2, or 3, or 4, or 5, or 6, or 7, or 10 or 14, or 21, or 28 days for example.

An example of a dosage for a 60 kilogram person comprises administering a compound of the formula (II) as defined herein, for example the free base of compound 4-(2,6-dichloro-benzoylamlno)-1H-pyrazole-3-carboxylic acid piperidin-4-ylamide at a starting dosage of 4.5-10.8 mg/60kg/day (equivalent to 75-180ug/kg/day) and subsequently by an efficacious dose of 44-97 mg/60kg/day (equivalent to 0.7-1.6 mg/kg/day) or an efficacious dose of 72-274 mg/60kg/day (equivalent to 1.2-4.6 mg/kg/day). The mg/kg dose would scale pro-rata for any given body weight.

An example of a dosage for the mesylate salt Is, at a starting dosage of 5.6-13.5 mg/60 kg/day (equivalent to 93-225 µg/kg/day/person) and subsequently by an efficacious dose of 55-122 mg/60 kg/day (equivalent to 0.9-2.0mg/kg/day/person) or an efficacious dose of 90-345 mg/60 kg/day (equivalent to 1.5-5.8 mg/kg/day/person).

In one particular dosing schedule, a patient will be given an infusion of a compound of the formula (II) for periods of one hour daily for up to ten days in particular up to five days for one week, and the treatment repeated at a desired interval such as two to four weeks, in particular every three weeks.

More particularly, a patient may be given an infusion of a compound of the formula (II) for periods of one hour daily for 5 days and the treatment repeated every three weeks.

In another particular dosing schedule, a patient is given an infusion over 30 minutes to 1 hour followed by maintenance infusions of variable duration, for example 1 to 5 hours, e.g. 3 hours.

In a further particular dosing schedule, a patient is given a continuous infusion for a period of 12 hours to 5 days, an in particular a continuous infusion of 24 hours to 72 hours.

Ultimately, however, the quantity of compound administered, the type of composition used, and the timing and frequency of the adinstration of the two components will be commensurate with the nature of the disease or physiological condition being treated and will be at the discretion of the physician.

Accordingly, a person skilled in the art would know through their common general knowledge the dosing regimes and combination therapies to use. It will be appreciated that the preferred method and order of administration and the respective dosage amounts and regimes for each component of the combination will depend on the particular ancillary agent or compound of formula I being administered, their route of administration, the particular tumour being treated and the particular host being treated. The optimum method and order of administration and dosage amounts and regime can be readily determined by those skilled in the art using conventional methods and in view of the information set out herein.

As described infra, the compounds of the formula (II), (IV), (IVa), (Va), (Vb), (VIa) or (VIb) and sub-groups are administered in combination therapy with one of more other cytotoxic compounds, for example in the treatment of a particular disease state (for example a neoplastic disease such as a cancer as hereinbefore defined). Examples of suitable cytotoxic compounds that may be used in the combinations of the invention are described in detail above.

However, the combinations of the invention may also be further combined with other classes of therapeutic agents or treatments that may be administered together (whether concurrently or at different time intervals) with the combinations of the invention, including (but not limited to):
1. camptothecin compounds;
2. antimetabolites;
3. vinca alkaloids;
4. taxanes;
5. platinum compounds;
6. DNA binders and Topo II inhibitors (including anthracycline derivatives);
7. signalling inhibitors (including PKB signalling pathway inhibitors);
8. Other therapeutic or prophylactic agents, for example agents that reduce or alleviate some of the side effects associated with chemotherapy. Particular examples of such agents include anti-emetic agents and agents that prevent or decrease the duration of chemotherapy-associated neutropenia and prevent complications that arise from reduced levels of red blood cells or white blood cells, for example erythropoietin (EPO), granulocyte macrophage-colony stimulating factor (GM-CSF), granulocyte-colony stimulating factor (G-CSF). In other embodiments, the other therapeutic or prophylactic agents can be as described below.

Alternatively, the combinations of the invention may also be further combined with other classes of therapeutic agents or treatments that may be administered together (whether concurrently or at different time intervals) with the combinations of the invention, including (but not limited to):
1. hormones, hormone agonists, hormone antagonists and hormone modulating agents (including antiandrogens, antiestrogens and GNRAs);
2. monoclonal antibodies (e.g. monoclonal antibodies to cell surface antigen(s));
3. camptothecin compounds;
4. antimetabolites;
5. vinca alkaloids;
6. taxanes;
7. platinum compounds;
8. DNA binders and Topo II inhibitors (including anthracycline derivatives);
9. alkylating agents (including aziridine, nitrogen mustard and nitrosourea alkylating agents);
10. a combination of two or more of the foregoing classes (1)-(9).
11. signalling inhibitors (including PKB signalling pathway Inhibitors);
12. CDK inhibitors;
13. COX-2 inhibitors;
14. HDAC inhibitors;
15. DNA methylase inhibitors;
16. proteasome inhibitors;
17. a combination of two or more of the foregoing classes (11)-(16);
18. a combination of two or more of the foregoing classes (1)-(17);
19. Other therapeutic or prophylactic agents, for example agents that reduce or alleviate some of the side effects associated with chemotherapy. Particular examples of such agents include anti-emetic agents and agents that prevent or decrease the duration of chemotherapy-associated neutropenia and prevent complications that arise from reduced levels of red blood cells or white blood cells, for example erythropoietin (EPO), granulocyte macrophage-colony stimulating factor (GM-CSF), granulocyte-colony stimulating factor (G-CSF). In other embodiments, the other therapeutic or prophylactic agents can be as described below.

### Other therapeutic or prophylactic agents

The compositions may also include other therapeutic or prophylactic agents, for example agents that reduce or alleviate some of the side effects associated with chemotherapy. Particular examples of such agents include anti-emetic agents and agents that prevent or decrease the duration of chemotherapy-associated neutropenia and prevent complications that arise from reduced levels of red blood cells or white blood cells, for example erythropoietin (EPO), granulocyte macrophage-colony stimulating factor (GM-CSF), and granulocyte-colony stimulating factor (G-CSF).

Also included are agents that inhibit bone resorption such as bisphosphonate agents e.g. zoledronate, pamidronate and ibandronate, as well as agents that suppress inflammatory responses (such as dexamethazone, prednisone, and prednisolone). Also included are agents used to reduce blood levels of growth hormone and IGF-I in acromegaly patients such as synthetic forms of the brain hormone somatostatin, which includes octreotide acetate which is a long-acting octapeptide with pharmacologic properties mimicking those of the natural hormone somatostatin. Further included are agents such as leucovorin, which is used as an antidote to drugs that decrease levels of folic acid, or folinic acid it self. In one particular embodiment is the combination of 5FU and leucovorin or 5FU and folinic acid. In addition megestrol acetate can be used for the treatment of side-effects including oedema and thromoembolic episodes.

Therefore in one embodiment the combinations further include an additional agent selected from erythropoietin (EPO), granulocyte macrophage-colony stimulating factor (GM-CSF), granulocyte-colony stimulating factor (G-CSF), zoledronate, pamidronate, ibandronate, dexamethazone, prednisone, prednisolone, leucovorin, folinic acid and megestrol acetate.

In particular the combinations further include an additional agent selected from erythropoietin (EPO), granulocyte macrophage-colony stimulating factor (GM-CSF), granulocyte-colony stimulating factor (G-CSF), zoledronate, pamidronate, dexamethazone, prednisone, prednisolone, leucovorin, and folinic acid such as erythropoietin (EPO), granulocyte macrophage-colony stimulating factor (GM-CSF) and granulocyte-colony stimulating factor (G-CSF).

Zoledronic acid is available from Novartis under the Tradename Zometa®. It is used in the treatment of bone metastasis in a variety of tumor types and for the treatment of hypercalcemia.

Pamidronate disodium (APD) available from Novartis under the tradename Aredia is a bone-resorption inhibitor and is used in the treatment of moderate or severe hypercalcemia. Pamidronate disodium is for i.v. injection.

Octreotide acetate is available from Novartis as Sandostatin LAR ® (octreotide acetate for injectable suspension) and Sandostatin® (octreotide acetate for injection ampuls or for vials). Octreotide is known chemically as L-Cysteinamide, D-phenylalanyl-L-cysteinyl-L-phenylalanyl-D-tryptophyl-L-lysyl-L-threonyl-N-[2-hydroxy-1-(hydroxy-methyl) propyl]-, cyclic (2, 7)-disulfide; [R-(R*,R*)]. Synthetic forms of the brain hormone somatostatin, such as octreotide, work at the site of the tumour. They bind to sst-2/sst-5 receptors to regulate gastrointestinal hormone secretion and affect tumour growth.

Each of the compounds present in the combinations of the invention may be given in individually varying dose schedules and via different routes.

Thus, administration of the compound of the formula (II), (IV), (IVa), (Va), (Vb), (Via) or (Vlb) and sub-groups in combination therapy with one or more cytotoxic compounds may comprise simultaneous or sequential administration. When administered sequentially, they can be administered at closely spaced intervals (for example over a period of 5-10 minutes) or at longer intervals (for example 1, 2, 3, 4 or more hours apart, or even longer periods apart where required), the precise dosage regimen being commensurate with the properties of the therapeutic agent(s).

The combinations of the invention may also be administered in conjunction with non-chemotherapeutic treatments such as radiotherapy, photodynamic therapy, gene therapy, surgery and controlled diets.

The combination therapy may therefore involve the formulation of the compound of the formula (II), (IV), (IVa), (Va), (Vb), (VIa) or (VIb) and sub-groups with one, two, three, four or more other therapeutic agents (including at least one of the specific ancillary agents described herein). Such formulations can be, for example, a dosage form containing two, three, four or more therapeutic agents. In an alternative, the individual therapeutic agents may be formulated separately and presented together in the form of a kit, optionally with instructions for their use.

A person skilled in the art would know through their common general knowledge the dosing regimes and combination therapies to use.

### Methods of Diagnosis

Prior to administration of a compound of the formula (II), a patient may be screened to determine whether a disease or condition from which the patient is or may be suffering is one which would be susceptible to treatment with a compound having activity against cyclin dependent kinases or treatmnent with an ancillary agent as hereinabove described. For example, a biological sample taken from a patient may be analysed to determine whether a condition or disease, such as cancer, that the patient is or may be suffering from is one which is characterised by a genetic abnormality or abnormal protein expression which leads to over-activation of CDKs or to sensitisation of a pathway to normal CDK activity. Examples of such abnormalities that result in activation or sensitisation of the CDK2 signal include up-regulation of cyclin E, (Harwell RM, Mull BB, Porter DC, Keyomarsi K.; J Biol Chem. 2004 Mar 26;279(13):12695-705) or loss of p21 or p27, or presence of CDC4 variants (Rajagopalan H, Jallepalli PV, Rago C, Velculescu VE, Kinzler KW, Vogelstein B, Lengauer C.; Nature. 2004 Mar 4;428(6978):77-81). The term up-regulation includes elevated expression or over-expression, including gene amplification (i.e. multiple gene copies) and increased expression by a transcriptional effect, and hyperactivity and activation, including activation by mutations. Thus, the patient may be subjected to a diagnostic test to detect a marker characteristic of up-regulation of cyclin E, or loss of p21 or p27, or presence of CDC4 variants. The term diagnosis includes screening. By marker we include genetic markers including, for example, the measurement of DNA composition to identify mutations of CDC4. The term marker also includes markers which are characteristic of up regulation of cyclin E, including enzyme activity, enzyme levels, enzyme state (e.g. phosphorylated or not) and mRNA levels of the aforementioned proteins.

Tumours with upregulation of cyclin E, or loss of p21 or p27 may be particularly sensitive to CDK inhibitors. Tumours may preferentially be screened for upregulation of cyclin E, or loss of p21 or p27 prior to treatment. Thus, the patient may be subjected to a diagnostic test to detect a marker characteristic of up-regulation of cyclin E, or loss of p21 or p27. The diagnostic tests are typically conducted on a biological sample selected from tumour biopsy samples, blood samples (isolation and enrichment of shed tumour cells), stool biopsies, sputum, chromosome analysis, pleural fluid, peritoneal fluid, or urine.

It has been found, Rajagopalan et al (Nature. 2004 Mar 4;428(6978):77-81), that there were mutations present in CDC4 (also known as Fbw7 or Archipelago) in human colorectal cancers and endometrial cancers (Spruck et al, Cancer Res. 2002 Aug 15;62(16):4535-9). Identification of individual carrying a mutation in CDC4 may mean that the patient would be particularly suitable for treatment with a CDK inhibitor. Tumours may preferentially be screened for presence of a CDC4 variant prior to treatment. The screening process will typically involve direct sequencing, oligonucleotide microarray analysis, or a mutant specific antibody.

Methods of identification and analysis of mutations and up-regulation of proteins are known to a person skilled in the art. Screening methods could include, but are not limited to, standard methods such as reverse-transcriptase polymerase chain reaction (RT-PCR) or in-situ hybridisation.

In screening by RT-PCR, the level of mRNA in the tumour is assessed by creating a cDNA copy of the mRNA followed by amplification of the cDNA by PCR. Methods of PCR amplification, the selection of primers, and conditions for amplification, are known to a person skilled in the art. Nucleic acid manipulations and PCR are carried out by standard methods, as described for example in Ausubel, F.M. et al., eds. Current Protocols in Molecular Biology, 2004, John Wiley & Sons Inc., or Innis, M.A. et-al., eds. PCR Protocols: a guide to methods and applications, 1990, Academic Press, San Diego. Reactions and manipulations involving nucleic acid techniques are also described in Sambrook et al., 2001, 3rd Ed, Molecular Cloning: A Laboratory Manual, Cold Spring Harbor Laboratory Press. Alternatively a commercially available kit for RT-PCR (for example Roche Molecular Biochemicals) may be used, or methodology as set forth in United States patents 4,666,828; 4,683,202; 4,801,531; 5,192,659, 5,272,057, 5,882,864, and 6,218,529.

An example of an in-situ hybridisation technique for assessing mRNA expression would be fluorescence in-situ hybridisation (FISH) (see Angerer, 1987 Meth. Enzymol., 152: 649).

Generally, in situ hybridization comprises the following major steps: (1) fixation of tissue to be analyzed; (2) prehybridization treatment of the sample to increase accessibility of target nucleic acid, and to reduce nonspecific binding; (3) hybridization of the mixture of nucleic acids to the nucleic acid in the biological structure or tissue; (4) post-hybridization washes to remove nucleic acid fragments not bound in the hybridization, and (5) detection of the hybridized nucleic acid fragments. The probes used in such applications are typically labeled, for example, with radioisotopes or fluorescent reporters. Preferred probes are sufficiently long, for example, from about 50, 100, or 200 nucteotides to about 1000 or more nucleotides, to enable specific hybridization with the target nucleic acid(s) under stringent conditions. Standard methods for carrying out FISH are described in Ausubel, F.M. et al., eds. Current Protocols in Molecular Biology, 2004, John Wiley & Sons Inc and Fluorescence In Situ Hybridization: Technical Overview by John M. S. Bartlett in Molecular Diagnosis of Cancer, Methods and Protocols, 2nd ed.; ISBN: 1-59259-760-2; March 2004, pps. 077-088; Series: Methods in Molecular Medicine.

Alternatively, the protein products expressed from the mRNAs may be assayed by immunohistochemistry of tumour samples, solid phase immunoassay with microtiter plates, Western blotting, 2-dimensional SDS-polyacrylamide gel electrophoresis, ELISA, flow cytometry and other methods known in the art for detection of specific proteins. Detection methods would include the use of site specific antibodies. The skilled person will recognize that all such well-known techniques for detection of upregulation of cyclin E, or loss of p21 or p27, or detection of CDC4 variants could be applicable in the present case.

Therefore all of these techniques could also be used to identify tumours particularly suitable for treatment with combinations of CDK inhibitors and ancillary agent as hereinabove described. Patients with mantle cell lymphoma (MCL) could be selected for treatment with a CDK inhibitor using diagnostic tests outlined herein. MCL is a distinct clinicopathologic entity of non-Hodgkin's lymphoma, characterized by proliferation of small to medium-sized lymphocytes with co-expression of CD5 and CD20, an aggressive and incurable clinical course, and frequent t(11;14)(q13;q32) translocation. Over-expression of cyclin D1 mRNA, found in mantle cell lymphoma (MCL), is a critical diagnostic marker. Yatabe et al (Blood. 2000 Apr 1;95(7):2253-61) proposed that cyclin D1-positivity should be included as one of the standard criteria for MCL, and that innovative therapies for this incurable disease should be explored on the basis of the new criteria. Jones et al (J Mol Diagn. 2004 May;6(2):84-9) developed a real-time, quantitative, reverse transcription PCR assay for cyclin D1 (CCND1) expression to aid in the diagnosis of mantle cell lymphoma (MCL). Howe et al (Clin Chem. 2004 Jan;50(1):80-7) used real-time quantitative RT-PCR to evaluate cyclin D1 mRNA expression and found that quantitative RT-PCR for cyclin D1 mRNA normalized to CD19 mRNA can be used in the diagnosis of MCL in blood, marrow, and tissue. Alternatively, patients with breast cancer could be selected for treatment with a CDK inhibitor using diagnostic tests outline above. Tumour cells commonly overexpress cyclin E and it has been shown that cyclin E is over-expressed in breast cancer (Harwell et al, Cancer Res, 2000, 60, 481-489). Therefore breast cancer may in particular be treated with a CDK inhibitor.

### EXAMPLES

The invention will now be illustrated, but not limited, by reference to the specific embodiments described in the following examples.

In the examples, the compounds prepared were characterised by liquid chromatography and mass spectroscopy (LC-MS) using the system and operating conditions set out below. Where chlorine is present and a single mass is quoted, the mass quoted for the compound is for ³⁵Cl. The two systems were equipped with identical chromatography columns and were set up to run under the same operating conditions. The operating conditions used are also described below. In the examples, the retention times are given in minutes.

### Platform system

| | |
|---|---|
| System: | Waters 2790/Platform LC |
| Mass Spec Detector | Micromass Platform LC |
| PDA Detector | Waters 996 PDA |

### Analytical conditions:

| | |
|---|---|
| Eluent A: | 5% CH3CN in 95% H₂O (0.1% Formic Acid) |
| Eluent B: | CH₃CN (0.1% Formic Acid) |
| Gradient: | 10-95% eluent B |
| Flow: | 1.2 ml/min |
| Column: | Synergi 4µm Max-RP C₁₂, 80A, 50 x 4.6 mm (Phenomenex) |

### MS conditions:

| | |
|---|---|
| Capillary voltage: | 3.5 kV |
| Cone voltage: | 30 V |
| Source Temperature: | 120 °C |

### FractionLynx system

| | |
|---|---|
| System: | Waters FractionLynx (dual analytical/prep) |
| Mass Spec Detector: | Waters-Micromass ZQ |
| PDA Detector: | Waters 2996 PDA |

### Analytical conditions:

| | |
|---|---|
| Eluent A: | H₂O (0.1% Formic Acid) |
| Eluent B: | CH₃CN (0.1 % Formic Acid) |
| Gradient: | 5-95% eluent B |
| Flow: | 1.5 ml/min |

| | |
|---|---|
| Column: Synergi 4µm Max-RP C₁₂, 80A, 50 x 4.6 mm (Phenomenex) | |

### MS conditions:

| | |
|---|---|
| Capillary voltage: | 3.5 kV |
| Cone voltage: | 30 V |
| Source Temperature: | 120 °C |
| Desolvation Temperature: | 300 °C |

### Analytical LC-MS System

Several systems were used, as described below, and these were equipped with were set up to run under closely similar operating conditions. The operating conditions used are also described below.

| | |
|---|---|
| HPLC System: | Waters 2795 |
| Mass Spec Detector: | Micromass Platform LC |
| PDA Detector: | Waters 2996 PDA |

### Acidic Analytical conditions :

| | |
|---|---|
| Eluent A: | H₂O (0.1% Formic Acid) |
| Eluent B: | CH₃CN (0.1% Formic Acid) |
| Gradient: | 5-95% eluent B over 3.5 minutes |
| Flow: | 0.8 ml/min |
| Column: | Phenomenex Synergi 4µ MAX-RP 80A, 2.0 x 50 mm |

### Basic Analytical conditions:

| | |
|---|---|
| Eluent A: | H₂O (10mM NH₄HCO₃ buffer adjusted to pH=9.5 with NH₄OH) |
| Eluent B: | CH₃CN |
| Gradient: | 05-95% eluent B over 3.5 minutes |
| Flow: | 0.8 ml/min |
| Column: | Thermo Hypersil-Keystone BetaBasic-18 5µm 2.1 x 50 mm |
| or | |
| Column: | Phenomenex Luna C18(2) 5µm 2.0 x 50 mm |

### Polar Analytical conditions:

| | |
|---|---|
| Eluent A: | H₂O (0.1% Formic Acid) |
| Eluent B: | CH₃CN (0.1% Formic Acid) |
| Gradient: | 00-50% eluent B over 3 minutes |
| Flow: | 0.8 ml/min |
| Column: | Thermo Hypersil-Keystone HyPurity Aquastar, 5µ, 2.1 x 50 mm |
| or | |
| Column: | Phenomenex Synergi 4µ MAX-RP 80A, 2.0 x 50 mm or |

### Longer Analytical conditions:

| | |
|---|---|
| Eluent A: | H₂O (0.1 % Formic Acid) |
| Eluent B: | CH₃CN (0.1% Formic Acid) |
| Gradient: | 05-95% eluent B over 15 minutes |
| Flow: | 0.4 ml/min |
| Column: | Phenomenex Synergi 4µ MAX-RP 80A, 2.0 x 150 mm |

### MS conditions:

| | |
|---|---|
| Capillary voltage: | 3.6 kV |
| Cone voltage: | 30 V |
| Source Temperature: | 120 °C |
| Scan Range: | 165-700 amu |
| lonisation Mode: | ElectroSpray Positive or |
| | ElectroSpray Negative or |
| | ElectroSpray Positive & Negative |

### Mass Directed Purification LC-MS System

The following preparative chromatography systems can be used to purify the compounds of the invention.
• Hardware:
Waters Fractionlynx system:
2767 Dual Autosampler/Fraction Collector
2525 preparative pump
CFO (column fluidic organiser) for column selection
RMA (Waters reagent manager) as make up pump
Waters ZQ Mass Spectrometer
Waters 2996 Photo Diode Array detector

• Software: Masslynx 4.0
• Columns:
1. Low pH chromatography: Phenomenex Synergy MAX-RP, 10µ, 150 x 15mm (alternatively used same column type with 100 x 21.2mm dimensions).
2. High pH chromatography: Phenomenex Luna C18 (2), 10 µ, 100 x 21.2 mm (alternatively used Thermo Hypersil Keystone BetaBasic C18, 5 µ, 100 x 21.2 mm)

• Eluents:
1. Low pH chromatography:
   Solvent A: H₂O + 0.1% Formic Acid, pH 1.5
   Solvent B: CH₃CN + 0.1% Formic Acid
2. High pH chromatography:
   Solvent A: H₂0 + 10 mM NH₄HCO₃ + NH₄OH, pH 9.5
   Solvent B: CH₃CN
3. Make up solvent: MeOH + 0.1% formic acid (for both chromatography type)

• Methods:
Prior to using preparative chromatography to isolate and purify the product compounds, analytical LC-MS (see above) can first be used to determine the most appropriate conditions for preparative chromatography. A typical routine is to run an analytical LC-MS using the type of chromatography (low or high pH) most suited for compound structure. Once the analytical trace shows good chromatography, a suitable preparative method of the same type can be chosen. Typical running condition for both low and high pH chromatography methods are:
   Flow rate: 24 ml/min
   Gradient: Generally all gradients have an initial 0.4 min step with 95% A + 5% B. Then according to analytical trace a 3.6 min gradient is chosen in order to achieve good separation (e.g. from 5% to 50% B for early retaining compounds; from 35% to 80% B for middle retaining compounds and so on)
   Wash: 1 minute wash step is performed at the end of the gradient
   Re-equilibration: A 2.1 minute re-equilibration step is carried out to prepare the system for the next run
   Make Up flow rate: 1 ml/min

• Solvent:
All compounds were usually dissolved in 100% MeOH or 100% DMSO

• MS running conditions:

| | |
|---|---|
| Capillary voltage: | 3.2 kV |
| Cone voltage: | 25 V |
| Source Temperature: | 120 °C |
| Multiplier: | 500 V |
| Scan Range: | 125-800 amu |
| lonisation Mode: | ElectroSpray Positive |

The starting materials for each of the Examples are commercially available unless otherwise specified.

### EXAMPLE 1 (Comparative Example)

### 4-Amino-1H-pyrazole-3-carboxylic acid phenylamide

### 1A. 4-Nitro-1H-pyrazole-3-carboxylic acid phenylamide

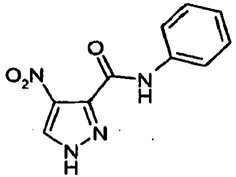

4-Nitropyrazole-3-carboxylic acid (2.5 g; 15.9 mmol) was added to a stirred solution of aniline (1.6 ml; 17.5 mmol), EDC (3.7 g; 19.1 mmol), and HOBt (2.6 g; 19.1 mmol) in N,N-dimethylformamide (DMF) (25 ml), then stirred at room temperature overnight. The solvent was removed by evaporation under reduced pressure and the residue triturated with ethyl acetate / saturated NaHCO₃ solution. The resultant solid was collected by filtration, washed with water and diethyl ether then dried under vacuum to give 2.85 g of the title compound (sodium salt) as a yellow / brown solid. (LC/MS: Rₜ 2.78, [M+H]⁺ 232.95).

### 1B. 4-Amino-1H-pyrazole-3-carboxylic acid phenylamide

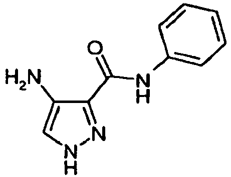

4-Nitro-1H-pyrazole-3-carboxylic acid phenylamide (100 mg; 0.43 mmol) was dissolved in ethanol (5 ml), treated with tin (II) chloride dihydrate (500 mg; 2.15 mmol) then heated at reflux overnight. The reaction mixture was cooled and evaporated. The residue was partitioned between ethyl acetate and brine, and the ethyl acetate layer was separated, dried (MgSO₄), filtered and evaporated. The crude product was purified by flash column chromatography eluting with 1:1 ethyl acetate /petroleum ether then 5% methanol / dichloromethane. Evaporation of product containing fractions followed by preparative LC/MS gave 15 mg of the product as an off white solid. (LC/MS: Rₜ 1.40, [M+H]⁺ 202.95).

### EXAMPLE 2 (comparative example)

### 4-Acetylamino-1H-pyrazole-3-carboxylic acid (4-fluoro-phenyl)-amide

### 2A. 4-Nitro-1H-pyrazole-3-carboxylic acid (4-fluoro-phenyl)-amide

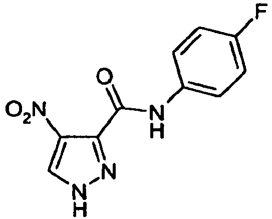

4-Nitropyrazole-3-carboxylic acid (10 g; 63.66 mmol) was added to a stirred solution of 4-fluoroaniline (6.7 ml; 70 mmol), EDC (14.6 g; 76.4 mmol), and HOBt (10.3 g; 76.4 mmol) in DMF (25 ml), then stirred at room temperature overnight. The solvent was removed by evaporation under reduced pressure and the residue triturated with ethyl acetate / saturated brine solution. The resultant yellow solid was collected by filtration, washed with 2M hydrochloric acid, then dried under vacuum to give 15.5 g of the title compound. (LC/MS: Rₜ 2.92 [M+H]⁺ 250.89).

### 2B. 4-Amino-1H-pyrazole-3-carboxylic acid (4-fluoro-phenyl)-amide

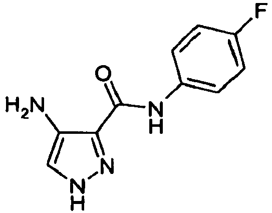

4-Nitro-1H-pyrazole-3-carboxylic acid (4-fluorophenyl)-amide (15 g) was dissolved in 200 ml of ethanol, treated with 1.5 g of 10% palladium on carbon under a nitrogen atmosphere, then hydrogenated at room temperature and pressure overnight The catalyst was removed by filtration through Celite and the filtrate evaporated. The crude product was dissolved in acetone / water (100 ml:100 ml) and after slow evaporation of the acetone the product was collected by filtration as a brown crystalline solid (8.1 g). (LC/MS: Rₜ 1.58, [M+H]⁺ 220.95).

### 2C. 4-Acetylamino-1H-pyrazole-3-carboxylic acid (4-fluoro-phenyl)-amide

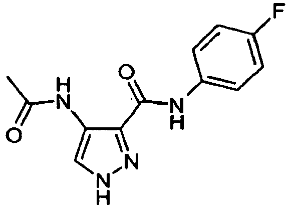

4-Amino-1H-pyrazole-3-carboxylic acid (4-fluorophenyl)-amide (500 mg; 2.27 mmol) was dissolved in 5 ml of pyridine, treated with acetic anhydride (240 µl, 2.5 mmol) then stirred at room temperature overnight. The solvent was removed by evaporation then dichloromethane (20 ml) and 2M hydrochloric acid (20 ml) were added. The undissolved solid was collected by filtration, washed with more dichloromethane and water then dried under vacuum. The product was isolated as an off white solid (275 mg). (LC/MS: Rₜ 2.96, [M+H]⁺ 262.91).

### EXAMPLE 3 (comparative example)

### 4-(2,2,2-Trifluoro-acetylamino)-1H-pyrazole-3-carboxylic acid (4-fluoro-phenyl)-amide

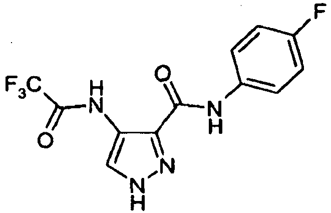

4-Amino-1H-pyrazole-3-carboxylic acid (4-fluorophenyl)-amide (Example 2B) (500 mg; 2.27 mmol) was dissolved in 5 ml of pyridine, treated with trifluoroacetic anhydride (320 µl, 2.5 mmol) then stirred at room temperature overnight. The solvent was removed by evaporation, the residue was partitioned between ethyl acetate (50 ml) and 2 M hydrochloric acid (50 ml), and the ethyl acetate layer was separated, washed with brine (50 ml), dried (MgSO₄), filtered and evaporated to give 560 mg of product as a brown solid. (LC/MS: [M+H]⁺ 317).

### EXAMPLE 4 (comparative example)

### 4-[(5-Oxo-pyrrolidine-2-carbonyl)-amino]-1H-pyrazole-3-carboxylic acid (4-fluoro-phenyl)-amide

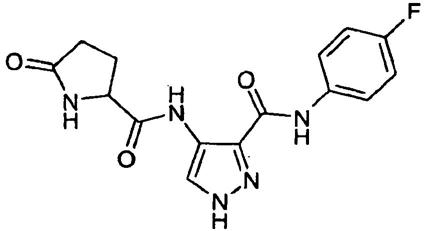

To a stirred solution of 4-amino-1H-pyrazole-3-carboxylic acid (4-fluorophenyl)-amide (Example 2B) (50 mg; 0.23 mmol), EDAC (52 mg; 0.27 mmol) and HOBt (37 mg; 0.27 mmol) in 5 ml of DMF was added 2-oxoproline (33 mg; 0.25 mmol), and the mixture was then left at room temperature overnight. The reaction mixture was evaporated and the residue purified by preparative LC/MS, to give 24 mg of the product as a white solid.
(LC/MS: Rₜ 2.27 [M+H]⁺ 332).

### EXAMPLE 5 (comparative example)

### 4-Phenylacetylamino-1H-pyrazole-3-carboxylic acid (4-fluoro-phenyl)-amide

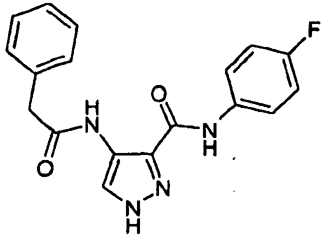

The reaction was carried out in a manner analogous to Example 4 but using phenylacetic acid (34mg; 0.23 mmol) as the starting material. The title compound (14 mg) was isolated as a white solid. (LC/MS: Rt 3.24 [M+H]⁺ 339).

### EXAMPLE 6 (comparative example)

### 4-(2-1H-Indol-3-yl-acetylamino)-1H-pyrazole-3-carboxylic acid (4-fluoro-phenyl)-amide

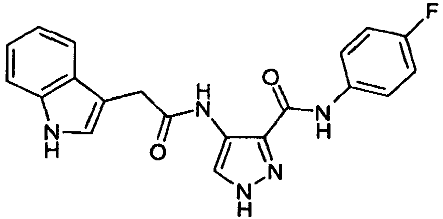

The reaction was carried out in a manner analogous to Example 4, but using indole-3-acetic acid (44 mg; 0.23 mmol) as the starting material. The title product (14 mg) was Isolated as a white solid. (LC/MS: Rₜ 3.05 [M+H]⁺ 378).

### EXAMPLE 7 (comparative example)

### 4-(2-Benzenesulphonyl-acetylamino)-1H-pyrazole-3-carboxylic acid (4-fluoro-phenyl)-amide

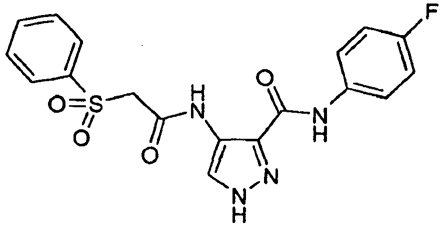

The reaction was carried out in a manner analogous to Example 4, but using 2-(phenylsulphonyl) acetic acid (50 mg; 0.23 mmol) as the starting material. The title compound (29 mg) was isolated as a white solid. (LC/MS: Rt 3.00 [M+H]⁺ 403).

### EXAMPLE 8 (comparative examples)

### 4-[2-(5-Amino-tetrazol-1-yl)-acetylaminol-1H-pyrazole-3-carboxylic acid (4-fluoro-phenyl)-amide

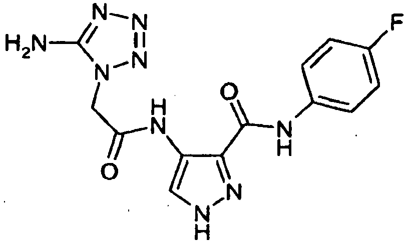

The reaction was carried out in a manner analogous to Example 4, but 5-aminotetrazole-1-acetic acid (36 mg; 0.23 mmol) was used as the starting material. The title compound (23 mg) was isolated as a white solid.
(LC/MS: Rₜ 2.37 [M+H]⁺ 346).

### EXAMPLE 9 (comparative example)

### N-[3-(4-Fluoro-phenylcarbamoyl)-1H-pyrazol-4-yl-6-hydroxy-nicotinamide

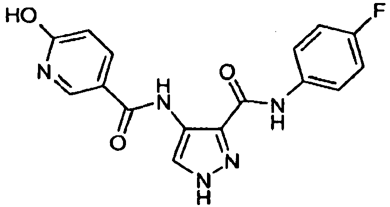

The reaction was carried out in a manner analogous to Example 4, but using 6-hydroxynicotinic acid (38 mg; 0.23 mmol) as the starting material. The title compound (17 mg) was isolated as a white solid. (LC/MS: Rₜ 2.32 [M+H]⁺ 342).

### EXAMPLE 10 (comparative example)

### 4-[3-(4-Chloro-phenyl)-propionylaminol-1H-pyrazole-3-carboxylic acid (4-fluoro-phenyl)-amide

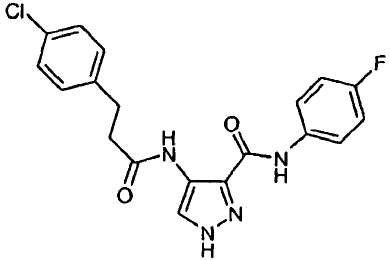

The reaction was carried out in a manner analogous to Example 4, but using 3-(4-chlorophenyl)propionic acid (46 mg; 0.23 mmol) as the starting material. The title compound (40 mg) was isolated as a white solid. (LC/MS: Rₜ 3.60 [M+H]⁺ 388).

### EXAMPLE 11 (comparative example)

### 4-(3-4H-[1,2,4]Triazol-3-yl-propionylamino)-1H-pyrazole-3-carboxylic acid (4-fluoro-phenyl)-amide

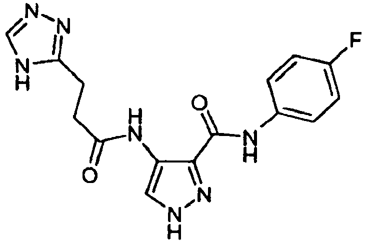

The reaction was carried out in a manner analogous to Example 4, but using 3triazol-3-yl propionic acid (36 mg; 0.23 mmol) as the starting material. The title compound (18 mg) was isolated as a white solid. (LC/MS: Rₜ 2.39 [M+H]⁺ 344).

### EXAMPLE 12 comparative example)

### 4-[2-(1-Methyl-1H-indol-3-yl)-acetylaminol-1H-pyrazole-3-carboxylic acid (4-fluoro-phenyl)-amide

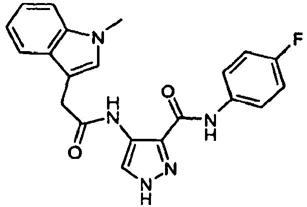

The reaction was carried out in a manner analogous to Example 4, but using N-methyl indole-3-acetic acid (48 mg; 0.23 mmol) as the starting material. The title compound (20 mg) was isolated as a white solid. (LC/MS: Rₜ 3.34 [M+H]⁺ 392).

### EXAMPLE 13 (comparative example)

### 4-[(1-Hydroxy-cyclopropanecarbonyl)-amino]-1H-pyrazole-3-carboxylic acid (4-fluoro-phenyl)-amide

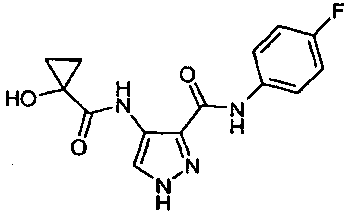

The reaction was carried out in a manner analogous to Example 4, but using 1-hydroxycyclopropane carboxylic acid (26 mg; 0.23 mmol) as the starting material. The title compound (24 mg) was isolated as a white solid.
(LC/MS: Rₜ 2.55 [M+H]⁺ 305).

### EXAMPLE 14 (comparative example)

### 1-Acetyl-piperidine-4-carboxylic acid [3-(4-fluoro-phenylcarbamoyl)-1H-pyrazol-4-yl]-amide

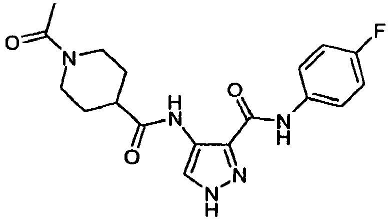

The reaction was carried out in a manner analogous to Example 4, but using N-acetylpiperidine acetic acid (43 mg; 0.23 mmol) as the starting material. The title compound (19 mg) was isolated as a white solid. (LC/MS: Rₜ 2.49 [M+H]⁺ 374).

### EXAMPLE 15 (comparative example)

### 4-[3-(4-Methyl-piperazin-1-yl)-propionylaminol-1H-pyrazole-3-carboxylic acid (4-fluoro-phenyl)-amide

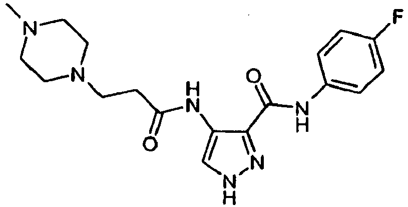

The reaction was carried out in a manner analogous to Example 4, but using 4-N-methylpiperazine-1-N-propionic acid (31 mg; 0.23 mmol) as the starting material. The title compound (19 mg) was isolated as a white solid. (LC/MS: Rₜ 1.77 [M+H]⁺ 375).

### EXAMPLE 16 (comparative example)

### 4-(2-1H-Imidazol-4-yl-acetylamino)-1H-pyrazole-3-carboxylic acid (4-fluorophenyl)-amide

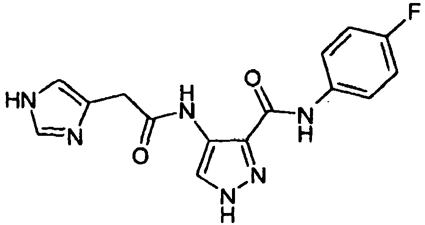

The reaction was carried out in a manner analogous to Example 4, but using imidazole-4-acetic acid (32 mg; 0.23 mmol) as the starting material. The title compound (35 mg) was isolated as a white solid. (LC/MS: Rₜ 1.82 [M+H]⁺ 329).

### EXAMPLE 17 (comparative example)

### 4-(3-Morpholin-4-yl-propionylamino)-1H-pyrazole-3-carboxylic acid (4-fluorophenyl)-amide

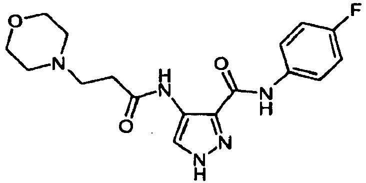

The reaction was carried out in a manner analogous to Example 4, but using 3-morpholin-4-yl-propionic acid (40 mg; 0.23 mmol) as the starting material. The title compound (15 mg)was isolated as a white solid. (LC/MS: Rₜ 1.84 [M+H]⁺ 362).

### EXAMPLE 18 (comparative example)

### 4-(3-piperidin-1-yl-propionylamino)-1H-pyrazole-3-carboxylic acid (4-fluoro-phenyl)-amide

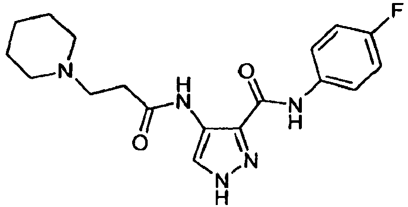

The reaction was carried out in a manner analogous to Example 4, but using 3-piperidine-4-yl-proplonic acid (39 mg; 0.23 mmol) as the starting material. The title compound (19 mg) was isolated as a white solid. (LC/MS: Rₜ 1.92 [M+H]⁺ 360).

### EXAMPLE 19 (Comparative Example)

### 4-Cyclohexylamino-1H-pyrazole-3-carboxylic acid (4-fluoro-phenyl)-amide

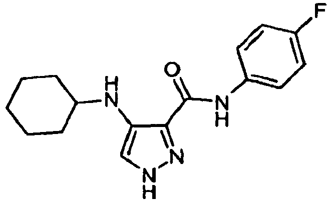

To a solution of 4-amino-1H-pyrazole-3-carboxylic acid (4-fluoro-phenyl)-amide (200 mg; 1 mmol) and cyclohexanone (107 mg; 1.1 mmol) in dichloromethane (10 ml) were added 3A molecular sieves (1 g) and sodium triacetoxyborohydride (315 mg; 1.5 mmol), and the mixture was then stirred at room temperature over the weekend. The reaction mixture was filtered through Celite^{®}, diluted with ethyl acetate, washed with brine, dried (MgSO₄) and evaporated to give the 48 mg of the product as a grey gum. (LC/MS: Rₜ 2.95, [M+H]⁺ 285).

### EXAMPLE 20 (Comparative Example)

### 4-isopropylamino-1H-pyrazole-3-carboxylic acid (4-fluoro-phenyl)-amide

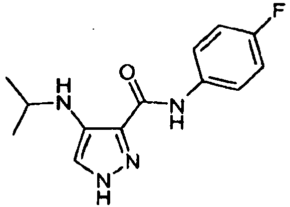

The title compound was prepared in a manner analogous to Example 19, but using acetone in place of cyclohexanone. (LC/MS: Rₜ 2.08, [M+H]⁺ 245).

### EXAMPLE 21 (Comparative Example)

### 4-(2-Hydroxy-1-methyl-ethylamino)-1H-pyrazole-3-carboxylic acid (4-fluorophenyl)-amide

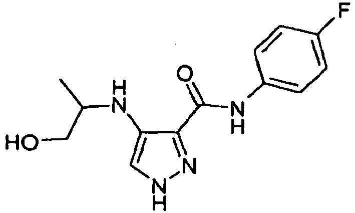

The compound was prepared in a manner analogous to Example 19, but using hydroxyacetone in place of cyclohexanone. ¹HNMR (400MHz, D6-DMSO): 9.9 (1H, br s), 7.8 (2H, dd), 7.3 (1H, s), 7.15 (2H, t), 5.15 (1H, d), 4.7 (1H, br s), 3.4 (2H, m), 3.2 (1H, m), 1.1 (3H, d).

### EXAMPLE 22 (Comparative Example)

### 4-(1-Ethyl-propylamino)-1H-pyrazole-3-carboxylic acid (4-fluoro-phenyl)-amide

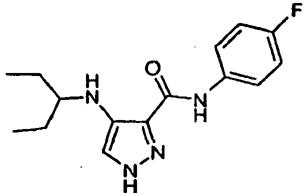

The compound was prepared in a manner analogous to Example 19, but using 3-pentanone In place of cyclohexanone. ¹HNMR (400MHz, D6-DMSO): 12.85 (1 h,br s), 9.9 (1H, br s), 7.8 (2H, br t), 7.3 (1 H, s), 7.15 (2H, t), 5.0 (1H, d), 2.9 (1H, br m), 1.5 (4H, m), 3.2 (1H, m), 0.9 (6H, t).

### EXAMPLE 23 (Comparative Example)

### 4-(3-Chloro-pyrazin-2-ylamino)-1H-pyrazole-3-carboxylic acid (4-fluoro-phenyl)-amide

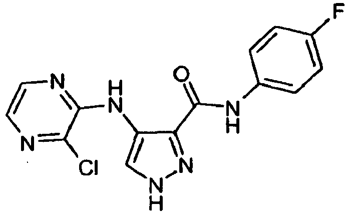

A mixture of 4-amino-1H-pyrazole-3-carboxylic acid (4-fluoro-phenyl)-amide (50 mg; 0.23 mmol) and 2,3-dichloropyrazine (140 mg; 0.92 mmol) was heated at 150°C (50W) for 20 minutes in a CEM Discover^{™} microwave synthesiser. The crude reaction mixture was purified by flash column chromatography eluting with ethyl acetate / hexane (1:3 then 1:2). Product containing fractions were combined and evaporated to give 15 mg of the title compound as a white solid. (LC/MS: Rₜ 4.06 M+H]⁺ 332).

### EXAMPLE 24 (Comparative Example)

### 4-(Pyrazin-2-ylamino)-1H-pyrazole-3-carboxylic acid (4-fluoro-phenyl)-amide

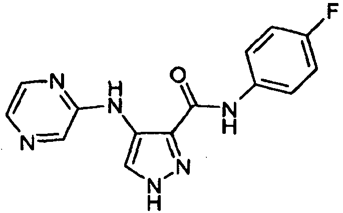

The compound was prepared in a manner analogous to Example 23, but using 2-chloropyrazine in place of 2,3-dichloropyrazine. (LC/MS: Rₜ 3.28 [M+H]⁺ 299).

### EXAMPLE 25 (comparative example)

### Synthesis of 4-(2-Methoxy-benzoylamino)-1H-pyrazole-3-carboxylic acid (4-fluoro-phenyl)-amide

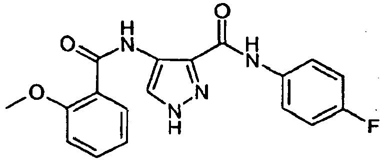

2-Methoxy-benzolc acid (38 mg, 0.25 mmol) was added to a solution of 4-amino-1H-pyrazole-3-carboxylic acid (4-fluoro-phenyl)-amide (50 mg, 0.23 mmol), EDC (53 mg, 0.27 mmol), and HOBt (37 mg, 0.27 mmol) in DMF (5ml). The reaction mixture was stirred at room temperature for 24 hours. The solvent was removed under reduced pressure. The residue was purified by preparative LC/MS and, after evaporation of product-containing fractions, yielded the product as a pinkish solid (12 mg, 15%). (LC/MS: Rₜ 4.00, [M+H]⁺ 354.67).

### EXAMPLE 26 (comparative example)

### Synthesis of 4-Benzoylamino-1H-pyrazole-3-carboxylic acid (4-fluoro-phenyl)-amide

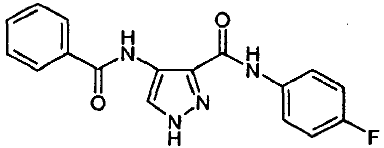

The experiment was carried out in a manner analogous to that of Example 25 using benzoic acid (31 mg, 0.25 mmol) as starting acid. The product was isolated as a pink solid (26 mg, 35%). (LC/MS: Rₜ 3.96, [M+H]⁺ 324.65).

### EXAMPLE 27 (comparative example)

### Synthesis of 4-(Cyclohexanecarbonyl-amino)-1H-pyrazole-3-carboxylic acid (4-fluoro-phenyl)-amide

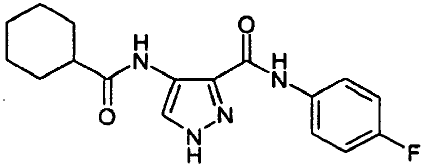

The experiment was carried out in a manner analogous to that of Example 25 using cyclohexanecarboxylic acid (32 mg, 0.25 mmol) as starting acid. The product was isolated as a pink solid (28 mg, 37%). (LC/MS: Rₜ 4.16, [M+H]⁺ 330.70).

### EXAMPLE 28 (comparative example)

### Synthesis of 4-[(1-Methyl-cyclopropanecarbonyl)-amino]-1H-pyrazole-3-carboxylic acid (4-fluoro-phenyl)-amide

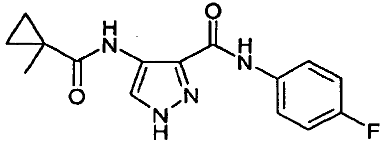

The experiment was carried out in a manner analogous to that of Example 25 using 1-methyl-cyclopropanecarboxylic acid (25 mg, 0.25 mmol) as starting acid. The product was isolated as a pink solid (24 mg, 35%). (LC/MS: Rₜ 3.72, [M+H]⁺ 302.68).

### EXAMPLE 29 (comparative example)

### Synthesis of 4-(2-Hydroxy-acetylamino)-1H-pyrazole-3-carboxylic acid (4-fluoro-phenyl)-amide

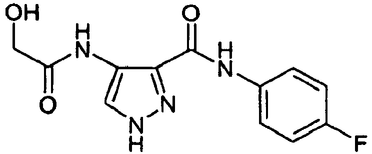

The experiment was carried out in a manner analogous to that of Example 25 using hydroxy-acetic acid (19 mg, 0.25 mmol) as starting acid. The product was isolated as a white solid (26 mg, 41%). (LC/MS: Rₜ 2.65, [M+H]⁺ 278.61).

### EXAMPLE 30 (comparative example)

### Synthesis of 4-(2,2-Dimethyl-propionylamino)-1H-pyrazole-3-carboxylic acid (4-fluoro-phenyl)-amide

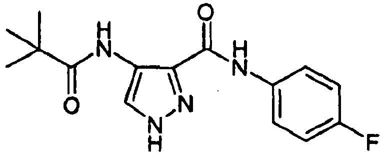

The experiment was carried out in a manner analogous to that of Example 25 using 2,2-dimethyl-propionic acid (26 mg, 0.25 mmol) as starting acid. The product was isolated as a pink solid (21 mg, 30%). (LC/MS: Rₜ 3.83, [M+H]⁺ 304.68).

### EXAMPLE 31 (comparative example)

### Synthesis of 4-(3-Hydroxy-propionylamino)-1H-pyrazole-3-carboxylic acid (4-fluoro-phenyl)-amide

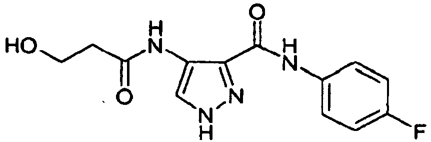

The experiment was carried out in a manner analogous to that of Example 25 using 3-hydroxy-propionic acid (75.1 mg, 0.25 mmol) as starting acid. The product was isolated as a beige solid (5 mg, 8%). (LC/MS: Rₜ 2.58, [M+H]⁺ 292.65).

### EXAMPLE 32 (comparative example)

### Synthesis of 4-(2-Fluoro-benzoylaminol-1H-pyrazole-3-carboxylic acid (4-fluoro-phenyl)-amide

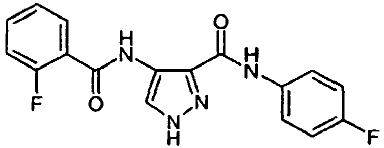

2-Fluorobenzoic acid (36 mg, 0.25 mmol) was added to a solution of 4-amino-1H-pyrazole-3-carboxylic acid (4-fluoro-phenyl)-amide (50 mg, 0.23 mmol), EDC (53 mg, 0.27 mmol) and HOBt (37 mg, 0.27 mmol) in DMSO (1 ml). The reaction mixture was stirred at room temperature for 24 hours and purified by preparative LC/MS. Evaporation of product-containing fractions yielded the product as a white solid (15 mg, 19 %). (LC/MS: Rₜ 3.91, [M+H]⁺ 342.66).

### EXAMPLE 33 (comparative example)

### Synthesis of 4-(3-Fluoro-benzoylamino)-1H-pyrazole-3-carboxylic acid (4-fluoro-phenyl)-amide

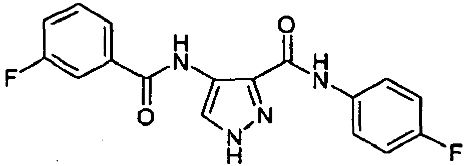

The experiment was carried out in a manner analogous to that of Example 32 using 3-fluorobenzoic acid (36 mg, 0.25 mmol) as starting acid. The product was isolated as a white solid (19 mg, 24%). (LC/MS: Rₜ 4.03, [M+H]⁺ 342.67).

### EXAMPLE 34 (comparative example)

### Synthesis of 4-(3-Methoxy-benzoylamino)-1H-pyrole-3carboxylic acid (4-fluoro-phenyl)-amide

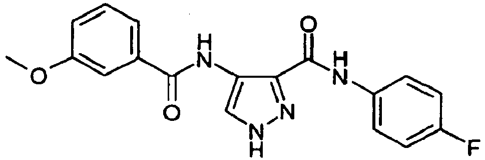

The experiment was carried out in a manner analogous to that of Example 32 using 3-methoxy-benzoic acid (39 mg, 0.25 mmol) as starting acid. The product was isolated as a white solid (20 mg, 25%). (LC/MS: Rₜ 3.97, [M+H]⁺ 354.68).

### EXAMPLE 35 (comparative example)

### Synthesis of 4-(2-Nitro-benzolamino)-1H-pyrazole-3-carboxylic acid (4-fluoro-phenyl)-amide

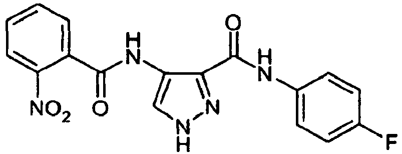

The experiment was carried out in a manner analogous to that of Example 32 using 2-nitrobenzoic acid (43 mg, 0.25 mmol) as starting acid. The product was isolated as a white solid (17 mg, 20%). (LC/MS: Rₜ 3.67, [M+H]⁺ 369.66).

### EXAMPLE 36 (comparative example)

### Synthesis of 4-(4-Nitro-benzoylamino)-1H-pyrazole-3-carboxylic acid (4-fluoro-phenyl)-amide

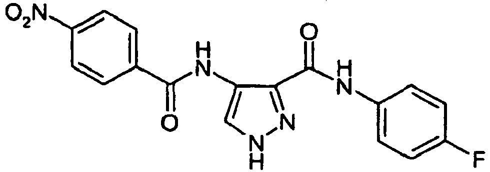

The experiment was carried out in a manner analogous to that of Example 32 using 4-nitrobenzoic acid (43 mg, 0.25 mmol) as starting acid. The product was isolated as a white solid (15 mg, 18%). (LC/MS: Rₜ 3.98, [M+H]⁺ 369.63).

### EXAMPLE 37 (comparative example)

### Synthesis of 4-[(3-Methyl-furan-2-carbonyl)-amino]-1H-pyrazole-3-carboxylic acid (4-fluoro-phenyl)-amide

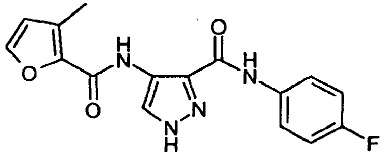

The experiment was carried out in a manner analogous to that of Example 32 using 3-methyl-2-furoic acid (32 mg, 0.25 mmol) as starting acid. The product was isolated as a white solid (15 mg, 20%). (LC/MS: Rₜ 3.86, [M+H]⁺ 328.68).

### EXAMPLE 38 (comparative example)

### Synthesis of 4-[(Furan-2-carbonyl)-aminol-1H-pyrazole-3-carboxylic acid (4-fluoro-phenyl)-amide

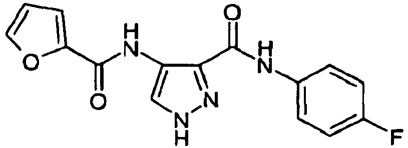

The experiment was carried out in a manner analogous to that of Example 32 using 2-furoic acid (29 mg, 0.25 mmol) as starting acid. The product was isolated as a white solid (18 mg, 25%). (LC/MS: Rₜ 3.56, [M+H]⁺ 314.64).

### EXAMPLE 39 (comparative example)

### Synthesis of 4-[(3H-Imidazole-4-carbonyl)-amino]-1H-pyrazole-3-carboxylic acid (4-fluoro-phenyl)-amide

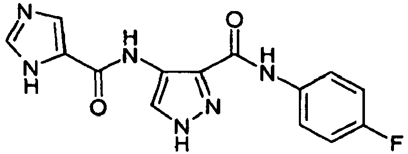

The experiment was carried out in a manner analogous to that of Example 32 using 1H-imidazole-4-carboxylic acid (29 mg, 0.25 mmol) as starting acid. The product was isolated as a white solid (16 mg, 22%). (LC/MS: Rₜ 2.59, [M+H]⁺ 314.65).

### EXAMPLE 40 (comparative example)

### Synthesis of 4-(4-Fluoro-benzoylamlno)-1H-pyrazole-3-carboxylic acid (4-fluoro-phenyl)-amide

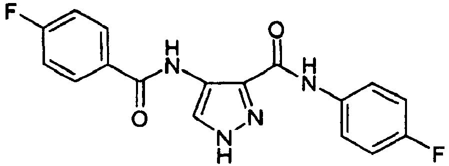

The experiment was carried out in a manner analogous to that of Example 32 using 4-fluorobenzoic acid (36 mg, 0.25 mmol) as starting acid. The product was isolated as a cream coloured solid (23 mg, 29%). (LC/MS: Rₜ 4.00, [M+H]⁺ 342.67).

### EXAMPLE 41 (comparative example)

### Synthesis of 4-(2,6-Difluoro-benzoylamino)-1H-pyrazole-3-carboxylic acid (4-fluoro-phenyl)-amide

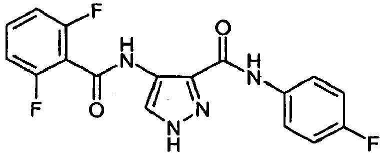

The experiment was carried out in a manner analogous to that of Example 32 using 2,6-difluorobenzoic acid (40 mg, 0.25 mmol) as starting acid. The product was isolated as a cream coloured solid (25 mg, 30%).
(LC/MS: Rₜ 3.76, [M+H]⁺ 360.66).

### EXAMPLE 42 (comparative example)

### Synthesis of 4-(3-Nitro-benzoylamino)-1H-pyrazole-3-carboxylic acid (4-fluoro-phenyl)-amide

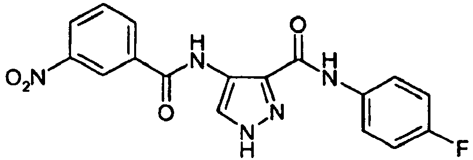

The experiment was carried out in a manner analogous to that of Example 32 using 3-nitrobenzoic acid (43 mg, 0.25 mmol) as starting acid. The product was isolated as a cream coloured solid (15 mg, 18%). (LC/MS: Rₜ 3.94, [M+H]⁺ 369.65).

### EXAMPLE 43 (comparative example)

### Synthesis of 1H-Indole-3-carboxylic acid [3-(4-fluoro-phenylcarbamoyl)-1H-pyrazol-4-yl]-amide

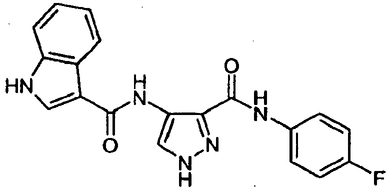

The experiment was carried out in a manner analogous to that of Example 32 using indole-3-carboxylic acid (41 mg, 0.25 mmol) as starting acid. The product was isolated as a rust coloured solid (14 mg, 17%). (LC/MS: Rₜ 3.60, [M+H]⁺ 363.66).

### EXAMPLE 44 (comparative example)

### Synthesis of 4-(4-Hydroxymethyl-benzoylamino)-1H-pyrazole-3-carboxylic acid (4-fluoro-phenyl)-amide

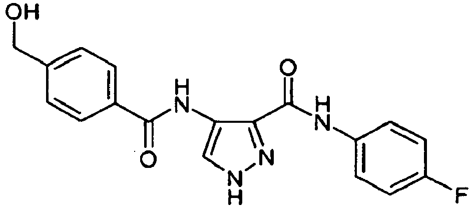

The experiment was carried out In a manner analogous to that of Example 32 using 4-hydroxymethylbenzoic acid (39 mg, 0.25 mmol) as starting acid. The product was isolated as a white solid (19 mg, 23%). (LC/MS: Rₜ 3.12, [M+H]⁺ 354.68).

### EXAMPLE 45 (comparative example)

### Synthesis of 4-(3-Methyl-benzoylamino)-1H-pyrazole-3-carboxylic acid (4-fluoro-phenyl)-amide

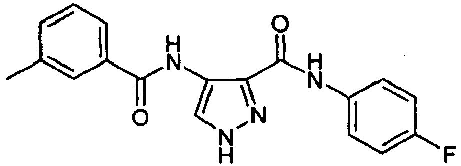

The experiment was carried out in a manner analogous to that of Example 32 using 3-methylbenzoic acid (35 mg, 0.25 mmol) as starting acid. The product was isolated as an off- white solid (21 mg, 27%). (LC/MS: Rₜ 4.13, [M+H]⁺ 338.71).

### EXAMPLE 46 (comparative example)

### Synthesis of 4-(2-Methyl-benzoylamino)-1H-pyrazole-3-carboxylic acid (4-fluoro-phenyl)-amide

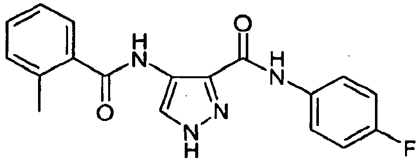

The experiment was carried out in a manner analogous to that of Example 32 using 2-methylbenzoic acid (35 mg, 0.25 mmol) as starting acid. The product was Isolated as an off-white solid (20 mg, 26%). (LC/MS: Rₜ 4.05, [M+H]⁺ 338.69).

### EXAMPLE 47 (comparative example)

### Synthesis of 4-(4-Methyl-benzoylamino)-1H-pyrazole-3-carboxylic acid (4-fluoro-phenyl)-amide

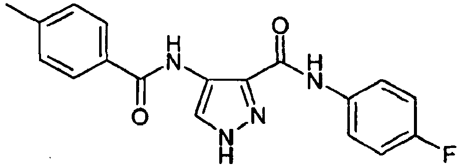

The experiment was carried out in a manner analogous to that of Example 32 using 4-methylbenzoic acid (35 mg, 0.25 mmol) as starting acid. The product was isolated as an off- white solid (19 mg, 24%). (LC/MS: Rₜ 4.16, [M+H]⁺ 338.70).

### EXAMPLE 48 (comparative example)

### Synthesis of 4-[(2-Methyl-thiophene-3-carbonyl)-aminol-1H-pyrazole-3-carboxylic acid (4-fluoro-phenyl)-amide

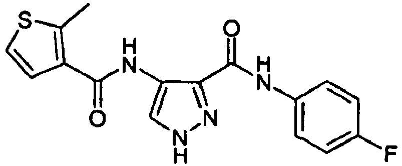

2-Methyl-3-thiophenecarboxylic acid (36 mg, 0.25 mmol) was added to a solution of 4-amino-1H-pyrazole-3-carboxylic acid (4-fluoro-phenyl)-amide (Example 2B) (50 mg, 0.23 mmol), EDC (53 mg, 0.27 mmol), and HOBt (37 mg, 0.27 mmol) in DMSO (1 ml). The reaction mixture was stirred at room temperature for 24 hours. The reaction mixture was added dropwise to water (30 ml) and the resultant solid was collected by filtration, washed with water and sucked dry. The title compound was obtained as a beige solid (15 mg, 19%). (LC/MS: Rₜ 4.08, [M+H]⁺ 344.67).

### EXAMPLE 49 (comparative example)

### Synthesis of Quinoline-2-carboxylic acid [3-(4-fluoro-phenylcarbamoyl)-1H-pyrazol-4-yl]-amide

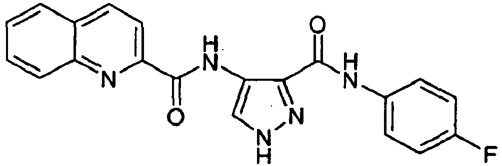

The experiment was carried out in a manner analogous to that of Example 48 using quinaldic acid (44 mg, 0.25 mmol) as starting acid. The product was isolated as a brown solid (16 mg, 19%). (LC/MS: Rₜ 4.29, [M+H]⁺ 375.66).

### EXAMPLE 50 (comparative example)

### Synthesis of 4-[(Thiophene-3-carbonyl)-amino]-1H-pyrazole-3-carboxylic acid (4-fluoro-phenyl)-amide

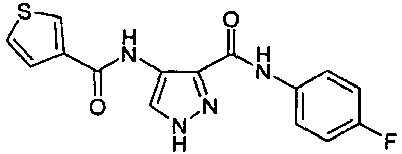

The experiment was carried out in a manner analogous to that of Example 48 using thiophene-3-carboxylic acid (33 mg, 0.25 mmol) as starting acid. The product was isolated as a beige solid (15 mg, 20%). (LC/MS: Rₜ 3.77, [M+H]⁺ 330.61).

### EXAMPLE 51 (comparative example)

### 4-(2-fluoro-3-methoxy-benzoylamino)-1H-pyrazole-3-carboxylicacid (4-fluoro-phenyl)-amide

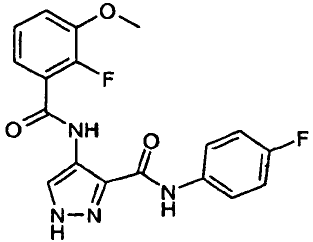

2-Fluoro-3-methoxybenzoic acid (0.047 g, 0.28 mmol), 4-amino-1H-pyrazole-3-carboxylic acid (4-fluorophenyl)-amide (Example 2B) (0.055 g, 0.25 mmol), EDC (0.58 g, 0.30 mmol) and HOBt (0.041 g, 0.30 mmol) were stirred at room temperature in DMSO (1.25 ml) for 5 hours. The reaction mixture was poured into water (30 ml) and the resultant solid was collected by filtration and dried in a vacuum oven to give the title compound as a grey solid (0.058 g, 63 %). (LC/MS: Rₜ 3.99, [MH]⁺ 372.98).

### EXAMPLE 52 (comparative example)

### Synthesis of 4-[2-(2-Pyrrolidin-l-yl-ethoxy)-benzoylamino]-1H-pyrazole-3-carboxylic acid 4-fluorophenylamide

### 52A 2-(2-Pyrrolidin-1-yl-ethoxy)-benzoic acid methyl ester

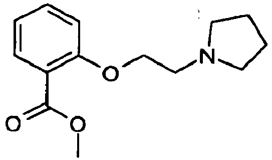

Diisopropylazodicarboxylate (0.404 g, 2 mmol) was added dropwise to a solution of triphenylphosphine (0.524 g, 2 mmol) in THF (10 ml). Methyl salicylate (0.304 g, 2 mmol) was added dropwise and the resultant mixture was stirred at room temperature for 1 hour. 1,2-Hydroxyethyl pyrrolidine (0.230 g, 2 mmol) was added dropwise and the reaction mixture was left stirring at room temperature for a further 1.5 hours. The resulting solution was reduced in vacuo and subject to flash column chromatography, eluting with hexane: ethyl acetate (5:1, 1:1) then ethyl acetate: methanol (4:1) to give the product as a clear yellow oil (0.104 g, 21 %). (LC/MS: Rₜ 0.69, 1.62, [MH]⁺ 250.02).

### 52B. 4-[2-(2-Pyrrolidin-1-yl-ethoxy)-benzoylamino1-1H-pyrazole-3-carboxylic acid 4-fluorophenylamide

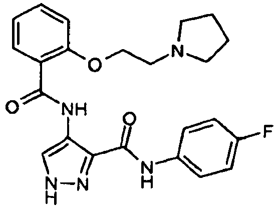

2-(2-Pyrrolidln-1-yl-ethoxy)-benzoic acid methyl ester (0.104 g. 0.42 mmol) was treated with 2 M aqueous NaOH (20 ml) and water (20 ml). The reaction mixture was stirred at room temperature for 20 hours, then reduced in vacuo and azeotroped with toluene (3 x 5 ml). Water (50 ml) was added and the mixture taken to pH 5 using 1M aqueous HCl. The resulting solution was reduced in vacuo and azeotroped with toluene (3 x 5 ml) to give a white solid, which was combined with 4-amino-1H-pyrazole-3-carboxylic acid (4-fluoro-phenyl)-amide (Example 2B) (0.055 g, 0.25 mmol), EDC (0.058 g, 0.3 mmol) and HOBt (0.041g, 0.3 mmol) and stirred at room temperature in DMSO (3 ml) for 20 hours. The reaction mixture was poured into water (30 ml) and the resultant solid was collected by filtration and dried in a vacuum oven to give the title compound as a grey solid (0.015 g, 14 %). (LC/MS: Rₜ 2.18, [MH]⁺ 438.06).

### EXAMPLE 53 (comparative example)

### Synthesis of 4-(2,6-Difluoro-benzoylamino)-1H-pyrazole-3-carboxylic acid (1-methyl-piperidin-4-yl)-amide

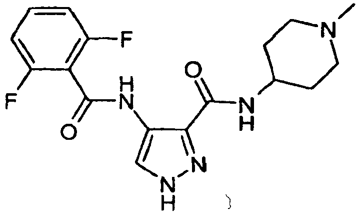

A mixture of 4-(2,6-difluoro-benzoylamino)-1H-pyrazole-3-carboxylic acid (134 mg, 0.50 mmol), 4-amino-N-methylpiperidine (50.0 µl, 0.45 mmol), EDAC (104 mg, 0.54 mmol) and HOBt (73.0 mg, 0.54 mmol) in DMF (3 ml) was stirred at ambient temperature for 16 hours. The mixture was reduced in vacuo, the residue taken up in EtOAc and washed successively with saturated aqueous sodium bicarbonate, water and brine. The organic portion was dried (MgSO₄) and reduced in vacuo to give 4-(2,6-difluoro-benzoylamino)-1 H-pyrazole-3-carboxylic acid (1-methyl-piperidin-4-yl)-amide as a white solid (113 mg, 69%). (LC/MS: Rₜ 2.52, [M+H]⁺ 364.19).

### EXAMPLE 54 (Comparative Example)

### Synthesis of 4-(Cyclohexyl-methyl-amino)-1H-pyrazole-3-carboxylic acid (4-fluoro-phenyl)-amide

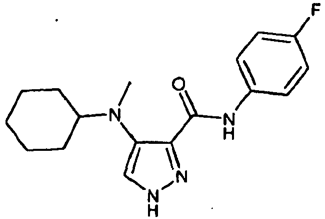

This compound was prepared in a manner analogous to the compound of Example 19 by succssive reductive alkylations using firstly cyclohexanone and then formaldehyde. (LC/MS: Rₜ 2.77 [MH]⁺ 316.71).

### EXAMPLE 55 (Comparative Example)

### 4-(Pyridin-2-ylamino)-1H-pyrazole-3-carboxylic acid (4-fluoro-phenyl)-amide

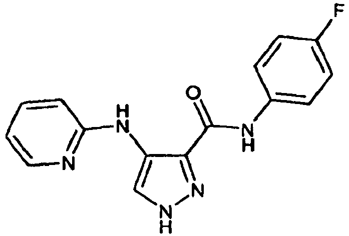

The title compound was prepared in a manner analogous to the compound of Example 23. (LC/MS: Rₜ 2.07 [MH]⁺ 298.03).

### EXAMPLES 56 - 81

By following the procedures described in the foregoing examples or methods analogous thereto, or by carrying out chemical transformations using the compounds described in the above examples and synthetic methods well known to the skilled person, the compounds set out in Table 3 were prepared.

**Table 3**

| Example No. | Structure | Prepared using method analogous to Example No | Differences to Example? | LCMS |
|---|---|---|---|---|
| 56* | 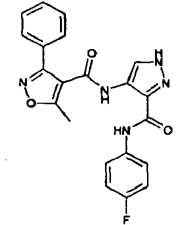 | 4 | | Rₜ 3.20 min [M+H]⁺ 406.07 |
| 57* | 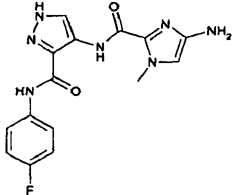 | 4 | Then removal of t-Boc protecting group with TFA as described in Example 82 | Rₜ 2.35 min m/z 343.72 |
| 58* | 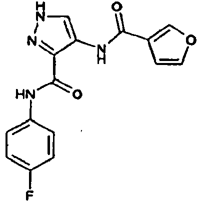 | 4 | Used DMSO instead of DMF as solvent | Rₜ 3.51 min m/z 314.62 |
| 59* | 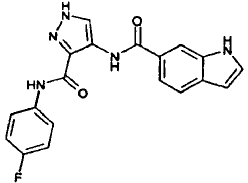 | 4 | Used DMSO instead of DMF as solvent | Rₜ 3.79 min m/z 363.67 |
| 60* | 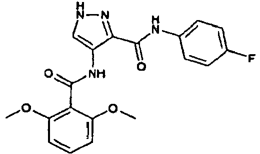 | 48 | Purified by column chromatography using EtOAC: Petroleum ether eluent | Rₜ 3.68 min m/z 384.69 |
| 61* | 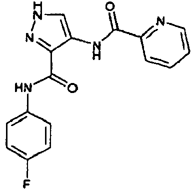 | 48 | Purified by column chromatography using EtOAC: Petroleum ether eluent | Rₜ 3.61 min m/z 326.10 |
| 62* | 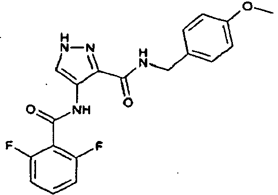 | 48 | Purified by column chromatography using EtOAC: Petroleum ether eluent | Rₜ 3.51 min m/z 387.11 |
| 63* | 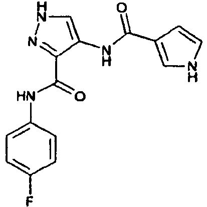 | 48 | | Rₜ 3.11 min m/z 313.65 |
| 64* | 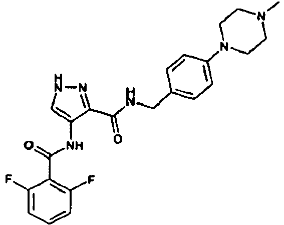 | 48 | Purified by column chromatography using EtOAC: Petroleum ether eluent | Rₜ 2.20 min m/z 455.19 |
| 65 | 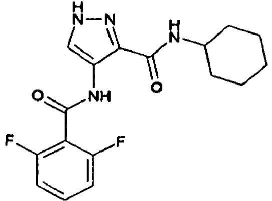 | 53 | | Rₜ 3.95 min m/z 349.09 |
| 66 | 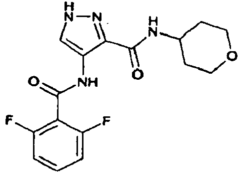 | 48 | Purified by column chromatography using EtOAC: Petroleum ether eluent | Rₜ 2.39 min m/z 351.07 |
| 67 | 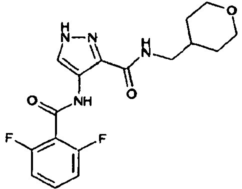 | 48 | Purified by column chromatography using EtOAC: Petroleum ether eluent | Rₜ 2.83 min m/z 365.13 |
| 68* | 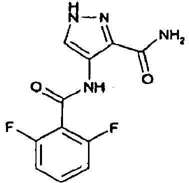 | Removal of PMB group from the compound of Example 62 using TFA-anisole | | Rₜ 2.10 min m/z 266.97 |
| 69 | 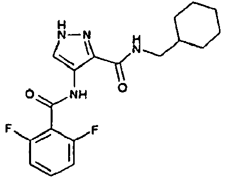 | 48 | Used DMF instead of DMSO as solvent | Rₜ 3.22 min m/z 363.10 |
| 70* | 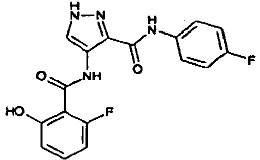 | 48 | | Rₜ 4.48 min m/z 358.96 |
| 71* | 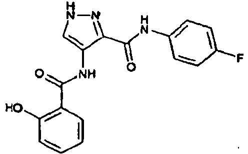 | 48 | | Rₜ 3.93 min m/z 340.96 |
| 72* | 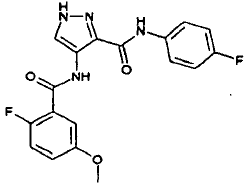 | 48 | | Rₜ 4.11 min m/z 373.01 |
| 73* | 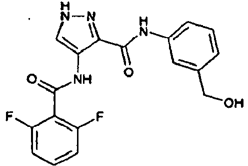 | 48 | Used DMF instead of DMSO as solvent | Rₜ 2.56 min m/z 373.05 |
| 74* | 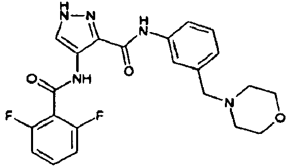 | Obtained by oxidation and then reductive amination of Example 73 | | Rₜ 1.99 min m/z 442.09 |
| 75 | 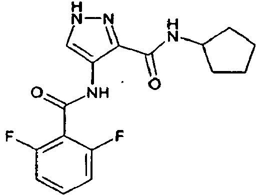 | 53 | Purified by column chromatography using DCM:MeOH (1:0 to 19:1) eluent | Rₜ 3.65 min m/z 335.03 |
| 76 | 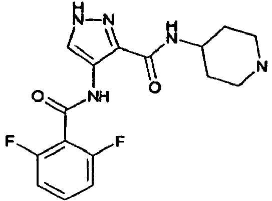 | 25 | Purified by column chromatography, Then removal of t-Boc protecting group with saturated ethyl acetate/HCl | Rₜ 1.57 min m/z 350.10 |
| 77 | 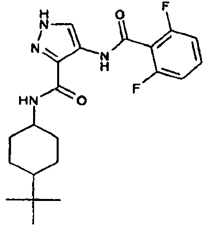 | 53 | | Rₜ 5.05 min m/z 405.14 |
| 78* | 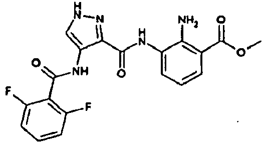 | 53 | | Rₜ 2.87 min m/z 416.07 |
| 79 | 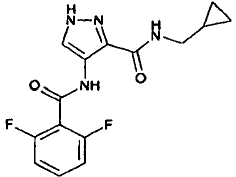 | 53 | Purified by column chromatography using EtOAC: Petroleum ether eluent (1:1) | Rₜ 3.41 min m/z 321.03 |
| 80* | 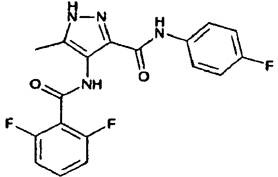 | 2A, 2B & 53 | Commercially available 5-methyl-pyrazole-1 H-3-carboxylic acid used as starting material. Purified by column chromatography using EtOAc: Hexane eluent (1:3 to 1:1) | Rₜ 3.42 min m/z 375.05 |
| 81* | 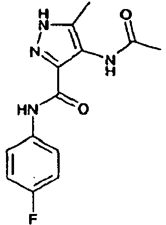 | 2C | Purified by column chromatography using EtOAC: Hexane eluent (1:1 to 1:0) | Rₜ 2.37 min m/z 277.04 |

| | | | | |
|---|---|---|---|---|
| * = comparative example | | | | |

### EXAMPLE 82 (comparative example)

### 4-[(4-Amino-1-methyl-1H-imidazole-2-carbonyl-amino)-1H-pyrazole-3-carboxylic acid (4-fluoro-phenyl)-amid

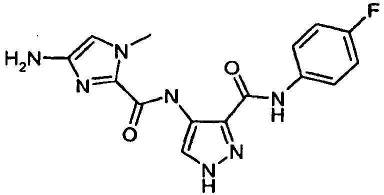

Trifluoroacetic acid (200 µl) was added to a stirred suspension of {2-[3-(4-fluoro-phenylcarbamoyl)-1H-pyrazol-4-ylcarbamoyl]-1-methyl-1H-imidazol-4-yl}-carbamic acid tert-butyl ester (30 mg) in dichloromethane (5 ml), then stirred at room temperature for 2 hours. The solvent was evaporated then re-evaporated with toluene (2 x 10 ml). The residue was triturated with diethyl ether and the resultant solid collected by filtration. The solid was washed with diethyl ether then dried under vacuum to give 15 mg of 4-[(4-amino-1-methyl-1H-imidazole-2-carbonyl)-amino]-1H-pyrazole-3-carboxylic acid (4-fluoro-phenyl)-amide as an off-white solid. (LC/MS: [M+H]⁺ 343.72).

### EXAMPLE 83

### Synthesis of 4-{[4-(2,6-Difluoro-benzoylamino)-1H-pyrazole-3-carbonyl]-amino}-cyclohexanecarboxylic acid

### 83A. 4-{[4-(2,6-difluoro-benzoylamino)1H-pyrazole-3-carbonyl]-amino}-cyclohexanecarboxylic acid ethyl ester

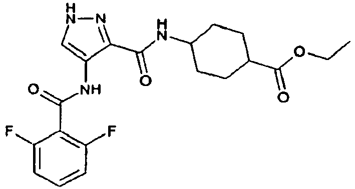

Thionyl chloride (0.32 ml, 4.40 mmol) was slowly added to a mixture of 4-aminocyclohexanecarboxylic acid (572 mg, 4.00 mmol) in EtOH (10 ml) and stirred at ambient temperature for 16 hours. The mixture was reduced in vacuo, azeotroping with toluene, to give the corresponding ethyl ester (650 mg) as a pale solid.

A mixture of the ethyl ester (103 mg, 0.60 mmol), 4-(2,6-difluoro-benzoylamino-1H-pyrazole-3-carboxylic acid (134 mg, 0.50 mmol), EDC (115 mg, 0.60 mmol) and HOBt (81 mg, 0.60 mmol) in DMF (5 ml) was stirred at ambient temperature for 16 hours. The mixture was reduced in vacuo, the residue taken up in EtOAc and washed successively with saturated aqueous sodium bicarbonate, water and brine. The organic portion was dried (MgSO₄) and reduced in vacuo to give 4-{(4-(2,6-difluoro-benzoylamino)-1H-pyrazole-3-carbonyl]-amino}-cyclohexanecarboxylic acid ethyl ester (112 mg).

### 83B. 4-{[4-(2,6-difluoro-benzoylamino)-1H-pyrazole-3-carbonyl]-amino}-cyclohexanecarboxylic acid

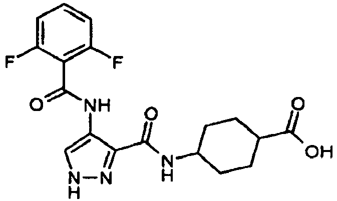

A mixture of the ester (45 mg) (from 83A) in MeOH (2.5 ml) and 2M aqueous NaOH (2.5 ml) was stirred at ambient temperature for 16 hours. The volatiles were removed in vacuo, water (10 ml) added and the mixture taken to pH 5 using 1 M aqueous HCl. The precipitate formed was collected by filtration and purified by column chromatography using EtOAc/MeOH (1:0 - 9:1) to give 4-{[4-(2,6-difluoro-benzoylamino)-1H-pyrazole-3-carbonyl]-amlno}-cyclohexanecarboxylic acid (11 mg) as a white solid and mixture of cis-/trans-isomers.
(LC/MS: Rₜ 2.78 and 2.96, [M+H]⁺ 393.09).

### EXAMPLES 84 - 152

### General Procedure A

### Preparation of Amide from Pyrazole Carboxylic Acid

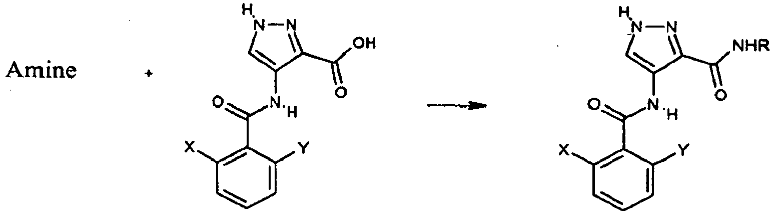

A mixture of the appropriate benzoylamino-1H-pyrazole-3-carboxylic acid (0.50 mmol), EDAC (104 mg, 0.54 mmol), HOBt (73.0 mg, 0.54 mmol) and the corresponding amine (0.45 mmol) in DMF (3 ml) was stirred at ambient temperature for 16 hours. The mixture was reduced in vacuo, the residue taken up in EtOAc and washed successively with saturated aqueous sodium bicarbonate, water and brine. The organic portion was dried (MgSO₄) and reduced in vacuo to give the desired product

### General Procedure B

### Preparation of Amide from Amino-Pyrazole

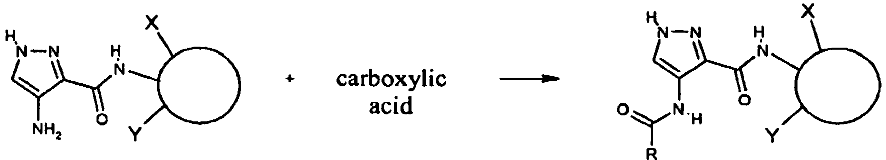

To a stirred solution of the appropriate 4-amino-1H-pyrazole-3-carboxylic acid amide (0.23 mmol), EDAC (52 mg; 0.27 mmol) and HOBt (37 mg; 0.27 mmol) in 5 ml of N,N-dimethylformamide was added the corresponding carboxylic acid (0.25 mmol), and the mixture was then left at room temperature overnight The reaction mixture was evaporated and the residue purified by preparative LC/MS, to give the product.

### General Procedure C

### Deprotection of Piperidine Ring Nitrogen by Removal of tert-Butoxycarbonyl Group

A product of Procedure A or Procedure B containing a piperidine group bearing an N-tert-butoxycarbonyl (t-Boc) protecting group (40 mg) was treated with saturated ethyl acetate/HCl, and stirred at room temperature for 1 hour. A solid precipitated out of the reaction mixture, which was filtered off, washed with ether, and then dried to give 25 mg product (LC/MS: [M+H]⁺ 364).

### Procedure L

### Preparation of Amine Starting Materials

The following method was used to prepare the following amines:
4-thiomorpholine-4-yl-cyclohexylamine;
4-(1,1-dioxo-thiomorpholine-4-yl)-cyclohexylamine;
N- (tetrahydro-pyran-4-yl)-cyclohexane-1,4-diamine;
4-(4-methyl-piperazin-1-yl)-cyclohexylamine;
1'-methyl-[1,4']bipiperidinyl-4-ylamine; and
4-morpholin-4-yl-cyclohexylamine.

A solution of N-4-Boc-aminocyclohexanone (0.5 g, 2.3 mmol) in THF (10 ml) was treated with the appropriate amine, e.g. thiomorpholine (0.236 g, 2.3 mmol), and sodium triacetoxyborohydride (0.715 g, 2.76 mmol) and acetic acid (0.182 ml). The reaction was stirred overnight at room temperature, then diluted with CH₂Cl₂ and washed with saturated sodium carbonate. The organic layer was dried over MgSO₄ and evaporated to give a white solid which was used without further purification in the next step. The white solid was treated with with saturated HCl/EtOAc, stirred at room temperature for 1 hour, evaporated to dryness and then re-evaporated with toluene. The resulting amines were isolated as the hydrochloride salt. (LC/MS: Rₜ 1.75, [M+H]⁺ 201).

By following General Procedures A, B, C and L, modified where stated, the compounds set out in Table 4 were prepared.

**Table 4**

| Example No. | | Method of Preparation | LCMS |
|---|---|---|---|
| 84 | 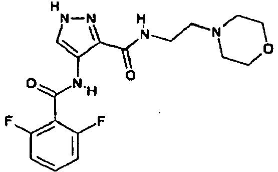 | Procedure A | [M+H]⁺ 380 Rₜ 1.42 |
| 85 | 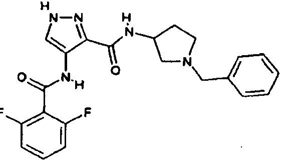 | Procedure A | [M+H]⁺ 426 Rₜ 1.93 |
| 86 | 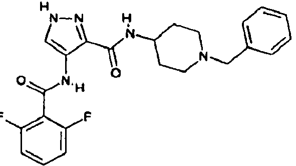 | Procedure A | [M+H]⁺ 440 Rₜ 1.87 |
| 87 | 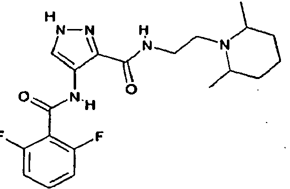 | Procedure A | [M+H]⁺ 406 Rₜ 2.78 |
| 88 | 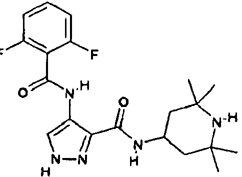 | Procedure A | [M+H]⁺ 406 Rₜ 2.55 |
| 89* | 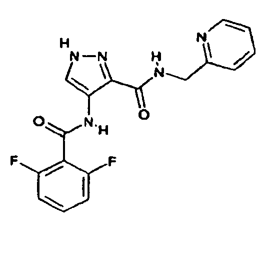 | Procedure A | [M+H]⁺ 358 Rₜ .98 |
| | | DMSO instead of DMF | |
| 90* | 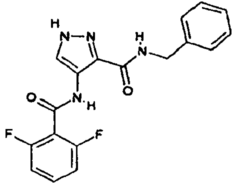 | Procedure A | [M+H]⁺ 357 Rₜ 3.37 |
| | | DMSO instead of DMF | |
| 91* | 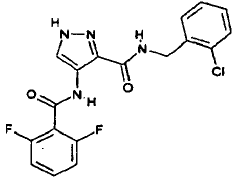 | Procedure A | [M+H]⁺ 391 Rₜ 3.16 |
| | | DMSO instead of DMF | |
| 92* | 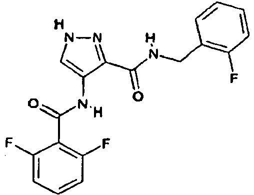 | Procedure A | [M+H]⁺ 375 Rₜ 3.02 |
| | | DMSO instead of DMF | |
| 93* | 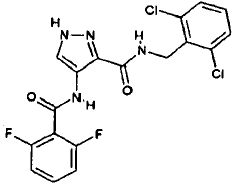 | Procedure A | [M+H]⁺ 425 Rₜ 3.27 |
| | | DMSO Instead of DMF | |
| 94* | 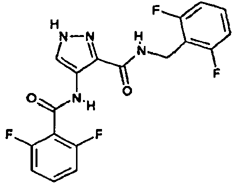 | Procedure A | [M+H]⁺ 393 Rₜ 3.01 |
| | | DMSO instead of DMF | |
| 95 | 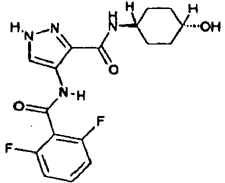 | Procedure A | [M+H]⁺ 365 Rₜ 2.22 |
| | | DMSO instead of DMF | |
| 96* | 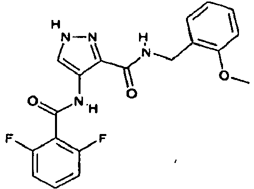 | Procedure A | [M+H]⁺ 387 Rₜ 3.05 |
| | | DMSO instead of DMF | |
| 97 | 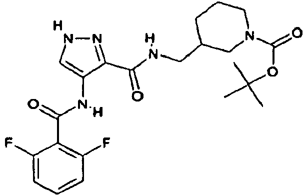 | Procedure A | [M+H]⁺ 464 Rₜ 3.17 |
| | | DMSO instead of DMF | |
| 98 | 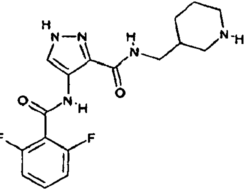 | Procedure C using the product of | [M+H]⁺ 364 Rₜ 1.76 |
| | | Example 97 as starting material | |
| 99* | 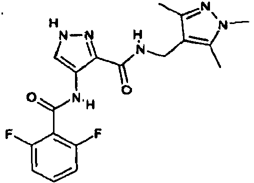 | Procedure A | [M+H]⁺ 389 Rₜ 2.36 |
| | | DMSO instead of DMF | |
| 100 | 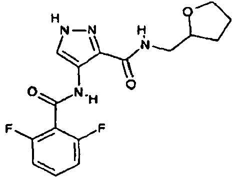 | Procedure A | [M+H]⁺ 351 Rₜ 2.55 |
| | | DMSO instead of DMF | |
| 101* | 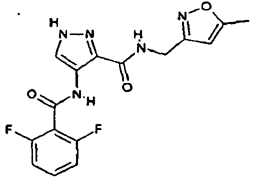 | Procedure A | [M+H]⁺ 362 Rₜ 2.63 |
| | | DMSO instead of DMF | |
| 102 | 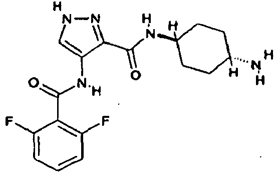 | Procedure A | [M+H]⁺ 364 Rₜ 1.75 |
| | | DMSO instead of DMF | |
| | | Starting amine prepared according to Procedure L | |
| 103* | 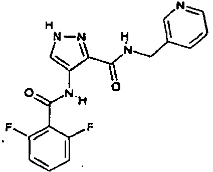 | Procedure A | [M+H]⁺ 358 Rₜ3.2 |
| | | DMSO instead of DMF | |
| 104* | 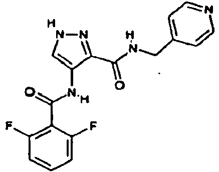 | Procedure A | [M+H]⁺ 358 Rₜ 1.77 |
| | | DMSO instead of DMF | |
| 105* | 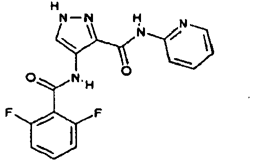 | Procedure A | [M+H]⁺ 344 Rₜ 2.71 |
| | | DMSO instead of DMF | |
| 106 | 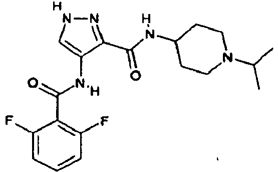 | Procedure A | [M+H]⁺ 392 Rₜ 2.57 |
| | | DMSO instead of DMF | |
| 107* | 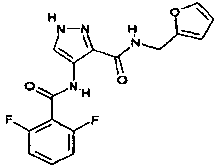 | Procedure A | [M+H]⁺ 347 Rₜ 2.8 |
| | | DMSO instead of DMF | |
| 108* | 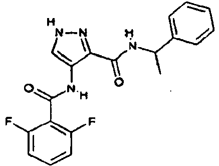 | Procedure A | [M+H]⁺ 371 Rₜ3.1 |
| | | DMSO instead of DMF | |
| 109 | 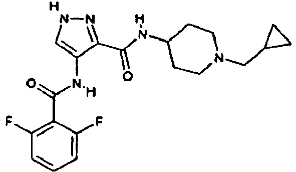 | Procedure A | [M+H]⁺ 404 Rₜ 2.7 |
| | | Et₃N 1 equiv., DMSO instead of DMF | |
| 110* | 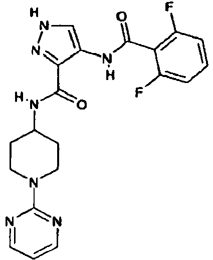 | Procedure A | [M+H]⁺ 428 Rₜ 2.63 |
| | | Et₃N 2 equiv., HOAt instead of HOBt. | |
| | | DMSO instead of DMF | |
| 111 | 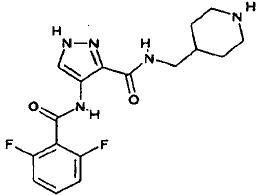 | Procedure Procedure A followed by Procedure C | [M+H]⁺ 364 Rₜ 1.75 |
| | | Et₃N 2 equiv., | |
| | | HOAt instead of HOBt, DMSO instead of DMF | |
| 112 | 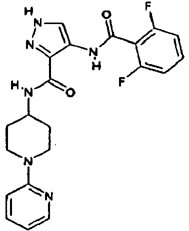 | Procedure A | [M+H]⁺ 427 Rₜ 2.71 |
| | | Et₃N 2 equiv., HOAt instead of HOBt, | |
| | | DMSO instead of DMF | |
| 113 | 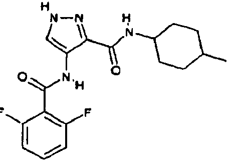 | Procedure A | [M+H]⁺ 363 Rₜ 3.34 |
| | | HOAt instead of HOBt, | |
| | | DMSO instead of DMF | |
| 114 | 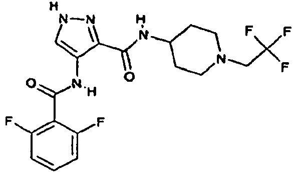 | Procedure A | [M+H]⁺ 432 Rₜ 2.63 |
| | | Et₃N 2 equiv., HOAt instead of HOBt, | |
| | | DMSO instead of DMF | |
| 115 | 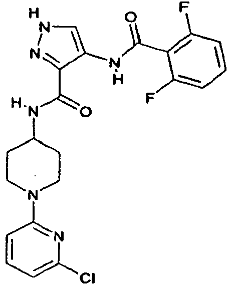 | Procedure A | [M+H]⁺ 461 Rₜ 3.3 |
| | | | |
| 116 | 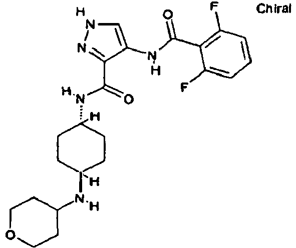 | Procedure A | [M+H]⁺ 448 Rₜ 1.87 |
| | | DMSO instead of DMF, Et₃N 2 equiv | |
| | | Starting amine prepared according to Procedure L | |
| 117 | 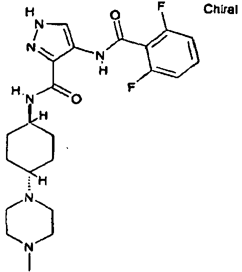 | Procedure A | [M+H]⁺ 447 Rₜ 1.65 |
| | | DMSO instead of DMF, Et₃N 2 equiv | |
| | | Starting amine prepared according to Procedure L | |
| 118 | 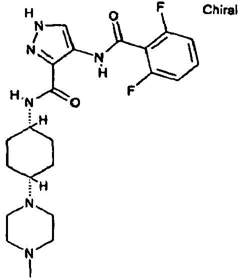 | Procedure A | [M+H]⁺ 447 Rₜ 1.72 |
| | | DMSO instead of DMF, Et₃N 2 equiv | |
| | | Starting amine prepared according to Procedure L | |
| 119 | 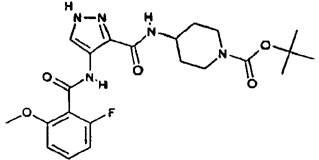 | Procedure B | [M+H]⁺ 462 Rₜ 2.97 |
| | | | |
| 120 | 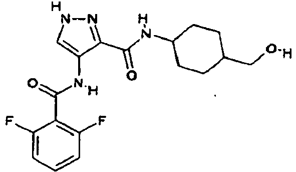 | Procedure A | [M+H]⁺ 379 Rₜ 2.45 |
| | | N-ethyl-morpholine (NEM) 2 equiv | |
| 121 | 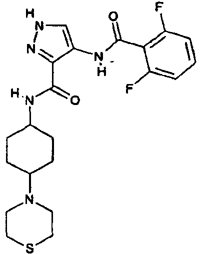 | Procedure A | [M+H]⁺ 450 Rₜ 1.97 |
| | | HOAt instead of HOBt. Et₃N 2 equiv | |
| | | Starting amine prepared according to Procedure L | |
| 122 | 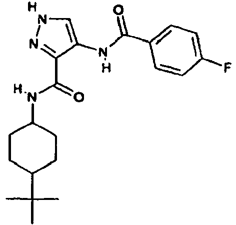 | Procedure B | [M+H]⁺ 387 Rₜ 3.83 |
| 123 | 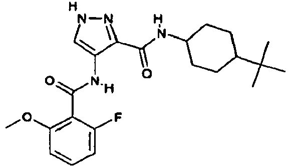 | Procedure B | [M+H]⁺ 417 Rₜ 3.65 |
| 124 | 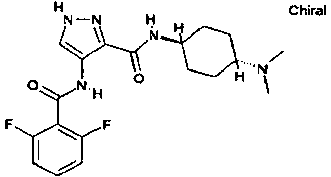 | Procedure A | [M+H]⁺ 392 Rₜ 1.85 |
| | | HOAt instead of HOBt, Et₃N 2 equiv | |
| 125 | 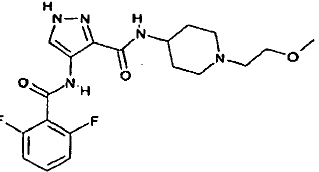 | Procedure A | [M+H]⁺ 408 Rₜ 1.82 |
| | | HOAt instead of HOBt, Et₃N 2 equiv | |
| 126 | 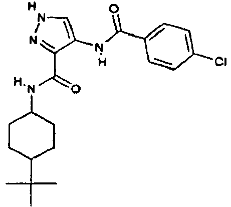 | Procedure B | [M+H]⁺ 403 Rₜ 4.02 |
| 127 | 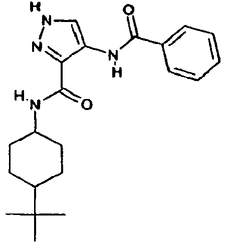 | Procedure B | [M+H]⁺ 369 Rₜ 3.78 |
| 128 | 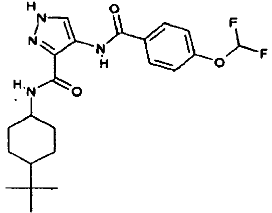 | Procedure B | [M+H]⁺ 435 Rₜ 3.83 |
| 129 | 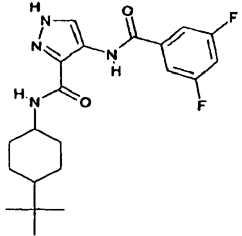 | Procedure B | [M-t-H]⁺ 405 Rₜ 3.96 |
| 130 | 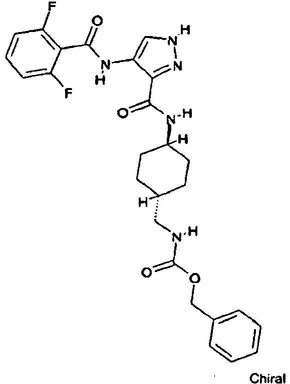 | Procedure A | [M+H]⁺ 512 Rₜ3.1 |
| | | HOAt instead of HOBt | |
| 131 | 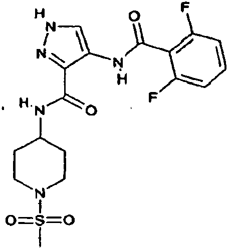 | Procedure A | [M+H]⁺ 428 Rₜ2.45 |
| | | HOAt instead of HOBt, | |
| 132 | 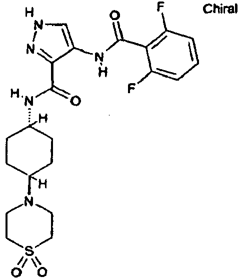 | Procedure A | [M+H]⁺ 482 Rₜ1.96 |
| | | HOAt instead of HOBt, Et₃N 2 equiv. | |
| | | Cis and trans isomers separated after amide coupling step | |
| | | Starting amine prepared according to Procedure L | |
| 133 | 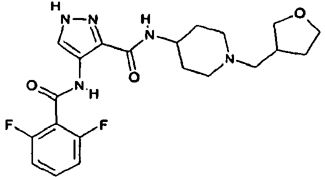 | Procedure A | [M+H]⁺ 434 Rₜ2.3 |
| | | HOAt instead of HOBt, DMSO instead of DMF | |
| 134 | 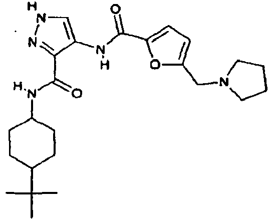 | Procedure B | [M+H]⁺ 442 Rₜ 2.39 |
| 135 | 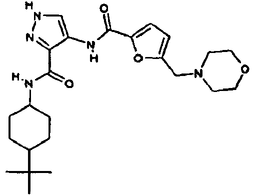 | Procedure B | [M+H]⁺ 458 Rₜ 2.26 |
| 136 | 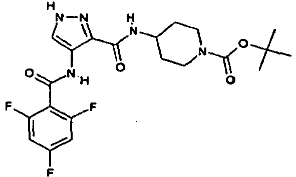 | Procedure B | [M+H]⁺ 468 Rₜ3.07 |
| | | HOAt instead of HOBt, | |
| 137 | 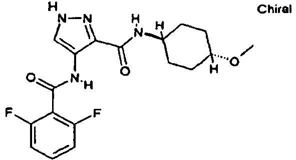 | Procedure A | [M+H]⁺ 379 Rₜ2.6 |
| | | Et₃N 2 equiv., HOAt Instead of HOBt, | |
| 138 | 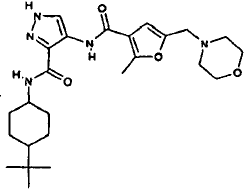 | Procedure B | [M+H]⁺ 472 Rₜ 2.40 |
| 139 | 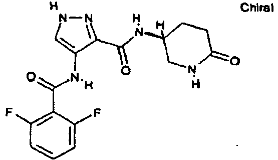 | Procedure A | [M+H]⁺ 364 Rₜ2.1 |
| | | Et₃N 2 equiv., HOAt instead of HOBt, | |
| | | DMSO instead of DMF | |
| 140 | 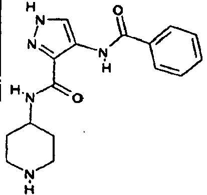 | Procedure B followed by Procedure C | [M+H]⁺ 314 Rₜ 1.78 |
| 141 | 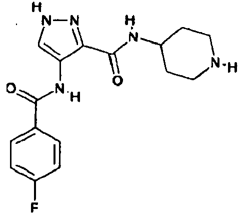 | Procedure B followed by Procedure C | [M+H]⁺ 332 Rₜ 1.89 |
| 142 | 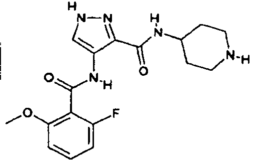 | Procedure B followed by Procedure C | [M+H]⁺ 362 Rₜ 1.78 |
| 143 | 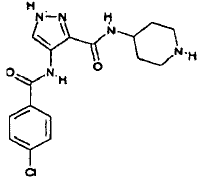 | Procedure B followed by Procedure C | [M+H]⁺ 348 Rₜ 2.01 |
| 144 | 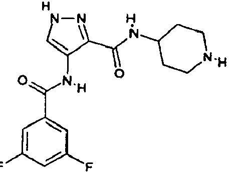 | Procedure B followed by Procedure C | [M+H]⁺ 350 Rₜ 1.97 |
| 145 | 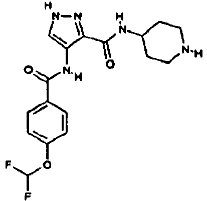 | Procedure B followed by Procedure C | [M+H]⁺ 380 Rₜ 2.01 |
| 146 | 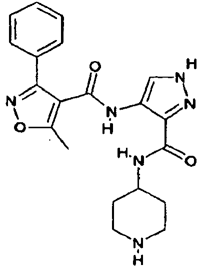 | Procedure B followed by Procedure C | [M+H]⁺ 395 Rₜ 1.94 |
| 147 | 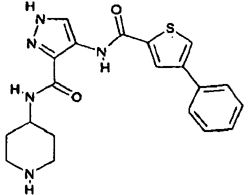 | Procedure B followed by Procedure C | [M+H]⁺ 396 Rₜ 2.11 |
| 148 | 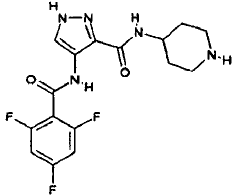 | Procedure B followed by Procedure C | [M+H]⁺ 368 Rₜ1.76 |
| | | HOAt instead of HOBt | |
| 149 | 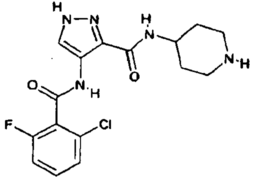 | Procedure B followed by Procedure C | [M+H]⁺ 366 Rₜ 1.78 |
| 150 | 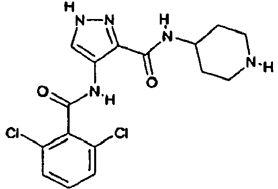 | Procedure B followed by | [M+H]⁺ 383 Rₜ 1.87 |
| | | Procedure C | |
| 151 | 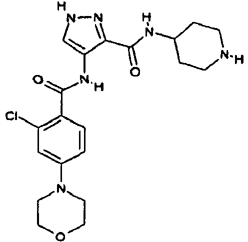 | Procedure B followed by Procedure C | [M+H]⁺ 433 Rₜ 1.89 |
| | | | |
| 152 | 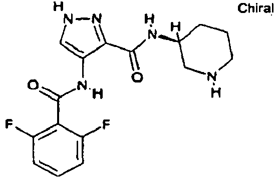 | Procedure A followed by Procedure C | [M+H]⁺ 350 Rₜ1.76 |
| | | HOAt instead of HOBt | |

### EXAMPLES 153 - 165

### General Procedure D

### Preparation of Protected 4-Amino-pyrazol-3-yl carboxylic acid 4-hydroxy-cyclohexylamide

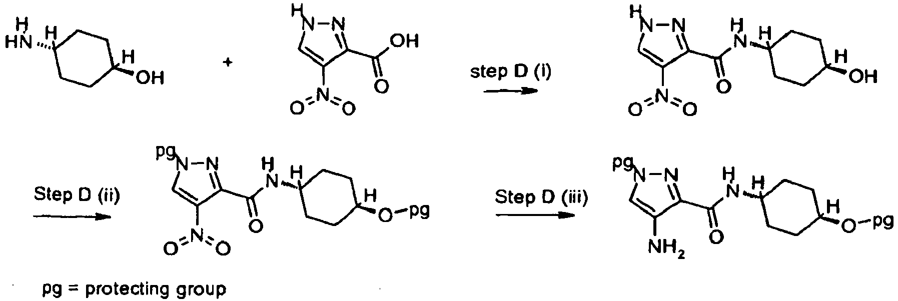

### Ste D (i):

A mixture of 4-nitro-3-pyrazolecarboxylic acid (4.98 g, 31.7 mmol), trans 4-aminocyclohexanol (3.65 g, 31.7 mmol), EDAC (6.68 g, 34.8 mmol) and HOBt (4.7 g, 34.8 mmol) in DMF (120 ml) was stirred at ambient temperature for 16 hours. The mixture was reduced in vacuo, the residue taken up in CH₂Cl₂ and washed successively with 5% citric acid, saturated aqueous sodium bicarbonate, water and brine. The product was found to be mainly in the citric acid wash, which was basified and extracted with EtOAc. The organic layer was dried over MgSO₄, filtered and evaporated to give a white solid, which was triturated with CHCl₃ to give 1.95 g of 4-nitro-1H-pyrazole-3-carboxylic acid 4-hydroxy-cyclohexylamide. (LC/MS: Rₜ 1.62, [M+H]⁺ 255).

### Step D (ii):

### Introduction of Tetrahydro-pyran-2-yl Protecting Group

A solution of 4-nitro-1H-pyrazole-3-carboxylic acid 4-hydroxy-cyclohexylamide (1.95 g; 7.67 mmol) in a mix of THF (50 ml) and chloroform (100 m1), was treated with 3,4-dihydro-2H-pyran (1.54 ml, 15.34 mmol) and p-toluenesulphonic acid monohydrate (100 mg). The reaction mixture was stirred at room temperature overnight, and then excess pyran (0.9 ml) was added in total to bring reaction to completion. The reaction mixture was diluted with CH₂Cl₂ and washed successively with saturated aqueous sodium bicarbonate, water and brine. The resulting solution was reduced in vacuo and subject to Biotage column chromatography, eluting with hexane (2 column lengths) followed by 30% ethyl acetate: hexane (10 column lengths), 70% ethyl acetate: hexane (10 column lengths) to give 1.25 g of 4- nitro-1-(tetrahydro-pyran-2-yl-1H-pyrazole-3-carboxylic acid [4-(tetrahydro-pyran-2-yloxy)-cyclohexyl]-amide. (LC/MS: Rt 2.97, [M+H]⁺ 423).

### Step D (iii):

A solution of 4- nitro-1- (tetrahydro-pyran-2-yl)-1H-pyrazole-3-carboxylic acid [4-(tetrahydro-pyran-2-yloxy)-cyclohexyl]-amide (0.3 g; 0.71 mmol) in methanol (25 ml), was treated with 10% palladium on carbon (30 mg) then hydrogenated at room temperature and pressure overnight. The catalyst was removed by filtration and washed three times with methanol. The filtrate was evaporated to give 0.264 g of the required product.
(LC/MS: Rₜ 2.39, [M+H]⁺ 393).

### General Procedure E

### Procedure for Removal of a Tetrahydropyran-2-yl Protecting Group

To a suspension of 4-(2-methoxy- benzoylamino)-1- (tetrahydro-pyran-2-yl-1H-pyrazole-3-carboxylic acid (4-(tetrahydro-pyran-2-yloxy)-cyclohexyl]-amide (0.125 g, 0.23 mmol) in EtOH (10 ml) was added p-toluene sulphonic acid hydrate (90 mg, 0.46 mmol). The reaction mixture was heated at 70 °C for 30 mins. The reaction was diluted with EtOAc and washed successively with saturated aqueous sodium bicarbonate, water and brine. The resulting solution was reduced in vacuo to give a white solid, which contained traces of p-toluene sulphonic acid hydrate. The solid was then taken up in EtOAc and washed with 1 M NaOH and then brine. The resulting solution was reduced in vacuo and then triturated with ether/ hexane to give 10 mg of required product. (LC/MS: Rₜ 2.29, [M+H]⁺ 359)

### General Procedure F

### Preparation of a Urea from a 4-Amino-pyrazole-3-carboxylic acid amide

To a solution of 4-amino-1- (tetrahydro-pyran-2-yl-1H-pyrazole-3-carboxylic acid [4-(tetrahydro-pyran-2-yloxy)-cyclohexyl]-amide (80 mg, 0.2 mmol) in toluene (2 ml) was added phenyl isocyanate (929 mg, 0.24 mmol). The reaction mixture was heated at 70 °C for 1 hour. The reaction was diluted with EtOAc and washed successively with water and brine. The resulting solution was reduced in vacuo to give yellow oil. This was used without further purification. (LC/MS: Rₜ 2.28, [M+H]⁺ 344).

### General Procedure G

### Conversion of a 4-Amino-pyrazole group to a 4-(Morpholine-4-carbonylamino)-Pyrazole Group

To a solution of 4-amino-1- (tetrahydro-pyran-2-yl-1H-pyrazole-3-carboxylic acid [4-(tetrahydro-pyran-2-yloxy)-cyclohexyl]-amide (0.1 g. 0.255 mmol) in CH₂Cl₂ (5 ml) at -10 °C was added in a dropwise manner a 20% solution of phosgene in toluene. The reaction mixture was stirred at -10 °C for 15 mins and then morpholine (0.765 mmol) was added. The reaction mixture was allowed to warm up to room temperature over 1 hour then stirred at room temperature overnight. The reaction was diluted with CH₂Cl₂ and washed successively with saturated sodium bicarbonate and brine. The resulting solution was reduced in vacuo to give a yellow oil which was used without further purification. (LC/MS: Rₜ 1.68,[M+H]⁺ 338).

### General Procedure H

### Preparation of N-Oxides

To a suspension of the compound of Example 53 (7.7 mg, 0.02 mmol) in CH₂Cl₂ (0.5 ml) was added meta-chloroperbenzoic acid (MCPBA) (3.6 mg, 0.02 mmol). The reaction mixture was stirred at room temperature overnight, and then evaporated. The residue was purified by preparative LC/MS, to give 3 mg of the required product. (LC/MS: Rₜ 1.83, [M+H]⁺ 380)

### General Procedure I

### Removal of a Benzyloxycarbonyl Protecting Group

A solution of the compound of Example 130 (0.2 g; 0.39 mmol) in EtOAc (40 ml) was treated with 10% palladium on carbon (20 mg) then hydrogenated at room temperature and pressure for 3 hours. The catalyst was removed by filtration and washed three times with EtOAc. The filtrate was evaporated and the residue was subjected to chromatography using 10% MeOH-CH₂Cl₂ then 20% MeOH- CH₂Cl₂ to give 80 mg of the required product. (LC/MS: Rₜ 1.88, [M+H]⁺ 378).

### General Procedure J

### Mesylation of an Amine

To a solution of the compound of Example 163 (20 mg, 0.05 mmol) in CH₃CN (3 ml) added methane-sulphonyl chloride (0.0045 ml, 0.058 mmol) followed by Hunig's Base (0.018 ml, 0.1 mmol). The reaction mixture was stirred at room temperature for 2 hours and was then evaporated down. The residue was purified by preparative LC/ MS to give 8mg of the required product. (LC/MS: Rₜ 2.54, [M+H]⁺ 456).

By following Procedures A to L, the compounds set out in Table 5 were prepared.

**Table 5**

| Example No. | | Method of Preparation | LCMS |
|---|---|---|---|
| 153 | 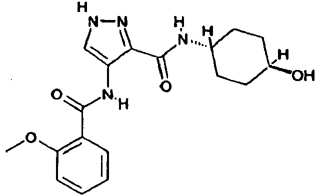 | Procedure D followed by B then E | [M+H]⁺ 359 Rₜ 2.29 |
| | | HOAt instead of HOBt, | |
| | | CH₂Cl₂ instead of DMF | |
| 154 | 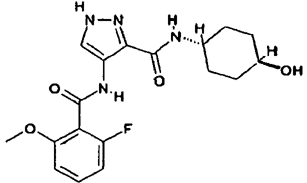 | Procedure D followed by B then E | [M+H]⁺ 377 Rₜ 2.22 |
| | | HOAt instead of HOBt, | |
| | | CH₂Cl₂ instead of DMF | |
| 155 | 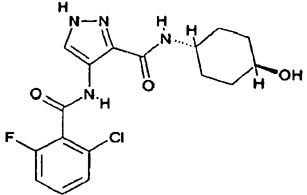 | Procedure D followed by B then E | [M+H]⁺ 381 Rₜ 2.34 |
| | | HOAt instead of HOBt, | |
| | | CH₂Cl₂ instead of DMF | |
| 156 | 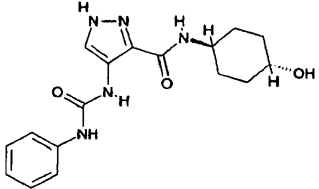 | Procedure D followed by F then E | [M+H]⁺ 344 Rₜ 2.28 |
| 157 | 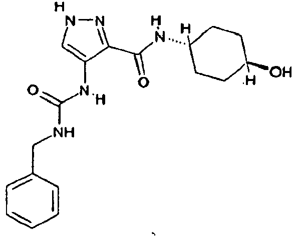 | Procedure D followed by F then E | [M+H]⁺ 358 Rₜ 2.22 |
| 158 | 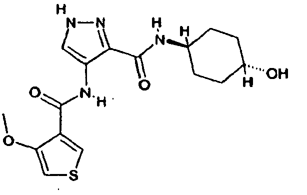 | Procedure D followed by B then E | [M+H]⁺ 365 Rₜ 2.21 |
| | | HOAt instead of HOBt, | |
| | | CH₂Cl₂ instead of DMF | |
| 159 | 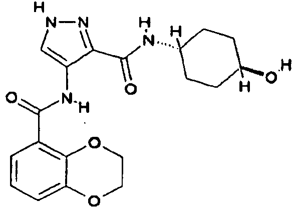 | Procedure D followed by B then E | [M+H]⁺ 387 Rₜ 2.29 |
| | | HOAt instead of HOBt, | |
| | | CH₂Cl₂ instead of DMF | |
| 160 | 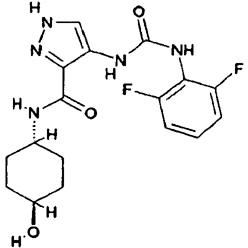 | Procedure D followed by F then E | [M+H]⁺ 380 Rₜ 2.17 |
| 161 | 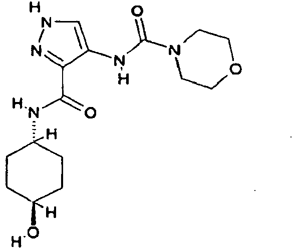 | Procedure D followed by G then E | [M+H]⁺ 338 Rₜ 1.68 |
| 162 | 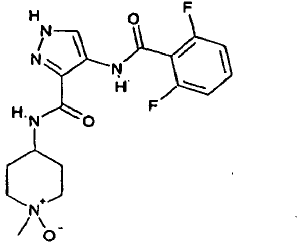 | Procedure H | [M+H]⁺ 380 Rₜ 1.83 |
| 163 | 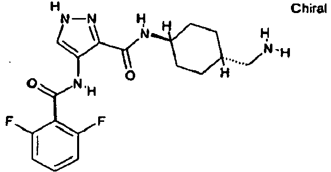 | Procedure A (HOAt instead of HOBt) to give the compound of Example 130 followed by Procedure 1. | [M+H]⁺ 378 Rₜ 1.78 |
| 164 | 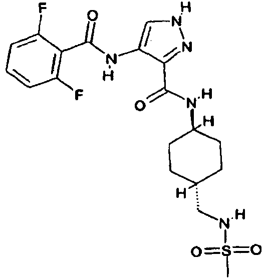 | Procedures A(HOAt instead of HOBt) and I to give the compound of Example 163 followed by Procedure J | [M+H]⁺ 456 Rₜ 2.54 |

### General Procedure M

### Formation of pyrazole 4-amide group

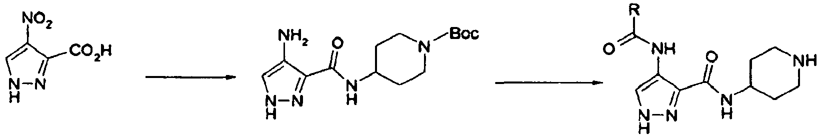

4-Nitropyrazole-3-carboxylic acid (7.3 g; 15.9 mmol) was added to a stirred solution of 4-amino-1-Boc-piperidine (10.2 mg; 51 mmol), EDC (10.7 g; 55.8 mmol), and HOAt (55.8 g; 19.1 mmol) in DMF (100 ml), and then stirred at room temperature overnight. The solvent was removed by evaporation under reduced pressure and the residue triturated with water (250ml). The resultant cream solid was collected by filtration, washed with water then dried under vacuum to give 13.05 g of 4-[(4-nitro-1H-pyrazole-3-carbonyl)-amino]-piperidine-1-carboxylic acid tert-butyl ester (LC/MS: Rₜ 2.50, [M+H]⁺ 340).

4-[(4-Nitro-1H-pyrazole-3-carbonyl)-amino]-piperidine-1-carboxylic acid tert-butyl ester (13.05 g) was dissolved in ethanol / DMF (300 ml / 75 ml), treated with 10% palladium on carbon (500 mg) then hydrogenated at room temperature and pressure overnight. The catalyst was removed by filtration through Celite and the filtrate evaporated and re-evaporated with toluene. The crude material was purified by flash column chromatography eluting with EtOAc then 2% MeOH / EtOAc then 5% MeOH / EtOAc. Product containing fractions were combined and evaporated to give 8.78 g of 4-[(4-amino-1H-pyrazole-3-carbonyl)amino]-piperidine-1-carboxylic acid tert-butyl ester as a brown foam. (LC/MS: Rₜ 1.91, [M+H]⁺ 310).

To a stirred solution of 4-[(4-amino-1H-pyrazole-3-carbonyl)-amino]-piperidine-1-carboxylic acid tert-butyl ester (200 mg; 0.65 mmol), EDAC (150 mg; 0.78 mmol) and HOBt (105 mg; 0.78 mmol) in 5 ml of N,N-dimethylformamide was added the corresponding carboxylic acid (0.25 mmol), and the mixture was then left at room temperature overnight. The reaction mixture was diluted with saturated aqueous sodium bicarbonate solution and the product collected by filtration and dried under vacuum. The Boc-protected compound was dissolved In saturated HCl / EtOAc and stirred at room temperature for 3 hours. The product was collected by filtration, washed with diethyl ether and dried under vacuum.

### General Procedure N

### Preparation of 1-tert-Butyl-piperidin-4-ylamine

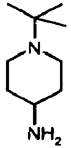

### Step N (i)

To a solution of 1-ethyl-4-oxopiperldine (25 g, 0.197 mol) in acetone (250 ml) at RT in a water bath was added methyl iodide (15.5 ml, 0.25 mol) at such a rate to keep the temperature below 30 °C. The mixture was filtered and the precipitate washed with acetone and dried to yield 1- ethyl-1-methyl-4-oxopiperidinium iodide (45 g) (LC/MS: Rₜ 0.38, [M+H]⁺ 143).

### Step N (ii)

To a solution of t-butylamine (78.2 ml, 0.74 mol) in toluene (400 ml) was added a solution of 1-ethyl-1-methyl-4-oxopiperidinium iodide (40g, 0.148 mol) and sodium bicarbonate (1.245 g, 0.014 mol) in water (60 ml). The reaction mixture was heated at 78 °C for 6 hours and then allowed to cool to ambient temperature. The layers were separated and the aqueous layer was washed with EtOAc. The organics were combined and washed with brine dried (MgSO₄), filtered and reduced in vacuo to yield 1-tert-butyl-4-oxopiperidine (14g) (LC/MS: Rₜ 0.39, [M+H]⁺ 156).

### Ste N (iii)

A solution of 1-tert-butyl-4-oxopiperidine (3.6g, 23.1), benzylamine (5.1 ml, 46.8 mmol), acetic acid (1.5 ml) and sodium triacetoxyborohydride (7.38 g, 34.8 mmol) was stirred at ambient for 2 days. Reaction mixture reduced in vacuo, residue partitioned between aqueous K₂CO₃ and EtOAc. The organic portion was dried (Na₂SO₄). filtered and reduced in vacuo. The residue was subjected to chromatography using CH₂Cl₂/MeOH/NH₄OH (87/12/1)as the eluent to yield N-benzyl-1-tert-butylpiperidin-4-amine (1.5g) (LC/MS: Rₜ 0.45, [M+H]⁺ 247).

### Step N (iv)

A solution of N-benzyl-1-tert-butylpiperidin-4-amine (1.56 g) and 10% palladium on carbon (2 g) in MeOH (250 ml) was hydrogenated in a Parr shaker at 50 psi for 16 hours. The solution was filtered and the reaction mixture reduced in vacuo, to yield 1-tert-butylpiperidin-4-amine (0.64 g) (LC/MS: Rₜ 02.31, no [M+H]⁺).

### EXAMPLE 165 (comparative example)

### Synthesis of 4-(2,6-difluoro-benzoylamino)-1H-pyrazole-3-carboxylic acid [5-fluoro-2-(1-methyl-piperidin-4-yloxy)-phenyl]-amide

### 165A. Synthesis of 4-nitro-1H-pyrazole-3-carboxylic acid ethyl ester

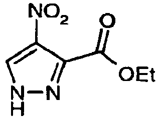

Thionyl chloride (2.90 ml, 39.8 mmol) was slowly added to a mixture of 4-nitro-3-pyrazolecarboxylic acid (5.68 g, 36.2 mmol) in EtOH (100 ml) at ambient temperature and the mixture stirred for 48 h. The mixture was reduced in vacuo and dried through azeotrope with toluene to afford 4-nitro-1H-pyrazole-3-carboxylic acid ethyl ester as a white solid (6.42 g, 96%). (¹H NMR (400 MHz, DMSO-d₆) □ 14.4 (s, 1H), 9.0 (s, 1 H), 4.4 (q, 2H), 1.3 (t, 3H)).

### 165B. Synthesis of 4-amino-1H-pyrazole-3-carboxylic acid ethyl ester

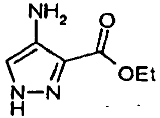

A mixture of 4-nitro-1H-pyrazole-3-carboxylic acid ethyl ester (6.40 g, 34.6 mmol) and 10% Pd/C (650 mg) in EtOH (150ml) was stirred under an atmosphere of hydrogen for 20 h. The mixture was filtered through a plug of Celite, reduced in vacuo and dried through azeotrope with toluene to afford 4-amino-1 H-pyrazole-3-carboxylic acid ethyl ester as a pink solid (5.28 g, 98%). (' H NMR (400 MHz, DMSO-d₆) □ 12.7 (s, 1H), 7.1 (s, 1H), 4.8 (s, 2H), 4.3 (q, 2H), 1.3 (t, 3H)).

### 165C. Synthesis of 4-(2,6-difluoro-benzoylamino)-1H-pyrazole-3-carboxylic acid ethyl ester

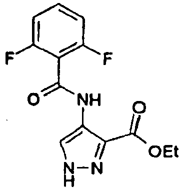

A mixture of 2,6-difluorobenzoic acid (6.32 g, 40.0 mmol), 4-amino-1H-pyrazole-3-carboxylic acid ethyl ester (5.96 g, 38.4 mmol), EDC (8.83 g, 46.1 mmol) and HOBt (6.23 g, 46.1 mmol) in DMF (100 ml) was stirred at ambient temperature for 6 h. The mixture was reduced in vacuo, water added and the solid formed collected by filtration and air-dried to give 4-(2,6-difluoro-benzoylamino)-1H-pyrazole-3-carboxylic acid ethyl ester as the major component of a mixture (15.3 g). (LC/MS: Rₜ 3.11, [M+H]⁺ 295.99).

### 165D. Synthesis of 4-(2,6-difluoro-benzoylamino)-1H-pyrazole-3-carboxylic acid

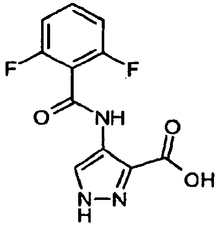

A mixture of 4-(2,6-difluoro-benzoylamino)-1H-pyrazole-3-carboxylic acid ethyl ester (10.2 g) in 2 M aqueous NaOH/MeOH (1:1, 250 ml) was stirred at ambient temperature for 14 h. Volatile materials were removed in vacuo, water (300 ml) added and the mixture taken to pH 5 using 1 M aqueous HCl. The resultant precipitate was collected by filtration and dried through azeotrope with toluene to afford 4-(2,6-difluoro-benzoylamino)-1H-pyrazole-3-carboxylic acid as a pink solid (5.70 g). (LC/MS: Rₜ 2.33, [M+H]⁺ 267.96).

### 165E. Synthesis of 5-fluoro-2-(1-methyl-piperidin-4-yloxy)-phenylamine

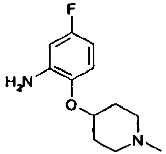

3,4-Dinitrofluorobenzene (1.86 g, 10 mmol) and 4-hydroxy-1-methylpiperidine (1.38 g. 12 mmol) were dissolved in THF (20 ml) and stirred at ambient temperature while sodium hydride (60 % dispersion in mineral oil, 0.40 g, 10 mmol) was added in several small portions. The reaction mixture was stirred for one hour and then reduced in vacuo, partitioned between ethyl acetate and water, and the organic phase washed with brine, dried (MgSO₄) and reduced in vacuo. The resulting residue was subject to column chromatography, eluting with 5% MeOH / DCM to give a yellow solid (1.76 g, 2:1 ratio of 4-(3,4-dinitro-phenoxy)-1-methyl-piperidine and a 4-(4-fluoro-2-nitro-phenoxy)-1-methyl-piperidine).

A sample of the mixture of products obtained (0.562 g) was dissolved in DMF (10 ml) under an atmosphere of nitrogen. Palladium on carbon (10 %, 0.056 g) was added and the reaction mixture was shaken under a hydrogen atmosphere for 40 hours. The solids were removed by filtration and the filtrate reduced in vacuo, taken up in ethyl acetate, washed (saturated aqueous ammonium chloride solution, then saturated aqueous brine), dried (MgSO₄) and reduced in vacuo to give 5-fluoro-2-(1-methyl-piperidin-4-yloxy)-phenylamine) as a brown oil (0.049 g, 7 %). (¹H NMR (400 MHz, MeOD-d₄) 0 6.6 (m, 2H), 6.4 (m, 1H), 4.3 (m, 1 H), 2.7 (m, 2H), 2.3 (m, 2H), 1.9 (m, 2H). 1.7 (m, 2H)).

### 165F. Synthesis of 4-(2,6-Difluoro-benzoylamino)-1H-pyrazole-3-carboxylic acid [5-fluoro-2-(1-methyl-piperidin-4-yloxy)-phenyl]-amide

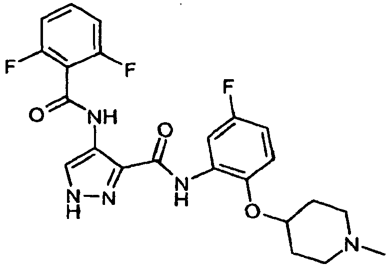

5-fluoro-2-(1-methyl-piperidin-4-yloxy)-phenylamine) (0.049 g, 0.22 mmol) was combined with 4-(2,6-difluorobenzoylamino)-1H-pyrazole-3-carboxylic acid (0.053 g, 0.20 mmol), EDC (0.048 g, 0.25 mmol), HOBt (0.034 g, 0.25 mmol) and DMF (1 ml) and the resulting reaction mixture was stirred at ambient temperature for 18 hours. The reaction mixture was reduced in vacuo and purified by preparative LC/MS to give 4-(2,6-Difluorobenzoylamino)-1H-pyrazole-3-carboxylic acid [5-fluoro-2-(1-methyl-piperidin-4-yloxy)-phenyl]-amide as a buff solid. (0.010 g, 11 %) (LC/MS: Rₜ 2.19, [M+H]⁺ 474.27).

### EXAMPLE 166 (comparative example)

### Synthesis of 4-(2,6-Difluoro-benzoylamino)-1H-pyrazole-3-carboxylic acid [5-fluoro-2-(2-pyrrolidin-1-yl-ethoxy) phenyl]-amide

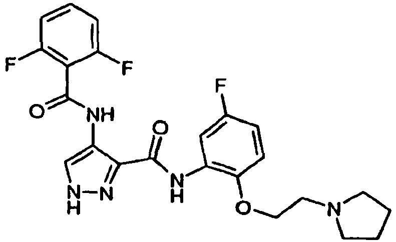

3,4-Dinitrofluorobenzene (0.93 g, 5 mmol) and 1-(2-hydroxyethylpyrrolidine) (0.69 g, 6 mmol) were dissolved in THF (10 ml) and stirred at ambient temperature while sodium hydride (60 % dispersion in mineral oil, 0.24 g, 6 mmol) was added in several small portions. The reaction mixture was stirred for 5 hours, diluted with ethyl acetate and the combined organics washed with water and brine, dried (MgSO₄) and reduced in vacuo. The resulting residue was subject to column chromatography, eluting with 5% MeOH / DCM to give an orange oil (0.94 g, 1:1 ratio of 1-[2-(3,4-dinitro-phenoxy)-ethyl]-pyrrolidine and 1-[2-(4-Fluoro-2-nitro-phenoxy)-ethyl]-pyrrolidine.

A sample of the mixture of products obtained (0.281 g) was dissolved in DMF (5 ml) under an atmosphere of nitrogen. Palladium on carbon (10 %, 0.028 g) was added and the reaction mixture was shaken under a hydrogen atmosphere for 20 hours. The solids were removed by filtration and the filtrate reduced in vacuo and combined with 4-(2,6-difluoro-benzoylamino)-1H-pyrazole-3-carboxylic acid (0.134 g, 0.50 mmol), EDC (0.116 g, 0.60 mmol), HOBt (0.081 g, 0.60 mmol) and DMF (2.5 ml) and the resulting reaction mixture was stirred at ambient temperature for 18 hours. The reaction mixture was reduced in vacuo and the residue partitioned between ethyl acetate (50 ml) and saturated aqueous sodium bicarbonate solution (50 ml). The organic layer was washed with brine, dried (MgSO₄) and reduced in vacuo to give the intermediate amides. Acetic acid (10 ml) was added to the crude amide and the mixture was heated at reflux for 3 hours and then reduced in vacuo. 4-(2,6-Difluoro-benzoylamino)-1H-pyrazole-3-carboxylic acid [5-fluoro-2-(2-pyrrolidin-1-yl-ethoxy)-phenyl]-amide was isolated from the residue by preparative LC/MS as an off white solid (0.040 g, 5.6 %). (LC/MS: Rₜ 2.38, [M+H]⁺ 474.33).

### EXAMPLES 167 - 223

By following the procedures described above, the compounds set out in Table 6 were prepared.

**Table 6**

| Example No. | Structure | Method | Differences | LCMS |
|---|---|---|---|---|
| 167 | 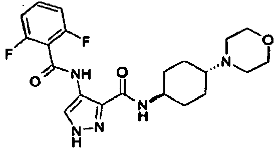 | A Starting amine prepared according to Procedure L | HOAt instead of HOBt DMSO as solvent instead of DMF Et₃N 2eq Purified by HPLC Cis/Trans Isomers separated after amine preparation (L) | [M+H]⁺ 434 Rₜ 1.97 |
| 168 | 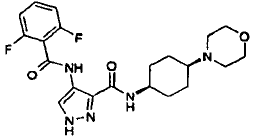 | A Starting amine prepared according to Procedure L | HOAt instead of HOBt DMSO as solvent instead of DMF Et₃N 2 eq Purified by chromatography 10% MeOH/CH₂Cl₂ Cis/Trans Isomers separated after amine preparation (L) | [M+H]⁺ 434 Rₜ 2.03 |
| 169* | 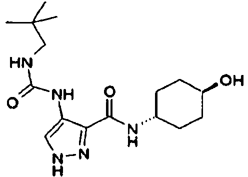 | Procedure D followed by G then E | | [M+H]⁺ 338 Rₜ 2.28 |
| 170 | 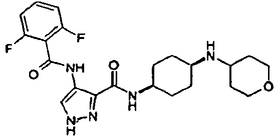 | A Starting amine prepared according to Procedure L | DMSO as solvent instead of DMF Et₃N eq Heated 80°C for 4 hours then RT O/N Purified by HPLC Cis/Trans isomers separated after final step | [M+H]⁺ 448 Rₜ 1.97 |
| 171* | 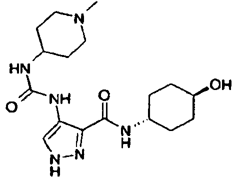 | Procedure D followed by G then E | | [M+H]⁺ 365 Rₜ 0.34 |
| 172 | 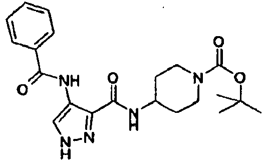 | B | Purified by column chromatography (pet. ether-EtOAc (1:1)) | [M+H]⁺ 414.13 Rₜ 3.05 |
| 173 | 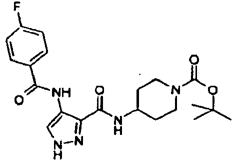 | B | Purified by column chromatography (pet. ether-EtOAc (1:1)) | [M+H]⁺ 432.12 Rₜ 3.12 |
| 174 | 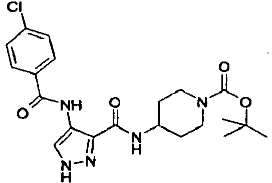 | B | Purified by column chromatography (pet. ether-EtOAc (1:1)) | [M+H]⁺ 448.06 Rₜ 3.33 |
| 175 | 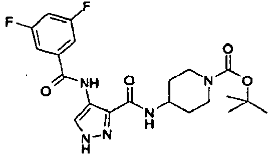 | B | Purified by column chromatography (pet. ether-EtOAc (1:1)) | [M+H]⁺ 450.08 Rₜ 3.29 |
| 176 | 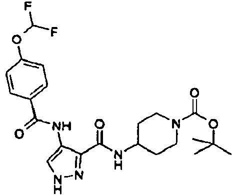 | B | Purified by column chromatography (pet. ether-EtOAc (1:1)) | [M+H]⁺ 480.05 Rₜ 3.18 |
| 177 | 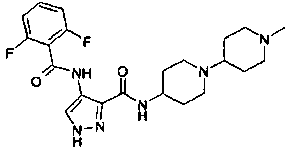 | A Starting amine prepared according to Procedure L | HOAt instead of HOBt DMSO as solvent instead of DMF Et₃N 2 eq Purified by HPLC and formation of HCl salt | [M+H]⁺ 447 Rₜ 2.01 |
| 178 | 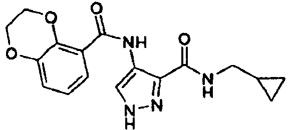 | B | | [M+H]⁺ 343.05 Rₜ 3.38 (polar method) |
| 179 | 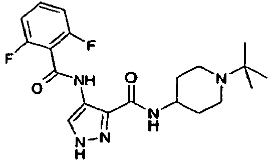 | A Butyl-piperidin-4-ylamine prepared by Procedure N | HOAt instead of HOBt Purified by trituration with MeOH | [M+H]⁺ 406 R, 1.85 |
| 180 | 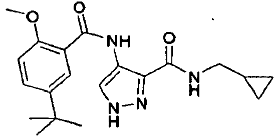 | B | | [M+H]⁺ 371.09 Rₜ 3.27 (polar method) |
| 181 | 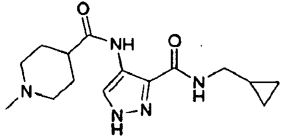 | B | | [M+H]⁺ 306.06 Rₜ 1.53 |
| 182 | 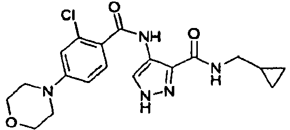 | B | | [M+H]⁺ 403.98 Rₜ 2.78 |
| 183 | 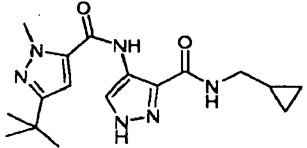 | B | | [M+H]⁺ 345.05 Rₜ 3.03 |
| 184^{*} | 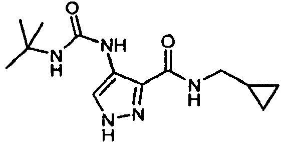 | B | | [M+H]⁺ 280.05 Rₜ 3.75 (basic method) |
| 185 | 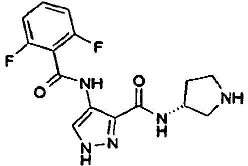 | A | HOAt instead of HOBt followed by EtOAc/HCl deprotection | [M+H]⁺ 336 Rₜ 1.67 |
| 186 | 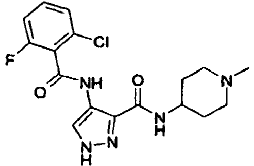 | A | | [M+H]⁺ 380.05 Rₜ 1.78 |
| 187 | 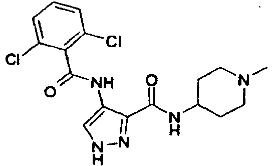 | A | | [M+H]⁺ 396.02 Rₜ 1.86 |
| 188 | 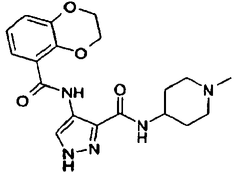 | A | | [M+H]⁺ 386.10 Rₜ 1.88 |
| 189 | 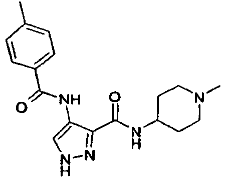 | A | | [M+H]⁺ 342.10 Rₜ 1.95 |
| 190 | 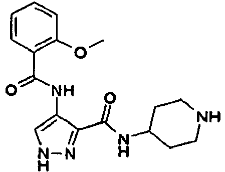 | M | | [M+H]⁺ = 344 Rₜ = 1.87 |
| 191 | 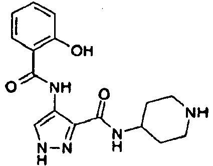 | M | | [M+H]⁺ = 330 Rₜ = 1.80 |
| 192 | 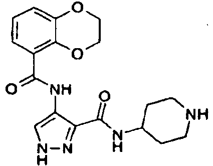 | M | | [M+H]⁺ = 372 Rₜ = 1.87 |
| 193 | 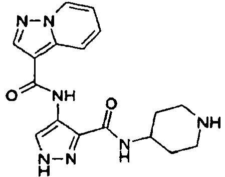 | M | | [M+H]⁺ = 354 Rₜ = 1.77 |
| 194 | 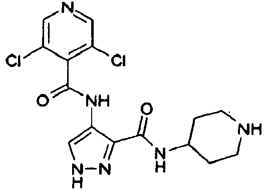 | M | Purified by flash chromatography eluting with dichloromethane 120ml, methanol 15, acetic acid 3ml, water 2ml (DMAW 120) | [M+H]⁺ = 383 / 385 Rₜ = 1.72 |
| 195 | 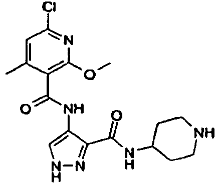 | M | Purified by flash chromatography eluting with DMAW 120 | [M+H]⁺ = 393 / 395 Rₜ = 1.86 |
| 196 | 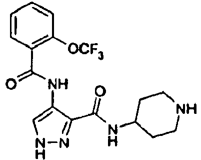 | M | | [M+H]⁺ = 398 Rₜ = 1.94 |
| 197 | 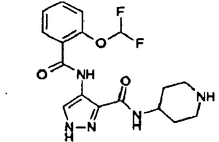 | M | | [M+H]⁺= 330 Rₜ = 1.80 |
| 198 | 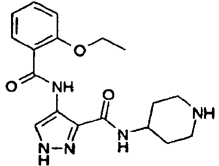 | M | | [M+H]⁺ = 358 Rₜ = 1.89 |
| 199 | 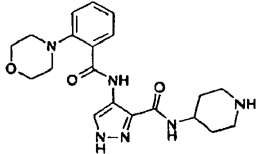 | M | | [M+H]⁺ = 399 Rₜ = 1.88 |
| 200 | 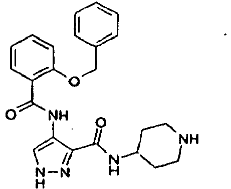 | M | | [M+H]⁺ = 420 Rₜ = 2.13 |
| 201 | 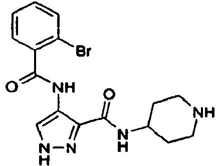 | M | | [M+H]⁺ = 392 / 394 Rₜ = 1.84 |
| 202 | 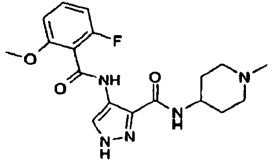 | B | Purified using flash chromatography (CH₂Cl₂-MeOH-AcOH-H₂O (90:18:3:2)) | [M+H]⁺ 376.14 Rₜ 1.78 |
| 203 | 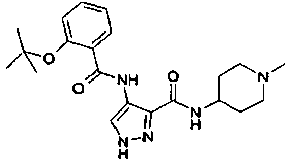 | B | Purified using flash chromatography (CH₂Cl₂-MeOH-AcOH-H₂O (90:18:3:2)) | [M+H]⁺ 400.17 Rₜ 2.08 |
| 204 | 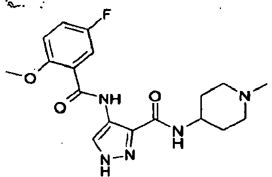 | B | Purified using flash chromatography (CH₂Cl₂-MeOH-AcOH-H₂O (90:18:3:2)) | [M+H]⁺ 376.15 Rₜ 1.92 |
| 205 | 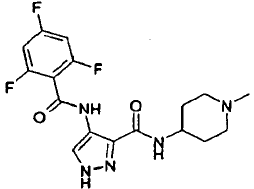 | B | Purified using column chromatography (CH₂Cl₂-MeOH-AcOH-H₂O (90:18:3:2)) | [M+H]⁺ 382.12 Rₜ 1.77 |
| 206 | 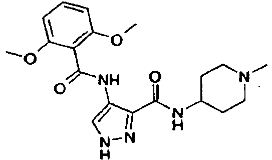 | B | Purified using column chromatography (CH₂Cl₂-MeOH-AcOH-H₂O (90:18:3:2)) | [M+H]⁺ 388.18 Rₜ 1.73 |
| 207 | 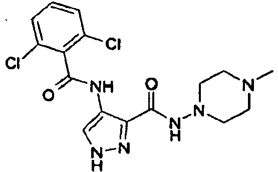 | A | Purified by flash chromatography eluting with DMAW 120 | [M+H]⁺ = 397 / 399 Rt = 1.83 |
| 208 | 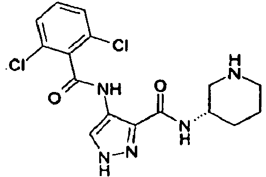 | A | Coupling using (S)-3-amino-1-N-BOC-piperidine. Deprotection as procedure M. Purified using column chromatography (CH₂Cl₂-MeOH-AcOH-H₂O (90:18:3:2)) | [M+H]⁺ 382.02 Rₜ 1.82 |
| 209 | 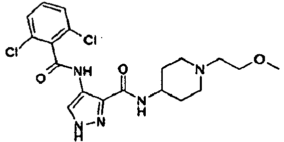 | A | | [M+H]⁺ 440.22 Rₜ 1.92 |
| 210 | 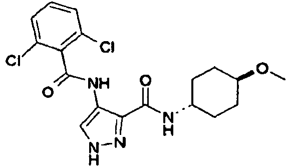 | A | | [M+H]⁺ 411.20 Rₜ 2.97 |
| 211 | 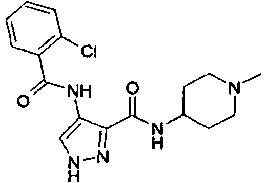 | A | Purified by prep. LCMS after work-up | [M+H]⁺ 362.11 Rₜ 1.91 |
| 212 | 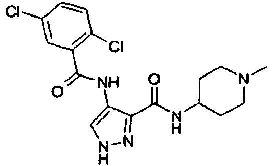 | A | Purified by prep. LCMS after work-up | [M+H]⁺ 396.08 Rₜ 2.06 |
| 213 | 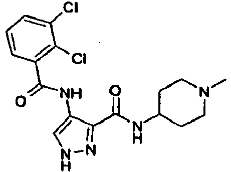 | A | Purified by prep. LCMS after work-up | [M+H]⁺ 396.06 Rₜ 2.04 |
| 214 | 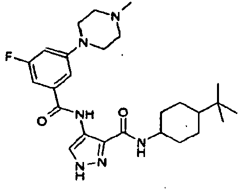 | B | The mixture was reduced in vacuo, the residue taken up in EtOAc and washed successively with saturated aqueous sodium bicarbonate, water and brine. The organic portion was dried (MgSO₄) and reduced in vacuo to give the desired product | [M+H]⁺ 485 Rₜ 2.59 |
| 215 | 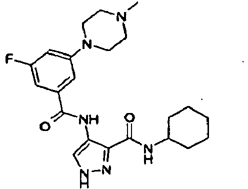 | B | The mixture was reduced in vacuo, the residue taken up in EtOAc and washed successively with saturated aqueous sodium bicarbonate, water and brine. The organic portion was dried (MgSO₄) and reduced in vacuo to give the desired product | [M+H]⁺ 429 Rₜ 2.25 |
| 216 | 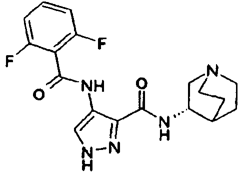 | A | Purified by flash chromatography eluting with DMAW 120 | [M+H]⁺ = 376 Rₜ = 1.85 |
| 217 | 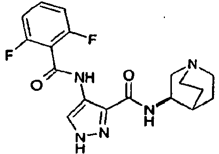 | A | Purified by flash chromatography eluting with DMAW 120 | [M+H]⁺ = 376 Rₜ = 1.87 |
| 218* | 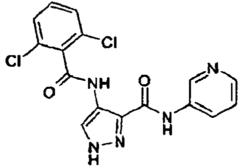 | A | Purified by flash chromatography eluting with 5% then 10% MeOH / DCM | [M+H]⁺ = 376 / 378 Rₜ = 2.23 |
| 219 | 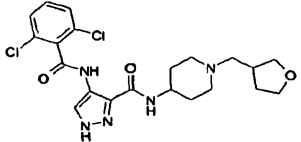 | A Starting amine prepared according to Procedure L | Purified by flash chromatography eluting with DMAW 90 | [M+H]⁺ = 466 / 468 Rₜ = 1.98 |
| 220* | 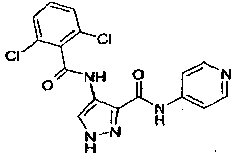 | A | Purified by flash chromatography eluting with 5% then 10% MeOH / DCM | [M+H]⁺ = 376 / 378 Rₜ = 2.09 |
| 221 | 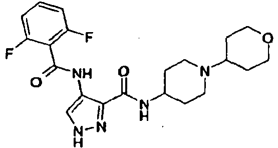 | A Starting amine prepared according to Procedure L | Purified by flash chromatography eluting with DMAW 90 | [M+H]⁺ = 434 Rₜ = 1.82 |
| 222* | 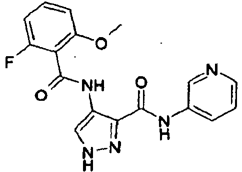 | A | Purified by flash chromatography eluting with 5% then 10% MeOH / DCM | [M+H]⁺ = 356 Rₜ = 2.11 |
| 223* | 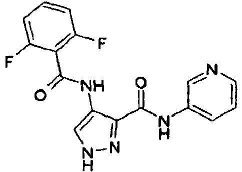 | A | Purified by flash chromatography eluting with 5% then 10% MeOH / DCM | [M+H]⁺ = 344 Rₜ = 2.09 |

### EXAMPLE 224 (Comparative Example) 4-(4-Methyl-piperazin-1-yl)-1H-pyrazole-3-carboxylic acid (4-fluoro-phenyl)amid

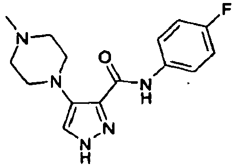

Bis(2-chloroethyl)methylamine hydrochloride (97mg; 0.5mmol) was added to a stirred solution of 4-amino-1H-pyrazole-3-carboxylic acid (4-fluoro-phenyl)-amide (100mg; 0.45mmol), tetrabutylammonium iodide (20mg; 0.045mmol) and dlisopropyethylamine (200ul) 1.13mmol) in DMF (5ml), and the resulting mixture was heated at 200°C (100W) for 30 minutes in a CEM Discover^{™} microwave synthesiser. The DMF was removed under vacuum, then purified by flash column chromatography, eluting with dichloromethane / methanol / acetic acid / water (90:18:3:2). Product containing fractions were combined and evaporated, treated with HCl in ethyl acetate and then re-evaporated with toluene (2x20ml) to give an off white solid (27mg). (LC/MS: Rₜ 1.64, [M+H]⁺ 378).

### EXAMPLE 225 (Comparative Example)

### 4-Morpholin-4-yl-1H-pyrazole-3-carboxylic acid (4-fluoro-phenyl)-amide

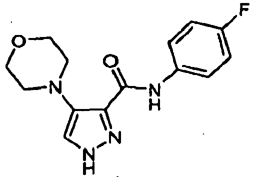

The compound was prepared in a manner analogous to Example 224, but using bis(2-chloroethyl)ether In place of bis(2-chloroethyl)methylamine hydrochloride. (LC/MS: Rₜ 2.48 [M+H]⁺ 291).

### EXAMPLE 226 (Comparative Example)

### 4-(2,4-Dichloro-phenyl)-1H-pyrazole-3-carboxylic acid 4-(4-methyl-piperazin-1-yl)-benzylamide

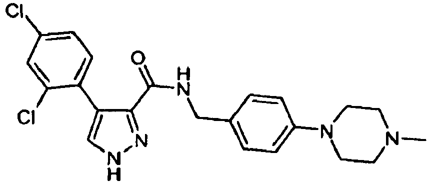

### 226A. Preparation of 4-(2,4-dichloro-phenyl)-1H-pyrazole-3-carboxylic acid

A solution of 4-(2,4-dichloro-phenyl)-1H-pyrazole-3-carboxylic acid ethyl ester (205 mg; 0.72 mmol) and lithium hydroxide monohydrate (125 mg; 2.9 mmol) in 1:1 THF/water (10 ml) was heated at 60 °C overnight. The THF was removed by evaporation, the aqueous phase acidified with 1 M hydrochloric acid then extracted with ethyl acetate (20 ml). The ethyl acetate layer was dried (MgSO₄), filtered and evaporated to give 200 mg of 4-(2,4-dichloro-phenyl)-1H-pyrazole-3-carboxylic acid. (LC/MS: [M+H]⁺ 256.85).

### 226B. Preparation of 4-(2,4-dichloro-phenyl)-1H-pyrazole-3-carboxylic acid 4-(4-methyl-piperazin-1-yl)-benzylamide

A solution of 4-(2,4-dichloro-phenyl)-1H-pyrazole-3-carboxylic acid (70 mg; 0.27 mmol), 4-(4-methyl-piperazin-1-yl)-benzylamine (62 mg; 0.3 mmol), EDAC (63 mg; 0.33 mmol) and HOBt (45 mg; 0.33 mmol) in 5 ml of DMF was stirred at room temperature for 48 hours. The reaction was evaporated and the residue partitioned between ethyl acetate and brine. The ethyl acetate layer was separated, dried (MgSO₄), filtered, evaporated then dried further under vacuum to give 34 mg of 4-(2,4-dichloro-phenyl)-1H-pyrazole-3-carboxylic acid 4-(4-methyl-piperazin-1-yl)-benzylamide. (LC/MS: Rₜ 2.42 [M+H]⁺ 444).

### EXAMPLE 227 (Comparative Examples)

### 4-(2,4-Dichloro-phenyl)-1H-pyrazole-3-carboxylic acid 4-methylsulphamoylmethyl-benzylamide

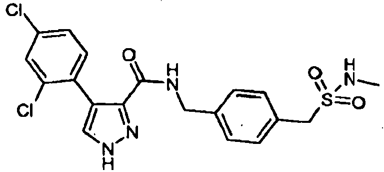

The title compound was prepared in a manner analogous to Example 226, but using (4-aminomethyl-phenyl)-N-methyl-methanesulphonamide as the starting material. 6 mg of product were isolated as a white solid. (LC/MS: Rₜ 3.56 [M+H]⁺ 440).

### EXAMPLE 228 (Comparative Examples)

### 4-Phenyl-1H-pyrazole-3-carboxylic acid amide

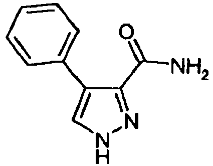

### 228A. 2-Benzylidene-but-3-yne nitrile

To a solution of benzaldehyde (2 g; 18.9 mmol) and malononitrile (1.37 g; 20.7 mmol) in ethanol (40 ml) was added 5 drops of piperidine and the mixture was heated at reflux overnight. The reaction was cooled, evaporated then purified by flash column chromatography eluting with 1:9 ethyl acetate/hexane and the product containing fractions combined and evaporated to give 930 mg of 2-benzylidene-but-3-yne nitrile.

### 228B. 4-phenyl-5-trimethylsilanyl-1H-pyrazole-3-carbonitrile

n-Butyl lithium (2.7 M solution in heptane) (3.3 ml, 9 mmol) was added drop wise to a stirred solution of trimethylsilyl diazomethane (2 M solution in diethyl ether) (4.5 ml, 9 mmol) In anhydrous THF (10 ml) at -78 °C. under a nitrogen atmosphere, then stirred for a further 30 minutes. To this was added drop wise a solution of 2-benzylidene-but-3-yne nitrile (920 mg; 6 mmol) in anhydrous THF (5ml), the mixture stirred for 30 minutes at -78 °C then gradually allowed to warm to room temperature overnight. The reaction mixture was diluted with ethyl acetate (30 ml) then washed with saturated ammonium chloride solution followed by brine. The ethyl acetate layer was separated, dried (MgSO₄), filtered and evaporated. The crude product was purified by flash column chromatography eluting with 1:8 then 1:4 ethyl acetate/hexane and the product containing fractions combined and evaporated to give 1.0 g of 4-phenyl-5-trimethylsilanyl-1H-pyrazole-3-carbonitrile.

### 228C. 4-phenyl-1H-pyrazole-3-carboxylic acid amide

4-Phenyl-5-trimethylsilanyl-1H-pyrazole-3-carbonitrile (500 mg; 2.1 mmol) was dissolved in 1 ml of ethanol, treated with potassium hydroxide (600 mg) in water (3 ml) then heated at 150 °C (100 W) for 30 minutes then 170 °C (100 W) for 20 minutes in a CEM Discover^{™} microwave synthesiser. The reaction mixture was acidified to pH1 with concentrated hydrochloric acid, diluted with water (40 ml) then extracted with ethyl acetate (2 x 40 ml). The combined ethyl acetate layers were separated, dried (MgSO₄), filtered and evaporated to give a 3:1 mixture of 4-phenyl-1H-pyrazole-3-carboxylic acid and 4-phenyl-1 H-pyrazole-3-carboxylic acid amide. A 50 mg batch of the crude material was purified by flash column chromatography eluting with 5% methanol/dichloromethane, and the product containing fractions combined and evaporated to give 15 mg of 4-phenyl-1H-pyrazole-3-carboxylic acid amide as a white solid. (LC/MS: Rₜ 2.15 [M+H]⁺ 188).

### EXAMPLE 229 (Comparative Example)

### 4-phenyl-1H-pyrazole-3-carboxylic acid phenylamide

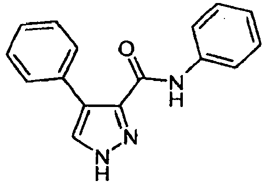

A solution of 4-phenyl-1H-pyrazole-3-carboxylic acid (75 mg; 0.4 mmol) (prepared according to Example 228C), aniline (45 µl; 0.48 mmol), EDAC (92 mg; 0.48 mmol) and HOBt (65 mg; 0.48 mmol) in 5 ml of DMF was stirred at room temperature overnight. The reaction was evaporated then purified by flash column chromatography eluting with 1:3 then 1:2 ethyl acetate/hexane. Product containing fractions were combined and evaporated to give 30 mg of 4-phenyl-1H-pyrazole-3-carboxylic acid phenylamide as a white solid. (LC/MS: Rₜ 3.12 [M+H]⁺ 264).

### EXAMPLE 230 (Comparative Example)

### 4-Phenyl-1H-pyrazole-3-carboxylic acid 4-(4-methyl-piperazin-1-yl)-benzylamide

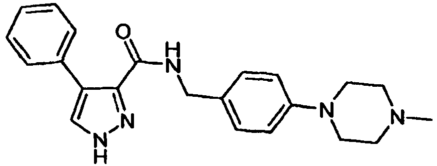

The compound was prepared in a manner analogous to Example 229, but using 4-(4-methyl-piperazin-1-yl)-benzylamine as the starting material. 6 mg of product were isolated as a white solid. (LC/MS: Rₜ 2.05 [M+H]⁺ 376).

### EXAMPLE 231 (Comparative Example)

### 4-Phenyl-1H-pyrazole-3-carboxylic acid (6-methoxy-pyridin-3-yl) amide

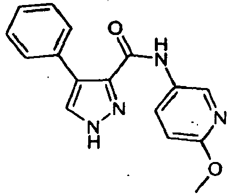

The compound was prepared in a manner analogous to Example 230, but using 3-amino-6-methoxypyridine as the amine fragment. 100 mg of product were Isolated as a pale brown solid. (LC/MS: Rₜ 3.17 [M+H]⁺ 295).

### EXAMPLE 232 (Comparative Example)

### 4-(3-benzyloxy-phenyl)-1H-pyrazole-3-carboxylic acid 4-(4-methyl-piperazin-1-yl)-benzylamide

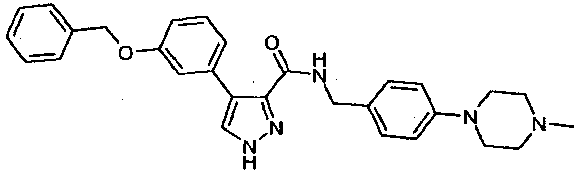

The compound was prepared in a manner analogous to Example 226. The product was isolated as a white solid. (LC/MS: Rₜ 2.65 [M+H]⁺ 482).

### EXAMPLE 233 (Comparative Example)

### 4-(3-Hydroxy-phenyl)-1H-pyrazole-3-carboxylic acid 4-(4-methyl-piperazin-1-yl)-benzylamide

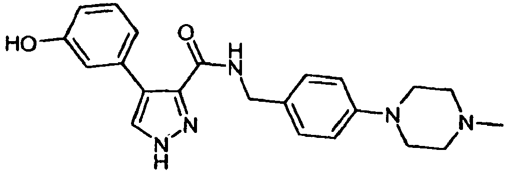

A solution of 4-(3-benzyloxy-phenyl)-1H-pyrazole-3-carboxylic acid 4-(4-methyl-piperazin-1-yl)-benzylamide (25 mg; 0.05mmol) in methanol (5 ml), was treated with 10% palladium on carbon (10 mg) then hydrogenated at room temperature and pressure overnight. The catalyst was removed by filtration through Celite and the filtrate evaporated. Purification by preparative LC/MS gave 8 mg of the required product as a cream solid. (LC/MS: Rₜ 1.67 [M+H]⁺ 392).

### EXAMPLE 234 (Comparative Example)

### 4-(-5-Methyl-3H-imidazol-4-yl)-1H-pyrazole-3-carboxylic acid (4-fluoro-phenyl)-amide

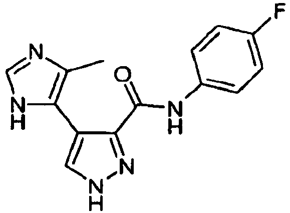

The compound was prepared in a manner analogous to Example 226, but using 4-methyl-5-formylimidazole as the starting material in the condensation step. The product (6 mg) was isolated as a white solid. (LC/MS: Rₜ 2.00 [M+H]⁺ 286).

### EXAMPLE 235 (Comparative Example)

### 4-(2.5-Dimethyl-pyrrol-1-yl)-1H-pyrazole-3-carboxylic acid (4-fluoro-phenyl)-amide

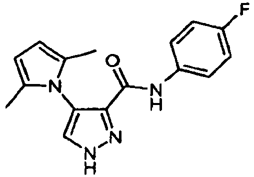

A mixture of 4-amino-1H-pyrazole-3-carboxylic acid (4-fluoro-phenyl)-amide (100 mg) and Montmorillonite KSF clay (100 mg) in acetonylacetone (1 ml) was heated at 120 °C (50 W) for 15 minutes in a CEM discover microwave synthesiser. The reaction mixture was diluted with 5% methanol/dichloromethane, filtered and evaporated. The crude product was purified by flash column chromatography eluting with 1:2 ethyl acetate/hexane, and the product containing fractions were combined and evaporated to give 65 mg of the target molecule as a pale brown solid. (LC/MS: Rₜ 3.75 [M+H]⁺ 299).

### EXAMPLE 236 (Comparative Example)

### 4-(3-Hydroxymethyl-phenyl)-1H-pyrazole-3-carboxylic acid phenylamide

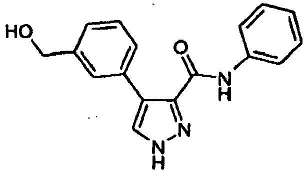

### 236A. 4-iodo-1H-pyrazole-3-carboxylic acid phenylamide

An aqueous solution of sodium nitrite (760 mg) in 2 ml of water was added drop wise to a stirred suspension of 4-amino-1H-pyrazole-3-carboxylic acid phenylamide (2 g; 10 mmol) in concentrated hydrochloric acid (20 ml) at 0 °C, then stirred at 0 °C for a further 60 minutes. The reaction mixture was diluted with acetone (10 ml) then treated with potassium iodide (1.8 g) and copper (I) iodide (2.1 g) and stirred at room temperature for 90 minutes. The reaction mixture was diluted with brine and ethyl acetate then washed with saturated sodium thiosulphate solution. The ethyl acetate layer was separated, dried (MgSO₄), filtered and evaporated to give 680 mg of 4-iodo-1H-pyrazole-3-carboxylic acid phenylamide.

### 236B. 4-iodo-1-(4-methoxy-benzyl)-1H-pyrazole-3-carboxylic acid phenylamide

A solution of 4-iodo-1H-pyrazole-3-carboxylic acid phenylamide (670 mg; 2.14 mmol) in acetonitrile (10 ml) was treated with potassium carbonate (360 mg; 2.57mmol)) followed by 4-methoxybenzyl chloride (320 µl; 2.35 mmol). The mixture was stirred at room temperature overnight then evaporated under reduced pressure. The residue was partitioned between ethyl acetate and brine; the ethyl acetate layer was separated, dried (MgSO₄), filtered and evaporated. The crude material was purified by flash column chromatography eluting with 1:3 ethyl acetate/hexane and the product containing fractions combined and evaporated to give 660 mg of 4-iodo-1-(4-methoxy-benzyl)-1 H-pyrazole-3-carboxylic acid phenylamide.

### 236C. 4-(3-hydroxymethyl-phenyl)-1-(4-methoxy-benzyl)-1H-pyrazole-3-carboxylic acid phenylamide

A mixture of 4-iodo-1-(4-methoxy-benzyl)-1H-pyrazole-3-carboxylic acid phenylamide (50 mg; 0.11 mmol), bis(tri-tert-butylphosphine)palladium (12 mg), potassium carbonate (100 mg; 0.66 mmol) and 3-(hydroxmethyl)benzene boronic acid (21mg,; 0.14mmol) in ethanol/toluene/water (4 m1:1 ml:1 ml) was heated at 120 °C (50 W)for 15 minutes in a CEM Discover microwave synthesiser. The reaction was evaporated and the residue partitioned between ethyl acetate and brine. The ethyl acetate layer was separated, dried (MgSO₄), filtered and evaporated and the crude material purified by flash column chromatography eluting with 1:2 then 2:1 ethyl acetate/hexane. Product containing fractions were combined and evaporated to give 60 mg of 4-(3-hydroxymethyl-phenyl)-1-(4-methoxy-benzyl)-1H-pyrazole-3-carboxylic acid phenylamide.

### 236D. 4-(3-Hydroxymethyl-phenyl)-1H-pyrazole-3carboxylicacid phenylamide

A mixture of 4-(3-hydroxymethyl-phenyl)-1-(4-methoxy-benzyl)-1H-pyrazole-3-carboxylic acid phenylamide (20 mg) and anisole (20 µl) in trifluoroacetic acid (1 ml) was heated at 120 °C (50 W) for 15 minutes in a CEM Discover microwave synthesiser. The reaction was evaporated then purified by flash column chromatography eluting with 2:1 ethyl acetate/hexane. Product containing fractions were combined and evaporated to give 5 mg of product. (LC/MS: Rₜ 2.55 [M+H]⁺ 294).

### EXAMPLE 237

### Preparation of 4-(2,6-dichloro-benzoylamino)-1H-pyrazole-3-carboxylic acid piperidin-4-ylamide hydrochloride

### 237A. 4-(2,6-dichloro-benzoylamino)-1H-pyrrazole-3-carboxylic acid

2,6-dichlorobenzoyl chloride (8.2 g; 39.05 mmol) was added cautiously to a solution of 4-amino-1H-pyrazole-3-carboxylic acid methyl ester (prepared in a manner analogous to 165B) (5 g; 35.5 mmol) and triethylamine (5.95 ml; 42.6 mmol) in dioxan (50 ml) then stirred at room temperature for 5 hours. The reaction mixture was filtered and the filtrate treated with methanol (50 ml) and 2M sodium hydroxide solution (100 ml), heated at 50 °C for 4 hours, and then evaporated. 100 ml of water was added to the residue then acidified with concentrated hydrochloric acid. The solid was collected by filtration, washed with water (100 ml) and sucked dry to give 10.05 g of 4-(2,6-dichloro-benzoylamino)-1H-pyrazole-3-carboxylic acid as a pale violet solid.

### 237B. 4-{[4-(2,6-dichloro-benzoylamino)-1H-pyrazole-3-carbonyl]-amino}-piperidine-1-carboxylic acid tert-butyl ester

A mixture of 4-(2,6-dichloro-benzoylamino)-1H-pyrazole-3-carboxylic acid (6.5 g, 21.6 mmol), 4-amino-1-BOC-piperidine (4.76 g, 23.8 mmol), EDC (5.0 g, 25.9 mmol) and HOBt (3.5 g, 25.9 mmol) in DMF (75 ml) was stirred at room temperature for 20 hours. The reaction mixture was reduced In vacuo and the residue partitioned between ethyl acetate (100 ml) and saturated aqueous sodium bicarbonate solution (100 ml). The organic layer was washed with brine, dried (MgSO₄) and reduced in vacuo. The residue was taken up in 5 % MeOH-DCM (-30 ml). The insoluble material was collected by filtration and, washed with DCM and dried in vacuo to give 4-{[4-(2,6-dichloro-benzoylamino)-1H-pyrazole-3-carbonyl]-amino}-piperidine-1-carboxylic acid tert-butyl ester (5.38 g) as a white solid. The filtrate was reduced In vacuo and the residue purified by column chromatography using gradient elution 1:2 EtOAc / hexane to EtOAc to give further 4-{[4-(2,6-dichloro-benzoylamino)-1H-pyrazole-3-carbonyl)-amino}-piperidine-1-carboxylic acid tert-butyl ester (2.54 g) as a white solid.

### 237C. 4-(2,6-dichloro-benzoylamino)-1H-pyrazole-3-carboxylic acid piperidin-4-ylamid

A solution of 4-{[4-(2,6-dichloro-benzoylamino)-1H-pyrazole-3-carbonyl]-amino}-piperidine-1-carboxylic acid tert-butyl ester (7.9 g) in MeOH (50 mL) and EtOAc (50ml) was treated with sat. HCl-EtOAc (40 mL) then stirred at r.t. overnight. The product did not crystallise due to the presence of methanol, and therefore the reaction mixture was evaporated and the residue triturated with EtOAc. The resulting off white solid was collected by filtration, washed with EtOAc and sucked dry on the sinter to give 6.3g of 4-(2,6-dichloro-benzoylamino)-1H-pyrazole-3-carboxylic acid piperidin-4-ylamide as the hydrochloride salt. (LC/MS: Rₜ 5.89, [M+H]⁺ 382 / 384).

### EXAMPLE 238 (Comparative Example)

### 4-Methanesulfonylamino-1H-pyrazole-3-carboxylic acid (4-fluoro-phenyl)-amide

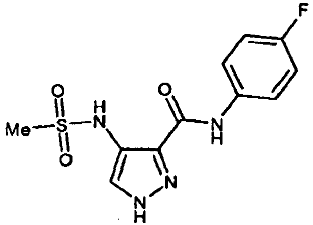

A solution of 4-amino-1H-pyrazole-3-carboxylic acid (4-fluorophenyl)-amide (50mg) (Example 2B) and methanesulphonic anhydride (45mg) in pyridine (1ml) was stirred at room temperature overnight then evaporated and purified by flash column chromatography eluting with 2:1 EtOAc / hexane. Evaporation of product containing fractions gave 20mg of the title compound. (LC/MS: Rt 2.87; {M+H+] 299).

### EXAMPLES 239 TO 245

The compounds of Examples 239 to 245 were prepared using the methods described above or methods closely analogous thereto.

### EXAMPLE 239

### 4-(2,6-Difluoro-benzoylaminol-1H-pyrazole-3-carboxylic acid [1-(2-fluoro-ethyl)-piperidin-4-yl]-amide

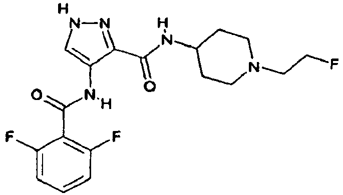

### EXAMPLE 240 (comparative example)

### 4-(2,6-Dichloro-benzoylamino)-1H-pyazole-3-carboxylic acid (6-chloro-pyridin-3-yl)-amide

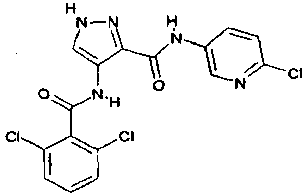

### EXAMPLE 241 (comparative example)

### 4-(2,6-Dichloro-benzoylamino)-1H-pyrazole-3-carboxylic acid (6-amino-pyridin-3-yl)-amide

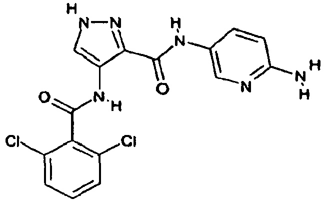

### EXAMPLE 242 (comparative example)

### 4-(2,6-Dichloro-benzoylaminol-1H-pyrazole-3-carboxylic acid (6-methoxy-pyridin-3-yl)-amide

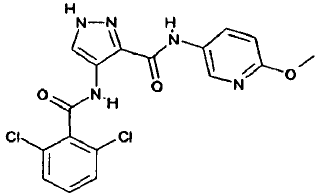

### EXAMPLE 243

### 4-[3-Chloro-5-(4-methyl-piperazin-1-yl)-benzoylamino]-1H-pyrazole-3-carboxylic acid cyclohexylamide

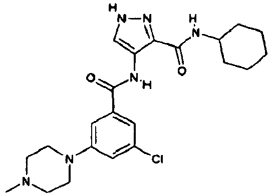

### EXAMPLE 244

### 4-(2,6-Difluoro-benzoylamino)-1H-pyrazole-3-carboxylic acid [1-(2,2-difluoro-ethyl)-piperidin-4-yl]-amide

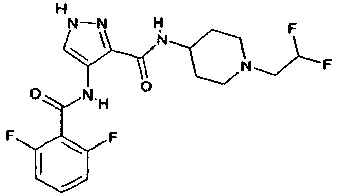

### EXAMPLE 245

### 4-[3-(4-Methyl-piperazin-1-yl)-benzoylamino]-1H-pyrazole-3-carboxylic acid cyclohexylamide

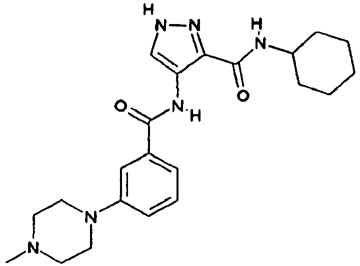

### EXAMPLE 246

### Preparation of 4-(2,6-dichloro-benzoylamino)-1H-pyrazole-3carboxylic acid piperidin-4-ylamide acetic acid salt

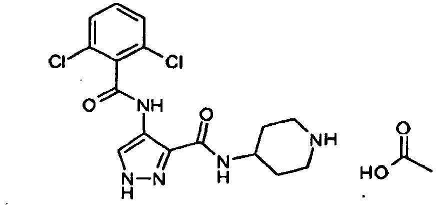

To a solution of 4-(2,6-dichloro-benzoylamino)-1H-pyrazole-3-carboxylic acid piperidin-4-ylamide hydrochloride salt (Example (237C) 20.6 g, 50 mmol) in water (500 ml) stirring at ambient temperature was added sodium bicarbonate (4.5 g, 53.5 mmol). The mixture was stirred for 1 hour and the solid formed collected by filtration and dried in vacuo azeotroping with toluene (x 3) to give the corresponding free base of 4-(2,6-dichloro-benzoylamino)-1H-pyrazole-3-carboxylic acid piperidin-4-ylamide.

¹H NMR (400 MHz, DMSO-d₆) □ 10.20 (s, 1 H), 8.30 (s, 1H), 8.25 (d, 1H), 7.60 - 7.50 (m, 3H), 3.70 (m, 1H), 3.00 (d, 2H), 2.50 (m, 2H), 1.70 (d, 2H), 1.50 (m, 2H).

To a stirred suspension of 4-(2,6-dichloro-benzoylamino)-1H-pyrazole-3-carboxylic acid piperidin-4-ylamide (10.0 g, 26.2 mmol) in methanol (150 ml) was added glacial acetic acid (15 ml, 262 mmol) at ambient temperature. After 1 h, a clear solution was obtained which was reduced in vacuo azeotroping with toluene (x 2). The residue was then triturated with acetonitrile (2 x 100 ml) and the solid dried in vacuo to give 4-(2,6-dichloro-benzoylamino)-1H-pyrazole-3-carboxylic acid piperidin-4-ylamide acetic acid salt (10.3 g) as a white solid.

¹H NMR (400 MHz, DMSO-d₆) □ 10.20 (s, 1H), 8.40 (d, 1 H), 8.35 (s, 1 H), 7.60 - 7.50 (m, 3H), 3.85 (m, 1H), 3.00 (d, 2H), 2.60 (t, 2H), 1.85 (s, 3H), 1.70 (d, 2H), 1.55 (m, 2H)

### EXAMPLE 247

### Synthesis of the methanesulphonic acid salt of 4-(2,6-dichloro-benzoylamino)-1H-pyrazole-3-carboxylic acid piperidin-4-ylamide

The methane sulphonic acid salt of 4-(2,6-dichloro-benzoylamino)-1H-pyrazole-3-carboxylic acid piperidin-4-ylamide may be prepared by the synthetic route shown in the Scheme below.

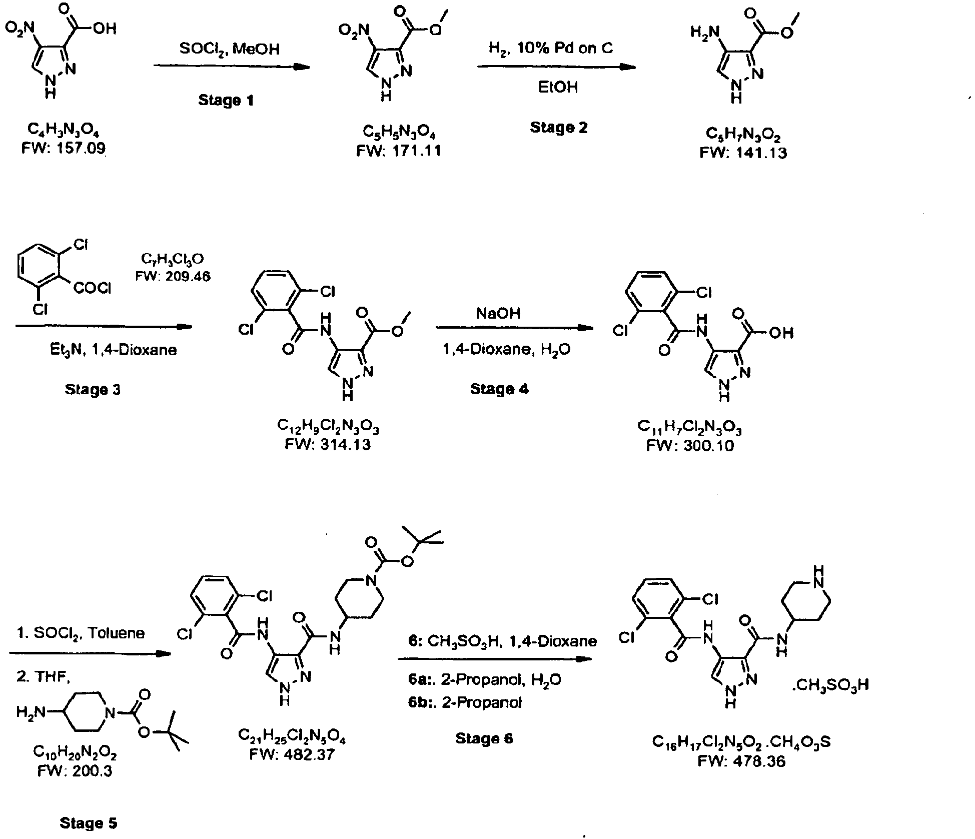

### Stage 1: Preparation of 4-nitro-1H-pyrazole-3-carboxylic acid methyl ester

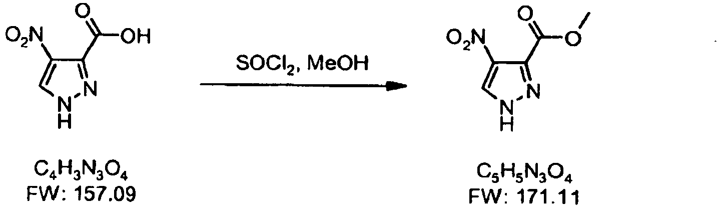

A 20L reaction vessel equipped with a digital thermometer and stirrer was charged with 4-nitro-1H-pyrazole-3-carboxylic acid (1.117 Kg, 7.11 mol, 1 wt) and methanol (8.950 L, 8 vol). The reaction mixture was stirred under nitrogen, cooled to 0 to 5 °C, thionyl chloride (0.581 L, 8.0 mol, 0.52 vol) added over 180 minutes and the resultant mixture allowed to warm to and stir at 18 to 22 °C overnight, after which time ¹H NMR analysis (d₆-DMSO) indicated reaction completion. The reaction mixture was concentrated under reduced pressure at 40 to 45 °C, the residue treated with toluene and re-concentrated (3x 2.250 L, 3x 2vol) under reduced pressure at 40 to 45 °C to give 4-nitro-1H-pyrazole-3-carboxylic acid methyl ester as an off-white solid (1.210 Kg, 99.5%).

### Stage 2: Preparation of 4-amino-1H-pyrazole-3-carboxylic acid methyl ester

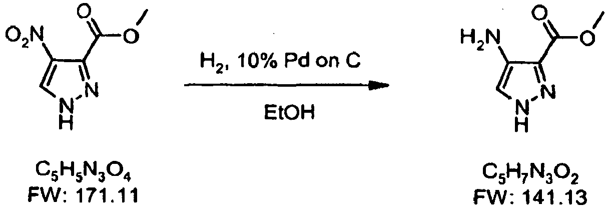

A 20 L reaction vessel equipped with a digital thermometer and stirrer was charged with palladium on carbon (10% wet paste, 0.170 Kg, 0.14 wt) under nitrogen. In a separate vessel a slurry of 4-nitro-1H-pyrazole-3-carboxylic acid methyl ester (1.210 Kg, 7.07 mol, 1 wt) in ethanol (12.10 L, 10 vol) was warmed to 30 to 35 °C to effect dissolution and the solution added to the catalyst under nitrogen. Following a nitrogen-hydrogen purge sequence an atmosphere of hydrogen was introduced and the reaction mixture maintained at 28 to 30 °C until reaction completion (5 to 10 hours) was noted by ¹H NMR analysis (d₆-DMSO). Following a purge cycle, the reaction mixture under nitrogen was filtered and the liquors concentrated under reduced pressure to give 4-amino-1H-pyrazole-3-carboxylic acid methyl ester (0.987 Kg, 98.9%).

### Stage 3: Preparation of 4-(2,6-dichlorobenzoylamino)-1H-pyrazole-3-carboxylic acid methyl ester

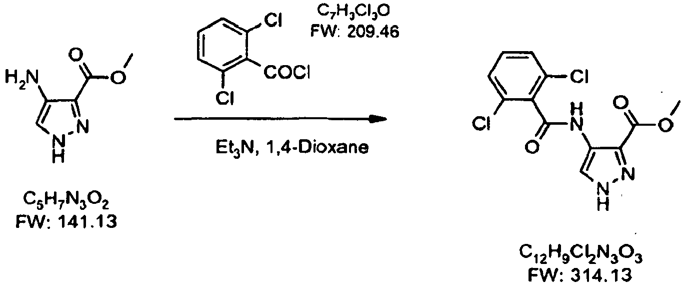

A solution of 4-amino-1H-pyrazole-3-carboxylic acid methyl ester (0.634 Kg, 4.49 mol, 1 wt) in 1,4-dioxane (8.90 L, 9 vol) under nitrogen was treated with triethylamine (0.761 L, 5.46 mol, 1.2 vol) followed by 2,6-dichlorobenzoyl chloride (0.710 L, 4.96 mol, 0.72 vol) such that the internal temperature was maintained in the range 20 to 25 °C. Residual 2,6-dichlorobenzoyl chloride was washed in with a line rinse of 1,4-dioxane (0.990 L, 1 vol) and the reaction mixture stirred at 18 to 25° C until complete (16 hours) by TLC analysis (eluent: ethyl acetate: heptanes 3:1; R_{f} amine 0.25, R_{f product} 0.65). The reaction mixture was filtered, the filter-cake washed with 1,4-dioxane (2x 0.990 L, 2x 1 vol) and the combined filtrates (red) progressed to Stage 4 without further isolation.

### Stage 4: Preparation of 4-(2,6-dichlorobenzoylamino)-1H-pyrazole-3-carboxylic acid

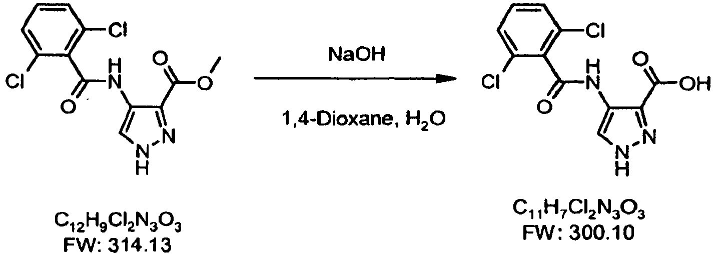

To a solution of sodium hydroxide (0.484 Kg, 12.1 mol) in water (6.05 L) was charged a solution of the Stage 3 ester in one portion: (1.099 Kg, 3.50 mol in 6.00 L). The reaction mixture was stirred to completion at 20 to 25 °C as determined by TLC analysis (eluent: ethyl acetate: heptanes 3:1; R_{f ester} 0.65, R_{f Stage 4} baseline). The reaction mixture was concentrated under reduced pressure at 45 to 50 °C, the oily residue diluted with water (9.90 L) and acidified to pH 1 with concentrated hydrochloric acid such that the temperature was maintained below 30 °C. The resulting precipitate was collected by filtration, washed with water (5.00 L), pulled dry on the filter and subsequently washed with heptanes (5.00 L). The filter-cake was charged to a 20 L rotary evaporator flask and drying completed azeotropically with toluene (2x 4.50 L) to afford 4-(2,6-dichlorobenzoylamino)-1H-pyrazole-3-carboxylic acid as a yellow solid (1.044 Kg, approx. 99.5%).

### Stage 5: Preparation of 4-{[4-(2,6-dichlorobenzoylamino)-1H-pyrazole-3-carbonyl]amino}piperidine-1-carboxylic acid tert-butyl ester

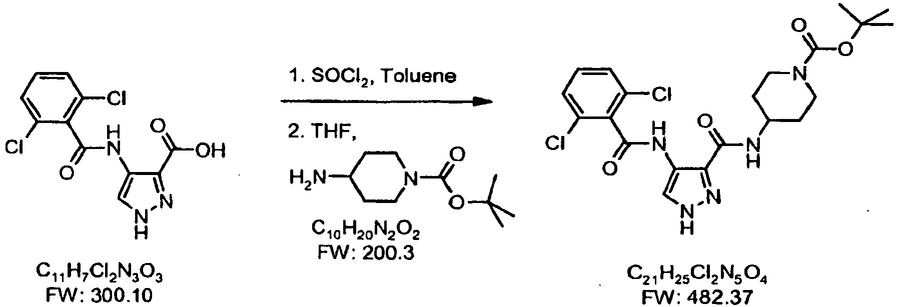

Stage 4 product (1.0 wt) and toluene (10.0 vol) were charged to a suitably sized flange flask equipped with a mechanical stirrer, dropping funnel and thermometer. The contents were stirred under nitrogen at 16 to 25 °C and thionyl chloride (0.3 vol) was added slowly. The contents were then heated to 80 to 100 °C and stirred at this temperature until the reaction was judged complete by ¹H NMR. Further toluene (up to 10 vol) could be added at this stage if the contents were to become too thick to stir. Once complete, the mixture was cooled to between 40 and 50 °C and then concentrated under vacuum at 45 to 50 °C to dryness. The residue was then azeo-dried with toluene (3x 2.0 vol).

The isolated solid was transferred to a suitably sized flask and tetrahydrofuran (5.0 vol) was charged. The contents were stirred under nitrogen at 16 to 25 °C and triethylamine (0.512 vol) was added. To a separate flask was charged 4-amino-piperidine-1-carboxylic acid tert-butyl ester (0.704 wt) and tetrahydrofuran (5.0 vol). The contents were agitated until complete dissolution was achieved and the solution was then charged to the reaction flask, maintaining the temperature between 16 and 30 °C. The reaction mixture was then heated to between 45 and 50 °C and the contents stirred until judged complete by ¹H NMR. The contents were then cooled to between 16 and 25 °C and water (5.0 vol) was charged. Mixed heptanes (0.5 vol) were added, the contents were stirred for up to 10 minutes and the layers were separated. The aqueous phase was then extracted with tetrahydrofuran:mixed heptanes [(9:1), 3x 5.0 vol]. The organic phases were combined, washed with water (2.5 vol) and then concentrated under vacuum at 40 to 45 °C. The residue was azeotroped with toluene (3x 5.0 vol) and concentrated to dryness to yield the crude Stage 5 product The solid was then transferred to a suitably sized flask, methanol: toluene [(2.5:97.5), 5.0 vol] was added and the slurry was stirred under nitrogen for 3 to 18 hours. The contents were filtered, the filter-cake was washed with toluene (2x 0.7 vol) and the solid was then dried under vacuum at 40 to 50 °C to yield 4-{(4-(2,6-dichlorobenzoylamino)-1H-pyrazole-3-carbonyl]amino}piperidine-1-carboxylic acid tert-butyl ester as an off-white solid.

Two batches of Stage 4 product (0.831 kg per batch) were processed in this way to give a total of 2.366 kg (88.6% yield) of 4-{[4-(2,6-dichlorobenzoylamino)-1H-pyrazole-3-carbonyl]amino}piperidine-1-carboxylic acid tert-butyl ester.

### Stage 6: Preparation of 4-(2,6-dichlorobenzoylamino)-1H-pyrazole-3-carboxylic acid piperidin-4-ylamide methanesulphonate

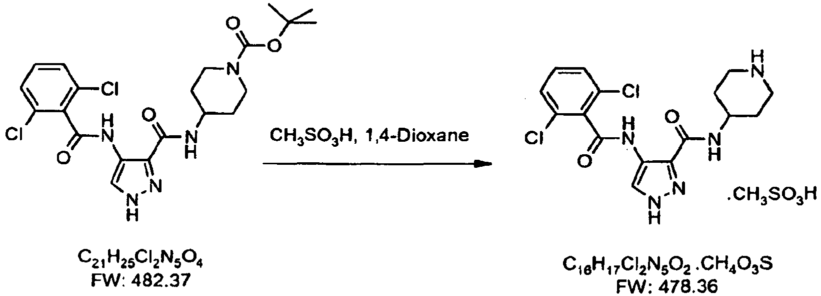

Stage 5 product (1.0 wt) and 1,4-dioxane (30.0 vol) were charged to a suitably sized flange flask equipped with a mechanical stirrer, dropping funnel and thermometer. The contents were stirred under nitrogen and heated to between 80 and 90 °C. Methanesulphonic acid (0.54 vol) was added over 30 to 60 minutes and the contents were then heated to 95 to 105 °C and stirred in this temperature range until the reaction was judged complete by ¹H NMR. Once complete, the contents were cooled to between 20 and 30 °C and the resultant precipitate collected by filtration. The filter-cake was washed with 2-propanol (2x 2.0 vol) and pulled dry on the filter for 3 to 24 hours to give crude 4-(2,6-dichlorobenzoylamino)-1H-pyrazole-3-carboxylic acid piperidin-4-ylamide methanesulphonate as a free-flowing off-white solid (80.0 to 120.0 %w/w, uncorrected for impurities or solutes).

Several batches of Stage 5 product were processed in this way and the details of the quantities of starting material and product for each batch are set out in Table 1 below.

**Table 1 - Yields from the deprotection step - Stage 6**

| Batch | Input (g) of (4-{[4-(2,6-Dichloro-benzoylamino)-1H-pyrazole-3-carbonyl]amino}-piperidine-1-carboxylic acid tert-butyl ester) | Output (g) of [4-(2,6-Dichlorobenzoyl-amino)-1H-pyrazole-3-carboxylic acid piperidin-4-ylamide methanesulphonate] | Chemical purity (HPLC % area) |
|---|---|---|---|
| 1 | 590.0 | 579.6 | 97.88 |
| | | 99.1%th, 98.2%w/w | |
| 2 | 521.0 | 532.7 | 98.09 |
| | | 103.1%th, 102.2%w/w | |
| 3 | 523.8 | 511.7 | 98.17 |
| | | 98.5%th, 97.7%w/w | |
| 4 | 518.4 | 596.3 | 98.24 |
| | | 116.0%th, 115.0%w/w | |
| 5 | 563.2 | 600.1 | 98.16 |
| | | 107.4%th, 106.6%w/w | |
| 6 | 563.1 | 565.2 | 98.49 |
| | | 101.2%th, 100.4%w/w | |
| 7 | 560.4 | 553.9 | 98.70 |
| | | 99.7%th, 98.8%w/w | |
| 8 | 569.7 | 560.6 | 98.41 |
| | | 99.2%th, 98.4%w/w | |

### Stage 6a: Recrystallisation of 4-(2,6-dichlorobenzoylamino)-1H-pyrazole-3-carboxylic acid piperidin-4-ylamide methanesulphonate

The product of Stage 6 was recrystallised to ensure that any residual levels of Boc-protected product of Stage 5 were no greater than 0.25%. Four batches of Stage 6 product were recrystallised using the following protocol.

Crude Stage 6 product and 2-propanol (10.0 vol) were charged to a suitably sized flask equipped with a mechanical stirrer, dropping funnel and thermometer. The contents were stirred under nitrogen and heated to between 75 and 85 °C. Water (up to 2.5 vol) was then charged to the contents until a clear solution was obtained. The contents were then cooled to between 40 and 60 °C and concentrated under vacuum at 40 to 50 °C until the reaction volume was reduced by approximately 50%. 2-Propanol (3.0 vol) was charged to the flask and the contents were concentrated at 40 to 50 °C until approximately 3.0 vol of solvent was removed. This process was then repeated twice more with 2-propanol (2x 3.0 vol) and the water content was checked. The resultant slurry was then cooled to between 0 and 5 °C and stirred at this temperature for 1 to 2 hours. The contents were filtered, the filter-cake was washed with 2-propanol (2x 1.0 vol) and then pulled dry on the filter for up to 24 hours. The solid was transferred to drying trays and dried under vacuum at 45 to 50 °C to constant weight to give 4-(2,6-dichlorobenzoylamino)-1H-pyrazole-3-carboxylic acid piperidin-4-ylamide methanesulphonate as an off-white solid (60.0 to 100.0% w/w).

The recrystallisation yields for the four batches ranged between 85.6% and 90.4% and the purities of the recrystallised product ranged from 99.29% to 99.39%. A second recrystallisation increased the purity still further.

The 4-(2,6-dichlorobenzoylamino)-1H-pyrazole-3-carboxylic acid piperidin-4-ylamide methanesulphonate produced by this route had a melting point (by DSC) of 379.8 °C.

### Removal of residual Boc-protected products of Stage 5

In some cases, when the methanesulphonate salt was dissolved in acetate buffer, a fine precipitate consisting of residual traces of the Boc-protected free base was observed. Several techniques may be used for removing or preventing the formation of the precipitate, as set out below.

### (a) Filtration

A mixture of the methanesulphonate salt in 200 mM acetate buffer was drawn from a vial into a 20 mL single-use syringe using a sterile needle, and a clinical grade 0.2 µm filter (a Sartorius Minisart sterile single use filter unit) was then attached to the syringe. The plunger of the syringe was slowly depressed and the filtrate collected in a clean, clear glass vial. The content of the vial was a clear, colourless solution of the methanesulphonate salt free of particulate matter.

### (b) Heating in aqueous acid

A mixture of the methanesulphonate salt and methanesulphonic acid (0.4 eq.) in water (10 vol) was heated at 100 °C for 4 hours, and then cooled to 60 °C. Analysis by TLC indicates that the methanesulphonate salt was present as a single component. 2-Propanol (10 vol) was added and the mixture cooled to 40 °C. The mixture was reduced *in vacuo* to approximately 10 volumes, then a further portion of 2-propanol added (10 vol) and the mixture again reduced to 10 volumes. This cycle was repeated a further three times. The mixture was cooled in an ice-bath and the solid formed collected by filtration, washed with 2-propanol (5 vol) and dried *in vacuo* to give the methanesulphonate salt as a white to off-white solid.

### (c) Organic-aqueous Extractions

A mixture of the methanesulphonate salt and methanesulphonic acid (0.4 eq.) in water (10 vol) was heated at 100 °C for 3 hours, and then cooled to ambient temperature. To this mixture was added THF-heptane (9:1, 10 vol) and the resultant mixture stirred vigorously to give a solution. The layers were separated and the aqueous phase washed with THF-heptane (9:1, 2 x 10 vol) then ethyl acetate (2 x 10 vol). To the aqueous phase was added 2-propanol (10 vol) and the solution was reduced *in vacuo* to approximately 5 volumes, then a further portion of 2-propanol added (10 vol) and the mixture again reduced to 5 volumes. This cycle was repeated a further three times. The solid formed was collected by filtration, washed with 2-propanol (5 vol) and dried *in vacuo* to give the methanesulphonate salt as a white to off-white solid.

### (d) Chromatography

The use of chromatographic techniques may provide a route for removing non-polar impurities from the methanesulphonate salt. It is envisaged that the use of reverse-phase methods will be particularly useful.

### BIOLOGICAL ACTIVITY

The biological activities of the compounds of formula (II) as inhibitors of CDK kinases, GSK-3 kinase and as inhibitors of cell growth are demonstrated by the examples set out below.

### EXAMPLE 248

### Measurement of CDK2 Kinase Inhibitory Activity (IC₅₀)

Compounds of the invention were tested for kinase inhibitory activity using either the following protocol or the activated CDK2/cyclin A kinase protocol described in Example 250.

1.7 µl of active CDK2/CyclinA (Upstate Biotechnology, 10U/µl) is diluted in assay buffer (250µl of 10X strength assay buffer (200mM MOPS pH 7.2, 250mM β-glycerophosphate, 50mM EDTA, 150mM MgCl₂). 11.27 µl 10mM ATP, 2.5 µl 1M DTT, 25 µl 100mM sodium orthovanadate, 708.53 µl H₂O), and 10 µl mixed with 10 µl of histone substrate mix (60 µl bovine histone H1 (Upstate Biotechnology, 5 mg/ml), 940 µl H₂O, 35 *Ci γ³³P-ATP) and added to 96 well plates along with 5 µl of various dilutions of the test compound in DMSO (up to 2.5%). The reaction is allowed to proceed for 5 hours before being stopped with an excess of ortho-phosphoric acid (30 µl at 2%).

γ³³P-ATP which remains unincorporated into the histone H1 is separated from phosphorylated histone H1 on a Millipore MAPH filter plate. The wells of the MAPH plate are wetted with 0.5% orthophosphoric acid, and then the results of the reaction are filtered with a Millipore vacuum filtration unit through the wells. Following filtration, the residue is washed twice with 200 µl of 0.5% orthophosphoric acid. Once the filters have dried, 25 µl of Microscint 20 scintillant is added, and then counted on a Packard Topcount for 30 seconds.

The % inhibition of the CDK2 activity is calculated and plotted in order to determine the concentration of test compound required to inhibit 50% of the CDK2 activity (IC₅₀).

By means of the protocol set out above, it was found that the compounds of Examples 2C to 87, 89-92, 94, 96-101, 104-105, 165, 166, 224, 225, 227, 229, 231, 233, 234 and 236 each have IC₅₀ values less than 20 µM or provide at least 50% inhibition of the CDK2 activity at a concentration of 10 µM. The compounds of Examples 88, 93, 226, 228, 230 and 235 each have IC₅₀ values less than 750 µM.

### EXAMPLE 249

### CDK Selectivity Assays

Compounds of the invention are tested for kinase inhibitory activity against a number of different kinases using the general protocol described in Example 247, but modified as set out below.

Kinases are diluted to a 10x working stock in 20mM MOPS pH 7.0, 1 mM EDTA, 0.1% γ-mercaptoethanol, 0.01% Brij-35, 5% glycerol, 1 mg/ml BSA. One unit equals the incorporation of 1 nmol of phosphate per minute into 0.1 mg/ml histone H1, or CDK7 substrate peptide at 30 °C with a final ATP concentration of 100 uM.

The substrate for all the CDK assays (except CDK7) is histone H1, diluted to 10X working stock in 20 mM MOPS pH 7.4 prior to use. The substrate for CDK7 is a specific peptide obtained from Upstate diluted to 10X working stock in deionised water.

### Assay Procedure for CDK1/cyclinB, CDK2/cyclinA, CDK2/cyclinE, CDK3/cyclinE, CDK5/p35, CDK6/cyclinD3:

In a final reaction volume of 25 µl, the enzyme (5-10 mU) is incubated with 8 mM MOPS pH 7.0, 0.2 mM EDTA, 0.1 mg/ml histone H1, 10 mM MgAcetate and [γ-³³P-ATP] (specific activity approx 500 cpm/pmol, concentration as required). The reaction is initiated by the addition of Mg²⁺ [γ-³³P-ATP]. After incubation for 40 minutes at room temperature the reaction is stopped by the addition of 5 µl of a 3% phosphoric acid solution. 10 ml of the reaction is spotted onto a P30 filter mat and washed 3 times for 5 minutes in 75 mM phosphoric acid and once in methanol prior to drying and counting.

In the CDK3/cyclinE assay, the compound of Example 150 had an IC₅₀ of less than 20 µM.

In the CDK5/p35 assay, the compounds of Examples 41 and 150 had an IC₅₀ of less than 20 µM.

In the CDK6/cyclinD3 assay, the compound of Example 150 had an IC₅₀ of less than 20 µM.

### Assay procedure for CDK7/cyclinH/MAT1

In a final reaction volume of 25 µl, the enzyme (5-10mU) is incubated with 8 mM MOPS pH 7.0, 0.2 mM EDTA, 500 µM peptide, 10 mM MgAcetate and [γ-³³P-ATP] (specific activity approx 500 cpm/pmol, concentration as required). The reaction is initiated by the addition of Mg²+[γ-³³P-ATP]. After incubation for 40 minutes at room temperature the reaction is stopped by the addition of 5 µl of a 3% phosphoric acid solution. 10 ml of the reaction is spotted onto a P30 filtermat and washed 3 times for 5 minutes in 75 mM phosphoric acid and once in methanol prior to drying and counting.

### EXAMPLE 250

### A. Measurement of Activated CDK2/CyclinA Kinase Inhibitory Activity Assay (IC₅₀)

Compounds of the invention were tested for kinase inhibitory activity using the following protocol.

Activated CDK2/CyclinA (Brown et al, Nat. Cell Biol., 1, pp438-443, 1999; Lowe, E.D., et al Biochemistry, 41, pp15625-15634, 2002) is diluted to 125 pM in 2.5X strength assay buffer (50 mM MOPS pH 7.2, 62.5 mM β-glycerophosphate, 12.5 mM EDTA, 37.5 mM MgCl₂, 112.5 mM ATP, 2.5 mM DTT, 2.5 mM sodium orthovanadate, 0.25 mg/ml bovine serum albumin), and 10 µl mixed with 10 µl of histone substrate mix (60 µl bovine histone H1 (Upstate Biotechnology, 5 mg/ml), 940 µl H₂O, 35 pCi γ³³P-ATP) and added to 96 well plates along with 5 µl of various dilutions of the test compound in DMSO (up to 2.5%). The reaction is allowed to proceed for 2 to 4 hours before being stopped with an excess of ortho-phosphoric acid (5 µl at 2%).

γ³³P-ATP which remains unincorporated into the histone H1 is separated from phosphorylated histone H1 on a Millipore MAPH filter plate. The wells of the MAPH plate are wetted with 0.5% orthophosphoric acid, and then the results of the reaction are filtered with a Millipore vacuum filtration unit through the wells. Following filtration, the residue is washed twice with 200 µl of 0.5% orthophosphoric acid. Once the filters have dried, 20 µl of Microscint 20 scintillant is added, and then counted on a Packard Topcount for 30 seconds.

The % inhibition of the CDK2 activity is calculated and plotted in order to determine the concentration of test compound required to inhibit 50% of the CDK2 activity (IC₅₀).

By means of the foregoing protocol, it was found that the compounds of Examples 95, 96, 99-104, 106-121, 123-125, 130-137, 139, 142-145, 147-150, 152-156, 158-160, 162-164, 167-173, 177-179, 181-182, 184-190, 194, 196-204, 208-213 and 215 have IC₅₀ values less than 20 µM. The compounds of Examples 122, 126-129, 140, 141, 146, 157 and 161 each have IC₅₀ values less than than 750 µM and most have IC₅₀ values of less than 100 µM.

### B. CDK1/CyclinB Assay.

CDK1/CyclinB assay is identical to the CDK2/CyclinA above except that CDK1/CyclinB (Upstate Discovery) is used and the enzyme is diluted to 6.25 nM.

In the CDK1 assay carried out as described above or by means of the protocol set out in Example 240, the compounds of Examples 2C, 41, 48, 53, 64, 65, 66, 73, 76, 77, 91, 95, 102, 106, 117, 123, 125, 133, 137, 142, 150, 152, 154, 167, 186, 187, 189, 190, 193, 194, 196, 199, 202-204, 207, 208-213, 215 AND 218-223 were found to have IC₅₀ values less than 20 µM, and the compounds of Examples 188 and 206, were found to have IC₅₀ values less than 100 µM.

### EXAMPLE 251

### Assay Procedure for CDK4

Assays for CDK4 inhibitory activity were carried out by Proqinase GmbH, Freiburg, Germany using their proprietary 33PanQinase^{®} Activity Assay. The assays were performed in 96 well FlashPlates™ (PerkinElmer). In each case, the reaction cocktail (50 µl final volume) is composed of; 20 µl assay buffer (final composition 60 mM HEPES-NaOH, pH 7.5, 3 mM MgCl₂, 3 µM Na-orthovanadate, 1.2mM DTT, 50 µg/ml PEG_{2000,} 5 µl ATP solution (final concentration 1 µM [γ-33P]-ATP (approx 5x10⁵ cpm per well)), 5 µl test compound (in 10% DMSO), 10 µl substrate/ 10 µl enzyme solution (premixed). The final amounts of enzyme and substrate were as below.

| Kinase | Kinase ng/50 µl | Substrate | Substrate ng/ 50µl |
|---|---|---|---|
| CDK4/CycD1 | 50 | Poly (Ala, Glu, Lys, Tyr) 6:2:5:1 | 500 |

The reaction cocktail was incubated at 30 °C for 80 minutes. The reaction was stopped with 50 µl of 2 % H₃PO₄, plates were aspirated and washed twice with 200 µl 0.9% NaCl. Incorporation of ³³P was determined with a microplate scintillation counter. Background values were subtracted from the data before calculating the residual activities for each well. IC₅₀s were calculated using Prism 3.03.

The compound of Example 150 has an IC50 of less than 5 µM in this assay.

### EXAMPLE 252

### Measurement of inhibitory activity against Glycogen Synthase Kinase-3 (GSK-3)

The activities of the compounds of the invention as inhibitors of GSK-3 were determined using either Protocol A or Protocol B below.

### Protocol A

GSK3- β (Upstate Discovery) is diluted to 7.5 nM in 25 mM MOPS, pH 7.00, 25 mg/ml BSA, 0.0025% Brij-35^{RTM}, 1.25% glycerol, 0.5 mM EDTA, 25 mM MgCl₂, 0.025% β-mercaptoethanol, 37.5 mM ATP and 10 µl mixed with 10 µl of substrate mix. The substrate mix is 12.5 µM phospho-glycogen synthase peptide-2 (Upstate Discovery) in 1 ml of water with 35 µCi γ³³P-ATP. Enzyme and substrate are added to 96 well plates along with 5 µl of various dilutions of the test compound in DMSO (up to 2.5%). The reaction is allowed to proceed for 3 hours before being stopped with an excess of ortho-phosphoric acid (5 µl at 2%). The filtration procedure is as for Activated CDK2/CyclinA assay above.

### Protocol B

GSK3β (human) is diluted to a 10x working stock in 50mM Tris pH 7.5, 0.1 mM EGTA, 0.1 mM sodium vanadate, 0.1% β-mercaptoethanol, 1 mg/ml BSA. One unit equals the incorporation of 1 nmol of phosphate per minute phospho-glycogen synthase peptide 2 per minute.

In a final reaction volume of 25µl, GSK3β (5-10 mU) is incubated with 8mM MOPS 7.0, 0.2mM EDTA, 20µM YRRAAVPPSPSLSRHSSPHQS(p)EDEEE (phospho GS2 peptide), 10mM MgAcetate and [γ-³³P-ATP] (specific activity approx 500cpm/pmol, concentration as required). The reaction is initiated by the addition of Mg²+[γ-³³P-ATP]. After incubation for 40 minutes at room temperature the reaction is stopped by the addition of 5µl of a 3% phosphoric acid solution. 10µl of the reaction is spotted onto a P30 filter mat and washed 3 times for 5 minutes in 50mM phosphoric acid and once in methanol prior to drying and counting.

From the results of the GSK3-B assays carried out using either of the two procols set out above, it was found that the compounds of Examples 2C, 26, 48, 53, 65, 76, 77, 84, 86, 95, 102, 106, 119, 122, 123, 126, 127, 128, 129, 131, 134, 135, 138, 140, 141, 142, 143, 144, 145, 146, 147, 149, 150 and 151 each have IC₅₀ values of less than 10 µM.

### EXAMPLE 253

### Anti-proliferative Activity

The anti-proliferative activities of the combinations of the invention, as well as the indidual components of the combinations, are determined by measuring the ability of the compounds to inhibition of cell growth in a number of cell lines. Inhibition of cell growth is measured using the Alamar Blue assay (Nociari, M. M, Shalev, A., Benias, P., Russo, C. Journal of Immunological Methods 1998, 213, 157-167). The method is based on the ability of viable cells to reduce resazurin to its fluorescent product resorufin. For each proliferation assay cells are plated onto 96 well plates and allowed to recover for 16 hours prior to the addition of inhibitor compounds for a further 72 hours. At the end of the incubation period 10% (v/v) Alamar Blue is added and incubated for a further 6 hours prior to determination of fluorescent product at 535nM ex / 590nM em. In the case of the non-proliferating cell assay cells are maintained at confluence for 96 hour prior to the addition of inhibitor compounds for a further 72 hours. The number of viable cells is determined by Alamar Blue assay as before. All cell lines are obtained from ECACC (European Collection of cell Cultures).

### HCT-116 cell line

In assays against the human colon carcinoma cell line HCT 116 (ECACC No. 91091005), the compounds of Examples 10, 25-27, 41, 44, 46, 48, 50, 52, 53, 60, 62, 64-67, 69, 73-77, 79, 80, 83A, 86, 90-93, 95-98, 100-104, 106, 107, 109-121, 123-125, 131-134, 136-143, 147-155, 158, 159, 162-164, 166, 167, 178, 179, 185-190, 192-205,207-215 and 218-223 have IC₅₀ values of less than 20 µM and the compounds of Examples 2C, 3, 29, 38, 39, 49, 51, 85, 89, 99, 108, 135, 160, 182, 183, 206 and 216 have IC₅₀ values of less than 100 µM.

### EXAMPLE 254

The effect of the compound 4-(2,6-dichloro-benzoylamino)-1H-pyrazole-3-carboxylic acid piperidin-4-ylamide ("Compound I") in combination with any ancillary agent (Compound II) can be assessed using the following technique:

### 1. IC₅₀ Shift Assay

Human colon carcinoma cell line HT29 (ECACC No. 91072201) cells were seeded onto 96-well tissue culture plates at a concentration of 5x10³ cells/well. Cells were allowed to recover overnight prior to addition of compound(s) or vehicle control (0.2% DMSO) as follows;

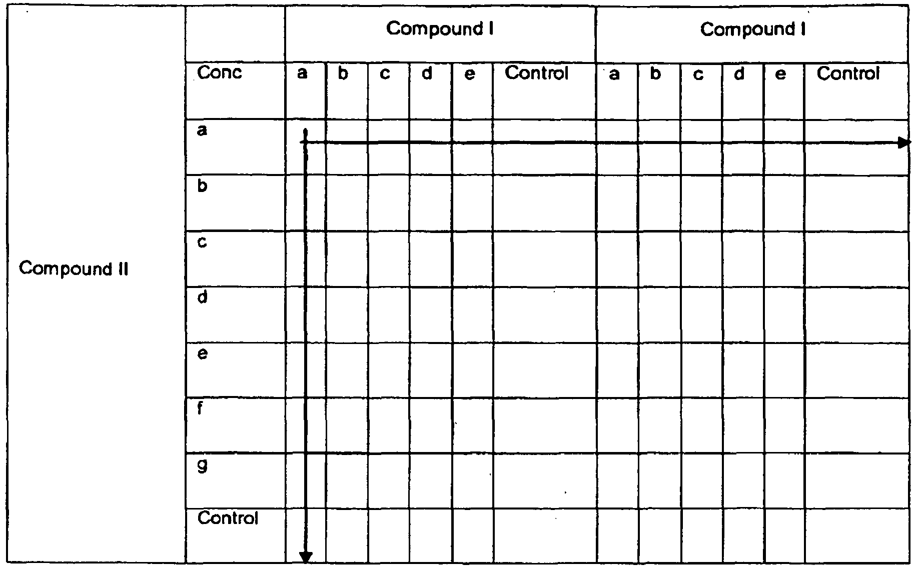

Compounds were added according to one of the following schedules;
a) Concurrent for 72 hours.
b) Compound I for 24 hours followed by Compound II for 48 hours.
c) Compound II for 24 hours followed by Compound I for 48 hours.

Following a total of 72 hours compound incubation, Alamar Blue^{™} was added to a final concentration of 10% (v/v) and incubated at 37 °C for 6 hours. Fluorescent product was quantified by reading at d535/25x (excitation) and d590/20m (emission) on a Fusion Reader (Perkin Elmer). The IC₅₀ for Compound II in the presence of varying doses of Compound I was determined. Synergy was determined when the IC₅₀ shifted down in the presence of sub-effective doses of Compound I. Additivity was determined when the response to Compound II and Compound I together resulted In an effect equivalent to the sum of the two compounds individually. Antagonistic effects were defined as those causing the IC₅₀ to shift upwards, i.e. those where the response to the two compounds was less than the sum of the effect of the two compounds individually.

### PHARMACEUTICAL FORMULATIONS

### EXAMPLE 255

### i) Lyophilised formulation I

Aliquots of formulated compound of formula (II) are put into 50 mL vials and lyophilized. During lyophilisation, the compositions are frozen using a one-step freezing protocol at (-45 °C). The temperature is raised to -10 °C for annealing, then lowered to freezing at -45 °C, followed by primary drying at +25 °C for approximately 3400 minutes, followed by a secondary drying with increased steps if temperature to 50 °C. The pressure during primary and secondary drying is set at 80 millitor.

### ii) Injectable formulatlon II

A formulation for i.v. delivery by injection or infusion can be prepared by dissolving the compound of formula (II) (e.g. in a salt form) in water at 20 mg/ml. The vial is then sealed and sterilised by autoclaving.

### iii) Injectable formulation III

A formulation for i.v. delivery by injection or infusion can be prepared by dissolving the compound of formula (II) (e.g. in a salt form) in water containing a buffer (e.g. 0.2 M acetate pH 4.6) at 20mg/ml. The vial is then sealed and sterilised by autoclaving.

### iv) Injectable Formulation IV

A parenteral composition for administration by injection can be prepared by dissolving a compound of the formula (II) (e.g. in a salt form) in water containing 10% propylene glycol to give a concentration of active compound of 1.5 % by weight. The solution is then sterilised by filtration, filled into an ampoule and sealed.

### (v) Injectable Formulation V

A parenteral composition for injection is prepared by dissolving in water a compound of the formula (II) (e.g. in salt form) (2 mg/ml) and mannitol (50 mg/ml), sterile filtering the solution and filling into sealable 1 ml vials or ampoules.

### (vi) Subcutaneous Injection Formulation VI

A composition for sub-cutaneous administration is prepared by mixing a compound of the formula (II) with pharmaceutical grade corn oil to give a concentration of 5 mg/ml. The composition Is sterilised and filled into a suitable container.

### (vii) Tablet Formulation

A tablet composition containing a compound of the formulae (I°) or (I) or an acid addition salt thereof as defined herein is prepared by mixing 50mg of the compound or its salt with 197mg of lactose (BP) as diluent, and 3mg magnesium stearate as a lubricant and compressing to form a tablet in known manner.

### (viii) Capsule Formulation

A capsule formulation is prepared by mixing 100mg of a compound of the formulae (I°) or (I) or an acid addition salt thereof as defined herein with 100mg lactose and filling the resulting mixture into standard opaque hard gelatin capsules.

### (ix) Lyophilised formulation

Aliquots of formulated compound of formulae (I°) or (I) or an acid addition salt thereof as defined herein are put into 50 mL vials and lyophilized. During lyophilisation, the compositions are frozen using a one-step freezing protocol at (-45 °C). The temperature is raised to -10 °C for annealing, then lowered to freezing at -45 °C, followed by primary drying at +25 °C for approximately 3400 minutes, followed by a secondary drying with increased steps if temperature to 50 °C. The pressure during primary and secondary drying is set at 80 millitor.

### (x) Concentrate for use in i.v. administration

An aqueous buffered solution is prepared by dissolving 4-(2,6-dichlorobenzoylamino)-1*H*-pyrazole-3-carboxylic acid piperidin-4-ylamide methanesulphonate at a concentration of 20 mg/ml in a 0.2M sodium acetate/acetic acid buffer at a pH of 4.6.

The buffered solution is filled, with filtration to remove particulate matter, into a container (such as class 1 glass vials) which is then sealed (e.g. by means of a Florotec stopper) and secured (e.g. with an aluminium crimp). if the compound and formulation are sufficiently stable, the formulation is sterilised by autoclaving at 121 °C for a suitable period of time. If the formulation is not stable to autoclaving, it can be sterilised using a suitable filter and filled under sterile conditions into sterile vials. For intravenous administration, the solution can be dosed as is, or can be injected into an infusion bag (containing a pharmaceutically acceptable excipient, such as 0.9% saline or 5% dextrose), before administration.

### EXAMPLE 256

### Determination of the crystal structure of 4-(2,6-dichlorobenzoylamino)-1H-pyrazole-3-carboxylic acid piperidin-4-ylamide methanesulphonate by X-ray diffraction

The compound 4-(2,6-dichlorobenzoylamino)-1*H*-pyrazole-3-carboxylic acid piperidin-4-ylamide methanesulphonate was prepared as described in Example 1. The crystal used for the diffraction experiment was a colourless plate with dimensions 0.05 x 0.08 x 0.14 mm³ obtained by precipitation from a water solution by 2-propanol. Crystallographic data were collected at 93 K using CuKα radiation (λ = 1.5418 Å) from a Rigaku rotating anode RU3HR, Osmic blue confocal optics and a Rigaku Jupiter CCD detector. Images were collected in two ω scans at 2θ=15 and 90° with a detector to crystal distance of 67 mm. Data collection was controlled by CrystalClear software and images were processed and scaled by Dtrek. Due to a high absorption coefficient (µ=4.01 mm⁻¹) data had to be corrected using 4^{th} order Fourier absorption correction. It was found that the crystals belong to an orthorhombic space group P*bca* (# 61) with crystal lattice parameters at 93 K *a*=8.90(10), *b*=12.44(10), c=38.49(4) A, α = β = γ = 90°. The numbers in brackets represents the deviation (s.u., standard uncertainty).

The crystals described above and the crystal structure form a further aspect of the invention.

The crystal structure was solved using direct methods implemented in SHELXS-97. Intensity data for a total of 2710 unique reflections in a resolution range from 20-0.9 Å (2.3<θ<58.87) were used in the refinement of 271 crystallographic parameters by SHELXL-97. Final statistical parameters were: wR2=0.2115 (all data), R1=0.0869 (data with I>2σ(I)) and goodness of fit S=1.264.

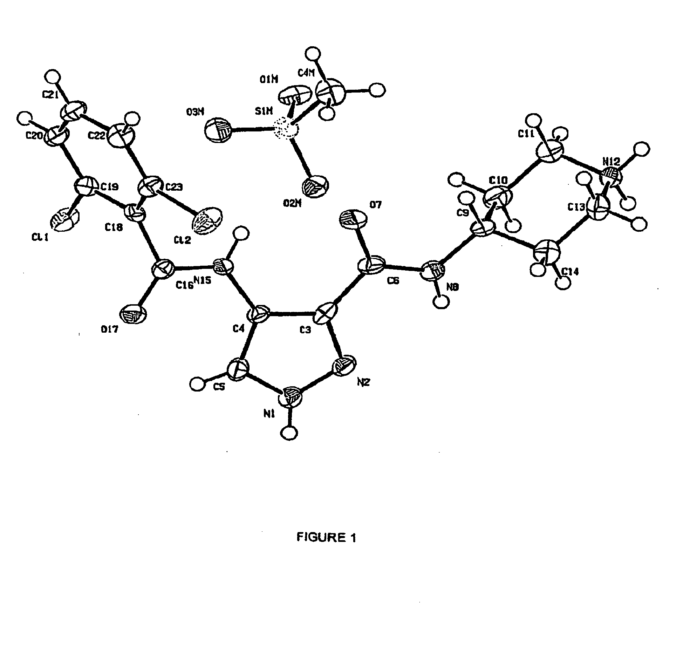

One molecule of protonated free base and one mesylate anion were found in the asymmetric unit. The elemental composition of the asymmetric unit was C₁₇H₂₁Cl₂N₅O₅S and the calculated density of the crystals is 1.49 Mg/m³. Hydrogen atoms were generated on geometrical grounds while the location of heteroatom bound hydrogen atoms was confirmed by inspection of Fo-Fc difference maps. The positional and thermal parameters of hydrogen atoms were constricted to ride on corresponding non-hydrogen atoms. The thermal motion of non-hydrogen atoms was modelled by anisotropical thermal factors (see Figure 1).

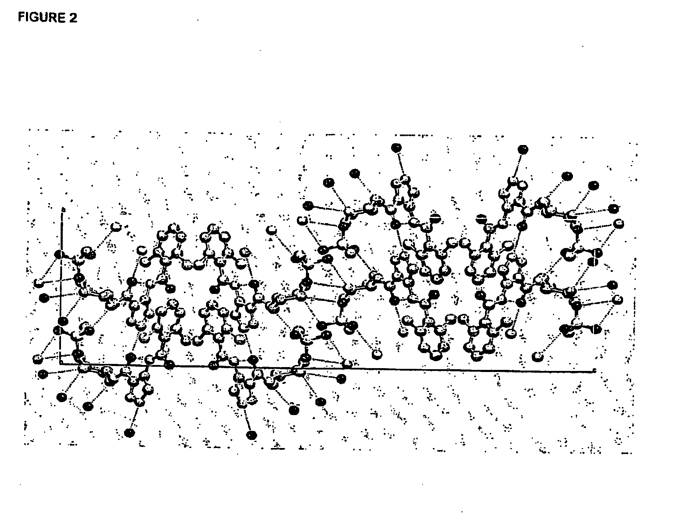

The crystal structure contains one intramolecular (N15H...O7 2.690 A) and five intermolecular hydrogen bonds (see packing figure Figure 2). Three of them link the protonated piperidine nitrogen with two mesylate anions. The first mesylate anion is linked through a single H-bond N12H12A...02M 2.771 Å. while the second is involved in a bifurcated H-bond with interactions N12H12B...01M 2.864 A and N12H12B...O2M 3.057 A. The remaining mesylate oxygen O3M is involved in a hydrogen bond N8H8...O3M 2.928 A. Neighbouring protonated free base molecules are linked together by a H-bond N15H15...O7 2.876 A, as well as by relatively long contact N15H15...N2 3.562 A and stacking of phenyl and pyrazole rings. These interactions are propagated infinitely along the b axis. Crystal packing contains 2D layers (in the ab plane) of mesylate anions sandwiched by an extensive network of charged H-bonds with two layers of protonated free base cations. The compact 2D sandwich layers are joined together along the c axis by stacking of phenyl rings and involving chlorine...phenyl interaction with CI2...C18 3.341 Å.

A graphical representation of the structure generated by the X-ray diffraction study is provided in Figure 2.

The coordinates for the atoms making up the structure of the 4-(2,6-dichlorobenzoylamino)-1*H*-pyrazole-3-carboxylic acid piperidin-4-ylamide methanesulphonate are as set out in Table 2.

### Table 2

space group: Pbca
unit cell at 93K with a, b & c having 5% s.u.:
a= 8.9
b=12.4
c=38.5
alpha=beta=gamma=90

Coordinates in cif format:
loop_
_atom_site_label
_atom_site_type_symbol
_atom_site_fract_x
_atom_site_fract_y
_atom_site_fract_z
_atom_site_U_iso_or_equiv
_atom_site_adp_type
_atom_site_occupancy
_atom_site_symmetry_multiplicity
_atom_site_calc_flag
_atom_site_refinement_flags
_atom_site_disorder_assembly
_atom_aite_disorder_group
S1M S 0.13517(17) 0.18539(13) 0.03193(5) 0.0286(5) Uani 1 1 d ...
O1M O 0.1193(5) 0.2208(3) -0.00409(14) 0.0326(13) Uani 1 1 d ...
O2M O 0.1551(5) 0.0681(3) 0.03330(13) 0.0331(13) Uani 1 1 d ...
O3M O 0.0151(5) 0.2217(4) 0.05453(14) 0.0368(13) Uani 1 1 d ...
C4M C 0.3036(8) 0.2420(6) 0.0475(2) 0.0355(19) Uani 1 1 d ...
H4M1 H 0.3855 0.2197 0.0329 0.053 Uiso 1 1 calc R ..
H4M2 H 0.3212 0.2181 0.0708 0.053 Uiso 1 1 calc R ..
H4M3 H 0.2959 0.3189 0.0471 0.053 Uiso 1 1 calc R ..
C11 C1 0.26158(17) 0.18137(12) 0.34133(5) 0.0325(5) Uani 1 1 d . . .
C12 C1 0.75698(19) 0.16766(13) 0.26161(5) 0.0366(6) Uani 1 1 d . . .
N1 N 0.6277(6) -0.2419(4) 0.34903(16) 0.0276(14) Uani 1 1 d . . .
H1 H 0.5932 -0.3064 0.3484 0.033 Uiso 1 1 calc R ..
N2 N 0.7505(5) -0.2150(4) 0.36663(16) 0.0286(15) Uani 1 1 d . . .
C3 C 0.7635(7) -0.1082(5) 0.36163(19) 0.0265(17) Uani 1 1 d . . .
C4 C 0.6453(7) -0.0708(5) 0.34039(18) 0.0211(16) Uani 1 1 d . . .
C5 C 0.5616(7) -0.1594(5) 0.3322(2) 0.0277(18) Uani 1 1 d . . .
H5 H 0.4770 -0.1623 0.3181 0.033 Uiso 1 1 calc R ..
C6 C 0.8878(7) -0.0454(5) 0.3760(2) 0.0269(17) Uani 1 1 d . . .
07 O 0.9037(5) 0.0506(3) 0.36722(14) 0.0368(13) Uani 1 1 d . . .
N8 N 0.9821(6) -0.0939(4) 0.39821(15) 0.0267(14) Uani 1 1 d . . .
H8 H 0.9626 -0.1584 0.4048 0.032 Uiso 1 1 calc R ..
C9 C 1.1147(7) -0.0417(5) 0.41139(19) 0.0253(17) Uani 1 1 d . . .
H9 H 1.1272 0.0261 0.3987 0.030 Uiso 1 1 calc R ..
C10 C 1.1019(8) -0.0148(5) 0.4502(2) 0.0330(18) Uani 1 1 d . . .
H10A H 1.0156 0.0315 0.4540 0.040 Uiso 1 1 calc R . .
H10B H 1.0866 -0.0804 0.4633 0.040 Uiso 1 1 calc R ..
C11 C 1.2429(7) 0.0412(5) 0.4630(2) 0.0349(19) Uani 1 1 d . . .
H11A H 1.2533 0.1102 0.4515 0.042 Uiso 1 1 calc R ..
H11B H 1.2355 0.0538 0.4878 0.042 Uiso 1 1 calc R ..
N12 N 1.3784(6) -0.0279(4) 0.45532(16) 0.0258(14) Uani 1 1 d . . .
H12A H 1.4618 0.0069 0.4623 0.031 Uiso 1 1 calc R ..
H12B H 1.3716 -0.0892 0.4676 0.031 Uiso 1 1 calc R ..
C13 C 1.3929(7) -0.0546(6) 0.4181(2) 0.0314(18) Uani 1 1 d . . .
H13A H 1.4790 -0.1013 0.4147 0.038 Uiso 1 1 calc R ..
H13B H 1.4098 0.0107 0.4049 0.038 Uiso 1 1 calc R ..
C14 C 1.2538(7) -0.1097(6) 0.4049(2) 0.0356(19) Uani 1 1 d . . .
H14A H 1.2425 -0.1785 0.4165 0.043 Uiso 1 1 calc R ..
H14B H 1.2639 -0.1231 0.3802 0.043 Uiso 1 1 calc R ..
N15 N 0.6215(5) 0.0371(4) 0.33108(16) 0.0256(14) Uani 1 1 d . . .
H15 H 0.6768 0.0852 0.3408 0.031 Uiso 1 1 calc R ..
C16 C 0.5183(7) 0.0697(5) 0.30805(18) 0.0213(15) Uani 1 1 d . . .
017 O 0.4336(5) 0.0082(3) 0.29260(13) 0.0309(12) Uani 1 1 d . . .
C18 C 0.5120(6) 0.1890(5) 0.30170(17) 0.0195(15) Uani 1 1 d . . .
C19 C 0.3923(7) 0.2486(5) 0.31620(19) 0.0252(16) Uani 1 1 d . . .
C20 C 0.3785(7) 0.3569(5) 0.30904(19) 0.0267(17) Uani 1 1 d . . .
H20 H 0.2991 0.3957 0.3185 0.032 Uiso 1 1 calc R ..
C21 C 0.4814(7) 0.4078(5) 0.28805(19) 0.0270(17) Uani 1 1 d . . .
H21 H 0.4708 0.4808 0.2834 0.032 Uiso 1 1 calc R ..
C22 C 0.6005(7) 0.3518(5) 0.27375(19) 0.0294(18) Uani 1 1 d . . .
H22 H 0.6702 0.3865 0.2597 0.035 Uiso 1 1 calc R ..
C23 C 0.6142(7) 0.2425(5) 0.2807(2) 0.0286(17) Uani 1 1 d . . .

### EXAMPLE 257

### Preparation of 4-(2,6-dichloro-benzoylamino)-1H-pyrazole-3-carboxylic acid piperidin-4-ylamide acetic acid salt

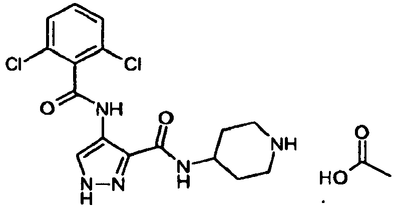

To a solution of 4-(2,6-dichloro-benzoylamino)-1H-pyrazole-3-carboxylic acid piperidin-4-ylamide hydrochloride salt (20.6 g, 50 mmol) in water (500 ml) stirring at ambient temperature was added sodium bicarbonate (4.5 g, 53.5 mmol). The mixture was stirred for 1 hour and the solid formed collected by filtration and dried *in vacuo* azeotroping with toluene (x 3) to give the corresponding free base of 4-(2,6-dichloro-benzoylamino)-1H-pyrazole-3-carboxylic acid piperidin-4-ylamide.

¹N NMR (400 MHz, DMSO-*d*₆) δ 10.20 (s, 1 H), 8.30 (s, 1H), 8.25 (d, 1 H), 7.60 - 7.50 (m, 3H), 3.70 (m, 1 H), 3.00 (d, 2H), 2.50 (m, 2H), 1.70 (d, 2H), 1.50 (m, 2H).

To a stirred suspension of 4-(2,6-dichloro-benzoylamino)-1H-pyrazole-3-carboxylic acid piperidin-4-ylamide (10.0 g, 26.2 mmol) in methanol (150 ml) was added glacial acetic acid (15 ml, 262 mmol) at ambient temperature. After 1 h, a clear solution was obtained which was reduced *in vacuo* azeotroping with toluene (x 2). The residue was then triturated with acetonitrile (2 x 100 ml) and the solid dried *in vacuo.* to give 4-(2,6-dichloro-benzoylamino)-1H-pyrazole-3-carboxylic acid piperidin-4-ylamide acetic acid salt (10.3 g) as a white solid.

¹H NMR (400 MHz, DMSO-*d*₆) δ 10.20 (s, 1 H), 8.40 (d, 1H), 8.35 (s, 1 H), 7.60 - 7.50 (m, 3H), 3.85 (m, 1H), 3.00 (d, 2H), 2.60 (t, 2H), 1.85 (s, 3H), 1.70 (d, 2H), 1.55 (m, 2H)

## Claims

1. A combination comprising an ancillary agent and a compound of the formula (II) : 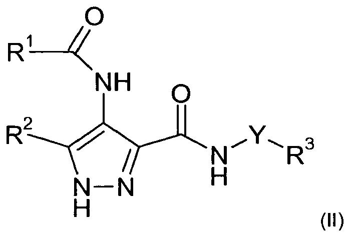 or salts or tautomers or N-oxides or solvates thereof;
wherein
the ancillary agent is selected from:
(a) a monoclonal antibody to a cell surface antigen; or
(b) an alkylating agent wherein said alkylating agent is selected from nitrogen mustards; nitrosoureas; bifunctional alkylating agents having a bis-alkanesulfonate group; and aziridine compounds; and wherein the alkylating agents are other than mitomycin C or cyclophosphamide: or
(c) an anticancer agent selected from COX-2 inhibitors, HDAC inhibitors, DNA methyltransferase (methylase) inhibitors and proteasome inhibitors; or
(d) a further CDK inhibitor; and
(e) an anti-androgen including an anti-oestrogen;
Y is a bond or an alkylene chain of 1, 2 or 3 carbon atoms in length;
R¹ is a carbocyclic or heterocyclic group having from 3 to 12 ring members, wherein the carbocyclic or heterocyclic group is unsubstituted or substituted by one or more substituent groups R¹⁰; or a C₁₋₈ hydrocarbyl group optionally substituted by one or more substituents selected from fluorine, hydroxy, C₁₋₄ hydrocarbyloxy, amino, mono- or di-C₁₋₄ hydrocarbylamino, and carbocyclic or heterocyclic groups having from 3 to 12 ring members wherein the carbocyclic or heterocyclic groups are unsubstituted or substituted by one or more substituent groups R¹⁰;
R² is hydrogen or methyl;
R³ is selected from non-aromatic carbocyclic and heterocyclic groups having from 3 to 12 ring members, wherein the carbocyclic or heterocyclic group is unsubstituted or substituted by one or more substituent groups R¹⁰; and
R¹⁰ is selected from halogen, hydroxy, trifluoromethyl, cyano, nitro, carboxy, amino, mono- or di-C₁₋₄ hydrocarbylamino, carbocyclic and heterocyclic groups having from 3 to 12 ring members; a group R^{a}-R^{b} wherein R^{a} is a bond, O, CO, X¹C(X²), C(X²)X¹, X¹C(X²)X¹, S, SO, SO₂, NR^{c}, SO₂NR^{c} or NR^{c}SO₂; and R^{b} is selected from hydrogen, carbocyclic and heterocyclic groups having from 3 to 12 ring members, and a C₁₋₈ hydrocarbyl group optionally substituted by one or more substituents selected from hydroxy, oxo, halogen, cyano, nitro, carboxy, amino, mono- or di-C₁₋₄ hydrocarbylamino, carbocyclic and heterocyclic groups having from 3 to 12 ring members;
R^{c} is selected from hydrogen and C₁₋₄ hydrocarbyl; and
X¹ is O, S or NR^{c} and X² is =O, =S or =NR^{c};
and provided that where the substituent group R¹⁰ comprises or includes a carbocyclic or heterocyclic group, the said carbocyclic or heterocyclic group may be unsubstituted or may itself be substituted with one or more further substituent groups R¹⁰ and wherein (a) such further substituent groups R¹⁰ include carbocyclic or heterocyclic groups, which are not themselves further substituted; or (b) the said further substituents do not include carbocyclic or heterocyclic groups but are otherwise selected from the groups listed above in the definition of R¹⁰.

2. A combination according to claim 1 wherein Y is a bond.

3. A combination according to claim 1 or claim 2 wherein the carbocyclic and heterocyclic groups are monocyclic.

4. A combination according to claim 3 wherein R¹ is selected from unsubstituted phenyl, 2-fluorophenyl, 2-hydroxyphenyl, 2-methoxyphenyl, 2-methylphenyl, 2-(2-(pyrrolidin-1-yl)ethoxy)-phenyl, 3-fluorophenyl, 3-methoxyphenyl, 2,6-difluorophenyl, 2-fluoro-6-hydroxyphenyl, 2-fluoro-3-methoxyphenyl, 2-fluoro-5-methoxyphenyl, 2-chloro-6-methoxyphenyl, 2-fluoro-6-methoxyphenyl, 2,6-dichlorophenyl and 2-chloro-6-fluorophenyl; and is optionally further selected from 5-fluoro-2-methoxyphenyl.

5. A combination according to claim 1 comprising an ancillary agent and a compound having
the formula (IV): 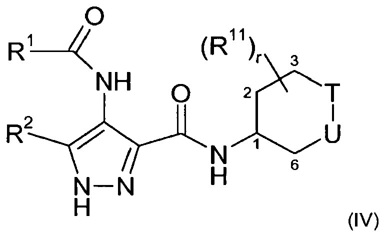 or salts or tautomers or N-oxides or solvates thereof;
wherein the ancillary agent and R¹ and R² are as defined in any one of claims 1 to 4;
an optional second bond may be present between carbon atoms numbered 1 and 2;
one of U and T is selected from CH₂, CHR¹³, CR¹¹R¹³, NR¹⁴, N(O)R¹⁵, O and S(O)ₜ; and the other of U and T is selected from NR¹⁴, O, CH₂, CHR¹¹, C(R¹¹)₂, and C=O; r is 0, 1, 2, 3 or 4; t is 0, 1 or 2;
R¹¹ is selected from hydrogen, halogen , C₁₋₃ alkyl and C₁₋₃ alkoxy ;
R¹³ is selected from hydrogen, NHR¹⁴, NOH, NOR¹⁴ and R^{a}-R^{b};
R¹⁴ is selected from hydrogen and R^{d}-R^{b};
R^{d} is selected from a bond, CO, C(X²)X¹, SO₂ and SO₂NR^{c};
R^{a} is a bond, O, CO, X¹C(X²), C(X²)X¹, X¹C(X²)X¹, S, SO, SO₂, NR^{c}, SO₂NR^{c} or NR^{c}SO₂; R^{b} is selected from hydrogen, carbocyclic and heterocyclic groups having from 3 to 12 ring members, and a C₁₋₈ hydrocarbyl group optionally substituted by one or more substituents selected from hydroxy, oxo, halogen, cyano, nitro, carboxy, amino, mono- or di-C₁₋₄ hydrocarbylamino, carbocyclic and heterocyclic groups having from 3 to 12 ring members;
R^{c} is selected from hydrogen and C₁₋₄ hydrocarbyl;
X¹ is O, S or NR^{c} and X² is =O, =S or =NR^{c}; and
R¹⁵ is selected from C₁₋₄ saturated hydrocarbyl optionally substituted by hydroxy, C₁₋₂ alkoxy, halogen or a monocyclic 5- or 6-membered carbocyclic or heterocyclic group, provided that U and T cannot be O simultaneously.

6. A combination according to claim 5 comprising an ancillary agent and a compound having the formula (IVa): 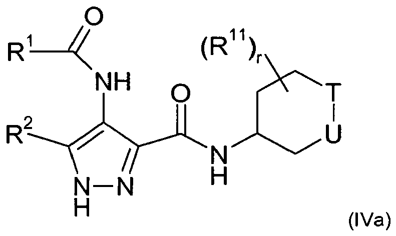 or salts or tautomers or N-oxides or solvates thereof;
wherein
the ancillary agent is as defined in claim 1;
one of U and T is selected from CH₂, CHR¹³, CR¹¹R¹³, NR¹⁴, N(O)R¹⁵, O and S(O)ₜ; and the other of U and T is selected from CH₂, CHR¹¹, C(R¹¹)₂, and C=O; r is 0, 1 or 2; t is 0, 1 or 2; R¹¹ is selected from hydrogen and C₁₋₃ alkyl;
R¹³ is selected from hydrogen and R^{a}-R^{b};
R¹⁴ is selected from hydrogen and R^{d}-R^{b};
R^{d} is selected from a bond, CO, C(X²)X¹, SO₂ and SO₂NR^{c};
R¹⁵ is selected from C₁₋₄ saturated hydrocarbyl optionally substituted by hydroxy, C₁₋₂ alkoxy, halogen or a monocyclic 5- or 6-membered carbocyclic or heterocyclic group; and
R¹, R², R^{a}, R^{b} and R^{c} are as defined in claim 5.

7. A combination according to claim 6 comprising an ancillary agent and a compound having
the formula (Va): 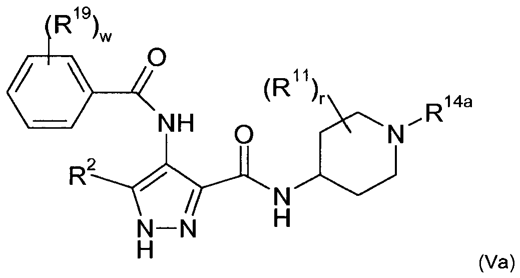 or salts or tautomers or N-oxides or solvates thereof; wherein
the ancillary agent is as defined in claim 1;
R^{14a} is selected from hydrogen, C₁₋₄ alkyl optionally substituted by fluoro , cyclopropylmethyl, phenyl-C₁₋₂ alkyl , C₁₋₄ alkoxycarbonyl , phenyl-C₁₋₂ alkoxycarbonyl , C₁₋₂-alkoxy-C₁₋₂ alkyl , and C₁₋₄ alkylsulphonyl , wherein the phenyl moieties when present are optionally substituted by one to three substituents selected from fluorine, chlorine, C₁₋₄ alkoxy optionally substituted by fluoro or C₁₋₂-alkoxy, and C₁₋₄ alkyl optionally substituted by fluoro or C₁₋₂-alkoxy;
w is 0, 1, 2 or 3;
R² is hydrogen or methyl;
R¹¹ and rare as defined in claim 6; and
R¹⁹ is selected from fluorine; chlorine; C₁₋₄ alkoxy optionally substituted by fluoro or C₁₋₂-alkoxy; and C₁₋₄ alkyl optionally substituted by fluoro or C₁₋₂-alkoxy.

8. A combination according to claim 6 comprising an ancillary agent and a compound of the
formula (VIa): 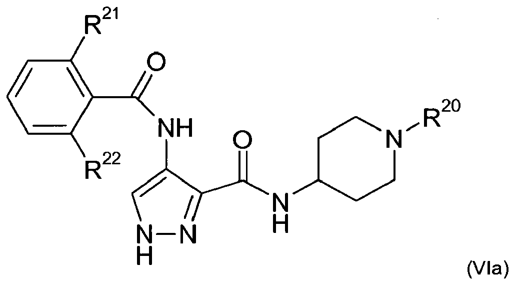 or salts or tautomers or N-oxides or solvates thereof;
wherein
the ancillary agent is as defined in claim 1;
R²⁰ is selected from hydrogen and methyl;
R²¹ is selected from fluorine and chlorine; and
R²² is selected from fluorine, chlorine and methoxy; or
one of R²¹ and R²² is hydrogen and the other is selected from chlorine, methoxy, ethoxy, difluoromethoxy, trifluoromethoxy and benzyloxy.

9. A combination according to claim 8 comprising an ancillary agent and a compound of the
formula (VIb): 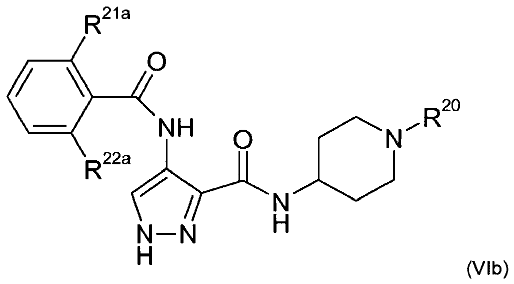 or salts or tautomers or N-oxides or solvates thereof;
wherein
the ancillary agent is as defined in claim 1;
R²⁰ is selected from hydrogen and methyl;
R^{21a} is selected from fluorine and chlorine; and
R^{22a} is selected from fluorine, chlorine and methoxy.

10. A combination according to claim 9 wherein the compound of the formula (VIb) is selected from:
4-(2,6-difluoro-benzoylamino)-1H-pyrazole-3-carboxylic acid piperidin-4-ylamide;
4-(2,6-difluoro-benzoylamino)-1H-pyrazole-3-carboxylicacid (1-methyl-piperidin-4-yl)-amide;
4-(2,6-dichloro-benzoylamino)-1H-pyrazole-3-carboxylic acid piperidin-4-ylamide; and
4-(2-fluoro-6-methoxy-benzoylamino)-1H-pyrazole-3-carboxylic acid piperidin-4-ylamide.

11. A combination according to claim 10 wherein the compound of the formula (VIb) is 4-(2,6-dichloro-benzoylamino)-1H-pyrazole-3-carboxylic acid piperidin-4-ylamide.

12. A combination according to claim 11 wherein the 4-(2,6-dichloro-benzoylamino)-1H-pyrazole-3-carboxylic acid piperidin-4-ylamide is in the form of a salt.

13. A combination according to claim 12 wherein the salt of 4-(2,6-dichloro-benzoylamino)-1H-pyrazole-3-carboxylic acid piperidin-4-ylamide is the salt formed with methanesulphonic acid.

14. A combination according to claim 1 wherein the ancillary agent and compound of formula (II) are formulated together, and they are (i) intimately mixed, or (ii) physically separated within a formulation.

15. The combination of claim 1 wherein the ancillary agent and compound of formula (II) are formulated separately for separate or concurrent administration.

16. The combination of claim 1 in the form of a pharmaceutical kit or patient pack.

17. A combination as defined in any one of claims 1 to 16 for use in inhibiting tumour growth in a mammal.

18. A combination as defined in any one of claims 1 to 16 for use in treating a cancer in a patient.

19. A combination as defined in any one of claims 1 to 16 for use in preventing, treating or managing cancer in a patient.

20. The use of a compound of Formula (II) as defined in claim 1 for the manufacture of a medicament for enhancing or potentiating the response rate in a patient suffering from a cancer where the patient is being treated with an ancillary agent as defined in claim 1.

21. A combination according to any one of claims 1 to 16 wherein the ancillary agent is an antiandrogen or an antiestrogen.

22. A combination according to claim 21 wherein the antiandrogen is an aromatase inhibitor.

23. A combination according to claim 21 wherein the ancillary agent is an antiandrogen selected from tamoxifen, fulvestrant, raloxifene, toremifene, droloxifene, letrazole, anastrozole, exemestane, bicalutamide, leuprolide acetate,megestrol acetate and aminoglutethimide.

24. A combination according to claim 21 wherein the ancillary agent is a GnRH analog.

25. A combination according to any one of claims 1 to 16 wherein the ancillary agent is a monoclonal antibody to cell surface antigens (or an anti-CD antibody).

26. A combination according to claim 25 wherein the monoclonal antibody to cell surface antigens is (a) selected from CD20, CD22, CD33 and CD52; or (b) selected from rituximab, tositumomab and gemtuzumab ozogamicin.

27. A combination according to any one of claims 1 to 16 wherein the alkylating agent is selected from a nitrogen mustard compound, nitrosourea compound and busulfan.

28. A combination according to any one of claims 1 to 16 wherein the anticancer agent is a HDAC inhibitor which is selected from TSA, SAHA, JNJ-16241199, LAQ-824, MGCD-0103 and PXD-101.

29. A combination according to any one of claims 1 to 16 wherein the anticancer agent is a COX-2 inhibitor which is celecoxib.

30. A combination according to any one of claims 1 to 16 wherein the anticancer agent is a DNA methylation inhibitor which is temozolomide.

31. A combination according to any one of claims 1 to 16 wherein the anticancer agent is a proteasome inhibitor which is bortezomib.

32. A combination according to any one of claims 1 to 16 wherein the further CDK inhibitor is one or more compounds of formula (II) as defined in claim 1.

33. A combination according to any one of claims 1 to 16 wherein the further CDK inhibitor is selected from seliciclib, alvocidib, 7- hydroxystaurosporine, JNJ-7706621, BMS-387032, Pha533533, PD332991, ZK-304709 and AZD-5438.

## Patentansprüche

1. Kombination, die ein Hilfsmittel und eine Verbindung der Formel (II) : 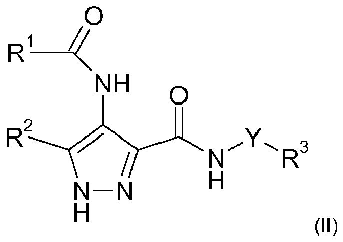 oder Salze oder Tautomere oder N-Oxide oder Solvate davon umfasst;
wobei
das Hilfsmittel ausgewählt ist aus:
(a) einem monoklonalen Antikörper zu einem Zelloberflächenantigen oder
(b) einem Alkylans, wobei das Alkylans aus Stickstoff-Senfgasen; Nitrosoharnstoffen; bifunktionellen Alkylantien mit einer Bisalkansulfonatgruppe und Aziridinverbindungen ausgewählt ist; und wobei es sich bei den Alkylantien nicht um Mitomycin C oder Cyclophosphamid handelt; oder
(c) einem Mittel gegen Krebs, das aus COX-2-Hemmern, HDAC-Hemmern, DNA-Methyltransferase-Hemmern (Methylasehemmern) und Proteasomen-Hemmern ausgewählt ist; oder
(d) einem weiteren CDK-Hemmer und
(e) einem Antiandrogen, einschließlich eines Antiöstrogens;
Y eine Bindung oder eine Alkylenkette mit einer Länge von 1, 2 oder 3 Kohlenstoffatomen ist;
R¹ eine carbocyclische oder heterocyclische Gruppe mit 3 bis 12 Ringgliedern, wobei die carbocyclische oder heterocyclische Gruppe unsubstituiert oder durch eine oder mehrere Substituentengruppen R¹⁰ substituiert ist; oder eine C₁₋₈-Hydrocarbylgruppe ist, die gegebenenfalls durch einen oder mehrere Substituenten substituiert ist, die aus Fluor, Hydroxy, C₁₋₄-Hydrocarbyloxy, Amino, Mono- oder Di-C₁₋₄-hydrocarbylamino und carbocyclischen oder heterocyclischen Gruppen mit 3 bis 12 Ringgliedern ausgewählt sind, wobei die carbocyclischen oder heterocyclischen Gruppen unsubstituiert oder durch eine oder mehrere Substituentengruppen R¹⁰ substituiert sind;
R² Wasserstoff oder Methyl ist;
R³ aus nicht aromatischen carbocyclischen oder heterocyclischen Gruppen mit 3 bis 12 Ringgliedern ausgewählt ist, wobei die carbocyclische oder heterocyclische Gruppe unsubstituiert oder durch eine oder mehrere Substituentengruppen R¹⁰ substituiert ist; und
R¹⁰ aus Halogen, Hydroxy, Trifluormethyl, Cyano, Nitro, Carboxy, Amino, Mono- oder Di-C₁₋₄-hydrocarbylamino, carbocyclischen oder heterocyclischen Gruppen mit 3 bis 12 Ringgliedern und einer Gruppe R^{a}-R^{b} ausgewählt ist, wobei R^{a} eine Bindung, 0, CO, X¹C(X²), C(X²)X¹, X¹C(X²)X¹, S, SO, SO₂, NR^{c}, SO₂NR^{c} oder NR^{c}SO₂ ist und R^{b} aus Wasserstoff, carbocyclischen und heterocyclischen Gruppen mit 3 bis 12 Ringgliedern und einer C₁₋₈-Hydrocarbylgruppe ausgewählt ist, die gegebenenfalls durch einen oder mehrere Substituenten substituiert ist, die aus Hydroxy, Oxo, Halogen, Cyano, Nitro, Carboxy, Amino, Mono- oder Di-C₁₋₄-Hydrocarbylamino, carbocyclischen und heterocyclischen Gruppen mit 3 bis 12 Ringgliedern ausgewählt sind;
R^{c} aus Wasserstoff und C₁₋₄-Hydrocarbyl ausgewählt ist und
X¹ 0, S oder NR^{c} ist und X² =O, =S oder =NR^{c} ist;
und vorausgesetzt, dass, wenn die Substituentengruppe R¹⁰ eine carbocyclische oder heterocyclische Gruppe umfasst oder enthält, die carbocyclische oder heterocyclische Gruppe unsubstituiert sein kann oder selbst mit einer oder mehreren weiteren Substituentengruppen R¹⁰ substituiert sein kann, und wobei (a) derartige weitere Substituentengruppen R¹⁰ carbocyclische oder heterocyclische Gruppen enthalten, die selbst nicht weiter substituiert sein; oder (b) die weiteren Substituenten keine carbocyclischen oder heterocyclischen Gruppen enthalten, jedoch anderweitig aus den oben in der Definition von R¹⁰ aufgeführten Gruppen ausgewählt sind.

2. Kombination nach Anspruch 1, wobei Y eine Bindung ist.

3. Kombination nach Anspruch 1 oder 2, wobei die carbocyclischen und heterocyclischen Gruppen monocyclisch sind.

4. Kombination nach Anspruch 3, wobei R¹ aus unsubstituiertem Phenyl, 2-Fluorphenyl, 2-Hydroxyphenyl, 2-Methoxyphenyl, 2-Methylphenyl, 2-(2-(Pyrrolidin-1-yl)ethoxy)phenyl, 3-Fluorphenyl, 3-Methoxyphenyl, 2,6-Difluorphenyl, 2-Fluor-6-hydroxyphenyl, 2-Fluor-3-methoxyphenyl, 2-Fluor-5-methoxyphenyl, 2-Chlor-6-methoxyphenyl, 2-Fluor-6-methoxyphenyl, 2,6-Dichlorphenyl und 2-Chlor-6-fluorphenyl ausgewählt ist und gegebenenfalls weiterhin aus 5-Fluor-2-methoxyphenyl ausgewählt ist.

5. Kombination nach Anspruch 1, die ein Hilfsmittel und eine Verbindung der Formel (IV): 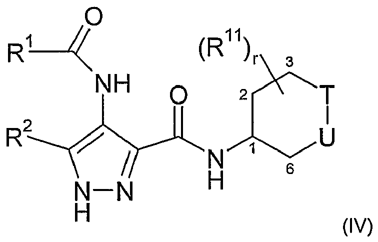
oder Salze oder Tautomere oder N-Oxide oder Solvate davon umfasst;
wobei das Hilfsmittel und R¹ und R² wie in einem der Ansprüche 1 bis 4 definiert sind;
eine fakultative zweite Bindung zwischen Kohlenstoffatomen,
die mit 1 und 2 nummeriert sind, vorliegen kann;
eines von U und T aus CH₂, CHR¹³, CR¹¹R¹³, NR¹⁴, N(O)R¹⁵, O und S(O)ₜ ausgewählt ist und das andere von U und T aus NR¹⁴, 0, CH₂, CHR¹¹, C(R¹¹)₂ und C=O ausgewählt ist; r 0, 1, 2, 3 oder 4 ist; t 0, 1 oder 2 ist;
R¹¹ aus Wasserstoff, Halogen, C₁₋₃-Alkyl und C₁₋₃-Alkoxy ausgewählt ist;
R¹³ aus Wasserstoff, NHR¹⁴, NOH, NOR¹⁴ und R^{a}-R^{b} ausgewählt ist;
R¹⁴ aus Wasserstoff und R^{d}-R^{b} ausgewählt ist;
R^{d} aus einer Bindung, CO, C(X²)X¹, SO₂ und SO₂NR^{c} ausgewählt ist;
R^{a} eine Bindung, 0, CO, X¹C(X²), C(X²)X¹, X¹C(X²)X¹, S, SO, SO₂, NR^{c}, SO₂NR^{c} oder NR^{c}SO₂ ist;
R^{b} aus Wasserstoff, carbocyclischen und heterocyclischen Gruppen mit 3 bis 12 Ringgliedern und einer C₁₋₈-Hydrocarbylgruppe ausgewählt ist, die gegebenenfalls durch einen oder mehrere Substituenten substituiert ist, die aus Hydroxy, Oxo, Halogen, Cyano, Nitro, Carboxy, Amino, Mono- oder Di-C₁₋₄-Hydrocarbylamino, carbocyclischen und heterocyclischen Gruppen mit 3 bis 12 Ringgliedern ausgewählt sind, und wobei ein oder mehrere Kohlenstoffatome der C₁₋₈-Hydrocarbylgruppe gegebenenfalls durch O, S, SO, SO₂, NR^{c}, X¹C(X²), C(X²)X¹ oder X¹C(X²)X¹ ersetzt sein können;
R^{c} aus Wasserstoff und C₁₋₄-Hydrocarbyl ausgewählt ist;
X¹ 0, S oder NR^{c} ist und X² =0, =S oder =NR^{c} ist und
R¹⁵ aus gesättigtem C₁₋₄-Hydrocarbyl ausgewählt ist, das gegebenenfalls durch Hydroxy, C₁₋₂-Alkoxy, Halogen oder eine monocyclische 5- oder 6-gliedrige carbocyclische oder heterocyclische Gruppe substituiert ist, vorausgesetzt,
dass U und T nicht gleichzeitig 0 sein können.

6. Kombination nach Anspruch 5, die ein Hilfsmittel und eine Verbindung der Formel (IVa): 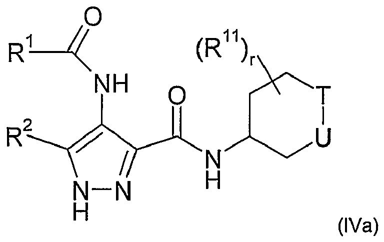 oder Salze oder Tautomere oder N-Oxide oder Solvate davon umfasst;
wobei
das Hilfsmittel wie in Anspruch 1 definiert ist; eines von U und T aus CH₂, CHR¹³, CR¹¹R¹³, NR¹⁴, N(O)R¹⁵, O und S(O)ₜ ausgewählt ist und das andere von U und T aus CH₂, CHR¹¹, C(R¹¹) und C=O ausgewählt ist; r 0, 1 oder 2 ist; t 0, 1 oder 2 ist;
R¹¹ aus Wasserstoff und C₁₋₃-Alkyl ausgewählt ist;
R¹³ aus Wasserstoff und R^{a}-R^{b} ausgewählt ist;
R¹⁴ aus Wasserstoff und R^{d}-R^{b} ausgewählt ist;
R^{d} aus einer Bindung, CO, C(X²)X¹, SO₂ und SO₂NR^{c} ausgewählt ist;
R¹⁵ aus gesättigtem C₁₋₄-Hydrocarbyl ausgewählt ist, das gegebenenfalls durch Hydroxy, C₁₋₂-Alkoxy, Halogen oder eine monocyclische 5- oder 6-gliedrige carbocyclische oder heterocyclische Gruppe substituiert ist; und
R¹, R², R^{a}, R^{b} und R^{c} wie in Anspruch 5 definiert sind.

7. Kombination nach Anspruch 6, die ein Hilfsmittel und eine Verbindung der Formel (Va): 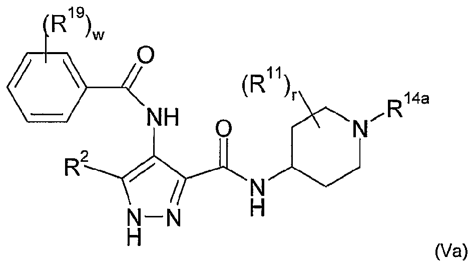 oder Salze oder Tautomere oder N-Oxide oder Solvate davon umfasst;
wobei
das Hilfsmittel wie in Anspruch 1 definiert ist;
R^{14a} aus Wasserstoff, C₁₋₄-Alkyl, das gegebenenfalls durch Fluor substituiert ist, Cyclopropylmethyl, Phenyl-C₁₋₂-alkyl, C₁₋₄-Alkoxycarbonyl, Phenyl-C₁₋₂-alkoxycarbonyl, C₁₋₂-Alkoxy-C₁₋₂-alkyl und C₁₋₄-Alkylsulfonyl ausgewählt ist,
wobei die Phenyleinheiten, wenn sie vorliegen, gegebenenfalls durch einen bis drei Substituenten substituiert sind, die aus Fluor, Chlor, C₁₋₄-Alkoxy, das gegebenenfalls durch Fluor oder C₁₋₂-Alkoxy substituiert ist, und C₁₋₄-Alkyl, das gegebenenfalls durch Fluor oder C₁₋₂-Alkoxy substituiert ist, ausgewählt sind;
w 0, 1, 2 oder 3 ist;
R² Wasserstoff oder Methyl ist;
R¹¹ und r wie in Anspruch 6 definiert sind und
R¹⁹ aus Fluor; Chlor; C₁₋₄-Alkoxy, das gegebenenfalls durch Fluor oder C₁₋₂-Alkoxy substituiert ist; und C₁₋₄-Alkyl, das gegebenenfalls durch Fluor oder C₁₋₂-Alkoxy substituiert ist, ausgewählt ist.

8. Kombination nach Anspruch 6, die ein Hilfsmittel und eine Verbindung der Formel (VIa): 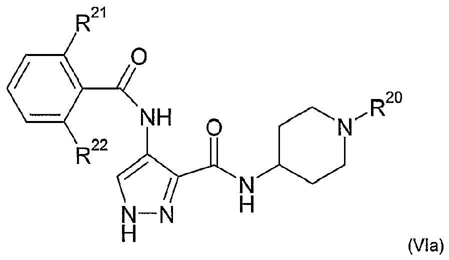 oder Salze oder Tautomere oder N-Oxide oder Solvate davon umfasst;
wobei
das Hilfsmittel wie in Anspruch 1 definiert ist;
R²⁰ aus Wasserstoff und Methyl ausgewählt ist;
R²¹ aus Fluor und Chlor ausgewählt ist und
R²² aus Fluor, Chlor und Methoxy ausgewählt ist; oder
einer von R²¹ und R²² Wasserstoff ist und der andere aus Chlor, Methoxy, Ethoxy, Difluormethoxy, Trifluormethoxy und
Benzyloxy ausgewählt ist.

9. Kombination nach Anspruch 8, die ein Hilfsmittel und eine Verbindung der Formel (VIb): 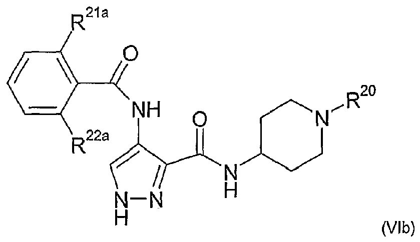 oder Salze oder Tautomere oder N-Oxide oder Solvate davon umfasst;
wobei
das Hilfsmittel wie in Anspruch 1 definiert ist;
R²⁰ aus Wasserstoff und Methyl ausgewählt ist;
R^{21a} aus Fluor und Chlor ausgewählt ist und
R^{22a} aus Fluor, Chlor und Methoxy ausgewählt ist.

10. Kombination nach Anspruch 9, wobei die Verbindung der Formel (VIb) ausgewählt ist aus:
4-(2,6-Difluorbenzoylamino)-1H-pyrazol-3-carbonsäurepiperidin-4-ylamid;
4-(2,6-Difluorbenzoylamino)-1H-pyrazol-3-carbonsäure-(1-methylpiperidin-4-yl)amid;
4-(2,6-Dichlorbenzoylamino)-1H-pyrazol-3-carbonsäurepiperidin-4-ylamid und
4-(2-Fluor-6-methoxybenzoylamino)-1H-pyrazol-3-carbonsäurepiperidin-4-ylamid.

11. Kombination nach Anspruch 10, wobei die Verbindung der Formel (VIb) 4-(2,6-Dichlorbenzoylamino)-1H-pyrazol-3-carbonsäurepiperidin-4-ylamid ist.

12. Kombination nach Anspruch 11, wobei das 4-(2,6-Dichlorbenzoylamino)-1H-pyrazol-3-carbonsäurepiperidin-4-ylamid in der Form eines Salzes ist.

13. Kombination nach Anspruch 12, wobei das Salz von 4-(2,6-Dichlorbenzoylamino)-1H-pyrazol-3-carbonsäurepiperidin-4-ylamid das Salz ist, das mit Methansulfonsäure gebildet wird.

14. Kombination nach Anspruch 1, wobei das Hilfsmittel und die Verbindung der Formel (II) zusammen formuliert werden und sie (i) innig gemischt oder (ii) innerhalb einer Formulierung physikalisch getrennt werden.

15. Kombination nach Anspruch 1, wobei das Hilfsmittel und die Verbindung der Formel (II) zur separaten oder gleichzeitigen Verabreichung separat formuliert werden.

16. Kombination nach Anspruch 1 in der Form eines Kits oder einer Patientenpackung.

17. Kombination nach einem der Ansprüche 1 bis 16 zur Verwendung bei der Hemmung von Tumorwachstum in einem Säugetier.

18. Kombination nach einem der Ansprüche 1 bis 16 zur Verwendung bei der Behandlung einer Krebserkrankung in einem Patienten.

19. Kombination nach einem der Ansprüche 1 bis 16 zur Verwendung bei der Prävention, Behandlung oder Kontrolle von Krebs in einem Patienten.

20. Verwendung einer Verbindung der Formel (II) nach Anspruch 1 zur Herstellung eines Arzneimittels zur Förderung oder Steigerung der Ansprechrate in einem Patienten, der an einer Krebserkrankung leidet, wobei der Patient mit einem Hilfsmittel nach Anspruch 1 behandelt wird.

21. Kombination nach einem der Ansprüche 1 bis 16, wobei das Hilfsmittel ein Antiandrogen oder ein Antiöstrogen ist.

22. Kombination nach Anspruch 21, wobei das Antiandrogen ein Aromatasehemmer ist.

23. Kombination nach Anspruch 21, wobei das Hilfsmittel ein Antiandrogen ist, das aus Tamoxifen, Fulvestrant, Raloxifen, Toremifen, Droloxifen, Letrozol, Anastrozol, Exemestan, Bicalutamid, Leuprolid-Acetat, Megestrol-Acetat und Aminoglutethimid ausgewählt ist.

24. Kombination nach Anspruch 21, wobei das Hilfsmittel ein GnRH-Analogon ist.

25. Kombination nach einem der Ansprüche 1 bis 16, wobei das Hilfsmittel ein monoklonaler Antikörper zu Zelloberflächenantigenen (oder ein Anti-CD-Antikörper) ist.

26. Kombination nach Anspruch 25, wobei der monoklonale Antikörper zu Zelloberflächenantigenen (a) aus CD20, CD22, CD33 und CD52 ausgewählt ist oder (b) aus Rituximab, Tositumomab und Gemtuzumab-Ozogamicin ausgewählt ist.

27. Kombination nach einem der Ansprüche 1 bis 16, wobei das Alkylans aus einer Stickstoff-Senfgas-Verbindung, einer Nitrosoharnstoff-Verbindung und Busulfan ausgewählt ist.

28. Kombination nach einem der Ansprüche 1 bis 16, wobei das Mittel gegen Krebs ein HDAC-Hemmer ist, der aus TSA, SAHA, JNJ-16241199, LAQ-824, MGCD-0103 und PXD-101 ausgewählt ist.

29. Kombination nach einem der Ansprüche 1 bis 16, wobei das Mittel gegen Krebs ein COX-2-Hemmer ist, der Celecoxib ist.

30. Kombination nach einem der Ansprüche 1 bis 16, wobei das Mittel gegen Krebs ein DNA-Methylierungshemmer ist, der Temozolomid ist.

31. Kombination nach einem der Ansprüche 1 bis 16, wobei das Mittel gegen Krebs ein Proteasomen-Hemmer ist, der Bortezomib ist.

32. Kombination nach einem der Ansprüche 1 bis 16, wobei der weitere CDK-Hemmer eine oder mehrere Verbindungen der Formel (II) nach Anspruch 1 ist.

33. Kombination nach einem der Ansprüche 1 bis 16, wobei der weitere CDK-Hemmer aus Seliciclib, Alvocidib, 7-Hydroxystaurosporin, JNJ-7706621, BMS-387032, Pha533533, PD332991, ZK-304709 und AZD-5438 ausgewählt ist.

## Revendications

1. Combinaison comprenant un agent auxiliaire et un composé de la formule (II) : 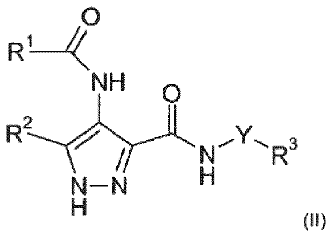 ou des sels ou tautomères ou N-oxydes ou solvates de celui-ci ;
dans laquelle
l'agent auxiliaire est sélectionné parmi :
(a) un anticorps monoclonal à un antigène de surface cellulaire ; ou
(b) un agent d'alkylation, dans laquelle ledit agent d'alkylation est sélectionné parmi des moutardes à l'azote ; des nitrosourées ; des agents d'alkylation bifonctionnels ayant un groupe bis-alcanesulfonate ;
et des composés d'aziridine ; et dans laquelle les agents d'alkylation sont autres que la mitomycine C ou le cyclophosphamide ; ou
(c) un agent anticancéreux sélectionné parmi des inhibiteurs de COX-2, des inhibiteurs de HDAC, des inhibiteurs d'ADN méthyltransférase (méthylase) et des inhibiteurs de protéasome ; ou
(d) un autre inhibiteur de CDK ; et
(e) un anti-androgène incluant un anti-oestrogène ;
Y est une liaison ou une chaîne alkylène de 1, 2 ou 3 atomes de carbone de longueur ;
R¹ est un groupe carbocyclique ou hétérocyclique ayant 3 à 12 éléments d'anneau, dans laquelle le groupe carbocyclique ou hétérocyclique est non substitué ou substitué par un ou plusieurs groupes substituants R¹⁰ ; ou un groupe hydrocarbyle en C₁₋₈ substitué en option par un ou plusieurs substituants sélectionnés parmi fluor, hydroxy, hydrocarbyloxy en C₁₋₄, amino, mono- ou di-C₁₋₄-hydrocarbylamino, et des groupes carbocycliques ou hétérocycliques ayant 3 à 12 éléments d'anneau, dans laquelle les groupes carbocycliques ou hétérocycliques sont non substitués ou substitués par un ou plusieurs groupes substituants R¹⁰ ;
R² est hydrogène ou méthyle ;
R³ est sélectionné parmi des groupes carbocycliques et hétérocycliques non aromatiques ayant 3 à 12 éléments d'anneau, dans laquelle le groupe carbocyclique ou hétérocyclique est non substitué ou substitué par un ou plusieurs groupes substituants R¹⁰ ; et
R¹⁰ est sélectionné parmi halogène, hydroxy, trifluorométhyle, cyano, nitro, carboxy, amino, mono- ou di-C₁₋₄-hydrocarbylamino, des groupes carbocycliques et hétérocycliques ayant 3 à 12 éléments d'anneau ; un groupe R^{a}-R^{b}, dans laquelle R^{a} est une liaison, 0, CO, X¹C(X²), C(X²)X¹, X¹C(X²)X¹, S, SO, SO₂, NR^{c}, SO₂NR^{c} ou NR^{c}SO₂ ; et R^{b} est sélectionné parmi hydrogène, des groupes carbocycliques et hétérocycliques ayant 3 à 12 éléments d'anneau et un groupe hydrocarbyle en C₁₋₈ substitué en option par un ou plusieurs substituants sélectionnés parmi hydroxy, oxo, halogène, cyano, nitro, carboxy, amino, mono- ou di-C₁₋₄-hydrocarbylamino, des groupes carbocycliques ou hétérocycliques ayant 3 à 12 éléments d'anneau ;
R^{c} est sélectionné parmi hydrogène et hydrocarbyle en C₁₋₄ ; et
X¹ est 0, S ou NR^{c} et X² est =0, =S ou =NR^{c} ;
et à condition que, lorsque le groupe substituant R¹⁰ comprend ou inclut un groupe carbocyclique ou hétérocyclique, ledit groupe carbocyclique ou hétérocyclique puisse être non substitué ou puisse lui-même être substitué par un ou plusieurs autres groupes substituants R¹⁰ et dans laquelle (a) de tels autres groupes substituants R¹⁰ incluent des groupes carbocycliques ou hétérocycliques qui ne sont pas eux-mêmes davantage substitués ; ou (b) lesdits autres substituants n'incluent pas de groupes carbocycliques ou hétérocycliques mais sont autrement sélectionnés parmi les groupes énumérés ci-dessus dans la définition de R¹⁰.

2. Combinaison selon la revendication 1, dans laquelle Y est une liaison.

3. Combinaison selon la revendication 1 ou la revendication 2, dans laquelle les groupes carbocycliques et hétérocycliques sont monocycliques.

4. Combinaison selon la revendication 3, dans laquelle R¹ est sélectionné parmi phényle, 2-fluorophényle, 2-hydroxyphényle, 2-méthoxyphényle, 2-méthylphényle, 2-(2-(pyrrolidin-1-yl)éthoxy)-phényle, 3-fluorophényle, 3-méthoxyphényle, 2,6-difluorophényle, 2-fluoro-6-hydroxyphényle, 2-fluoro-3-méthoxyphényle, 2-fluoro-5-méthoxyphényle, 2-chloro-6-méthoxyphényle, 2-fluoro-6-méthoxyphényle, 2,6-dichlorophényle et 2-chloro-6-fluorophényle non substitués ; et est en outre sélectionné en option parmi 5-fluoro-2-méthoxyphényle.

5. Combinaison selon la revendication 1, comprenant un agent auxiliaire et un composé ayant la formule (IV) : 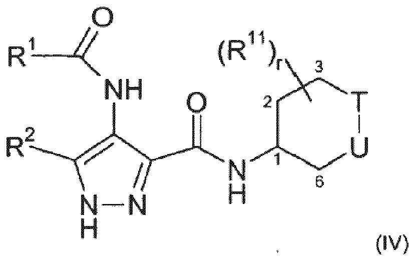 ou des sels ou tautomères ou N-oxydes ou solvates de celui-ci ;
dans laquelle l'agent auxiliaire et R¹ et R² sont tels que définis dans l'une quelconque des revendications 1 à 4 ;
une seconde liaison optionnelle peut être présente entre les atomes de carbone numérotés 1 et 2 ;
un de U et T est sélectionné parmi CH₂, CHR¹³, CR¹¹R¹³, NR¹⁴, N(O)R¹⁵, 0 et S(O)ₜ ; et l'autre de U et T est sélectionné parmi NR¹⁴, 0, CH₂, CHR¹¹, C(R¹¹)₂ et C=O ; r est 0, 1, 2, 3 ou 4 ; t est 0, 1 ou 2 ;
R¹¹ est sélectionné parmi hydrogène, halogène, alkyle en C₁₋₃ et alcoxy en C₁₋₃ ;
R¹³ est sélectionné parmi hydrogène, NHR¹⁴, NOH, NOR¹⁴ et R^{a}-R^{b} ;
R¹⁴ est sélectionné parmi hydrogène et R^{d}-R^{b} ;
R^{d} est sélectionné parmi une liaison, CO, C(X²)X¹, SO₂ et SO₂NR^{c} ;
R^{a} est une liaison, O, CO, X¹C(X²), C(X²)X¹, X¹C(X²)X¹, S, SO, SO₂, NR^{c}, SO₂NR^{c} ou NR^{c}SO₂ ;
R^{b} est sélectionné parmi hydrogène, des groupes carbocycliques et hétérocycliques ayant 3 à 12 éléments d'anneau et un groupe hydrocarbyle en C₁₋₈ substitué en option par un ou plusieurs substituants sélectionnés parmi hydroxy, oxo, halogène, cyano, nitro, carboxy, amino, mono- ou di-C₁₋₄-hydrocarbylamino, des groupes carbocycliques et hétérocycliques ayant 3 à 12 éléments d'anneau et dans laquelle un ou plusieurs atomes de carbone du groupe hydrocarbyle en C₁₋₈ peuvent être remplacés en option par 0, S, SO, SO₂, NR^{c}, X¹C(X²), C(X²)X¹ ou X¹C(X²)X¹ ;
R^{c} est sélectionné parmi hydrogène et hydrocarbyle en C₁₋₄ ;
X¹ est 0, S ou NR^{c} et X² est =0, =S ou =NR^{c} ; et
R¹⁵ est sélectionné parmi hydrocarbyle saturé en C₁₋₄ substitué en option par hydroxy, alcoxy en C₁₋₂, halogène ou un groupe carbocyclique ou hétérocyclique monocyclique à 5 ou 6 éléments, à condition que U et T ne puissent pas être 0 simultanément.

6. Combinaison selon la revendication 5, comprenant un agent auxiliaire et un composé ayant la formule (IVa) : 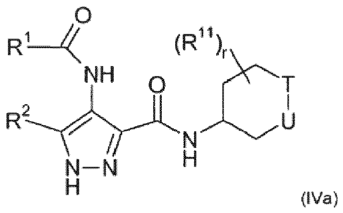 ou des sels ou tautomères ou N-oxydes ou solvates de celui-ci ;
dans laquelle
l'agent auxiliaire est tel que défini dans la revendication 1 ;
un de U et T est sélectionné parmi CH₂, CHR¹³, CR¹¹R¹³, NR¹⁴, N(O)R¹⁵, 0 et S(O)ₜ ; et l'autre de U et T est sélectionné parmi CH₂, CHR¹¹, C(R¹¹)₂ et C=O ; r est 0, 1 ou 2 ; t est 0, 1 ou 2 ;
R¹¹ est sélectionné parmi hydrogène et alkyle en C₁₋₃ ; R¹³ est sélectionné parmi hydrogène et R^{a}-R^{b} ;
R¹⁴ est sélectionné parmi hydrogène et R^{d}--R^{b} ;
R^{d} est sélectionné parmi une liaison, CO, C(X²)X¹, SO₂ et SO₂NR^{c} ;
R¹⁵ est sélectionné parmi hydrocarbyle saturé en C₁₋₄ substitué en option par hydroxy, alcoxy en C₁₋₂, halogène ou un groupe carbocyclique ou hétérocyclique monocyclique à 5 ou 6 éléments ; et
R¹, R², R^{a}, R^{b} et R^{c} sont tels que définis dans la revendication 5.

7. Combinaison selon la revendication 6, comprenant un agent auxiliaire et un composé ayant la formule (Va) : 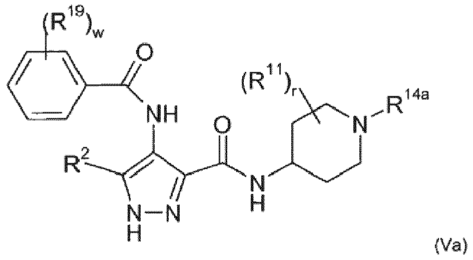 ou des sels ou tautomères ou N-oxydes ou solvates de celui-ci ;
dans laquelle
l'agent auxiliaire est tel que défini dans la revendication 1 ;
R^{14a} est sélectionné parmi hydrogène, alkyle en C₁₋₄ substitué en option par fluoro, cyclopropylméthyle, phényl-C₁₋₂-alkyle, alcoxycarbonyle en C₁₋₄, phényl-C₁₋₂-alcoxycarbonyle, C₁₋₂-alcoxy-C₁₋₂-alkyle et alkylsulfonyle en C₁₋₄, dans laquelle les fragments phényle, lorsqu'ils sont présents, sont substitués en option par un à trois substituants sélectionnés parmi fluor, chlore, alcoxy en C₁₋₄ substitué en option par fluoro ou alcoxy en C₁₋₂, et alkyle en C₁₋₄ substitué en option par fluoro ou alcoxy en C₁₋₂ ;
w est 0, 1, 2 ou 3 ;
R² est hydrogène ou méthyle ;
R¹¹ et r sont tels que définis dans la revendication 6 ; et
R¹⁹ est sélectionné parmi fluor ; chlore ; alcoxy en C₁₋₄ substitué en option par fluoro ou alcoxy en C₁₋₂ ; et alkyle en C₁₋₄ substitué en option par fluoro ou alcoxy en C₁₋₂.

8. Combinaison selon la revendication 6, comprenant un agent auxiliaire et un composé de la formule (VIa) : 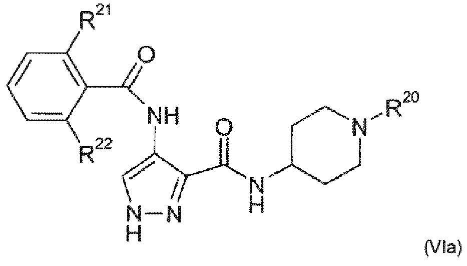 ou des sels ou tautomères ou N-oxydes ou solvates de celui-ci ;
dans laquelle
l'agent auxiliaire est tel que défini dans la revendication 1 ;
R²⁰ est sélectionné parmi hydrogène et méthyle ;
R²¹ est sélectionné parmi fluor et chlore ; et
R²² est sélectionné parmi fluor, chlore et méthoxy ; ou
un de R²¹ et R²² est hydrogène et l'autre est sélectionné parmi chlore, méthoxy, éthoxy, difluorométhoxy, trifluorométhoxy et benzyloxy.

9. Combinaison selon la revendication 8, comprenant un agent auxiliaire et un composé de la formule (VIb) : 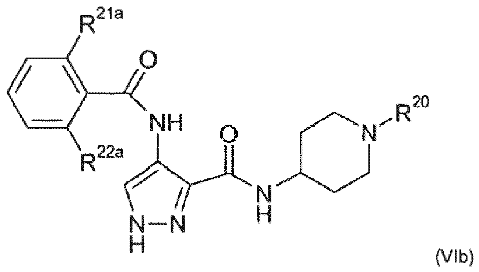 ou des sels ou tautomères ou N-oxydes ou solvates de celui-ci ;
dans laquelle
l'agent auxiliaire est tel que défini dans la revendication 1 ;
R²⁰ est sélectionné parmi hydrogène et méthyle ;
R^{21a} est sélectionné parmi fluor et chlore ; et
R^{22a} est sélectionné parmi fluor, chlore et méthoxy.

10. Combinaison selon la revendication 9, dans laquelle le composé de la formule (VIb) est sélectionné parmi :
pipéridin-4-ylamide d'acide 4-(2,6-difluoro-benzoylamino)-1H-pyrazole-3-carboxylique ;
(1-méthyl-pipéridin-4-yl)-amide d'acide 4-(2,6-difluoro-benzoylamino)-1H-pyrazole-3-carboxylique ;
pipéridin-4-ylamide d'acide 4-(2,6-dichloro-benzoylamino)-1H-pyrazole-3-carboxylique ; et pipéridin-4-ylamide d'acide 4-(2-fluoro-6-méthoxy-benzoylamino)-1H-pyrazole-3-carboxylique.

11. Combinaison selon la revendication 10, dans laquelle le composé de la formule (VIb) est le pipéridin-4-ylamide d'acide 4-(2,6-dichloro-benzoylamino)-1H-pyrazole-3-carboxylique.

12. Combinaison selon la revendication 11, dans laquelle le pipéridin-4-ylamide d'acide 4-(2,6-dichloro-benzoylamino)-1H-pyrazole-3-carboxylique est sous la forme d'un sel.

13. Combinaison selon la revendication 12, dans laquelle le sel de pipéridin-4-ylamide d'acide 4-(2,6-dichloro-benzoylamino)-1H-pyrazole-3-carboxylique est le sel formé avec l'acide méthanesulfonique.

14. Combinaison selon la revendication 1, dans laquelle l'agent auxiliaire et le composé de formule (II) sont formulés ensemble, et ils sont (i) mélangés intimement ou (ii) séparés physiquement au sein d'une formulation.

15. Combinaison selon la revendication 1, dans laquelle l'agent auxiliaire et le composé de formule (II) sont formulés séparément pour l'administration séparée ou concomitante.

16. Combinaison selon la revendication 1 sous la forme d'un pharmaceutique kit ou emballage à destination du patient.

17. Combinaison telle que définie dans l'une quelconque des revendications 1 à 16 destinée à être utilisée dans l'inhibition de la croissance tumorale chez un mammifère.

18. Combinaison telle que définie dans l'une quelconque des revendications 1 à 16 destinée à être utilisée dans le traitement d'un cancer chez un patient.

19. Combinaison telle que définie dans l'une quelconque des revendications 1 à 16 destinée à être utilisée dans la prévention, le traitement ou la gestion du cancer chez un patient.

20. Utilisation d'un composé de Formule (II) tel que défini dans la revendication 1 pour la fabrication d'un médicament pour amplifier ou potentialiser le taux de réponse chez un patient souffrant d'un cancer où le patient est traité avec un agent auxiliaire tel que défini dans la revendication 1.

21. Combinaison selon l'une quelconque des revendications 1 à 16, dans laquelle l'agent auxiliaire est un anti-androgène ou un anti-oestrogène.

22. Combinaison selon la revendication 21, dans laquelle l'anti-androgène est un inhibiteur d'aromatase.

23. Combinaison selon la revendication 21, dans laquelle l'agent auxiliaire est un anti-androgène sélectionné parmi le tamoxifène, le fulvestrant, le raloxifène, le torémifène, le droloxifène, le létrazole, l'anastrazole, l'exémestane, le bicalutamide, l'acétate de leuprolide, l'acétate de mégestrol et l'aminoglutéthimide.

24. Combinaison selon la revendication 21, dans laquelle l'agent auxiliaire est un analogue de GnRH.

25. Combinaison selon l'une quelconque des revendications 1 à 16, dans laquelle l'agent auxiliaire est un anticorps monoclonal à des antigènes de surface cellulaire (ou un anticorps anti-CD).

26. Combinaison selon la revendication 25, dans laquelle l'anticorps monoclonal à des antigènes de surface cellulaire est (a) sélectionné parmi CD20, CD22, CD33 et CD52 ; ou (b) sélectionné parmi le rituximab, tositumomab et l'ozogamicine de gemtuzumab.

27. Combinaison selon l'une quelconque des revendications 1 à 16, dans laquelle l'agent d'alkylation est sélectionné parmi un composé de moutarde à l'azote, un composé de nitrosourée et le busulfan.

28. Combinaison selon l'une quelconque des revendications 1 à 16, dans laquelle l'agent anticancéreux est un inhibiteur de HDAC qui est sélectionné parmi TSA, SAHA, JNJ-16241199, LAQ-824, MGCD-0103 et PXD-101.

29. Combinaison selon l'une quelconque des revendications 1 à 16, dans laquelle l'agent anticancéreux est un inhibiteur de COX-2 qui est le célécoxib.

30. Combinaison selon l'une quelconque des revendications 1 à 16, dans laquelle l'agent anticancéreux est un inhibiteur de méthylation d'ADN qui est le témozolomide.

31. Combinaison selon l'une quelconque des revendications 1 à 16, dans laquelle l'agent anticancéreux est un inhibiteur de protéasome qui est le bortézimib.

32. Combinaison selon l'une quelconque des revendications 1 à 16, dans laquelle l'autre inhibiteur de CDK est un ou plusieurs composés de formule (II) tels que définis dans la revendication 1.

33. Combinaison selon l'une quelconque des revendications 1 à 16, dans laquelle l'autre inhibiteur de CDK est sélectionné parmi le séliciclib, l'alvocidib, la 7-hydroxystaurosporine, JNJ-7706621, BMS-387032, Pha533533, PD332991, ZK-304709 et AZD-5438.
